(19) **Europäisches Patentamt / European Patent Office / Office européen des brevets**

(11) **EP 3 779 031 A1**

(12) **EUROPEAN PATENT APPLICATION**
published in accordance with Art. 153(4) EPC

(43) Date of publication:
**17.02.2021 Bulletin 2021/07**

(21) Application number: **19782456.8**

(22) Date of filing: **03.04.2019**

(51) Int Cl.:
**D06M 11/01** *(2006.01)*  **D01F 4/02** *(2006.01)*
**D03D 15/00** *(2021.01)*  **D06B 5/00** *(2006.01)*

(86) International application number:
**PCT/JP2019/014897**

(87) International publication number:
**WO 2019/194262 (10.10.2019 Gazette 2019/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.04.2018 JP 2018071892**

(71) Applicant: **Spiber Inc.**
**Tsuruoka-shi, Yamagata 997-0052 (JP)**

(72) Inventor: **IKEDA, Atsushi**
**Tsuruoka-shi, Yamagata 997-0052 (JP)**

(74) Representative: **Handley, Matthew Edward et al**
**Venner Shipley LLP**
**200 Aldersgate**
**London EC1A 4HD (GB)**

(54) **HIGH-DENSITY WOVEN FABRIC AND METHOD FOR MANUFACTURING SAME**

(57)    An object of the present invention is to provide a high-density woven fabric containing a protein fiber and a method for manufacturing the same. A method for manufacturing a high-density woven fabric according to the present invention comprises steps of: weaving a fiber that at least partially comprises a protein fiber to obtain a raw-material woven fabric; and shrinking the raw-material woven fabric by bringing the raw-material woven fabric into contact with aqueous moisture to obtain a high-density woven fabric.

*Fig.1*

**Description**

**Technical Field**

The present invention relates to a high-density woven fabric and a method for manufacturing the high-density woven fabric.

**Background Art**

Woven fabrics have been widely used in various industrial products, some medical devices, and the like, including clothing and bedding.

In such woven fabrics, so-called high densification that involves increasing the weaving density to reduce the gap between the fibers is required in some cases. For example, a high density is required for a woven fabric for clothing and the like in order to enhance windproofness and dustproofness, and for a woven fabric for a filter, which is one type of industrial products, and the like in order to enhance scavenging ability.

When obtaining a high-density woven fabric, a method that involves densely weaving fine yarns is generally adopted, but this method has a limitation. Therefore, for example, Patent Literature 1 proposes a method for improving the weaving density of a woven fabric by treating the woven fabric obtained by weaving cellulose fibers with hot water.

**Citation List**

**Patent Literature**

[Patent Literature 1] Japanese Unexamined Patent Publication No. 2013-209756

**Summary of Invention**

**Technical Problem**

However, with respect to a woven fabric containing a protein fiber, sufficient studies have not been conducted. An object of the present invention is to provide a high-density woven fabric containing a protein fiber and a method for manufacturing the same.

**Solution to Problem**

The present invention relates, for example, to each of the following inventions.

[1] A method for manufacturing a high-density woven fabric, comprising steps of:

weaving a fiber that at least partially comprises a protein fiber to obtain a raw-material woven fabric; and shrinking the raw-material woven fabric by bringing the raw-material woven fabric into contact with aqueous moisture to obtain a high-density woven fabric.

[2] The manufacturing method according to [1], wherein the protein fiber is capable of shrinking by 2% or more when being brought into contact with the aqueous moisture.

[3] The manufacturing method according to [1] or [2], wherein the protein fiber is capable of shrinking by more than 7% when being brought into contact with the aqueous moisture and subsequently dried.

[4] The manufacturing method according to any one of [1] to [3], wherein a temperature of the aqueous moisture is lower than a boiling point thereof.

[5] The manufacturing method according to any one of [1] to [4], wherein the weaving step comprises adjusting a weaving density of the raw-material woven fabric.

[6] The manufacturing method according to any one of [1] to [5], wherein the protein fiber is a fibroin fiber.

[7] The manufacturing method according to [6], wherein the fibroin fiber is a modified spider silk fibroin fiber.

[8] A high-density woven fabric, which is a woven fabric comprising a fiber that at least partially comprises a protein fiber, wherein the fiber is shrunk by aqueous moisture.

[9] A high-density woven fabric, which is a woven fabric comprising a fiber that at least partially comprises a protein fiber, wherein the woven fabric is shrunk by aqueous moisture.

[10] A high-density woven fabric, which is a woven fabric comprising a fiber that at least partially comprises a protein fiber, wherein a weaving density of the woven fabric exceeds a maximum value of a weaving density achievable by weaving the fiber.

[11] The high-density woven fabric according to any one of [8] to [10], wherein the protein fiber is a fibroin fiber.

[12] The high-density woven fabric according to [11], wherein the fibroin fiber is a modified spider silk fibroin fiber.

[13] The manufacturing method according to any one of [1] to [7] or the high-density woven fabric according to any one of [8] to [12], wherein

the protein fiber comprises a modified fibroin,

the modified fibroin comprises a domain sequence represented by

Formula 1: $[(A)_n \text{ motif-REP}]_m$,

and

the domain sequence has an amino acid sequence with a reduced content of $(A)_n$ motifs, which corresponds to an amino acid sequence in which at least one or a plurality of $(A)_n$ motifs are deleted, as compared with a naturally occurring fibroin,

wherein in Formula 1, the $(A)_n$ motif represents an amino acid sequence composed of 2 to 27 amino acid residues and the number of alanine residues with respect to a total number of amino acid residues in the $(A)_n$ motif is 83% or more;

REP represents an amino acid sequence composed of 10 to 200 amino acid residues;

m represents an integer of 2 to 300;

a plurality of the $(A)_n$ motifs may have the same amino acid sequence or amino acid sequences different from each other; and

a plurality of REPs may have the same amino acid sequence or amino acid sequences different from each other.

[14] The manufacturing method or the high-density woven fabric according to [13], wherein the domain sequence has an amino acid sequence that corresponds to an amino acid sequence in which at least one $(A)_n$ motif for every one to three $(A)_n$ motifs from the N-terminal side to the C-terminal side is deleted, as compared with a naturally occurring fibroin.

[15] The manufacturing method or the high-density woven fabric according to [13], wherein the domain sequence has an amino acid sequence that corresponds to an amino acid sequence in which at least deletion of two consecutive $(A)_n$ motifs and deletion of one $(A)_n$ motif are repeated in this order from the N-terminal side to the C-terminal side, as compared with a naturally occurring fibroin.

[16] The manufacturing method according to any one of [1] to [7] or the high-density woven fabric according to any one of [8] to [12], wherein

the protein fiber comprises a modified fibroin,

the modified fibroin comprises a domain sequence represented by

Formula 1: $[(A)_n \text{ motif-REP}]_m$,

and

when the numbers of amino acid residues of REP of two adjacent $[(A)_n \text{ motif-REP}]$ units are sequentially compared from the N-terminal side to the C-terminal side, x/y is 50% or more, where x is a maximum value of a total value obtained by adding the numbers of amino acid residues of two adjacent $[(A)_n \text{ motif-REP}]$ units wherein when the number of amino acid residues of one REP having less amino acid residues is taken as 1, the ratio of the number of amino acids residues of the other REP satisfies 1.8 to 11.3; and where y is a total number of amino acid residues of the domain sequence,

wherein in Formula 1, the $(A)_n$ motif represents an amino acid sequence composed of 2 to 27 amino acid residues and the number of alanine residues with respect to a total number of amino acid residues in the $(A)_n$ motif is 83% or more;

REP represents an amino acid sequence composed of 10 to 200 amino acid residues;

m represents an integer of 2 to 300;

a plurality of the $(A)_n$ motifs may have the same amino acid sequence or amino acid sequences different from each other; and

a plurality of REPs may have the same amino acid sequence or amino acid sequences different from each other.

[17] The manufacturing method according to any one of [1] to [7] or the high-density woven fabric according to any one of [8] to [12], wherein

the protein fiber comprises a modified fibroin,

the modified fibroin comprises a domain sequence represented by

Formula 1:      [(A)$_n$ motif-REP]$_m$,

and

the domain sequence has an amino acid sequence with a reduced content of glycine residues, which corresponds to an amino acid sequence in which at least one or a plurality of glycine residues in REP are substituted with other amino acid residues, as compared with a naturally occurring fibroin,

wherein in Formula 1, the (A)$_n$ motif represents an amino acid sequence composed of 2 to 27 amino acid residues and the number of alanine residues with respect to a total number of amino acid residues in the (A)$_n$ motif is 83% or more;

REP represents an amino acid sequence composed of 10 to 200 amino acid residues;

m represents an integer of 2 to 300;

a plurality of the (A)$_n$ motifs may have the same amino acid sequence or amino acid sequences different from each other; and

a plurality of REPs may have the same amino acid sequence or amino acid sequences different from each other.

[18] The manufacturing method or the high-density woven fabric according to [17], wherein the domain sequence has an amino acid sequence that corresponds to an amino acid sequence in which, in at least one motif sequence selected from GGX and GPGXX (where X represents an amino acid residue other than glycine) in REP, at least one glycine residue in one or a plurality of the motif sequences is substituted with another amino acid residue, as compared with a naturally occurring fibroin.

[19] The manufacturing method or the high-density woven fabric according to [18], wherein a proportion of the motif sequence in which a glycine residue is substituted with another amino acid residue is 10% or more with respect to all motif sequences.

[20] The manufacturing method according to any one of [1] to [7] or the high-density woven fabric according to any one of [8] to [12], wherein

the protein fiber comprises a modified fibroin,

the modified fibroin comprises a domain sequence represented by

Formula 1:      [(A)$_n$ motif-REP]$_m$,

and

z/w is 50.9% or more, where z is the total number of amino acid residues of an amino acid sequence consisting of XGX (where X represents an amino acid residue other than glycine) contained in all REPs in the domain sequence excluding the sequence between the (A)$_n$ motif positioned closest to the C-terminal side and the C-terminal of the domain sequence; and where w is the total number of amino acid residues in the domain sequence excluding the sequence between the (A)$_n$ motif positioned closest to the C-terminal side and the C-terminal of the domain sequence, wherein in Formula 1, the (A)$_n$ motif represents an amino acid sequence composed of 2 to 27 amino acid residues and the number of alanine residues with respect to a total number of amino acid residues in the (A)$_n$ motif is 83% or more;

REP represents an amino acid sequence composed of 10 to 200 amino acid residues;

m represents an integer of 2 to 300;

a plurality of the (A)$_n$ motifs may have the same amino acid sequence or amino acid sequences different from each other; and

a plurality of REPs may have the same amino acid sequence or amino acid sequences different from each other.

[21] The manufacturing method or the high-density woven fabric according to any one of [17] to [20], wherein the modified fibroin has an amino acid sequence that corresponds to an amino acid sequence in which one or a plurality of amino acid residues are further substituted, deleted, inserted, and/or added, in addition to substitution of one or a plurality of glycine residues in REP with other amino acid residues, as compared with a naturally occurring fibroin.

[22] The manufacturing method according to any one of [1] to [7] or the high-density woven fabric according to any one of [8] to [12], wherein

the protein fiber comprises a modified fibroin,

the modified fibroin comprises a domain sequence represented by

Formula 1:      [(A)$_n$ motif-REP]$_m$,

and

the domain sequence has an amino acid sequence comprising a region having a locally high hydropathy index, which corresponds to an amino acid sequence in which one or a plurality of amino acid residues in REP are substituted with an amino acid residue having a high hydropathy index and/or one or a plurality of amino acid residues having a high hydropathy index is inserted into REP, as compared with a naturally occurring fibroin,

wherein in Formula 1, the $(A)_n$ motif represents an amino acid sequence composed of 2 to 27 amino acid residues and the number of alanine residues with respect to a total number of amino acid residues in the $(A)_n$ motif is 83% or more;

REP represents an amino acid sequence composed of 10 to 200 amino acid residues;

m represents an integer of 2 to 300;

a plurality of the $(A)_n$ motifs may have the same amino acid sequence or amino acid sequences different from each other; and

a plurality of REPs may have the same amino acid sequence or amino acid sequences different from each other.

[23] The manufacturing method or the high-density woven fabric according to [22], wherein the region having a locally high hydropathy index is composed of 2 to 4 consecutive amino acid residues.

[24] The manufacturing method or the high-density woven fabric according to [22] or [23], wherein the amino acid residues having a high hydropathy index are selected from isoleucine (I), valine (V), leucine (L), phenylalanine (F), cysteine (C), methionine (M), and alanine (A).

[25] The manufacturing method according to any one of [1] to [7] or the high-density woven fabric according to any one of [8] to [12], wherein

the protein fiber comprises a modified fibroin,

the modified fibroin comprises a domain sequence represented by

Formula 1: $[(A)_n \text{ motif-REP}]_m$,

and

p/q is 6.2% or more, where p is the total number of amino acid residues contained in a region where an average value of hydropathy indices of the four consecutive amino acid residues is 2.6 or more in all REPs contained in the domain sequence excluding the sequence between the $(A)_n$ motif positioned closest to the C-terminal side and the C-terminal of the domain sequence; and where q is the total number of amino acid residues contained in the domain sequence excluding the sequence between the $(A)_n$ motif positioned closest to the C-terminal side and the C-terminal of the domain sequence,

wherein in Formula 1, the $(A)_n$ motif represents an amino acid sequence composed of 2 to 27 amino acid residues and the number of alanine residues with respect to a total number of amino acid residues in the $(A)_n$ motif is 83% or more;

REP represents an amino acid sequence composed of 10 to 200 amino acid residues;

m represents an integer of 2 to 300;

a plurality of the $(A)_n$ motifs may have the same amino acid sequence or amino acid sequences different from each other; and

a plurality of REPs may have the same amino acid sequence or amino acid sequences different from each other.

[26] The manufacturing method or the high-density woven fabric according to any one of [22] to [25], wherein the modified fibroin has an amino acid sequence that corresponds to an amino acid sequence in which one or a plurality of amino acid residues are substituted, deleted, inserted, and/or added, in addition to substitution of one or a plurality of amino acid residues in REP with amino acid residues having a high hydropathy index and/or insertion of one or a plurality of amino acid residues having a high hydropathy index into REP, as compared with a naturally occurring fibroin.

[27] The manufacturing method according to any one of [1] to [7] or the high-density woven fabric according to any one of [8] to [12], wherein

the protein fiber comprises a modified fibroin,

the modified fibroin comprises a domain sequence represented by

Formula 1: $[(A)_n \text{ motif-REP}]_m$

or

Formula 2: $[(A)_n \text{ motif-REP}]_m\text{-}(A)_n$

motif, and

the domain sequence has an amino acid sequence with a reduced content of glutamine residues, which corresponds to an amino acid sequence in which one or a plurality of glutamine residues in REP are deleted or substituted with other amino acid residues, as compared with a naturally occurring fibroin,

wherein in Formula 1 and Formula 2, the $(A)_n$ motif represents an amino acid sequence composed of 2 to 27 amino acid residues and the number of alanine residues with respect to a total number of amino acid residues in the $(A)_n$ motif is 80% or more;

REP represents an amino acid sequence composed of 10 to 200 amino acid residues;

m represents an integer of 2 to 300;

a plurality of the $(A)_n$ motifs may have the same amino acid sequence or amino acid sequences different from each other; and

a plurality of REPs may have the same amino acid sequence or amino acid sequences different from each other.

[28] The manufacturing method or the high-density woven fabric according to [27], wherein the modified fibroin contains a GPGXX (where X represents an amino acid residue other than a glycine residue) motif in REP, and a GPGXX motif content rate is 10% or more.

[29] The manufacturing method or the high-density woven fabric according to [27] or [28], wherein a glutamine residue content rate of the modified fibroin is 9% or less.

[30] The manufacturing method or the high-density woven fabric according to any one of [27] to [29], wherein the other amino acid residues are amino acid residues selected from the group consisting of isoleucine (I), valine (V), leucine (L), phenylalanine (F), cysteine (C), methionine (M), alanine (A), glycine (G), threonine (T), serine (S), tryptophan (W), tyrosine (Y), proline (P), and histidine (H).

[31] The manufacturing method or the high-density woven fabric according to any one of [27] to [30], wherein the modified fibroin has a degree of hydrophobicity of REP of -0.8 or more.

[32] The manufacturing method or the high-density woven fabric according to any one of [27] to [31], wherein the modified fibroin has an amino acid sequence that corresponds to an amino acid sequence in which one or a plurality of amino acid residues are substituted, deleted, inserted, and/or added, in addition to deletion of one or a plurality of glutamine residues in REP or substitution with other amino acid residues, as compared with a naturally occurring fibroin.

[33] The manufacturing method according to any one of [1] to [7] or the high-density woven fabric according to any one of [8] to [12], wherein

the protein fiber comprises a modified fibroin, and

the modified fibroin has a limiting oxygen index (LOI) value of 26.0 or more.

[34] The manufacturing method according to any one of [1] to [7] or the high-density woven fabric according to any one of [8] to [12], wherein

the protein fiber comprises a modified fibroin, and

a maximum hygroscopic heat generation, as determined according to the following formula A, of the modified fibroin is more than 0.025°C/g:

$$\text{Formula A: Maximum hygroscopic heat generation} = \{(\text{Highest temperature of a sample when the sample has been transferred to a high humidity environment after being placed in a low humidity environment until a temperature of the sample reaches equilibrium}) - (\text{Temperature of the sample when the sample is being transferred to the high humidity environment after being placed in the low humidity environment until the temperature of the sample reaches equilibrium})\} \, (°C) / \text{Sample weight (g)},$$

wherein in Formula A, the low humidity environment means an environment of a temperature of 20°C and a relative humidity of 40%, and the high humidity environment means an environment of a temperature of 20°C and a relative humidity of 90%.

**Advantageous Effects of Invention**

According to the present invention, a high-density woven fabric containing a protein fiber and a method for manufacturing the same are provided. In particular, according to the present invention, it is possible to provide a woven fabric having a high density without adding special restrictions or conditions (for example, the use of a thin yarn or weaving with a high weaving density) in the weaving step. Further, according to the present invention, it is possible to provide a woven fabric having a weaving density exceeding the weaving limit.

**Brief Description of Drawings**

Fig. 1 is a schematic diagram illustrating an example of a domain sequence of a modified fibroin.
Fig. 2 is a diagram illustrating a distribution of z/w (%) values of a naturally occurring fibroin.
Fig. 3 is a diagram illustrating a distribution of x/y (%) values of a naturally occurring fibroin.
Fig. 4 is a schematic diagram illustrating an example of a domain sequence of a modified fibroin.
Fig. 5 is a schematic diagram illustrating an example of a domain sequence of a modified fibroin.
Fig. 6 is an explanatory diagram schematically illustrating an example of a spinning device for manufacturing a protein fiber.
Fig. 7 is a graph illustrating an example of the results of a hygroscopic heat generation test.

**Description of Embodiments**

Hereinafter, embodiments for carrying out the present invention will be described in detail. However, the present invention is not limited to the following embodiments.

[Method for manufacturing high-density woven fabric]

A method for manufacturing a high-density woven fabric according to the present embodiment includes: weaving a fiber that at least partially contains a protein fiber to obtain a raw-material woven fabric (weaving step); and bringing the raw-material woven fabric into contact with aqueous moisture to obtain a high-density woven fabric (shrinking step).

<Weaving step>

In the weaving step, the raw-material woven fabric is obtained by weaving a fiber that at least partially contains a protein fiber.

<Protein>

The protein may be, for example, a structural protein or a modified structural protein derived from the structural protein. The structural protein means a protein that forms or retains a structure, morphology, and the like in a living body. Examples of the structural protein include fibroin, collagen, resilin, elastin, and keratin.

As the fibroin, for example, the following modified fibroin may be used. The modified fibroin according to the present embodiment is a protein containing a domain sequence represented by Formula 1: $[(A)_n$ motif-REP$]_m$ or Formula 2: $[(A)_n$ motif-REP$]_m$-$(A)_n$ motif. In the modified fibroin, an amino acid sequence (an N-terminal sequence and a C-terminal sequence) may be further added to either or both of the N-terminal side and the C-terminal side of the domain sequence. The N-terminal sequence and the C-terminal sequence are typically, but not limited to, regions that do not have a repetition of amino acid motifs characteristic of a fibroin, and that consist of amino acids of about 100 residues.

The "modified fibroin" in the present specification means an artificially produced fibroin (an artificial fibroin). The modified fibroin may be a fibroin in which the domain sequence is different from an amino acid sequence of a naturally occurring fibroin or the same as the amino acid sequence of a naturally occurring fibroin. The "naturally occurring fibroin" referred to in the present specification also is a protein containing a domain sequence represented by Formula 1: $[(A)_n$ motif-REP$]_m$ or Formula 2: $[(A)_n$ motif-REP$]_m$-$(A)_n$ motif.

The "modified fibroin" may be a modified fibroin having an amino acid sequence of a naturally occurring fibroin as it is, may be a modified fibroin whose amino acid sequence has been modified based on the amino acid sequence of a naturally occurring fibroin (for example, a modified fibroin whose amino acid sequence has been modified by altering a cloned gene sequence of a naturally occurring fibroin), or may be a modified fibroin that is artificially designed and synthesized independently of a naturally occurring fibroin (for example, a modified fibroin having a desired amino acid sequence by chemically synthesizing a nucleic acid encoding a designed amino acid sequence).

The "domain sequence" in the present specification is an amino acid sequence that produces a crystalline region (typically corresponding to the $(A)_n$ motif of an amino acid sequence) and an amorphous region (typically corresponding to REP of an amino acid sequence) unique to a fibroin, and refers to an amino acid sequence represented by Formula 1: [(A)n motif-REP]m or Formula 2: [(A)n motif-REP]m-(A)n motif. Here, the (A)n motif represents an amino acid sequence mainly containing alanine residues, and the number of amino acid residues in the (A)n motif is 2 to 27. The number of amino acid residues in the (A)n motif may be an integer of 2 to 20, 4 to 27, 4 to 20, 8 to 20, 10 to 20, 4 to 16, 8 to 16, or 10 to 16. Further, the proportion of the number of alanine residues with respect to the total number of amino acid residues in the (A)n motif may be 40% or more, and may be 60% or more, 70% or more, 80% or more, 83% or more, 85% or more, 86% or more, 90% or more, 95% or more, or 100% (meaning that the (A)n motif is composed of only alanine residues). Among a plurality of (A)n motifs present in the domain sequence, at least seven of the (A)n motifs may be composed only of alanine residues. REP represents an amino acid sequence composed of 2 to 200 amino acid residues. REP may be an amino acid sequence composed of 10 to 200 amino acid residues, and may be an amino acid sequence composed of 10 to 40, 10 to 60, 10 to 80, 10 to 100, 10 to 120, 10 to 140, 10 to 160, or 10 to 180 amino acid residues. m represents an integer of 2 to 300, and may be an integer of 8 to 300, 10 to 300, 20 to 300, 40 to 300, 60 to 300, 80 to 300, 100 to 300, 10 to 200, 20 to 200, 20 to 180, 20 to 160, 20 to 140, or 20 to 120. A plurality of (A)n motifs may have the same amino acid sequence or amino acid sequences different from each other. A plurality of REPs may have the same amino acid sequence or amino acid sequences different from each other.

The modified fibroin according to the present embodiment may be obtained, for example, by subjecting a cloned gene sequence of a naturally occurring fibroin to a modification of an amino acid sequence corresponding to, for example, substitution, deletion, insertion, and/or addition of one or a plurality of amino acid residues. The substitution, deletion, insertion, and/or addition of an amino acid residue may be performed by methods well known to those skilled in the art, such as site-directed mutagenesis. Specifically, the modification may be performed in accordance with the method described in literature such as Nucleic Acid Res. 10, 6487 (1982) and Methods in Enzymology, 100, 448 (1983).

A naturally occurring fibroin is a protein containing a domain sequence represented by Formula 1: [(A)n motif-REP]m or Formula 2: [(A)n motif-REP]m-(A)n motif, and specific examples thereof include a fibroin produced by insects or spiders.

Examples of the fibroin produced by insects include silk proteins produced by silkworms such as Bombyx mori, Bombyx mandarina, Antheraea yamamai, Anteraea pernyi, Eriogyna pyretorum, Philosamia cynthia ricini, Samia cynthia, Caligula japonica, Antheraea mylitta, and Antheraea assama; and hornet silk proteins discharged by larvae of Vespa simillima xanthoptera.

More specific examples of the fibroin produced by insects include a silkworm fibroin L chain (GenBank Accession No. M76430 (nucleotide sequence), and AAA27840.1 (amino acid sequence)).

Examples of the fibroin produced by spiders include spider silk proteins produced by spiders belonging to the genus Araneus such as Araneus ventricosus, Araneus diadematus, Araneus pinguis, Araneus pentagrammicus and Araneus nojimai, spiders belonging to the genus Neoscona such as Neoscona scylla, Neoscona nautica, Neoscona adianta and Neoscona scylloides, spiders belonging to the genus Pronus such as Pronous minutes, spiders belonging to the genus Cyrtarachne such as Cyrtarachne bufo and Cyrtarachne inaequalis, spiders belonging to the genus Gasteracantha such as Gasteracantha kuhli and Gasteracantha mammosa, spiders belonging to the genus Ordgarius such as Ordgarius hobsoni and Ordgarius sexspinosus, spiders belonging to the genus Argiope such as Argiope amoena, Argiope minuta and Argiope bruennich, spiders belonging to the genus Arachnura such as Arachnura logio, spiders belonging to the genus Acusilas such as Acusilas coccineus, spiders belonging to the genus Cyrtophora such as Cyrtophora moluccensis, Cyrtophora exanthematica and Cyrtophora unicolor, spiders belonging to the genus Poltys such as Poltys illepidus, spiders belonging to the genus Cyclosa such as Cyclosa octotuberculata, Cyclosa sedeculata, Cyclosa vallata and Cyclosa atrata, and spiders belonging to the genus Chorizopes such as Chorizopes nipponicus; and spider silk proteins produced by spiders belonging to the genus Tetragnatha such as Tetragnatha praedonia, Tetragnatha maxillosa, Tetragnatha extensa and Tetragnatha squamata, spiders belonging to the genus Leucauge such as Leucauge magnifica, Leucauge blanda and Leucauge subblanda, spiders belonging to the genus Nephila such as Nephila clavata and Nephila pilipes, spiders belonging to the genus Menosira such as Menosira ornata, spiders belonging to the genus Dyschiriognatha such as Dyschiriognatha tenera, spiders belonging to the genus Latrodectus such as Latrodectus mactans, Latrodectus hasseltii, Latrodectus geometricus and Latrodectus tredecimguttatus, and spiders belonging to the family Tetragnathidae such as spiders belonging to the genus Euprosthenops. Examples of the spider silk proteins include traction yarn proteins such as MaSp (MaSp1 and MaSp2) and ADF (ADF3 and ADF4), and MiSp (MiSp1 and MiSp2).

More specific examples of the spider silk protein produced by spiders include fibroin-3 (adf-3) [derived from Araneus diadematus] (GenBank Accession No. AAC47010 (amino acid sequence), U47855 (nucleotide sequence)), fibroin-4 (adf-4) [derived from Araneus diadematus] (GenBank Accession No. AAC47011 (amino acid sequence), U47856 (nucleotide sequence)), dragline silk protein spidroin 1 [derived from Nephila clavipes] (GenBank Accession No. AAC04504 (amino acid sequence), U37520 (nucleotide sequence)), major ampullate spidroin 1 [derived from Latrodectus hesperus] (GenBank Accession No. ABR68856 (amino acid sequence), EF595246 (nucleotide sequence)), dragline silk protein spidroin 2 [derived from Nephila clavata] (GenBank Accession No. AAL32472 (amino acid sequence), AF441245 (nucleotide sequence)), major ampullate spidroin 1 [derived from Euprosthenops australis] (GenBank Accession No. CAJ00428 (amino acid sequence), AJ973155 (nucleotide sequence)) and major ampullate spidroin 2 [Euprosthenops australis] (GenBank Accession No. CAM32249.1 (amino acid sequence), AM490169 (nucleotide sequence)), minor ampullate silk protein 1 [Nephila clavipes] (GenBank Accession No. AAC14589.1 (amino acid sequence), minor ampullate silk protein 2 [Nephila clavipes] (GenBank Accession No. AAC14591.1 (amino acid sequence)), and minor ampullate spidroin-like protein [Nephilengys cruentata] (GenBank Accession No. ABR37278.1 (amino acid sequence)).

More specific examples of the naturally occurring fibroin further include a fibroin whose sequence information is registered in NCBI GenBank. For example, sequences thereof may be confirmed by extracting sequences that has a keyword of spidroin, ampullate, fibroin, "silk and polypeptide", or "silk and protein" in DEFINITION, among sequences containing INV as DIVISION in sequence information registered in NCBI GenBank; sequences that have a specific character string of a product from CDS; or sequences that have a specific character string described for TISSUE TYPE from SOURCE.

The modified fibroin according to the present embodiment may be a modified silk fibroin (a modified fibroin obtained by modifying an amino acid sequence of a silk protein produced by silkworm), or may be a modified spider silk fibroin (a modified fibroin obtained by modifying an amino acid sequence of a spider silk protein produced by spiders). As the modified fibroin, a modified spider silk fibroin is preferable. The modified spider silk fibroin is also excellent in heat retention, hygroscopic exothermicity, and/or flame retardancy.

Specific examples of the modified fibroin include a modified fibroin derived from a spigot dragline silk protein produced in the major ampullate gland of a spider (first modified fibroin), a modified fibroin having a domain sequence with a reduced content of glycine residues (second modified fibroin), a modified fibroin having a domain sequence with a reduced content of $(A)_n$ motifs (third modified fibroin), a modified fibroin with a reduced content of glycine residues and $(A)_n$ motifs (fourth modified fibroin), a modified fibroin having a domain sequence containing a region having a locally high hydropathy index (fifth modified fibroin), and a modified fibroin having a domain sequence with a reduced content of glutamine residues (sixth modified fibroin).

Examples of the first modified fibroin include a protein containing a domain sequence represented by Formula 1: $[(A)_n$ motif-REP$]_m$. In the first modified fibroin, the number of amino acid residues of the $(A)_n$ motif is preferably an integer of 3 to 20, more preferably an integer of 4 to 20, still more preferably an integer of 8 to 20, even still more preferably an integer of 10 to 20, still further preferably an integer of 4 to 16, particularly preferably an integer of 8 to 16, and most preferably an integer of 10 to 16. In the first modified fibroin, the number of amino acid residues constituting REP in Formula 1 is preferably 10 to 200 residues, more preferably 10 to 150 residues, still more preferably 20 to 100 residues, and even still more preferably 20 to 75 residues. In the first modified fibroin, the total number of the glycine residues, the serine residues, and the alanine residues contained in the amino acid sequence represented by Formula 1: $[(A)_n$ motif-REP$]_m$ is preferably 40% or more, more preferably 60% or more, and still more preferably 70% or more with respect to the total number of amino acid residues.

The first modified fibroin may be a polypeptide that contains a unit of an amino acid sequence represented by Formula 1: $[(A)_n$ motif-REP$]_m$, and has the amino acid sequence set forth in any of SEQ ID NOs: 1 to 3 or an amino acid sequence having 90% or more homology with the amino acid sequence set forth in any of SEQ ID NOs: 1 to 3 as the C-terminal sequence.

The amino acid sequence set forth in SEQ ID NO: 1 is the same as the amino acid sequence consisting of 50 amino acid residues at the C-terminal of the amino acid sequence of ADF3 (GI: 1263287, NCBI), the amino acid sequence set forth in SEQ ID NO: 2 is the same as the amino acid sequence obtained by removing 20 residues from the C-terminal of the amino acid sequence set forth in SEQ ID NO: 1, and the amino acid sequence set forth in SEQ ID NO: 3 is the same as the amino acid sequence obtained by removing 29 residues from the C-terminal of the amino acid sequence set forth in SEQ ID NO: 1.

More specific examples of the first modified fibroin include a modified fibroin containing (1-i) the amino acid sequence set forth in SEQ ID NO: 4 (recombinant spider silk protein ADF3KaiLargeNRSH1), or (1-ii) an amino acid sequence having 90% or more sequence identity with the amino acid sequence set forth in SEQ ID NO: 4. The sequence identity is preferably 95% or more.

The amino acid sequence shown in SEQ ID NO 4 is obtained by performing mutation on the amino acid sequence of ADF3 in which an amino acid sequence (SEQ ID NO 5) consisting of a start codon, His10 tag, and HRV3C protease (Human rhinovirus 3C protease) recognition site is added to the N-terminal, such that the 1st to 13th repeat regions are approximately doubled, and the translation terminates at the 1154th amino acid residue. The amino acid sequence of the C-terminal of the amino acid sequence shown in SEQ ID NO 4 is the same as the amino acid sequence shown in SEQ ID NO 3.

The modified fibroin (1-i) may be a modified fibroin consisting of the amino acid sequence set forth in SEQ ID NO: 4.

The second modified fibroin has a domain sequence that has an amino acid sequence with a reduced content of glycine residues, as compared with a naturally occurring fibroin. It can be said that the second modified fibroin is a modified fibroin having an amino acid sequence that corresponds to an amino acid sequence in which, at least one or a plurality of glycine residues in REP are substituted with other amino acid residues, as compared with a naturally occurring fibroin.

The second modified fibroin may have a domain sequence that has an amino acid sequence corresponding to an amino acid sequence in which, in at least one motif sequence selected from GGX and GPGXX (where G represents a glycine residue, P represents a proline residue, and X represents an amino acid residue other than glycine) in REP, at least one glycine residue in one or a plurality of motif sequences is substituted with another amino acid residue, as compared with a naturally occurring fibroin.

In the second modified fibroin, the proportion of the above-described motif sequences in which a glycine residue is substituted with another amino acid residue may be 10% or more with respect to the all motif sequences.

The second modified fibroin may be a modified fibroin that contains a domain sequence represented by Formula 1: $[(A)_n$ motif-REP$]_m$, that has an amino acid sequence in which z/w is 30% or more, 40% or more, 50% or more, or 50.9% or more, where z is the total number of amino acid residues of an amino acid sequence consisting of XGX (where X represents an amino acid residue other than glycine) contained in all REPs in the domain sequence excluding the sequence between the $(A)_n$ motif positioned closest to the C-terminal side and the C-terminal of the domain sequence; and where w is the total number of amino acid residues in the domain sequence excluding the sequence between the $(A)_n$ motif positioned closest to the C-terminal side and the C-terminal of the domain sequence. The number of alanine residues with respect to a total number of amino acid residues in the $(A)_n$ motif may be 83% or more, but is preferably 86% or more, more preferably 90% or more, still more preferably 95% or more, and even still more preferably 100% (which means that the $(A)_n$ motif consists only of alanine residues).

The second modified fibroin is preferably a modified fibroin in which one glycine residue of the GGX motif is substituted with another amino acid residue to increase the content ratio of the amino acid sequence consisting of XGX. In the second modified fibroin, the content ratio of the amino acid sequence consisting of GGX in the domain sequence is preferably 30% or less, more preferably 20% or less, still more preferably 10% or less, even still more preferably 6 % or less, further still more preferably 4% or less, and particularly preferably 2% or less. The content ratio of the amino acid sequence consisting of GGX in the domain sequence can be calculated by the same method as the following method for calculating a content ratio (z/w) of the amino acid sequence consisting of XGX.

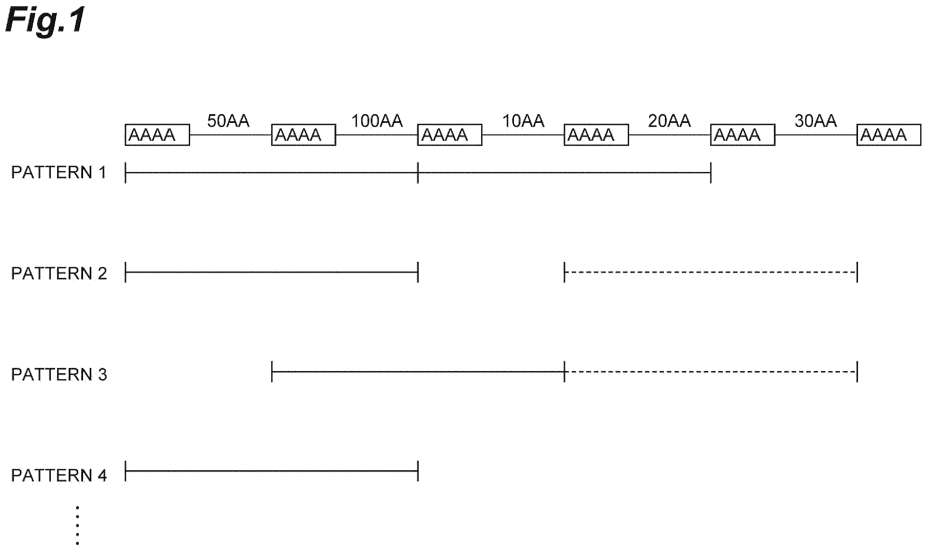

The method for calculating z/w will be described in more detail. First, in a fibroin (a modified fibroin or a naturally occurring fibroin) containing a domain sequence represented by Formula 1: $[(A)_n$ motif-REP$]_m$, an amino acid sequence consisting of XGX is extracted from all REPs contained in the domain sequence excluding the sequence between the $(A)_n$ motif positioned closest to the C-terminal side and the C-terminal of the domain sequence. The total number of amino acid residues constituting XGX is z. For example, in a case where 50 amino acid sequences consisting of XGX (without an overlap) are extracted, z is 50 $\times$ 3 = 150. Further, for example, in a case where there is X contained in two XGXs (X at the center), as in the case of an amino acid sequence consisting of XGXGX, the calculation is performed by deducting the overlap (in the case of XGXGX, 5 amino acid residues). w is the total number of amino acid residues contained in the domain sequence excluding the sequence between the $(A)_n$ motif positioned closest to the C-terminal side and the C-terminal of the domain sequence. For example, in the case of the domain sequence illustrated in Fig. 1, w is 4 + 50 + 4 + 100 + 4 + 10 + 4 + 20 + 4 + 30 = 230 (the $(A)_n$ motif positioned closest to the C-terminal side is excluded.). Next, z/w (%) can be calculated by dividing z by w.

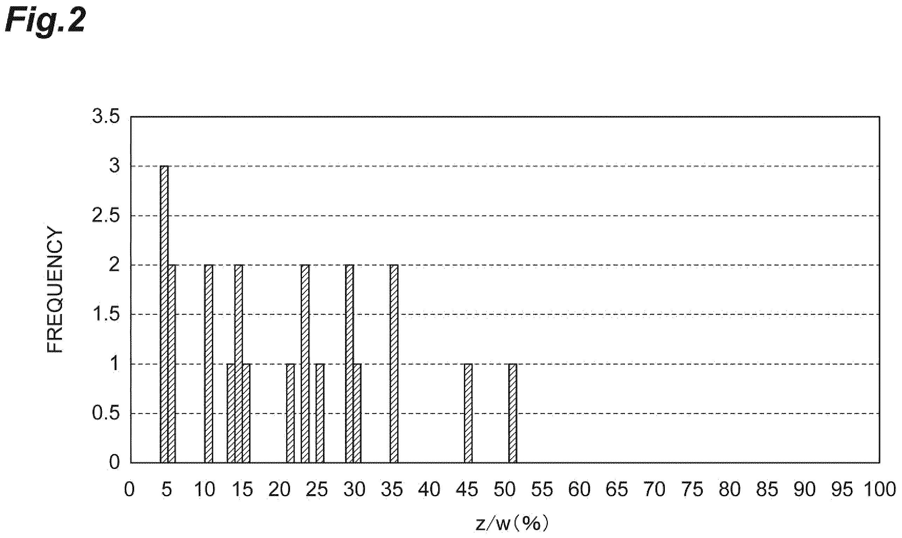

Here, z/w in a naturally occurring fibroin will be described. First, fibroins with amino acid sequence information registered in NCBI GenBank was checked by the exemplified method as described above, and 663 types of fibroins (of which 415 types were fibroins derived from spiders) were extracted. Among all the extracted fibroins, z/w was calculated from an amino acid sequence of a naturally occurring fibroin that contains a domain sequence represented by Formula 1: $[(A)_n$ motif-REP$]_m$, and that has a content ratio of the amino acid sequence consisting of GGX in the fibroin of 6% or less, using the above-described calculation method. The results are shown in Fig. 2. In Fig. 2, the horizontal axis represents z/w (%), and the vertical axis represents frequency. As apparent from Fig. 2, z/w in all the naturally occurring fibroin is less than 50.9% (highest value: 50.86%).

In the second modified fibroin, z/w is preferably 50.9% or more, more preferably 56.1% or more, still more preferably 58.7% or more, even still more preferably 70% or more, and still further preferably 80% or more. The upper limit of z/w is not particularly limited, but may be, for example, 95% or less.

The second modified fibroin may be obtained, for example, by modifying a cloned gene sequence of a naturally occurring fibroin such that at least a part of the nucleotide sequence encoding a glycine residue is substituted to encode another amino acid residue. In this case, one glycine residue in the GGX motif and the GPGXX motif may be selected as the glycine residue to be modified, and also, substitution may be performed so that z/w becomes 50.9% or more. In addition, the second modified fibroin may also be obtained, for example, by designing an amino acid sequence that satisfies the above embodiment based on the amino acid sequence of a naturally occurring fibroin, and chemically synthesizing a nucleic acid encoding the designed amino acid sequence. In any case, in addition to the modification that corresponds to substitution of the glycine residue in REP in the amino acid sequence of a naturally occurring fibroin with another amino acid residue, a modification of the amino acid sequence that corresponds to substitution, deletion, insertion, and/or addition of one or a plurality of amino acid residues may further be performed.

The above another amino acid residue is not particularly limited as long as it is an amino acid residue other than the glycine residue, but is preferably a hydrophobic amino acid residue such as a valine (V) residue, a leucine (L) residue, a isoleucine (I) residue, a methionine (M) residue, a proline (P) residue, a phenylalanine (F) residue, and a tryptophan (W) residue, or a hydrophilic amino acid residues such as a glutamine (Q) residue, an asparagine (N) residue, a serine (S) residue, a lysine (K) residue, and a glutamic acid (E) residue, more preferably a valine (V) residue, a leucine (L) residue, an isoleucine (I) residue, a phenylalanine (F) residue, and a glutamine (Q) residue, and still more preferably a glutamine (Q) residue.

More specific examples of the second modified fibroin include a modified fibroin containing (2-i) the amino acid sequence set forth in SEQ ID NO: 6 (Met-PRT380), SEQ ID NO: 7 (Met-PRT410), SEQ ID NO: 8 (Met-PRT525), or SEQ ID NO: 9 (Met-PRT799), or (2-ii) an amino acid sequence having 90% or more sequence identity with the amino acid sequence set forth in SEQ ID NO: 6, SEQ ID NO: 7, SEQ ID NO: 8, or SEQ ID NO: 9.

The modified fibroin (2-i) will be described. The amino acid sequence set forth in SEQ ID NO: 6 is an amino acid sequence in which all GGXs in REP of the amino acid sequence set forth in SEQ ID NO: 10 (Met-PRT313) corresponding to a naturally occurring fibroin are substituted with GQXs. The amino acid sequence set forth in SEQ ID NO: 7 is an amino acid sequence in which every third $(A)_n$ motif from the N-terminal side to the C-terminal side is deleted from the amino acid sequence set forth in SEQ ID NO: 6, and further, one $[(A)_n$ motif-REP] is inserted before the C-terminal sequence. The amino acid sequence set forth in SEQ ID NO: 8 is an amino acid sequence in which two alanine residues are inserted at the C-terminal side of each $(A)_n$ motif of the amino acid sequence set forth in SEQ ID NO: 7, and further some of the glutamine (Q) residues are substituted with a serine (S) residue, and some of the amino acids on the C-terminal side are deleted so that the molecular weight is almost the same as that of SEQ ID NO 7. The amino acid sequence set forth in SEQ ID NO: 9 is an amino acid sequence in which a predetermined hinge sequence and a His tag sequence are added to the C-terminal of a sequence having four-time repetition of a region of 20 domain sequences present in the amino acid sequence set forth in SEQ ID NO: 7 (however, with several amino acid residues on the C-terminal side of the region are substituted).

The value of z/w in the amino acid sequence set forth SEQ ID NO: 10 (corresponding to a naturally occurring fibroin) is 46.8%. The values of z/w in the amino acid sequence set forth in SEQ ID NO: 6, the amino acid sequence set forth in SEQ ID NO: 7, the amino acid sequence set forth in SEQ ID NO: 8, and the amino acid sequence set forth in SEQ ID NO: 9 are 58.7%, 70.1%, 66.1%, and 70.0%, respectively. In addition, the values of x/y at the Giza ratio (which will be described later) of 1:1.8 to 11.3 in the amino acid sequences set forth in SEQ ID NO: 10, SEQ ID NO: 6, SEQ ID NO: 7, SEQ ID NO: 8, and SEQ ID NO: 9 are 15.0%, 15.0%, 93.4%, 92.7%, and 89.8%, respectively.

The modified fibroin (2-i) may be a modified fibroin consisting of the amino acid sequence set forth in SEQ ID NO: 6, SEQ ID NO: 7, SEQ ID NO: 8, or SEQ ID NO: 9.

The modified fibroin (2-ii) is a modified fibroin containing an amino acid sequence having 90% or more sequence identity with the amino acid sequence set forth in SEQ ID NO: 6, SEQ ID NO: 7, SEQ ID NO: 8, or SEQ ID NO: 9. The modified fibroin (2-ii) also is a protein containing a domain sequence represented by Formula 1: $[(A)_n$ motif-REP$]_m$. The sequence identity is preferably 95% or more.

The modified fibroin (2-ii) preferably has 90% or more sequence identity with the amino acid sequence set forth in SEQ ID NO: 6, SEQ ID NO: 7, SEQ ID NO: 8, or SEQ ID NO: 9, and that z/w is 50.9% or more, where z is the total number of amino acid residues in the amino acid sequence consisting of XGX (where X represents an amino acid residue other than glycine) contained in REP; and where w is the total number of amino acid residues in REP in the domain sequence.

The second modified fibroin may contain a tag sequence at either or both of the N-terminal and C-terminal. This makes it possible to isolate, immobilize, detect, and visualize the modified fibroin.

The tag sequence may be, for example, an affinity tag utilizing specific affinity (binding property, affinity) with other molecules. Specific examples of the affinity tag include a histidine tag (His tag). The His tag is a short peptide in which about 4 to 10 histidine residues are arranged and has a property of specifically binding to a metal ion such as nickel, and thus, it can be used for isolation of a modified fibroin by a chelating metal chromatography. Specific examples of the tag sequence include an amino acid sequence set forth in SEQ ID NO: 11 (amino acid sequence containing a His tag sequence and a hinge sequence).

In addition, a tag sequence such as a glutathione-S-transferase (GST) that specifically binds to glutathione and a maltose binding protein (MBP) that specifically binds to maltose may also be used.

Furthermore, an "epitope tag" utilizing an antigen-antibody reaction may also be used. By adding a peptide (an epitope) showing antigenicity as a tag sequence, an antibody against the epitope can be bound. Examples of the epitope tag include an HA (peptide sequence of hemagglutinin of an influenza virus) tag, a myc tag, and a FLAG tag. The modified fibroin can be easily purified with high specificity by utilizing the epitope tag.

It is also possible to further use a tag sequence which can be cleaved with a specific protease. By treating a protein adsorbed via the tag sequence with a protease, it is also possible to recover a modified fibroin cleaved from the tag sequence.

More specific examples of the modified fibroin containing the tag sequence include a modified fibroin containing (2-iii) the amino acid sequence set forth in SEQ ID NO: 12 (PRT380), SEQ ID NO: 13 (PRT410), SEQ ID NO: 14 (PRT525), or SEQ ID NO: 15 (PRT799), or (2-iv) an amino acid sequence having 90% or more sequence identity with the amino acid sequence set forth in SEQ ID NO: 12, SEQ ID NO: 13, SEQ ID NO: 14, or SEQ ID NO: 15.

The amino acid sequences set forth in SEQ ID NO: 16 (PRT313), SEQ ID NO: 12, SEQ ID NO: 13, SEQ ID NO: 14, and SEQ ID NO: 15 are amino acid sequences in which the amino acid sequence (containing a His tag sequence and a hinge sequence) set forth in SEQ ID NO: 11 is added to the N-terminal of the amino acid sequences set forth in SEQ ID NO: 10, SEQ ID NO: 6, SEQ ID NO: 7, SEQ ID NO: 8, and SEQ ID NO: 9, respectively.

The modified fibroin (2-iii) may be a modified fibroin consisting of the amino acid sequence set forth in SEQ ID NO: 12, SEQ ID NO: 13, SEQ ID NO: 14, or SEQ ID NO: 15.

The modified fibroin (2-iv) is a modified fibroin containing an amino acid sequence having 90% or more sequence identity with the amino acid sequence set forth in SEQ ID NO: 12, SEQ ID NO: 13, SEQ ID NO: 14, or SEQ ID NO: 15. The modified fibroin (2-iv) also is a protein containing a domain sequence represented by Formula 1: $[(A)_n$ motif-REP$]_m$. The sequence identity is preferably 95% or more.

It is preferable that the modified fibroin (2-iv) has 90% or more sequence identity with the amino acid sequence set forth in SEQ ID NO: 12, SEQ ID NO: 13, SEQ ID NO: 14, or SEQ ID NO: 15, and that z/w is 50.9% or more, where z is the total number of amino acid residues in the amino acid sequence consisting of XGX (where X represents an amino acid residue other than glycine) contained in REP; and where w is the total number of amino acid residues in REP in the domain sequence.

The second modified fibroin may contain a secretory signal for releasing the protein produced in a recombinant protein production system to the outside of the host. The sequence of the secretory signal may be set as appropriate, depending on the type of the host.

The third modified fibroin has a domain sequence that has an amino acid sequence with a reduced content of $(A)_n$ motifs, as compared with a naturally occurring fibroin. It can be said that the domain sequence of the third modified fibroin is a domain sequence having an amino acid sequence that corresponds to an amino acid sequence in which at least one or a plurality of the $(A)_n$ motifs are deleted, as compared with a naturally occurring fibroin.

The third modified fibroin may be a modified fibroin having an amino acid sequence that corresponds to an amino acid sequence in which 10% to 40% of the $(A)_n$ motifs are deleted from a naturally occurring fibroin.

The domain sequence of the third modified fibroin may be a domain sequence having an amino acid sequence that corresponds to an amino acid sequence in which at least one $(A)_n$ motif for every one to three $(A)_n$ motifs from the N-terminal side to the C-terminal side is deleted, as compared with a naturally occurring fibroin.

The domain sequence of the third modified fibroin may be a domain sequence having an amino acid sequence that corresponds to an amino acid sequence in which at least deletion of two consecutive $(A)_n$ motifs and deletion of one $(A)_n$ motif are repeated in this order from the N-terminal side to the C-terminal side, as compared with a naturally occurring fibroin.

The domain sequence of the third modified fibroin may be a domain sequence having an amino acid sequence that corresponds to an amino acid sequence in which, at least, every third $(A)_n$ motif from the N-terminal side to the C-terminal side is deleted.

The third modified fibroin may be a modified fibroin that contains a domain sequence represented by Formula 1: $[(A)_n$ motif-REP$]_m$, and that has an amino acid sequence in which, when the numbers of amino acid residues of REP of two adjacent $[(A)_n$ motif-REP$]$ units are sequentially compared from the N-terminal side to the C-terminal side, x/y is 20% or more, 30% or more, 40% or more, or 50% or more, where x is a maximum value of a total value obtained by adding the numbers of amino acid residues of two adjacent $[(A)_n$ motif-REP$]$ units wherein when the number of amino acid residues of one REP having less amino acid residues is taken as 1, the ratio of the number of amino acids residues of the other REP satisfies 1.8 to 11.3; and where y is a total number of amino acid residues of the domain sequence. The number of alanine residues with respect to a total number of amino acid residues in the $(A)_n$ motif may be 83% or more, but is preferably 86% or more, more preferably 90% or more, still more preferably 95% or more, and even still more preferably 100% (which means that the $(A)_n$ motif consists only of alanine residues).

A method for calculating x/y will be described in more detail with reference to Fig. 1. Fig. 1 illustrates a domain sequence obtained by removing an N-terminal sequence and a C-terminal sequence from a modified fibroin. The domain sequence has a sequence of, from the N-terminal side (left side), $(A)_n$ motif-the first REP (50 amino acid residues)-$(A)_n$ motif-the second REP (100 amino acid residues)-$(A)_n$ motif-the third REP (10 amino acid residues)-$(A)_n$ motif-the fourth REP (20 amino acid residues)-$(A)_n$ motif-the fifth REP (30 amino acid residues)-$(A)_n$ motif.

The two adjacent $[(A)_n$ motif-REP$]$ units are sequentially selected from the N-terminal side to the C-terminal side so that there is no overlap. There may be $[(A)_n$ motif-REP$]$ unit that is not selected. Fig. 1 illustrates Pattern 1 (a comparison of the first REP and the second REP, and a comparison of the third REP and the fourth REP), Pattern 2 (a comparison of the first REP and the second REP, and a comparison of the fourth REP and the fifth REP), Pattern 3 (a comparison of the second REP and the third REP, and a comparison of the fourth REP and the fifth REP), and Pattern 4 (a comparison of the first REP and the second REP). There are selection methods other than these.

Next, for each pattern, the number of amino acid residues of each REP in the selected adjacent two $[(A)_n$ motif-REP] units is compared. The comparison is performed by determining the ratio, relative to one REP having less amino acid residues taken as 1, of the number of amino acid residues of the other REP. For example, in a case of comparing the first REP (50 amino acid residues) and the second REP (100 amino acid residues), the ratio of the number of amino acid residues of the second REP is 100/50 = 2, where the first REP having less amino acid residues is taken as 1. Similarly, in a case of comparing the fourth REP (20 amino acid residues) and the fifth REP (30 amino acid residues), the ratio of the number of amino acid residues of the fifth REP is 30/20 = 1.5, where the fourth REP having less amino acid residues is taken as 1.

In Fig. 1, a set of $[(A)_n$ motif-REP] units in which, where one REP having less amino acid residues is taken as 1, the ratio of the number of amino acid residues of the other REP is 1.8 to 11.3, is indicated by a solid line. In the present specification, this ratio is referred to as a Giza ratio. A set of $[(A)_n$ motif-REP] units in which, where a REP having less amino acid residues is taken as 1, the ratio of the number of amino acid residues of the other REP is less than 1.8 or more than 11.3, is indicated by a dotted line.

In each pattern, the numbers of all amino acid residues (not only REP but also the number of amino acid residues in the $(A)_n$ motif) of the two adjacent $[(A)_n$ motif-REP] units indicated by a solid line are added. Then, the total values obtained by addition are compared, and the total value of the pattern having the highest total value (a maximum value of the total value) is denoted as x. In the example illustrated in Fig. 1, the total value of Pattern 1 is the maximum.

Next, x/y (%) can be calculated by dividing x by the total number y of the amino acid residues of the domain sequence.

In the third modified fibroin, x/y is preferably 50% or more, more preferably 60% or more, still more preferably 65% or more, even still more preferably 70% or more, still further preferably 75% or more, and particularly preferably 80% or more. The upper limit of x/y is not particularly limited, but may be, for example, 100% or less. In a case where the Giza ratio is 1:1.9 to 11.3, x/y is preferably 89.6% or more, in a case where the Giza ratio is 1:1.8 to 3.4, x/y is more preferably 77.1% or more, in a case where the Giza ratio is 1:1.9 to 8.4, x/y is still more preferably 75.9% or more, and in a case where the Giza ratio is 1:1.9 to 4.1, x/y is even still more preferably 64.2% or more.

In a case where the third modified fibroin is a modified fibroin in which at least seven of a plurality of $(A)_n$ motifs present in the domain sequence are composed only of alanine residues, x/y is preferably 46.4% or more, more preferably 50% or more, still more preferably 55% or more, even still more preferably 60% or more, still further preferably 70% or more, and particularly preferably 80% or more. The upper limit of x/y is not particularly limited as long as it is 100% or less.

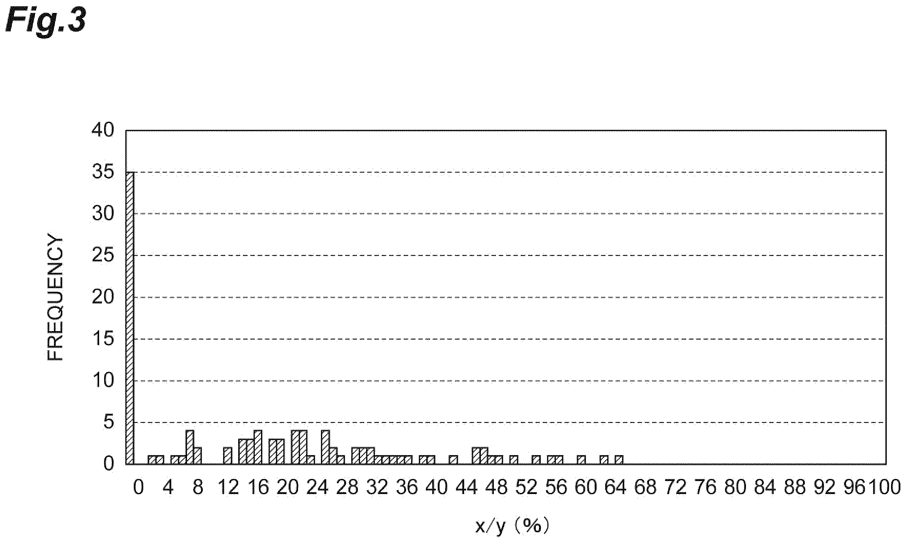

Here, x/y in a naturally occurring fibroin will be described. First, fibroins with amino acid sequence information registered in NCBI GenBank was checked by the exemplified method as described above, and 663 types of fibroins (of which 415 types were fibroins derived from spiders) were extracted. Among all the extracted fibroins, x/y was calculated from an amino acid sequence of a naturally occurring fibroin constituted with a domain sequence represented by Formula 1: $[(A)_n$ motif-REP]$_m$, using the above-described calculation method. The results in a case where the Giza ratio is 1:1.9 to 4.1 are shown in Fig. 3.

In Fig. 3, the horizontal axis represents x/y (%) and the vertical axis represents a frequency. As apparent from Fig. 3, x/y in all the naturally occurring fibroin is less than 64.2% (highest value: 64.14%).

The third modified fibroin may be obtained, for example, by deleting one or a plurality of sequences encoding $(A)_n$ motif from a cloned gene sequence of a naturally occurring fibroin so that x/y is 64.2% or more. In addition, the third modified fibroin may also be obtained, for example, by designing an amino acid sequence that corresponds to an amino acid sequence in which one or a plurality of $(A)_n$ motifs are deleted from an amino acid sequence of a naturally occurring fibroin so that x/y becomes 64.2% or more, and chemically synthesizing a nucleic acid encoding the designed amino acid sequence. In any case, in addition to the modification that corresponds to deletion of the $(A)_n$ motif from an amino acid sequence of a naturally occurring fibroin, a modification of the amino acid sequence that corresponds to substitution, deletion, insertion, and/or addition of one or a plurality of amino acid residues may further be performed.

More specific examples of the third modified fibroin include a modified fibroin containing an amino acid sequence having (3-i) the amino acid sequence set forth in SEQ ID NO: 17 (Met-PRT399), SEQ ID NO: 7 (Met-PRT410), SEQ ID NO: 8 (Met-PRT525), or SEQ ID NO: 9 (Met-PRT799), or (3-ii) an amino acid sequence having 90% or more sequence identity with the amino acid sequence set forth in SEQ ID NO: 17, SEQ ID NO: 7, SEQ ID NO: 8, or SEQ ID NO: 9.

The modified fibroin (3-i) will be described. The amino acid sequence set forth in SEQ ID NO: 17 is an amino acid sequence in which every third $(A)_n$ motif from the N-terminal side to the C-terminal side is deleted from the amino acid sequence set forth in SEQ ID NO: 10 (Met-PRT313) corresponding to a naturally occurring fibroin, and further, one $[(A)_n$ motif-REP] is inserted before the C-terminal sequence. The amino acid sequence set forth in SEQ ID NO: 7, SEQ ID NO: 8, or SEQ ID NO: 9 is as described for the second modified fibroin.

The value of x/y at a Giza ratio of 1:1.8 to 11.3 in the amino acid sequence set forth in SEQ ID NO: 10 (corresponding to a naturally occurring fibroin) is 15.0%. Both the values of x/y in the amino acid sequence set forth in SEQ ID NO: 17 and the amino acid sequence set forth in SEQ ID NO: 7 are 93.4%. The value of x/y in the amino acid sequence set forth in SEQ ID NO: 8 is 92.7%. The value of x/y in the amino acid sequence set forth in SEQ ID NO: 9 is 89.8%. The values of z/w in the amino acid sequences set forth in SEQ ID NO: 10, SEQ ID NO: 17, SEQ ID NO: 7, SEQ ID NO: 8, and SEQ ID NO: 9 are 46.8%, 56.2%, 70.1%, 66.1%, and 70.0%, respectively.

The modified fibroin (3-i) may be a modified fibroin consisting of the amino acid sequence set forth in SEQ ID NO: 17, SEQ ID NO: 7, SEQ ID NO: 8, or SEQ ID NO: 9.

The modified fibroin (3-ii) is a modified fibroin containing an amino acid sequence having 90% or more sequence identity with the amino acid sequence set forth in SEQ ID NO: 17, SEQ ID NO: 7, SEQ ID NO: 8, or SEQ ID NO: 9. The modified fibroin (3-ii) also is a protein containing a domain sequence represented by Formula 1: $[(A)_n$ motif-REP$]_m$. The sequence identity is preferably 95% or more.

It is preferable that the modified fibroin (3-ii) has 90% or more sequence identity with the amino acid sequence set forth in SEQ ID NO: 17, SEQ ID NO: 7, SEQ ID NO: 8, or SEQ ID NO: 9, and that, when the numbers of amino acid residues of REP of two adjacent $[(A)_n$ motif-REP$]$ units are sequentially compared from the N-terminal side to the C-terminal side, x/y is 64.2% or more, where x is a maximum value of a total value obtained by adding the numbers of amino acid residues of two adjacent $[(A)_n$ motif-REP$]$ units wherein when the number of amino acid residues of one REP having less amino acid residues is taken as 1, the ratio of the number of amino acids residues of the other REP satisfies 1.8 to 11.3 (the Giza ratio is 1:1.8 to 11.3); and where y is a total number of amino acid residues of the domain sequence.

The third modified fibroin may contain a tag sequence described above at either or both of the N-terminal and C-terminal.

A more specific example of the modified fibroin containing a tag sequence may be a modified fibroin containing (3-iii) the amino acid sequence set forth in SEQ ID NO: 18 (PRT399), SEQ ID NO: 13 (PRT410), SEQ ID NO: 14 (PRT525), or SEQ ID NO: 15 (PRT799), or (3-iv) an amino acid sequence having 90% or more sequence identity with the amino acid sequence set forth in SEQ ID NO: 18, SEQ ID NO: 13, SEQ ID NO: 14, or SEQ ID NO: 15.

The amino acid sequences set forth in SEQ ID NO: 18, SEQ ID NO: 13, SEQ ID NO: 14, and SEQ ID NO: 15 are amino acid sequences in which the amino acid sequence (containing a His tag sequence and a hinge sequence) set forth in SEQ ID NO: 11 is added to the N-terminal of the amino acid sequences set forth in SEQ ID NO: 17, SEQ ID NO: 7, SEQ ID NO: 8, and SEQ ID NO: 9, respectively.

The modified fibroin (3-iii) may be a modified fibroin consisting of the amino acid sequence set forth in SEQ ID NO: 18, SEQ ID NO: 13, SEQ ID NO: 14, or SEQ ID NO: 15.

The modified fibroin (3-iv) is a modified fibroin containing an amino acid sequence having 90% or more sequence identity with the amino acid sequence set forth in SEQ ID NO: 18, SEQ ID NO: 13, SEQ ID NO: 14, or SEQ ID NO: 15. The modified fibroin (3-iv) also is a protein containing a domain sequence represented by Formula 1: $[(A)_n$ motif-REP$]_m$. The sequence identity is preferably 95% or more.

It is preferable that the modified fibroin (3-iv) has 90% or more sequence identity with the amino acid sequence set forth in SEQ ID NO: 18, SEQ ID NO: 13, SEQ ID NO: 14 or SEQ ID NO: 15, and that, when the numbers of amino acid residues of REP of two adjacent $[(A)_n$ motif-REP$]$ units are sequentially compared from the N-terminal side to the C-terminal side, x/y is 64.2% or more, where x is a maximum value of a total value obtained by adding the numbers of amino acid residues of two adjacent $[(A)_n$ motif-REP$]$ units wherein when the number of amino acid residues of one REP having less amino acid residues is taken as 1, the ratio of the number of amino acids residues of the other REP satisfies 1.8 to 11.3; and where y is a total number of amino acid residues of the domain sequence.

The third modified fibroin may contain a secretory signal for releasing the protein produced in a recombinant protein production system to the outside of the host. The sequence of the secretory signal may be set as appropriate, depending on the type of the host.

The fourth modified fibroin has a domain sequence that has an amino acid sequence with a reduced content of glycine residues, in addition to the reduced content of $(A)_n$ motifs, as compared with a naturally occurring fibroin. It can be said that the domain sequence of the fourth modified fibroin is a domain sequence further having an amino acid sequence that corresponds to an amino acid sequence in which at least one or a plurality of glycine residues in REP are substituted with other amino acid residues, in addition to the amino acid sequence in which at least one or a plurality of the $(A)_n$ motifs are deleted, as compared with a naturally occurring fibroin. That is, the fourth modified fibroin is a modified fibroin having both the characteristics of the second modified fibroin and the characteristics of the third modified fibroin described above. Specific embodiments and the like are as described for the second modified fibroin and the third modified fibroin.

More specific examples of the fourth modified fibroin include a modified fibroin containing (4-i) the amino acid sequence set forth in SEQ ID NO: 7 (Met-PRT410), SEQ ID NO: 8 (Met-PRT525), SEQ ID NO: 9 (Met-PRT799), SEQ ID NO: 13 (PRT410), SEQ ID NO: 14 (PRT525), or SEQ ID NO: 15 (PRT799), or (4-ii) an amino acid sequence having 90% or more sequence identity with the amino acid sequence set forth in SEQ ID NO: 7, SEQ ID NO: 8, SEQ ID NO: 9, SEQ ID NO: 13, SEQ ID NO: 14, or SEQ ID NO: 15. Specific embodiments of the modified fibroin containing the amino acid sequence set forth in SEQ ID NO: 7, SEQ ID NO: 8, SEQ ID NO: 9, SEQ ID NO: 13, SEQ ID NO: 14, or SEQ ID NO: 15 are as described above.

The fifth modified fibroin may have a domain sequence that has an amino acid sequence containing a region having a locally high hydropathy index, which corresponds to an amino acid sequence in which one or a plurality of amino acid residues in REP are substituted with an amino acid residue having a high hydropathy index and/or one or a plurality of amino acid residues having a high hydropathy index is inserted into REP, as compared with a naturally occurring fibroin.

It is preferable that the region having a locally high hydropathy index is composed of 2 to 4 consecutive amino acid residues.

It is more preferable that the amino acid residues having a high hydropathy index are selected from isoleucine (I), valine (V), leucine (L), phenylalanine (F), cysteine (C), methionine (M), and alanine (A).

The fifth modified fibroin may further have a modification of an amino acid sequence that corresponds to substitution, deletion, insertion, and/or addition of one or a plurality of amino acid residues, as compared with a naturally occurring fibroin, in addition to the modification that corresponds to substitution of one or a plurality of amino acid residues in REP with amino acid residues having a high hydropathy index and/or insertion of one or a plurality of amino acid residues having a high hydropathy index into REP, as compared with a naturally occurring fibroin.

The fifth modified fibroin may be obtained, for example, from a cloned gene sequence of a naturally occurring fibroin, by substituting one or a plurality of hydrophilic amino acid residues in REP (for example, amino acid residues having a negative hydropathy index) with a hydrophobic amino acid residue (for example, amino acid residues having a positive hydropathy index), and/or inserting one or a plurality of hydrophobic amino acid residues into REP. In addition, the fifth modified fibroin may also be obtained, for example, by designing an amino acid sequence that corresponds to an amino acid sequence in which one or a plurality of hydrophilic amino acid residues in REP are substituted with hydrophobic amino acid residues and/or one or a plurality of hydrophobic amino acid residues are inserted into REP, from an amino acid sequence of a naturally occurring fibroin, and chemically synthesizing a nucleic acid encoding the designed amino acid sequence. In any case, in addition to the modification that corresponds to substitution of one or a plurality of hydrophilic amino acid residues in REP with hydrophobic amino acid residues and/or insertion of one or a plurality of hydrophobic amino acid residues into REP in an amino acid sequence of a naturally occurring fibroin, a modification of an amino acid sequence that corresponds to substitution, deletion, insertion, and/or addition of one or a plurality of amino acid residues may further be performed.

The fifth modified fibroin may be a modified fibroin that contains a domain sequence represented by Formula 1: $[(A)_n \text{ motif-REP}]_m$, and that has an amino acid sequence in which p/q is 6.2% or more, where p is the total number of amino acid residues contained in a region where an average value of hydropathy indices of the four consecutive amino acid residues is 2.6 or more in all REPs contained in the domain sequence excluding the sequence between the $(A)_n$ motif positioned closest to the C-terminal side and the C-terminal of the domain sequence; and where q is the total number of amino acid residues contained in the domain sequence excluding the sequence between the $(A)_n$ motif positioned closest to the C-terminal side and the C-terminal of the domain sequence.

With regard to the hydropathy index of an amino acid residue, known index from (Hydropathy index: Kyte J, & Doolittle R (1982) "A simple method for displaying the hydropathic character of a protein", J. Mol. Biol., 157, pp. 105-132) may be used. Specifically, the hydropathy index (hereinafter also referred to as "HI") of each amino acid is as shown in Table 1 below.

[Table 1]

| Amino acid | HI | Amino acid | HI |
|---|---|---|---|
| Isoleucine (Ile) | 4.5 | Tryptophan (Trp) | -0.9 |
| Valine (Val) | 4.2 | Tyrosine (Tyr) | -1.3 |
| Leucine (Leu) | 3.8 | Proline (Pro) | -1.6 |
| Phenylalanine (Phe) | 2.8 | Histidine (His) | -3.2 |
| Cysteine (Cys) | 2.5 | Asparagine (Asn) | -3.5 |
| Methionine (Met) | 1.9 | Aspartic acid (Asp) | -3.5 |
| Alanine (Ala) | 1.8 | Glutamine (Gln) | -3.5 |
| Glycine (Gly) | -0.4 | Glutamic acid (Glu) | -3.5 |
| Threonine (Thr) | -0.7 | Lysine (Lys) | -3.9 |
| Serine (Ser) | -0.8 | Arginine (Arg) | -4.5 |

A method for calculating p/q will be described in more detail. For calculation, a sequence (hereinafter also referred to as a "Sequence A") obtained by removing, from domain sequence represented by Formula 1: $[(A)_n$ motif-$REP]_m$, the sequence between the $(A)_n$ motif positioned closest to the C-terminal side and the C-terminal of the domain sequence is used. First, in all REPs contained in Sequence A, average values of hydropathy indices of the four consecutive amino acid residues are calculated. The average value of the hydropathy indices is obtained by dividing the sum of HI of each of the amino acid residues contained in the four consecutive amino acid residues by 4 (the number of amino acid residues). The average value of the hydropathy indices is obtained for all four consecutive amino acid residues (each of the amino acid residues is used for calculating the average value 1 to 4 times). Next, a region where the average value of the hydropathy indices of the four consecutive amino acid residues is 2.6 or more is identified. Even if a certain amino acid residues correspond to a plurality of the "four consecutive amino acid residues having an average value of the hydropathy indices of 2.6 or more", the amino acid residue is counted as one amino acid residue in the region. The total number of amino acid residues contained in the region is p. Further, the total number of amino acid residues contained in Sequence A is q.

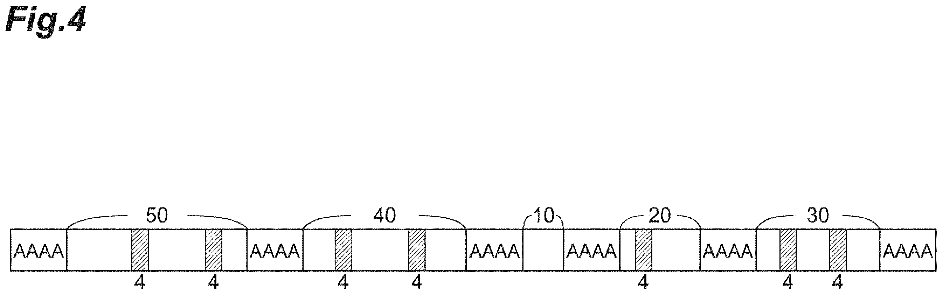

For example, in a case where 20 "four consecutive amino acid residues having an average value of the hydropathy indices of 2.6 or more " are extracted (without an overlap), there are 20 of the four consecutive amino acid residues (without an overlap) in the region in which the average value of the hydropathy indices of the four consecutive amino acid residues is 2.6 or more, and thus p is $20 \times 4 = 80$. In addition, for example, in a case where two "four consecutive amino acid residues having an average value of the hydropathy indices of 2.6 or more" overlap by only one amino acid residue, seven amino acid residues are contained in the region in which the average value of the hydropathy indices of the four consecutive amino acid residues is 2.6 or more ($p = 2 \times 4 - 1 = 7$; "-1" is deduction for overlap). For example, in a case of the domain sequence shown in Fig. 4, since there are seven "four consecutive amino acid residues having an average value of the hydropathy indices of 2.6 or more" without an overlap, p is $7 \times 4 = 28$. Further, for example, in a case of the domain sequence illustrated in Fig. 4, q is $4 + 50 + 4 + 40 + 4 + 10 + 4 + 20 + 4 + 30 = 170$ (the $(A)_n$ motif present at the end of the C-terminal side is not included). Next, p/q (%) can be calculated by dividing p by q. In the case of Fig. 4, p/q (%) is $28/170 = 16.47\%$.

In the fifth modified fibroin, p/q is preferably 6.2% or more, more preferably 7% or more, still more preferably 10% or more, even still more preferably 20% or more, and still further preferably 30% or more. The upper limit of p/q is not particularly limited, but may be, for example, 45% or less.

The fifth modified fibroin may be obtained, for example, by modifying an amino acid sequence of a cloned naturally occurring fibroin to an amino acid sequence containing a region having a locally high hydropathy index, by substituting one or a plurality of hydrophilic amino acid residues in REP (for example, amino acid residues having a negative hydropathy index) with hydrophobic amino acid residues (for example, amino acid residues having a positive hydropathy index), and/or inserting one or a plurality of hydrophobic amino acid residues into REP, so as to satisfy the above condition of p/q. In addition, the modified fibroin may also be obtained, for example, by designing an amino acid sequence satisfying the above-described condition of p/q based on the amino acid sequence of a naturally occurring fibroin, and chemically synthesizing a nucleic acid encoding the designed amino acid sequence. In any case, in addition to the modification that corresponds substitution of one or a plurality of amino acid residues in REP with amino acid residues having a high hydropathy index and/or insertion of one or a plurality of amino acid residues having a high hydropathy index, as compared with a naturally occurring fibroin, a modification that corresponds to substitution, deletion, insertion, and/or addition of one or a plurality of amino acid residues may further be performed.

The amino acid residue having a high hydropathy index is not particularly limited, but is preferably isoleucine (I), valine (V), leucine (L), phenylalanine (F), cysteine (C), methionine (M), and alanine (A), and more preferably valine (V), leucine (L), and isoleucine (I).

More specific examples of the fifth modified fibroin contains a modified fibroin containing (5-i) the amino acid sequence set forth in SEQ ID NO: 19 (Met-PRT720), SEQ ID NO: 20 (Met-PRT665), or SEQ ID NO: 21 (Met-PRT666), or (5-ii) an amino acid sequence having 90% or more sequence identity with the amino acid sequence set forth in SEQ ID NO: 19, SEQ ID NO: 20, or SEQ ID NO: 21.

The modified fibroin (5-i) will be described. The amino acid sequence set forth in SEQ ID NO: 19 is an amino acid sequence in which two amino acid sequences, each consisting of three amino acid residues (VLI), are inserted for every other REP excluding the terminal domain sequence on the C-terminal side; further, some of the glutamine (Q) residues are substituted with serine (S) residues; and some of the amino acids on the C-terminal side are deleted, with respect to the amino acid sequence set forth in SEQ ID NO: 7 (Met-PRT410). The amino acid sequence set forth in SEQ ID NO: 20 is an amino acid sequence in which one amino acid sequence consisting of three amino acid residues (VLI) is inserted for every other REP with respect to the amino acid sequence set forth in SEQ ID NO: 8 (Met-PRT525). The amino acid sequence set forth in SEQ ID NO: 21 is an amino acid sequence in which two amino acid sequences each consisting of three amino acid residues (VLI) are inserted for every other REP with respect to the amino acid sequence set forth in SEQ ID NO: 8.

The modified fibroin (5-i) may be a modified fibroin consisting of the amino acid sequence set forth in SEQ ID NO: 19, SEQ ID NO: 20, or SEQ ID NO: 21.

The modified fibroin (5-ii) is a modified fibroin containing an amino acid sequence having 90% or more sequence identity with the amino acid sequence set forth in SEQ ID NO: 19, SEQ ID NO: 20, or SEQ ID NO: 21. The modified fibroin (5-ii) also is a protein containing a domain sequence represented by Formula 1: $[(A)_n$ motif-REP$]_m$. The sequence identity is preferably 95% or more.

It is preferable that the modified fibroin (5-ii) has 90% or more sequence identity with the amino acid sequence set forth in SEQ ID NO: 19, SEQ ID NO: 20, or SEQ ID NO: 21, and that p/q is 6.2% or more, where p is the total number of amino acid residues contained in a region where an average value of hydropathy indices of the four consecutive amino acid residues is 2.6 or more in all REPs contained in the domain sequence excluding the sequence between the $(A)_n$ motif positioned closest to the C-terminal side and the C-terminal of the domain sequence; and where q is the total number of amino acid residues contained in the domain sequence excluding the sequence between the $(A)_n$ motif positioned closest to the C-terminal side and the C-terminal of the domain sequence.

The fifth modified fibroin may contain a tag sequence at either or both of the N-terminal and C-terminal.

More specific examples of the modified fibroin containing a tag sequence include a modified fibroin containing (5-iii) the amino acid sequence set forth in SEQ ID NO: 22 (PRT720), SEQ ID NO: 23 (PRT665), or SEQ ID NO: 24 (PRT666), or (5-iv) an amino acid sequence having 90% or more sequence identity with the amino acid sequence set forth in SEQ ID NO: 22, SEQ ID NO: 23, or SEQ ID NO: 24.

The amino acid sequences set forth in SEQ ID NO: 22, SEQ ID NO: 23, and SEQ ID NO: 24 are amino acid sequences in which the amino acid sequence (containing a His tag sequence and a hinge sequence) set forth in SEQ ID NO: 11 is added to the N-terminal of the amino acid sequences set forth in SEQ ID NO: 19, SEQ ID NO: 20, and SEQ ID NO: 21, respectively.

The modified fibroin (5-iii) may be a modified fibroin consisting of the amino acid sequence set forth in SEQ ID NO: 22, SEQ ID NO: 23, or SEQ ID NO: 24.

The modified fibroin (5-iv) is a modified fibroin containing an amino acid sequence having 90% or more sequence identity with the amino acid sequence set forth in SEQ ID NO: 22, SEQ ID NO: 23, or SEQ ID NO: 24. The modified fibroin (5-iv) also is a protein containing a domain sequence represented by Formula 1: $[(A)_n$ motif-REP$]_m$. The sequence identity is preferably 95% or more.

It is preferable that the modified fibroin (5-iv) has 90% or more sequence identity with the amino acid sequence set forth in SEQ ID NO: 22, SEQ ID NO: 23, or SEQ ID NO: 24, and that p/q is 6.2% or more, where p is the total number of amino acid residues contained in a region where an average value of hydropathy indices of the four consecutive amino acid residues is 2.6 or more in all REPs contained in the domain sequence excluding the sequence between the $(A)_n$ motif positioned closest to the C-terminal side and the C-terminal of the domain sequence; and where q is the total number of amino acid residues contained in the domain sequence excluding the sequence between the $(A)_n$ motif positioned closest to the C-terminal side and the C-terminal of the domain sequence.

The fifth modified fibroin may contain a secretory signal for releasing the protein produced in a recombinant protein production system to the outside of the host. The sequence of the secretory signal may be set as appropriate, depending on the type of the host.

The sixth modified fibroin has an amino acid sequence with a reduced content of glutamine residues, as compared with a naturally occurring fibroin.

It is preferable that the sixth modified fibroin contains at least one motif selected from a GGX motif and a GPGXX motif in the amino acid sequence of REP.

In a case where the sixth modified fibroin contains a GPGXX motif in REP, a GPGXX motif content rate is usually 1% or more, may be 5% or more, and is preferably 10% or more. The upper limit of the GPGXX motif content rate is not particularly limited, and it may be 50% or less, and may be 30% or less.

In the present specification, the "GPGXX motif content rate" is a value calculated by the following method.

In a fibroin (a modified fibroin or a naturally occurring fibroin) containing a domain sequence represented by Formula 1: $[(A)_n$ motif-REP$]_m$ or Formula 2: $[(A)_n$ motif-REP$]_m$-$(A)_n$ motif, the GPGXX motif content rate is calculated as s/t, where s is the number obtained by tripling the total number of the GPGXX motifs (namely, corresponds to the total number of G and P in the GPGXX motifs) contained in all REPs contained in the domain sequence excluding the sequence between the $(A)_n$ motif positioned closest to the C-terminal side and the C-terminal of the domain sequence; and where t is the total number of amino acid residues in all REPs obtained by excluding the sequence between the $(A)_n$ motif positioned closest to the C-terminal side and the C-terminal of the domain sequence from the domain sequence and further excluding the $(A)_n$ motifs.

For the calculation of the GPGXX motif content rate, a "domain sequence excluding the sequence between the $(A)_n$ motif positioned closest to the C-terminal side and the C-terminal of the domain sequence" is used, because "the sequence between the $(A)_n$ motif positioned closest to the C-terminal side and the C-terminal of the domain sequence (sequence corresponding to REP) may contain a sequence having a low correlation with the sequence characteristic of a fibroin, which influences the calculation result of the GPGXX motif content rate when m is small (that is, when the domain sequence is short), and thus, this effect is to be eliminated. In a case where a "GPGXX motif" is located at the C-terminal of REP, it is treated as "GPGXX motif" even if "XX" is, for example, "AA".

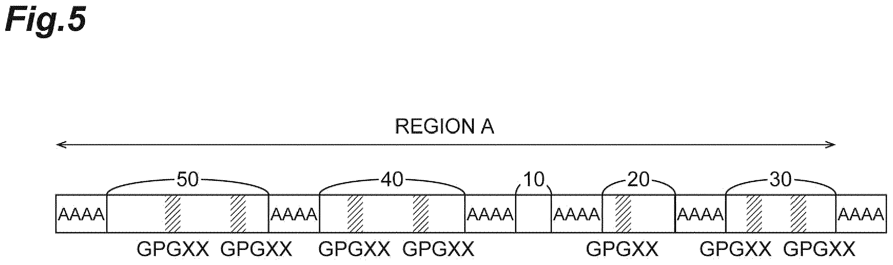

Fig. 5 is a schematic diagram illustrating a domain sequence of a modified fibroin. A method for calculating the GPGXX motif content rate will be specifically described with reference to Fig. 5. First, in a domain sequence of the modified fibroin (which is an [(A)$_n$ motif-REP]$_m$-(A)$_n$ motif] type) illustrated in Fig. 5, the number of GPGXX motifs for calculation of s is 7 and s is 7 × 3 = 21, because all REPs are contained in the "domain sequence excluding the sequence between the (A)$_n$ motif positioned closest to the C-terminal side and the C-terminal of the domain sequence" (indicated as "REGION A" in Fig. 5). Similarly, since all REPs are contained in the "domain sequence excluding the sequence between the (A)$_n$ motif positioned closest to the C-terminal side and the C-terminal of the domain sequence" (indicated as "REGION A" in Fig. 5), the total number, t, of amino acid residues in all REPs obtained by further excluding the (A)$_n$ motifs from the "domain sequence excluding the sequence between the (A)$_n$ motif positioned closest to the C-terminal side and the C-terminal of the domain sequence" is 50 + 40 + 10 + 20 + 30 = 150. Next, s/t (%) can be calculated by dividing s by t, and thus, in a case of the modified fibroin of Fig. 5, s/t (%) is 21/150 = 14.0%.

In the sixth modified fibroin, the glutamine residue content rate is preferably 9% or less, more preferably 7% or less, still more preferably 4% or less, and particularly preferably 0%.

In the present specification, the "glutamine residue content rate" is a value calculated by the following method.

In a fibroin (a modified fibroin or a naturally occurring fibroin) containing a domain sequence represented by Formula 1: [(A)$_n$ motif-REP]$_m$ or Formula 2: [(A)$_n$ motif-REP]$_m$-(A)$_n$ motif, the glutamine residue content rate is calculated as u/t, where u is the total number of glutamine residues contained in all REPs contained in the domain excluding the sequence between the (A)$_n$ motif positioned closest to the C-terminal side and the C-terminal of the domain sequence (sequence corresponding to the "REGION A" in Fig. 5); and where t is the total number of amino acid residues in all REPs obtained by excluding the sequence between the (A)$_n$ motif positioned closest to the C-terminal side and the C-terminal of the domain sequence from the domain sequence and further excluding the (A)$_n$ motifs. The reason as to why the "domain sequence excluding the sequence between the (A)$_n$ motif positioned closest to the C-terminal side and the C-terminal of the domain sequence" is used for the calculation of the glutamine residue content rate is the same as the reason described above.

The domain sequence of the sixth modified fibroin may be a domain sequence having an amino acid sequence that corresponds to an amino acid sequence in which one or a plurality of glutamine residues in REP are deleted or substituted with other amino acid residues, as compared with a naturally occurring fibroin.

The "other amino acid residues" may be amino acid residues other than the glutamine residue, but are preferably amino acid residues having a higher hydropathy index than that of the glutamine residue. The hydropathy indices of the amino acid residues are as shown in
Table 1.

As shown in Table 1, examples of the amino acid residues having a higher hydropathy index than a glutamine residue include an amino acid residue selected from isoleucine (I), valine (V), leucine (L), phenylalanine (F), cysteine (C), methionine (M), alanine (A), glycine (G), threonine (T), serine (S), tryptophan (W), tyrosine (Y), proline (P), and histidine (H). Among these, an amino acid residue selected from isoleucine (I), valine (V), leucine (L), phenylalanine (F), cysteine (C), methionine (M), and alanine (A) is more preferable, and an amino acid residue selected from isoleucine (I), valine (V), leucine (L), and phenylalanine (F) is still more preferable.

In the sixth modified fibroin, the degree of hydrophobicity of REP is preferably -0.8 or more, more preferably -0.7 or more, still more preferably 0 or more, even still more preferably 0.3 or more, and particularly preferably 0.4 or more. The upper limit of the degree of hydrophobicity of REP is not particularly limited, and it may be 1.0 or less, and may be 0.7 or less.

In the present specification, the "degree of hydrophobicity of REP" is a value calculated by the following method.

In a fibroin (a modified fibroin or a naturally occurring fibroin) containing a domain sequence represented by Formula 1: [(A)$_n$ motif-REP]$_m$ or Formula 2: [(A)$_n$ motif-REP]$_m$-(A)$_n$ motif, the degree of hydrophobicity of REP is calculated as v/t, where v is the total sum of the hydropathy index of each amino acid residue contained in all REPs contained in the domain a sequence excluding the sequence between the (A)$_n$ motif positioned closest to the C-terminal side and the C-terminal of the domain sequence (sequence corresponding to a "REGION A" in Fig. 5); and where t is the total number of amino acid residues in all REPs obtained by excluding the sequence between the (A)$_n$ motif positioned closest to the C-terminal side and the C-terminal of the domain sequence from the domain sequence and further excluding the (A)$_n$ motifs. The reason as to why the "domain sequence excluding the sequence between the (A)$_n$ motif positioned closest to the C-terminal side and the C-terminal of the domain sequence" is used for the calculation of the degree of hydrophobicity of REP is the same as the reason described above.

The domain sequence of the sixth modified fibroin may further have a modification of an amino acid sequence that corresponds to substitution, deletion, insertion, and/or addition of one or a plurality of amino acid residues, in addition to the modification that corresponds to deletion of one or a plurality of glutamine residues in REP and/or substitution of one or a plurality of glutamine residues in REP with other amino acid residues, as compared with a naturally occurring fibroin.

The sixth modified fibroin may be obtained, for example, from a cloned gene sequence of a naturally occurring fibroin, by deleting one or a plurality of glutamine residues in REP and/or substituting one or a plurality of glutamine residues in REP with other amino acid residues. In addition, the sixth modified fibroin may also be obtained by designing an amino acid sequence that corresponds to an amino acid sequence in which one or a plurality of glutamine residues in REP are deleted, and/or one or a plurality of glutamine residues in REP are substituted with other amino acid residues, based on the amino acid sequence of a naturally occurring fibroin, and chemically synthesizing a nucleic acid encoding the designed amino acid sequence.

More specific examples of the sixth modified fibroin include (6-i) a modified fibroin containing the amino acid sequence set forth in SEQ ID NO: 25 (Met-PRT888), SEQ ID NO: 26 (Met-PRT965), SEQ ID NO: 27 (Met-PRT889), SEQ ID NO: 28 (Met-PRT916), SEQ ID NO: 29 (Met-PRT918), SEQ ID NO: 30 (Met-PRT699), SEQ ID NO: 31 (Met-PRT698), or SEQ ID NO: 32 (Met-PRT966), or (6-ii) a modified fibroin containing an amino acid sequence having 90% or more sequence identity with the amino acid sequence set forth in SEQ ID NO: 25, SEQ ID NO: 26, SEQ ID NO: 27, SEQ ID NO: 28, SEQ ID NO: 29, SEQ ID NO: 30, SEQ ID NO: 31, or SEQ ID NO: 32.

The modified fibroin (6-i) will be described. The amino acid sequence set forth in SEQ ID NO: 25 is obtained by substituting all QQs in an amino acid sequence (Met-PRT410) set forth in SEQ ID NO: 7 with VLs. The amino acid sequence set forth in SEQ ID NO: 26 is obtained by substituting all QQs in the amino acid sequence set forth in SEQ ID NO: 7 with TSs, and substituting the remaining Qs with As. The amino acid sequence set forth in SEQ ID NO: 27 is obtained by substituting all QQs in the amino acid sequence set forth in SEQ ID NO: 7 with VLs, and substituting the remaining Qs with Is. The amino acid sequence set forth in SEQ ID NO: 28 is obtained by substituting all QQs in the amino acid sequence set forth in SEQ ID NO: 7 with VIs, and substituting the remaining Qs with Ls. The amino acid sequence set forth in SEQ ID NO: 29 is obtained by substituting all QQs in the amino acid sequence set forth in SEQ ID NO: 7 with VFs, and substituting the remaining Qs with Is.

The amino acid sequence set forth in SEQ ID NO: 30 is obtained by substituting all QQs in the amino acid sequence (Met-PRT525) set forth in SEQ ID NO: 8 with VLs. The amino acid sequence set forth in SEQ ID NO: 31 is obtained by substituting all QQs in the amino acid sequence set forth in SEQ ID NO: 8 with VLs, and substituting the remaining Qs with Is.

The amino acid sequence set forth in SEQ ID NO: 32 is a sequence obtained by substituting all QQs in a sequence obtained by repeating twice a region of 20 domain sequences present in the amino acid sequence (Met-PRT410) set forth in SEQ ID NO: 7 with VFs, and substituting the remaining Qs with Is.

The glutamine residue content rate of any of the amino acid sequences set forth in SEQ ID NO: 25, SEQ ID NO: 26, SEQ ID NO: 27, SEQ ID NO: 28, SEQ ID NO: 29, SEQ ID NO: 30, SEQ ID NO: 31, and SEQ ID NO: 32 is 9% or less (Table 2).

[Table 2]

| Modified fibroin | Glutamine residue content rate | GPGXX motif content rate | Degree of hydrophobicity of REP |
|---|---|---|---|
| Met-PRT410 (SEQ ID NO: 7) | 17.7% | 27.9% | -1.52 |
| Met-PRT888 (SEQ ID NO: 25) | 6.3% | 27.9% | -0.07 |
| Met-PRT965 (SEQ ID NO: 26) | 0.0% | 27.9% | -0.65 |
| Met-PRT889 (SEQ ID NO: 27) | 0.0% | 27.9% | 0.35 |
| Met-PRT916 (SEQ ID NO: 28) | 0.0% | 27.9% | 0.47 |
| Met-PRT918 (SEQ ID NO: 29) | 0.0% | 27.9% | 0.45 |
| Met-PRT699 (SEQ ID NO: 30) | 3.6% | 26.4% | -0.78 |
| Met-PRT698 (SEQ ID NO: 31) | 0.0% | 26.4% | -0.03 |

(continued)

| Modified fibroin | Glutamine residue content rate | GPGXX motif content rate | Degree of hydrophobicity of REP |
|---|---|---|---|
| Met-PRT966 (SEQ ID NO: 32) | 0.0% | 28.0% | 0.35 |

The modified fibroin (6-i) may be a modified fibroin consisting of the amino acid sequence set forth in SEQ ID NO: 25, SEQ ID NO: 26, SEQ ID NO: 27, SEQ ID NO: 28, SEQ ID NO: 29, SEQ ID NO: 30, SEQ ID NO: 31, or SEQ ID NO: 32.

The modified fibroin (6-ii) may be a modified fibroin containing an amino acid sequence having 90% or more sequence identity with the amino acid sequence set forth in SEQ ID NO: 25, SEQ ID NO: 26, SEQ ID NO: 27, SEQ ID NO: 28, SEQ ID NO: 29, SEQ ID NO: 30, SEQ ID NO: 31, or SEQ ID NO: 32. The modified fibroin (6-ii) also is a protein containing a domain sequence represented by Formula 1: $[(A)_n$ motif-REP$]_m$ or Formula 2: $[(A)_n$ motif-REP$]_m$-$(A)_n$ motif. The sequence identity is preferably 95% or more.

The modified fibroin (6-ii) preferably has a glutamine residue content rate of 9% or less. In addition, the modified fibroin (6-ii) preferably has a GPGXX motif content rate of 10% or more.

The sixth modified fibroin may contain a tag sequence at either or both of the N-terminal and C-terminal. This makes it possible to isolate, immobilize, detect, and visualize the modified fibroin.

More specific examples of the modified fibroin containing a tag sequence include a modified fibroin containing (6-iii) the amino acid sequence set forth in SEQ ID NO: 33 (PRT888), SEQ ID NO: 34 (PRT965), SEQ ID NO: 35 (PRT889), SEQ ID NO: 36 (PRT916), SEQ ID NO: 37. (PRT918), SEQ ID NO: 38 (PRT699), SEQ ID NO: 39 (PRT698), or SEQ ID NO: 40 (PRT966), or a modified fibroin containing (6-iv) an amino acid sequence having 90% or more sequence identity with the amino acid sequence set forth in SEQ ID NO: 33, SEQ ID NO: 34, SEQ ID NO: 35, SEQ ID NO: 36, SEQ ID NO: 37, SEQ ID NO: 38, SEQ ID NO: 39, or SEQ ID NO: 40.

The amino acid sequences set forth in SEQ ID NO: 33, SEQ ID NO: 34, SEQ ID NO: 35, SEQ ID NO: 36, SEQ ID NO: 37, SEQ ID NO: 38, SEQ ID NO: 39, and SEQ ID NO: 40 are amino acid sequences in which the amino acid sequence (containing a His tag sequence and a hinge sequence) set forth in SEQ ID NO: 11 is added to the N-terminal of the amino acid sequences set forth in SEQ ID NO: 25, SEQ ID NO: 26, SEQ ID NO: 27, SEQ ID NO: 28, SEQ ID NO: 29, SEQ ID NO: 30, SEQ ID NO: 31, and SEQ ID NO: 32, respectively. Since only the tag sequence is added to the N-terminal, the glutamine residue content rate is not changed, and any of the amino acid sequences set forth in SEQ ID NO:33, SEQ ID NO:34, SEQ ID NO:35, SEQ ID NO:36, SEQ ID NO:37, SEQ ID NO:38, SEQ ID NO:39, and SEQ ID NO: 40 has a glutamine residue content rate of 9% or less (Table 3).

[Table 3]

| Modified fibroin | Glutamine residue content rate | GPGXX motif content rate | Degree of hydrophobicity of REP |
|---|---|---|---|
| PRT888 (SEQ ID NO: 33) | 6.3% | 27.9% | -0.07 |
| PRT965 (SEQ ID NO: 34) | 0.0% | 27.9% | -0.65 |
| PRT889 (SEQ ID NO: 35) | 0.0% | 27.9% | 0.35 |
| PRT916 (SEQ ID NO: 36) | 0.0% | 27.9% | 0.47 |
| PRT918 (SEQ ID NO: 37) | 0.0% | 27.9% | 0.45 |
| PRT699 (SEQ ID NO: 38) | 3.6% | 26.4% | -0.78 |
| PRT698 (SEQ ID NO: 39) | 0.0% | 26.4% | -0.03 |
| PRT966 (SEQ ID NO: 40) | 0.0% | 28.0% | 0.35 |

The modified fibroin (6-iii) may be a modified fibroin consisting of the amino acid sequence set forth in SEQ ID NO: 33, SEQ ID NO: 34, SEQ ID NO: 35, SEQ ID NO: 36, SEQ ID NO: 37, SEQ ID NO: 38, SEQ ID NO: 39, or SEQ ID NO: 40.

The modified fibroin (6-iv) is a modified fibroin containing an amino acid sequence having 90% or more sequence identity with the amino acid sequence set forth in SEQ ID NO: 33, SEQ ID NO: 34, SEQ ID NO: 35, SEQ ID NO: 36, SEQ ID NO: 37, SEQ ID NO: 38, SEQ ID NO: 39, or SEQ ID NO: 40. The modified fibroin (6-iv) also is a protein containing a domain sequence represented by Formula 1: $[(A)_n \text{ motif-REP}]_m$ or Formula 2: $[(A)_n \text{ motif-REP}]_m\text{-}(A)_n$ motif. The sequence identity is preferably 95% or more.

The modified fibroin (6-iv) preferably has a glutamine residue content rate of 9% or less. In addition, the modified fibroin (6-iv) preferably has a GPGXX motif content rate of 10% or more.

The sixth modified fibroin may contain a secretory signal for releasing the protein produced in a recombinant protein production system to the outside of the host. The sequence of the secretory signal may be set as appropriate, depending on the type of the host.

The modified fibroin may be a modified fibroin that has at least two or more characteristics in combination among the characteristics of the first modified fibroin, the second modified fibroin, the third modified fibroin, the fourth modified fibroin, the fifth modified fibroin, and the sixth modified fibroin.

The limiting oxygen index (LOI) value of the modified fibroin according to the present embodiment may be 18 or more, 20 or more, 22 or more, 24 or more, 26 or more, 28 or more, 29 or more, or 30 or more. In the present specification, the LOI value is a value measured in accordance with "Test Method for Granular or Low-Melting-Point Synthetic Resin" described in "Notice No. 50 of the Office of Hazardous Materials Regulation (as of May 31, 1995)".

The maximum hygroscopic heat generation of the modified fibroin according to the present embodiment, which is determined according to Formula A, may be more than 0.025°C/g.

Formula A: Maximum hygroscopic heat generation = {(Highest temperature of a sample when the sample has been transferred to a high humidity environment after being placed in a low humidity environment until a temperature of the sample reaches equilibrium) - (Temperature of the sample when the sample is being transferred to the high humidity environment after being placed in the low humidity environment until the temperature of the sample reaches equilibrium)} (°C) / Sample weight (g)

Furthermore, in Formula A, the low humidity environment means an environment of a temperature of 20°C and a relative humidity of 40%, and the high humidity environment means an environment of a temperature of 20°C and a relative humidity of 90%.

The maximum hygroscopic heat generation of the modified fibroin according to the present embodiment is 0.026°C/g or more, 0.027°C/g or more, 0.028°C/g or more, 0.029°C/g or more, 0.030°C/g or more, 0.035°C/g or more, or 0.040°C/g or more. The upper limit of the maximum hygroscopic heat generation is not particularly limited, but is usually 0.060°C/g or less.

The modified fibroin according to the present embodiment may have a heat retention index determined according to the following Formula B of 0.20 or more.

Formula B: Heat retention index = Heat retention rate (%) / Basis weight of sample (g/m$^2$)

Here, in the present specification, the heat retention rate means a heat retention rate measured by a dry contact method using a Thermo Labo II type tester (under a wind at 30 cm/sec), and is a value measured by the method described in Reference Examples which will be described later.

The heat retention index of the modified fibroin according to the present embodiment may be 0.22 or more, may be 0.24 or more, may be 0.26 or more, may be 0.28 or more, may be 0.30 or more, and may be 0.32 or more. The upper limit of the heat retention index is not particularly limited, but may be, for example, 0.60 or less, or 0.40 or less.

Examples of the protein derived from collagen may include a protein that contains a domain sequence represented by Formula 3: $[REP2]_i$ (Here, in Formula 3, i represents an integer of 5 to 300. REP2 represents an amino acid sequence composed of Gly-X-Y, where X and Y represent any amino acid residues other than Gly. The plurality of REP2s may have the same amino acid sequence or amino acid sequences different from each other.). Specifically, a protein containing the amino acid sequence set forth in SEQ ID NO: 41 may be mentioned. The amino acid sequence set forth in SEQ ID NO: 41 is an amino acid sequence obtained by adding the amino acid sequence set forth in SEQ ID NO: 11 (a His tag sequence and a hinge sequence) to the N-terminal of the amino acid sequence from the 301st residue to the 540th residue, which corresponds to the repeat portion and motif, of the partial sequence of human collagen type 4 (NCBI GenBank Accession No.: CAA56335.1, GI: 3702452) obtained from the NCBI database.

Examples of the protein derived from resilin may include a protein containing a domain sequence represented by Formula 4: $[REP3]_j$ (Here, in Formula 4, j represents an integer of 4 to 300. REP3 represents an amino acid sequence composed of Ser-J-J-Tyr-Gly-U-Pro. J represents any amino acid residue and particularly preferably an amino acid residue selected from the group consisting of Asp, Ser, and Thr. U represents any amino acid residue and particularly preferably an amino acid residue selected from the group consisting of Pro, Ala, Thr, and Ser. The plurality of REP4s may have the same amino acid sequence or amino acid sequences different from each other.). Specifically, a protein containing the amino acid sequence set forth in SEQ ID NO: 42 may be mentioned. The amino acid sequence set forth in SEQ ID NO: 42 is an amino acid sequence obtained by adding the amino acid sequence set forth in SEQ ID NO: 11 (a His tag sequence and a hinge sequence) to the N-terminal of the amino acid sequence from 19th residue to 321 st residue of the amino acid sequence of resilin (NCBI GenBank Accession No. NP611157, G1: 24654243), in which Thr at the 87th residue is substituted with Ser, and Asn at the 95th residue is substituted with Asp.

Examples of the protein derived from elastin may include proteins having amino acid sequences such as NCBI GenBank Accession No.s, AAC98395 (human), 147076 (sheep), and NP786966 (bovine). Specifically, a protein containing the amino acid sequence set forth in SEQ ID NO: 43 may be mentioned. The amino acid sequence set forth in SEQ ID NO: 43 is an amino acid sequence obtained by adding the amino acid sequence set forth in SEQ ID NO: 11 (a His tag sequence and a hinge sequence) to the N-terminal of the amino acid sequence from 121st residue to 390th residue of the amino acid sequence of NCBI GenBank Accession No. AAC98395.

An example of the protein derived from keratin may include a type I keratin of Capra hircus. Specifically, a protein containing the amino acid sequence set forth in SEQ ID NO: 44 (the amino acid sequence of NCBI GenBank Accession No. ACY30466) may be mentioned.

The structural protein and modified structural protein derived from the structural protein described above may be used alone or in a combination of two or more thereof.

<Method for manufacturing protein>

The protein according to any of the embodiments can also be produced, for example, by expressing a nucleic acid by a host transformed with an expression vector having the nucleic acid sequence encoding the protein and one or a plurality of regulatory sequences operably linked to the nucleic acid sequence.

A method for producing a nucleic acid encoding a protein is not particularly limited. For example, the nucleic acid can be produced by a method for performing cloning using a gene encoding a natural fibroin by the amplification with polymerase chain reaction (PCR) or the like and modifying the gene by a genetic engineering method, or a method for chemically synthesizing the nucleic acid. The method for chemically synthesizing a nucleic acid is not particularly limited, and for example, a gene can be chemically synthesized by a method in which oligonucleotides are automatically synthesized by AKTA oligopilot plus 10/100 (GE Healthcare Japan Corporation) or the like, and are linked by PCR or the like, based on the amino acid sequence information of a protein obtained from the NCBI web database or the like. In this case, in order to facilitate purification and/or confirmation of the protein, a nucleic acid may be synthesized such that a protein having an amino acid sequence obtained by adding an amino acid sequence consisting of a start codon and a His 10 tag to the N-terminal of the above amino acid sequence is encoded.

The regulatory sequence is a sequence (for example, a promoter, an enhancer, a ribosome binding sequence, or a transcription termination sequence) that controls the expression of a modified fibroin in a host, and can be selected as appropriate, depending on the type of the host. As a promoter, an inducible promoter that functions in a host cell and is capable of inducing the expression of a modified fibroin may be used. An inducible promoter is a promoter that can control transcription by the presence of an inducer (an expression inducer), the absence of a repressor molecule, or physical factors such as an increase or decrease in temperature, osmotic pressure, or pH value.

The type of the expression vector such as a plasmid vector, a viral vector, a cosmid vector, a fosmid vector, or an artificial chromosome vector can be appropriately selected depending on the type of the host. As the expression vector, an expression vector that can autonomously replicate in a host cell or can be incorporated into a chromosome of a host and which contains a promoter at a position capable of transcribing the nucleic acid that encodes a protein is suitably used.

Both prokaryotes and eukaryotes such as yeast, filamentous fungi, insect cells, animal cells, and plant cells can be suitably used as a host.

Preferred examples of the prokaryotic host cells include bacteria belonging to the genus Escherichia, the genus Brevibacillus, the genus Serratia, the genus Bacillus, the genus Microbacterium, the genus Brevibacterium, the genus Corynebacterium, and the genus Pseudomonas. Examples of microorganisms belonging to the genus Escherichia may include Escherichia coli. Examples of microorganisms belonging to the genus Brevibacillus may include Brevibacillus agri. Examples of microorganisms belonging to the genus Serratia may include Serratia liquefaciens. Examples of microorganisms belonging to the genus Bacillus may include Bacillus subtilis. Examples of microorganisms belonging to the genus Microbacterium may include Microbacterium ammoniaphilum. Examples of microorganisms belonging to the genus Brevibacterium may include Brevibacterium divaricatum. Examples of microorganisms belonging to the genus Corynebacterium may include Corynebacterium ammoniagenes. Examples of microorganisms belonging to the genus Pseudomonas may include Pseudomonas putida.

In a case where a prokaryote is used as a host, examples of the vector into which a nucleic acid encoding a protein is introduced include pBTrp2 (manufactured by Boehringer Mannheim), pGEX (manufactured by Pharmacia), pUC18, pBluescriptII, pSupex, pET22b, pCold, pUB110, and pNCO2 (Japanese Unexamined Patent Publication No. 2002-238569).

Examples of the eukaryotic hosts include yeast and filamentous fungi (mold and the like). Examples of the yeasts include yeasts belonging to the genus Saccharomyces, the genus Pichia, and the genus Schizosaccharomyces. Examples of the filamentous fungi include filamentous fungi belonging to the genus Aspergillus, the genus Penicillium, and the genus Trichoderma.

In a case where an eukaryote is used as a host, examples of the vector into which a nucleic acid encoding a modified fibroin is introduced include YEp13 (ATCC37115) and YEp24 (ATCC37051). As a method for introducing an expression vector into the above host cell, any method may be used as long as the method introduces DNA into the host cell. Examples thereof include a method using calcium ions [Proc. Natl. Acad. Sci. USA, 69, 2110 (1972)], electroporation method, spheroplast method, protoplast method, lithium acetate method, and competent method.

As for the method for expressing a nucleic acid using a host transformed with an expression vector, secretory production, fusion protein expression, or the like, in addition to the direct expression, may be carried out in accordance with the methods described in Molecular Cloning, 2nd edition and the like.

The protein can be produced, for example, by culturing a host transformed with the expression vector in a culture medium, producing and accumulating the protein in the culture medium, and then collecting the modified fibroin from the culture medium. The method for culturing a host in a culture medium can be carried out according to a method commonly used for culturing a host.

When the host is a prokaryote such as Escherichia coli or a eukaryote such as yeast, any of a natural medium and a synthetic medium may be used as a culture medium, as long as the medium contains a carbon source, a nitrogen source, inorganic salts, and the like which can be utilized by the host and the medium allows for an efficient culturing of the host.

As the carbon source, any carbon source that can be utilized by the transformed microorganism may be used, and for example, glucose, fructose, sucrose, and molasses containing these; carbohydrates such as starch and a starch hydrolyzate; organic acids such as acetic acid and propionic acid; and alcohols such as ethanol and propanol may be used. As the nitrogen source, for example, ammonium salts of inorganic or organic acids such as ammonia, ammonium chloride, ammonium sulfate, ammonium acetate, and ammonium phosphate, other nitrogen-containing compounds, peptone, meat extract, yeast extract, corn steep liquor, casein hydrolysate, soybean cake and soybean cake hydrolysate, and various fermented microbial cells and digested products thereof may be used. As the inorganic salt, potassium dihydrogen phosphate, dipotassium phosphate, magnesium phosphate, magnesium sulfate, sodium chloride, ferrous sulfate, manganese sulfate, copper sulfate, and calcium carbonate may be used.

Culture of a prokaryote such as Escherichia coli or a eukaryote such as yeast may be carried out under aerobic conditions such as shaking culture or deep aeration stirring culture. The culture temperature is, for example, 15°C to 40°C. The culture time is usually 16 hours to 7 days. It is preferable to maintain the pH of the culture medium during the culture at 3.0 to 9.0. The pH of the culture medium may be adjusted using an inorganic acid, an organic acid, an alkali solution, urea, calcium carbonate, ammonia, or the like.

In addition, antibiotics such as ampicillin and tetracycline may be added to the culture medium as necessary during the culture. In a case of culturing a microorganism transformed with an expression vector in which an inducible promoter is used as the promoter, an inducer may be added to the medium as necessary. For example, in a case of culturing a microorganism transformed with an expression vector in which a lac promoter is used, isopropyl-$\beta$-D-thioga-

lactopyranoside or the like may be added to the medium, and in a case of culturing a microorganism transformed with an expression vector in which a trp promoter is used, indole acrylic acid or the like may be added to the medium.

The expressed protein can be isolated and purified by a commonly used method. For example, in a case where the protein is expressed in a dissolved state in cells, the host cells are recovered by centrifugation after the completion of the culture, suspended in an aqueous buffer solution, and then disrupted using an ultrasonicator, a French press, a Manton-Gaulin homogenizer, a Dyno-Mill, or the like to obtain a cell-free extract. From the supernatant obtained by centrifuging the cell-free extract, a purified preparation can be obtained by a method commonly used for protein isolation and purification, that is, a solvent extraction method, a salting-out method using ammonium sulfate or the like, a desalting method, a precipitation method using an organic solvent, an anion exchange chromatography method using a resin such as diethylaminoethyl (DEAE)-Sepharose or DIAION HPA-75 (manufactured by Mitsubishi Kasei Kogyo Kabushiki Kaisha), an cation exchange chromatography method using a resin such as S-Sepharose FF (manufactured by Pharmacia Corporation), a hydrophobic chromatography method using a resin such as butyl sepharose or phenyl sepharose, a gel filtration method using a molecular sieve, an affinity chromatography method, a chromatofocusing method, or an electrophoresis method such as isoelectric focusing or the like, using the above- described methods singly or in combination thereof.

In addition, in a case where the protein is expressed to form an insoluble body in the cell, similarly, the host cells are recovered, disrupted and centrifuged to recover the insoluble body of the protein as a precipitated fraction. The recovered insoluble body of the protein can be solubilized with a protein denaturing agent. After this operation, a purified preparation of the protein can be obtained by the same isolation and purification method as described above. In a case where the protein is secreted extracellularly, the protein can be recovered from the culture supernatant. That is, a culture supernatant is obtained by treating the culture by a method such as centrifugation, and a purified preparation can be obtained from the culture supernatant by using the same isolation and purification method as described above.

<Method for manufacturing protein fiber>

The protein fiber can be manufactured by a known spinning method. That is, for example, in a case of manufacturing a protein fiber containing a protein as the main component, first, the protein (for example, the modified fibroin) produced according to the above-described method is added to a solvent such as dimethyl sulfoxide (DMSO), N,N-dimethylformamide (DMF), formic acid, or hexafluoroisopropanol (HFIP), as necessary, together with an inorganic salt as a dissolution accelerator and dissolved to prepare a doping liquid. Then, using the doping liquid, spinning can be performed by a known spinning method such as wet type spinning, dry type spinning, dry-wet type spinning, or melt spinning to obtain the protein fiber of interest. A preferred spinning method is wet type spinning and dry-wet type spinning.

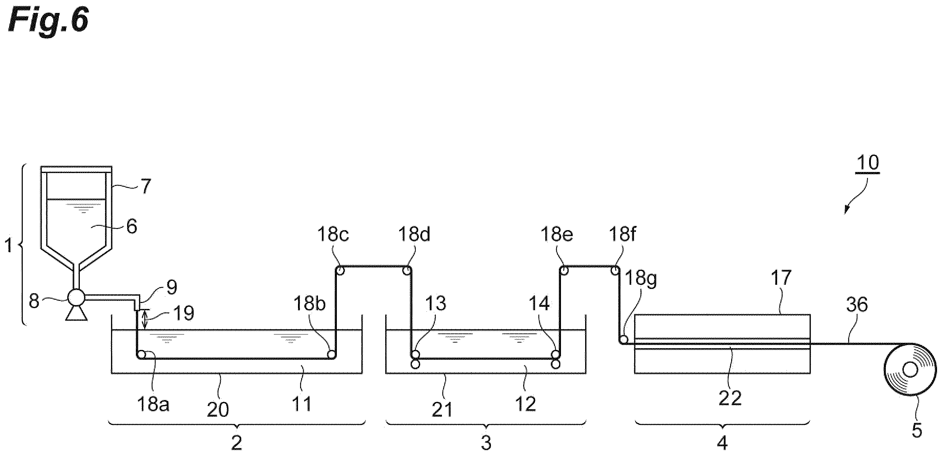

Fig. 6 is an illustrative view schematically illustrating one example of a spinning device for manufacturing a protein fiber. A spinning device 10 illustrated in Fig. 6 is one example of a spinning device for dry-wet type spinning and includes an extrusion device 1, an undrawn yarn manufacturing device 2, a wet heat drawing device 3, and a drying device 4.

A spinning method using the spinning device 10 will be described. First, a doping liquid 6 stored in a storage tank 7 is extruded from a spinneret 9 by a gear pump 8. In the laboratory scale, the doping liquid may be filled in a cylinder and extruded from a nozzle using a syringe pump. Next, the extruded doping liquid 6 is supplied into a coagulation liquid 11 in a coagulation liquid bath 20 through an air gap 19, the solvent is removed, a protein is coagulated, and a fibrous coagulate is formed. Then, the fibrous coagulate is supplied into warm water 12 in a drawing bath (washing bath) 21 and is drawn. A drawing rate is determined according to a speed ratio of a supply nip roller 13 to a withdrawing nip roller 14. Thereafter, the drawn fibrous coagulate is supplied to a drying device 4 and dried in a yarn path 22, and a protein fiber 36 is obtained as a wound yarn body 5. Reference signs 18a to 18g indicate yarn guides.

The coagulation liquid 11 may be any solvent that can be desolvated, and examples thereof include lower alcohols having 1 to 5 carbon atoms such as methanol, ethanol, and 2-propanol, and acetone. The coagulation liquid 11 may appropriately include water. The temperature of the coagulation liquid 11 is preferably 0°C to 30°C. In a case where a syringe pump having a nozzle with a diameter of 0.1 to 0.6 mm is used as the spinneret 9, the extrusion speed is preferably 0.2 to 6.0 ml/hour, and more preferably 1.4 to 4.0 ml/hour, per hole. The distance that the coagulated protein passes through the coagulation liquid 11 (substantially, the distance from the yarn guide 18a to the yarn guide 18b) may be a length that allows efficient desolvation, for example, 200 to 500 mm. The withdrawing speed of the undrawn yarn may be, for example, 1 to 20 m/min and preferably 1 to 3 m/min. The residence time in the coagulation liquid 11 may be, for example, 0.01 to 3 minutes, and is preferably 0.05 to 0.15 minutes. In addition, drawing (pre-drawing) may be performed in the coagulation liquid 11. The coagulation liquid bath 20 may be provided in multiple stages, and the drawing may be performed in each stage or in a specific stage as necessary.

As the drawing performed in a case of obtaining the protein fiber, for example, a pre-drawing performed in the coagulation liquid bath 20 and a wet heat drawing performed in the drawing bath 21 are employed, and a dry heat drawing is also employed.

The wet heat drawing may be performed in warm water, in a solution obtained by adding an organic solvent or the like to warm water, or in heated steam. The temperature may be, for example, 50°C to 90°C, and is preferably 75°C to 85°C. In the wet heat drawing, the undrawn yarn (or pre-drawn yarn) may be drawn, for example, by 1 to 10 times and preferably by 2 to 8 times.

The dry heat drawing may be performed using an electric tubular furnace, a dry heat plate, or the like. The temperature may be, for example, 140°C to 270°C, and is preferably 160°C to 230°C. In the dry heat drawing, the undrawn yarn (or pre-drawn yarn) may be drawn, for example, by 0.5 to 8 times and preferably by 1 to 4 times.

The wet heat drawing and the dry heat drawing may be performed independently or in combination or may be performed in multiple stages. That is, the wet heat drawing and the dry heat drawing may be performed in combination as appropriate, such as performing the first drawing by wet heat drawing and the second drawing by dry heat drawing; or performing the first drawing by wet heat drawing, the second drawing by wet heat drawing, and the third drawing by dry heat drawing.

The lower limit value of the final drawing rate with respect to the undrawn yarn (or pre-drawn yarn) is preferably any of more than 1 time, 2 times or more, 3 times or more, 4 times or more, 5 times or more, 6 times or more, 7 times or more, 8 times or more, or 9 times or more, and the upper limit value is preferably 40 times or less, 30 times or less, 20 times or less, 15 times or less, 14 times or less, 13 times or less 12 times or less, 11 times or less, or 10 times or less.

<Protein fiber>

The protein fiber according to the present embodiment contains a protein, and preferably contains a protein as the main component. The protein fiber may be a protein fiber obtained by spinning the above-described protein, in which case, may include the above-described protein as the main component. Hereinafter, depending on proteins used, the protein fiber may be simply referred to as the "modified protein fiber", "modified fibroin fiber", "modified spider silk fibroin fiber", or the like. The protein fiber may be a short fiber or a long fiber.

It is preferable that the protein fiber according to the present embodiment is shrinkable by merely being brought into contact with aqueous moisture to be in a wet state. Further, it is preferable that the protein fiber according to one embodiment is more highly shrinkable by being brought into contact with aqueous moisture to be in a wet state and subsequently being dried. The shrinkage rate of a protein fiber is defined by the following Formula I.

$$\text{Formula I: Shrinkage rate (\%)} = \{1 - (\text{Length of protein fiber that has been subjected to shrinking step including bringing into contact with aqueous moisture / Length of protein fiber before being subjected to shrinking step})\} \times 100$$

The protein fiber according to the present embodiment may have a shrinkage rate of 2.0% or more, 2.5% or more, 3% or more, 3.5% or more, 4% or more, 4.5% or more, 5% or more, 5.5% or more, or 6% or more. In other words, the shrinkage rate (see Formula II below) of the protein fiber due to being in a wet state may be 2.0% or more.

$$\text{Formula II: Shrinkage rate (\%) due to being in wet state} = \{1 - (\text{Length of protein fiber that has been brought into contact with aqueous moisture to be in wet state / Length of protein fiber before being brought into contact with aqueous moisture})\} \times 100$$

When being brought into contact with aqueous moisture to be in a wet state and then being dried, the protein fiber according to the present embodiment may shrink by a shrinkage rate of more than 7%, 15% or more, 25% or more, 32% or more, 40% or more, 48% or more, 56% or more, 64% or more, or 72% or more. In other words, the shrinkage rate (see Formula III below) of the protein fiber due to being in a wet state and a dry state may be more than 7%.

$$\text{Formula III: Shrinkage rate (\%) due to being in wet state and}$$
$$\text{dry state} = \{1 - (\text{Length of protein fiber that has been brought into}$$
$$\text{contact with aqueous moisture to be in wet state and then dried / Length}$$
$$\text{of protein fiber before being brought into contact with aqueous}$$
$$\text{moisture)}\} \times 100$$

The upper limit of the shrinkage rate is usually 80% or less.

The protein fiber according to the present embodiment may have of an LOI value of 18 or more, 20 or more, 22 or more, 24 or more, 26 or more, 28 or more, 29 or more, or 30 or more.

The protein fiber according to the present embodiment may have a maximum hygroscopic heat generation, as determined according to the following Formula A, of more than 0.025°C/g.

$$\text{Formula A: Maximum hygroscopic heat generation} = \{(\text{Highest}$$
$$\text{temperature of a sample when the sample has been transferred to a high}$$
$$\text{humidity environment after being placed in a low humidity environment}$$
$$\text{until a temperature of the sample reaches equilibrium}) - (\text{Temperature of}$$
$$\text{the sample when the sample is being transferred to the high humidity}$$
$$\text{environment after being placed in the low humidity environment until}$$
$$\text{the temperature of the sample reaches equilibrium})\} \ (°C) \ / \ \text{Sample}$$
$$\text{weight (g)}$$

In Formula A, the low-humidity environment means an environment of a temperature of 20°C and a relative humidity of 40%, and the high-humidity environment means an environment of a temperature of 20°C and a relative humidity of 90%.

The protein fiber according to the present embodiment may have a maximum hygroscopic heat generation of 0.026°C/g or more, 0.027°C/g or more, 0.028°C/g or more, 0.029°C/g or more, 0.030°C/g or more, 0.035°C/g or more, and 0.040°C/g or more. The upper limit of the maximum hygroscopic heat generation is not particularly limited, but is usually 0.060°C/g or less.

The protein fiber according to the present embodiment may have a heat retention index determined according to the following Formula B of 0.20 or more.

$$\text{Formula B: Heat retention index} = \text{Heat retention rate (\%) /}$$
$$\text{Basis weight of sample (g/m}^2)$$

The heat retention index of the protein fiber according to the present embodiment may be 0.22 or more, may be 0.24 or more, may be 0.26 or more, may be 0.28 or more, may be 0.30 or more, or may be 0.32 or more. The upper limit of the heat retention index is not particularly limited, but may be, for example, 0.60 or less, or 0.40 or less.

<Raw-material woven fabric>

The raw-material woven fabric may be obtained by weaving a fiber (also referred to as a material thread) that at least partially contains the protein fiber described above. As the material thread, a single yarn consisting only of a protein fiber and a composite yarn formed of a combination of a protein fiber and another fiber may be used alone or in a combination thereof. The single yarn and the composite yarn may be spun yarns in which short fibers are twisted together, or filament yarns in which long fibers are twisted together. Filament yarns are suitably used as the single yarn and the composite yarn. The single yarn may be formed of a fiber that uses only one kind of a protein or a fiber that uses a plurality of kinds of proteins. In addition, the above another fiber means a fiber containing no protein, and examples thereof include synthetic fibers such as nylon and polyester, regenerated fibers such as cupra and rayon, and natural fibers such as cotton and hemp. In a case where the protein fiber is used in combination with another fiber, the content of the protein fiber is preferably 5% or more by mass, more preferably 20% or more by mass, and still more preferably 50% or more by mass, based on the total amount of the fiber containing the protein fiber. By setting the content of the protein fiber in the above range, the shrinkage rate in the shrinking step of the raw-material woven fabric can be adjusted. Further, examples of the composite yarn includes a blended yarn, a blend fiber yarn, a covering yarn, and the like.

The raw-material woven fabric may be a woven fabric having any structure of plain weave, twill weave, and satin weave. Generally, the weaving density of the woven fabric (the weaving density of the warp thread and the weaving density of the weft thread) is greatly affected by the fineness of each of the warp thread and the weft thread (for example, the lower the fineness is, the higher the maximum weaving density is, and the higher the fineness is, the lower the higher the maximum weaving density is.), and the weaving density of the warp thread and the weaving density of the weft thread correlate with each other (for example, if the former becomes higher, the latter becomes lower, or if the former becomes lower, the latter becomes higher). The weaving density of one or both of the warp thread and the weft thread of the raw-material woven fabric used here may, for example, be 20 yarns/in or more or 25 yarns/in or more, when the fineness is 190 deniers. The weaving density is the number of warp threads or weft threads per inch (1 in corresponds to 2.54 cm) of the woven fabric. The weaving density of the warp thread and the weaving density of the weft thread are respectively called the warp thread density and the weft thread density, and respectively represent the number of the warp threads and the weft threads per 1 in of the woven fabric. The weaving density of the woven fabric may be measured, for example, by a method according to JIS L1096:2010.

The weaving method is not particularly limited, and for example, a material thread may be woven using a known loom. Examples of the loom to be used include shuttle looms, and shuttleless looms such as a gripper loom, a rapier loom, a water jet loom, and an air jet loom.

The weaving step may include a step of adjusting the weaving density of the raw-material woven fabric (hereinafter, also referred to as "weaving density adjusting step"). That is, the weaving step may include a step of adjusting the weaving density (both or either one of warp thread density and weft thread density) of the raw-material woven fabric in accordance with the weaving density of the desired high-density woven fabric. By including such a step, the weaving density of the high-density woven fabric obtained by shrinking the raw-material woven fabric can be easily adjusted. The weaving density of the raw-material woven fabric may be controlled by adjusting the number of reed dents of the loom, the number of warp threads passed through one reed dent (so-called "pull-in number"), and the fineness of the material thread (warp thread and weft thread).

More specifically, the weaving density adjusting step may, for example, be a step of adjusting the weaving density of the raw-material woven fabric so that the high-density woven fabric obtained in the shrinking step described later satisfies at least one of the following condition (i) or (ii).

(i) In a case where the fineness of the warp thread and weft thread of the high-density woven fabric is 190 deniers, the weaving density of one or both of the warp thread and the weft thread is 55 threads/in or more.
(ii) The rate of increase in the weaving density of the high-density woven fabric with respect to the weaving density of the raw-material woven fabric is more than 1 time.

In the weaving density adjusting step, the weaving density of one or both of the warp threads and the weft threads of the raw-material woven fabrics may be adjusted, for example, to 20 threads/in or more or 25 threads/in or more when the fineness is 190 deniers, so that the weaving density of the high-density woven fabric satisfies at least one of the above condition of (i) or (ii)

<Shrinking step>

In the shrinking step, the raw-material woven fabric is brought into contact with aqueous moisture to be in a wet state (hereinafter, also referred to as "contacting step"). The wet state means a state in which at least a part of protein fibers is wet with water. In the contacting step, by allowing the raw-material woven fabric to be in a wet state, the protein fiber can be shrunk (also referred to as water shrinkage) without depending on an external force, whereby the entire woven fabric is shrunk. In this step, it is considered that the shrinkage of the protein fiber that does not rely on the external force occurs due to following reasons. That is, one reason is considered to be due to the secondary structure or tertiary structure of the protein, and another reason is considered to be due to the relaxation of the residual stress by infiltration of the aqueous moisture between the fibers or into the fibers of the protein fibers having residual stress caused by drawing in the manufacturing step. Therefore, it is considered that the shrinkage rate of the protein fiber in the shrinking step can be controlled arbitrary, for example, in accordance with the value of the drawing rate in the manufacturing process of the protein fiber described above. For this reason, by using a material thread containing a protein fiber of which the shrinkage rate has been adjusted by adjusting the drawing rate in the manufacturing process of the protein fiber, the extent of shrinkage of the raw-material woven fabric, when brought into contact with aqueous moisture to be in a wet state, can be adjusted arbitrary.

As a result, it is speculated that a high-density woven fabric having a desired weaving density can be obtained.

It is also expected that the weaving density of the high-density woven fabric can be adjusted by appropriately selecting and combining appropriate threads from material threads having different shrinkage rates. Furthermore, by limiting the extent of shrinkage of the raw-material woven fabric due to being in a wet state, it is also possible to adjust the weaving density of the high-density woven fabric regardless of the kind of material thread. The method for limiting the extent of shrinkage of the raw-material woven fabric due to contacting with aqueous moisture is not particularly limited. Examples of the method for limiting the extent of shrinkage include a method that involves bringing the raw-material woven fabric into contact with aqueous moisture to be shrunk, while keeping the peripheral end portion of the raw-material woven fabric fixed. More specifically, the extent of shrinkage may be adjusted by bringing the raw-material woven fabric into contact with aqueous moisture while keeping the peripheral end portion of the entire periphery of the raw-material woven fabric fixed to a frame body, where the frame body has a size that is smaller than the original size of the raw-material woven fabric before being brought into contact with aqueous moisture and that is larger by a predetermined size than the size of the woven fabric obtained by bringing the raw-material woven fabric into contact with aqueous moisture while keeping the peripheral end portion free so as to shrink the raw-material woven fabric to the maximum extent (namely, while allowing the shrinkage only to an extent corresponding to the difference between the size of the frame body and the size of the raw-material woven fabric). The weaving density of the high-density woven fabric can be adjusted arbitrary by various adjusting the size of the frame body.

In the present specification, "aqueous moisture" means water in a state of either liquid or gas. In the contacting step, the method for bringing the raw-material woven fabric into contact with aqueous moisture is also not particularly limited. For example, a method that involves immersing the raw-material woven fabric in water, a method that involves spraying water in a steam state or the like that is at ordinary temperature or heated, and a method that involves exposing the raw-material woven fabric to a high-humidity environment filled with water vapor. Among these methods, the method that involves immersing the raw-material woven fabric in water is preferable since the shrinking time can be effectively shortened and the processing facilities can be simplified. Specific examples of the method that involves immersing the raw-material woven fabric in water include a method in which a raw-material woven fabric is put into a container containing water having a predetermined temperature.

In the contacting step, the temperature of the water when bringing the raw-material woven fabric into contact with aqueous moisture is not particularly limited, but is preferably, for example, lower than the boiling point of water. At such a temperature, the handling, workability, and the like of the shrinking step are improved. The upper limit value of the water temperature is preferably 90°C or lower and more preferably 80°C or lower. The lower limit value of the water temperature is preferably 10°C or higher, more preferably 40°C or higher, and further preferably 70°C or higher. The temperature of the water to be brought into contact with a raw-material woven fabric may be adjusted depending on the fiber constituting the raw-material woven fabric. In addition, while bringing aqueous moisture into contact with the raw-material woven fabric, the temperature of water may be constant, or the temperature of water may be altered so as to achieve a predetermined temperature.

In the contacting step, the duration of the raw-material woven fabric contacting aqueous moisture is not particularly limited and may be, for example, 1 minute or more. The duration may be 10 minutes or longer, 20 minutes or longer, and 30 minutes or longer. The upper limit of the duration is not particularly limited, but from the viewpoint of shortening the time of the manufacturing step and eliminating the risk of hydrolysis of protein fiber, and the like, it may, for example, be 120 minutes or less, 90 minutes or less, and 60 minutes or less.

In addition to the contacting step, the shrinking step may further include drying the raw-material woven fabric (hereinafter, also referred to as "drying step") after being brought into contact with aqueous moisture to be in a wet state.

The drying method in the drying step is not particularly limited and for example, may be air drying or may be forced drying using drying equipment. The drying temperature is not particularly limited as long as it is lower than the temperature at which the protein constituting the woven fabric is thermally damaged. The drying temperature is generally in the range of 20°C to 150°C, preferably in the range of 40 to 120°C, and more preferably in the range of 60°C to 100°C. Within such a temperature range, the woven fabric can be dried more quickly and efficiently without causing thermal damage to the protein. The drying time is selected as appropriate depending on the drying temperature and the like, and for example, a time in which the influence of overdrying of the protein fiber on the quality and physical properties of the woven fabric can be excluded as much as possible may be adopted.

According to the above-described manufacturing method, since the fiber that at least partially contains a protein fiber is shrunk by the aqueous moisture, a woven fabric (high-density woven fabric) having a weaving density higher than that of the conventional woven fabric obtained by simply weaving the fiber can be manufactured. In addition, it is possible to manufacture a high-density woven fabric having a weaving density satisfying at least one of the above-described conditions (i) and (ii). Further, it is possible to manufacture a woven fabric having a weaving density that exceeds the weaving density achievable with the conventional technique, while satisfying at least one of the above-described conditions (i) and (ii).

<High-density woven fabric>

The "high-density woven fabric" in the present specification means a woven fabric having a high weaving density.

Although both or one of the weaving density of the warp thread and the weft thread of the high-density woven fabric according to one embodiment is affected by the fineness of the warp thread and the weft thread or the like, the weaving density may be, for example, 55 threads/in or more, 60 threads/in or more, 65 threads/in or more, 70 threads/in or more, 75 threads/in or more, 80 threads/in or more, 85 threads/in or more, 90 threads/in or more, 95 threads/in or more, 100 threads/in or more, or 105 threads/in or more, when the fineness of the warp thread or the weft thread is 190 deniers. When the weaving density of both or one of the warp thread and the weft thread is equal to or higher than the lower limit value, the woven fabric can have moderate tension and stiffness. The upper limit value of the weaving density of the high-density woven fabric obtained according to the present invention is not particularly limited, and may be 150 threads/in or less. The weaving density of the high-density woven fabric may be controlled by adjusting the weaving density of the raw-material woven fabric, the shrinkage rate of the raw-material woven fabric due to contacting with aqueous moisture, the shrinkage rate of the fiber that constitutes the raw-material woven fabric and that at least partially contains a protein fiber, due to contacting with aqueous moisture, or the like.

The weaving density of both or one of the warp thread and weft thread of the high-density woven fabric according to one embodiment is increased in comparison with the weaving density of both or one of the warp thread and weft thread of the raw-material woven fabric, due to contacting of the raw-material woven fabric with aqueous moisture.

The rate of increase in the weaving density of the high-density woven fabric may be obtained by comparing the total numbers of warp threads and weft threads per 1 $in^2$ of woven fabric between the raw-material woven fabric before being subjected to the shrinking step and the woven fabric (high-density woven fabric) after being subjected to the shrinking step. That is, for example, the rate of increase in the weaving density of the high-density woven fabric with respect to the weaving density of the raw-material woven fabric is calculated according to Formula IV.

$$\text{Formula IV: Rate of increase in weaving density} = (N1s+N2s) / (N1d+N2d)$$

In the formula,

N1s represents the warp thread density of the high-density woven fabric after being subjected to the shrinking step,
N2s represents the weft thread density of the high-density woven fabric after being subjected to the shrinking step,
N1d represents the warp thread density of the raw-material woven fabric before being subjected to the shrinking step,
N2d represents the weft thread density of the raw-material woven fabric before being subjected to the shrinking step.

Accordingly, N1 s+N2s is the total number of warp threads and weft threads per 1 $in^2$ of the high-density woven fabric after being subjected to the shrinking step, and N1d+N2d is the total number of warp threads and weft threads per 1 1 $in^2$ of the raw-material woven fabric before being subjected to the shrinking step.

The rate of increase in the weaving density of the high-density woven fabric may be, for example, more than 1 time, 1.15 times, 1.5 times, 1.7 times, 2 times, or 2.5 times with respect to the weaving density of the raw-material woven fabric. The rate of increase in the weaving density of the high-density woven fabric may be, for example, 10 times or less with respect to the weaving density of the raw-material woven fabric. By performing the shrinking step such that the rate of increase in the weaving density is within the above range, the high-density woven fabric can have moderate tension and stiffness.

The high-density woven fabric according to one embodiment may be a woven fabric having a weaving density (both or either on of warp thread density and weft thread density) that exceeds the maximum value of the weaving density (theoretical density at the weaving limit) achievable by the conventional weaving step. Here, the maximum value of the weaving density of the warp thread and weft thread achievable in the weaving step, may be determined, for example, by a known method that uses respective cover factors of the warp thread and the weft thread which are obtained from the fineness and the weaving density, as well as weaving property line chart showing the relationship between the cover factors of the warp thread and the weft thread. That is, the theoretical weaving limit density of the weft thread in a case where the warp thread has a predetermined weaving density can be obtained by reading the maximum cover factor of the weft thread in a case where the warp thread has a predetermined cover factor from the weaving property line chart, or conversely, the theoretical weaving limit density of the warp thread in a case where the weft thread has a predetermined weaving density can be obtained by reading the maximum cover factor of the warp thread in a case where the weft thread has a predetermined cover factor from the weaving property line chart.

The cover factor is an index indicating how much the thread (warp thread or weft thread) covers the surface of the woven fabric, and a cover factor of a thread in a woven fabric called a jammed woven fabric that has no gap between the threads is the theoretical maximum value (limit value) of the cover factor. The maximum value of the cover factor of the warp thread and the weft thread can be obtained from a known weaving property line chart. The weaving property line chart is a chart in which the warp thread cover factor and the weft thread cover factor in the jammed woven fabric are plotted for each count ratio of the warp thread to the weft thread, and a unique weaving property line chart is drawn for each structure that constitutes the woven fabric.

The high-density woven fabric according to one embodiment may have of an LOI value of 18 or more, 20 or more, 22 or more, 24 or more, 26 or more, 28 or more, 29 or more, and 30 or more.

The high-density woven fabric according to one embodiment may have a maximum hygroscopic heat generation, as determined according to the following Formula A, of more than 0.025°C/g.

$$\text{Formula A: Maximum hygroscopic heat generation} = \{(\text{Highest temperature of a sample when the sample has been transferred to a high humidity environment after being placed in a low humidity environment until a temperature of the sample reaches equilibrium}) - (\text{Temperature of the sample when the sample is being transferred to the high humidity environment after being placed in the low humidity environment until the temperature of the sample reaches equilibrium})\} \ (°C) \ / \ \text{Sample weight (g)}$$

In Formula A, the low-humidity environment means an environment of a temperature of 20°C and a relative humidity of 40%, and the high-humidity environment means an environment of a temperature of 20°C and a relative humidity of 90%.

The high-density woven fabric according to one embodiment may have a maximum hygroscopic heat generation of 0.026°C/g or more, 0.027°C/g or more, 0.028°C/g or more, 0.029°C/g or more, 0.030°C/g or more, 0.035°C/g or more, and 0.040°C/g or more. The upper limit of the maximum hygroscopic heat generation is not particularly limited, but is usually 0.060°C/g or less.

The high-density woven fabric according to one embodiment may have a heat retention index determined according to the following Formula B of 0.20 or more.

$$\text{Formula B: Heat retention index} = \text{Heat retention rate (\%)} \ / \ \text{Basis weight of sample (g/m}^2\text{)}$$

The heat retention index of the high-density woven fabric according to one embodiment may be 0.22 or more, may be 0.24 or more, may be 0.26 or more, may be 0.28 or more, may be 0.30 or more, and may be 0.32 or more. The upper limit of the heat retention index is not particularly limited, but may be, for example, 0.60 or less, or 0.40 or less.

**Examples**

Hereinafter, the present invention will be described more specifically based on Examples and the like. However, the present invention is not limited to Examples below.

[(1) Production of modified spider silk fibroin (protein)]

(Synthesis of nucleic acid encoding modified spider silk fibroin and construction of expression vector)

A modified spider silk fibroin having the amino acid sequence set forth in SEQ ID NO: 18 (PRT399), a modified spider silk fibroin having the amino acid sequence set forth in SEQ ID NO: 12 (PRT380), a modified spider silk fibroin having the amino acid sequence set forth in SEQ ID NO: 13 (PRT410), and a modified spider silk fibroin (PRT799) having the amino acid sequence set forth in SEQ ID NO: 15 were designed.

Nucleic acids respectively encoding the four kinds of modified spider silk fibroins designed as above were synthesized. To the nucleic acids, an NdeI site was added to the 5' terminal and an EcoRI site was added downstream of the stop codon. These four kinds of nucleic acids were individually cloned into a cloning vector (pUC118). Thereafter, the nucleic acid was enzymatically cleaved by treatment with NdeI and EcoRI, and then recombinated into a protein expression vector pET-22b (+) to obtain an expression vector.

(Expression of modified spider silk fibroin)

Escherichia coli BLR (DE3) was transformed with each of the obtained recombinated pET-22b (+) expression vectors. The transformed Escherichia coli was cultured in 2 mL of an LB medium containing ampicillin for 15 hours. The culture solution was added to 100 mL of a seed culture medium (Table 4) containing ampicillin so that the $OD_{600}$ was 0.005. While maintaining the temperature of the culture solution at 30°C, flask culturing was performed (for about 15 hours) until the $OD_{600}$ reached 5, thereby obtaining a seed culture solution.

[Table 4]

| Seed culture medium | |
| --- | --- |
| Reagent | Concentration (g/L) |
| Glucose | 5.0 |
| $KH_2PO_4$ | 4.0 |
| $K_2HPO_4$ | 9.3 |
| Yeast Extract | 6.0 |
| Ampicillin | 0.1 |

To a jar fermenter to which 500 mL of a production medium (Table 5) had been added, the seed culture solution was added so that the $OD_{600}$ was 0.05. The culture was carried out while keeping the culture solution temperature at 37°C and controlling the pH constant at 6.9. Further, the concentration of dissolved oxygen in the culture solution was maintained at 20% of the dissolved oxygen saturation concentration.

[Table 5]

| Production medium | |
| --- | --- |
| Reagent | Concentration (g/L) |
| Glucose | 12.0 |
| $KH_2PO_4$ | 9.0 |
| $MgSO_4·7H_2O$ | 2.4 |
| Yeast Extract | 15 |
| $FeSO_4·7H_2O$ | 0.04 |
| $MnSO_4·5H_2O$ | 0.04 |
| $CaCl_2·2H_2O$ | 0.04 |
| ADEKANOL (ADEKA, LG-295S) | 0.1 (mL/L) |

Immediately after glucose in the production medium was completely consumed, a feed solution (455 g/1 L of glucose and 120 g/1 L of Yeast Extract) was added at a rate of 1 mL/min. The culture was carried out while keeping the culture solution temperature at 37°C and controlling the pH constant at 6.9. The culture was performed for 20 hours, while maintaining the concentration of dissolved oxygen in the culture solution at 20% of the dissolved oxygen saturation concentration. Thereafter, 1 M isopropyl-β-thiogalactopyranoside (IPTG) was added to the culture solution at a final concentration of 1 mM to induce the expression of the modified fibroin of interest. 20 hours after the addition of IPTG, the culture solution was centrifuged to recover the bacterial cell pellet. SDS-PAGE was performed using bacterial cell pellets prepared from the culture solution before the addition of IPTG and after the addition of IPTG, and the expression of the modified spider silk fibroin of interest was confirmed by the IPTG addition-dependent appearance of a band corresponding to the size of the modified fibroin of interest.

(Purification of modified spider silk fibroin)

The bacterial cell pellet recovered after 2 hours from the addition of IPTG was washed with a 20 mM Tris-HCl buffer solution (pH 7.4). The bacterial cell pellet after washing was suspended in a 20 mM Tris-HCl buffer solution (pH 7.4) containing about 1 mM PMSF, and the cell suspension was disrupted with a high-pressure homogenizer (manufactured by GEA Niro Soavi SpA). The disrupted cells were centrifuged to obtain a precipitate. The obtained precipitate was washed with a 20 mM Tris-HCl buffer solution (pH 7.4) until the obtained precipitate became highly pure. The precipitate after washing was suspended in an 8 M guanidine buffer solution (8 M guanidine hydrochloride, 10 mM sodium dihydrogen phosphate, 20 mM NaCl, 1 mM Tris-HCl, pH 7.0) so that the concentration was 100 mg/mL, and dissolved by stirring with a stirrer at 60°C for 30 minutes. After dissolution, dialysis was carried out in water using a dialysis tube (cellulose tube 36/32 manufactured by Sanko Junyaku Co., Ltd.). The white aggregated protein obtained after dialysis was recovered by centrifugation. The aqueous moisture was removed from the recovered aggregated protein with a freeze dryer to obtain a freeze-dried powder of the modified fibroin of interest.

[(2) Production of modified spider silk fibroin fiber (modified protein fiber)]

(Preparation of doping liquid)

Dimethyl sulfoxide (DMSO) in which LiCl was dissolved to be 4.0% by mass was prepared as a solvent, and the freeze-dried powder of the modified fibroin was added thereto to be a concentration of 18% by mass or 24% by mass and dissolved for 3 hours using a shaker. Thereafter, insoluble bodies and bubbles were removed, whereby a modified spider silk fibroin solution was obtained (see Tables 6 to 9).

(Spinning)

With the obtained modified spider silk fibroin solution used as a doping liquid (spinning stock solution), a modified spider silk fibroin fiber that is spun and drawn was manufactured by dry-wet type spinning using a spinning device based on the spinning device 10 illustrated in Fig.6. The spinning device used is a spinning device further having an additional second undrawn yarn manufacturing device (second bath) between the undrawn yarn manufacturing device 2 (first bath) and a wet heat drawing device 3 (third bath) in the spinning device 10 illustrated in Fig. 6. The dry-wet type spinning conditions are as follows.

    Extrusion nozzle diameter: 0.2 mm
    Coagulation bath (first and second bath) temperature: 2°C to 15°C
    Washing bath (third bath) temperature: 17°C
    Total drawing rate: 1 to 4 times
    Drying temperature: 60°C

[(3) Evaluation of modified spider silk fibroin fiber (modified protein fiber)]

(Shrinkage rate evaluation)

The shrinkage rate of each modified spider silk fibroin fiber obtained in Manufacture Examples 1 to 17 was evaluated. That is, the shrinking step in which each modified spider silk fibroin fiber was brought into contact with water to be in a wet state (contacting step) and subsequently dried (drying step) was performed, and the shrinkage rate of the modified spider silk fibroin fiber due to being in a wet state and the shrinkage rate of the modified spider silk fibroin fiber due to being in a wet state and a dry state was obtained.

<Contacting step>

A plurality of modified spider silk fibroin fibers each having a length of 30 cm were cut out for test from each of the wound products of the modified spider silk fibroin fibers obtained in Manufacture Examples 1 to 17. The plurality of modified spider silk fibroin fibers were bundled to obtain a modified spider silk fibroin fiber bundle having a fineness of 150 deniers. With 0.8 g of lead weight being attached to each of the modified spider silk fibroin fiber bundles, each of the modified spider silk fibroin fiber bundles was immersed in water for 10 minutes at a temperature shown in Tables 6 to 9. Thereafter, the length of each of the modified spider silk fibroin fiber bundles was measured in water. The measurement was carried out with the lead weight of 0.8 g being attached to the modified spider silk fibroin fiber bundle in order to eliminate the crimping of the modified spider silk fibroin fiber bundle. The shrinkage rate (%) of the modified spider silk fibroin fiber due to being in a wet state was calculated according to Formula V. In Formula V, Ld represents the length (30 cm) of the modified spider silk fibroin fiber bundle before being immersed in water, and Lw represents the length of the modified spider silk fibroin fiber bundle immersed in water to be in a wet state.

$$\text{Formula V: Shrinkage rate (\%) due to being in wet state} = \{1 - (Lw/Ld)\} \times 100$$

<Drying step>

The modified spider silk fibroin fiber bundle was taken out from water. The modified spider silk fibroin fiber bundle taken out was dried at room temperature for 2 hours with 0.8 g lead weight being attached. After drying, the length of each of the modified spider silk fibroin fiber bundles was measured. Subsequently, the shrinkage rate (%) of each of the modified spider silk fibroin fibers was calculated according to Formula VI. In Formula VI, Ld represents the length (30 cm) of the modified spider silk fibroin fiber bundle before being immersed in water, and Lwd represents the length of the modified spider silk fibroin fiber bundle brought into contact with water to be in a wet state and subsequently dried.

$$\text{Formula VI: Shrinkage rate (\%) due to being in wet state and dry state} = \{1 - (Lwd/Ld)\} \times 100$$

The results are shown in Tables 6 to 9. The total drawing rate in Tables 6 to 9 indicates the total drawing rate in the spinning step. In Tables 6 to 9, the "Shrinkage rate (%) (wet state)" indicates the shrinkage rate of the modified spider silk fibroin fiber due to being in a wet state, and the "Shrinkage rate (%) (dry state)" indicates the shrinkage rate of the modified spider silk fibroin fiber due to being in a wet state and a dry state.

[Table 6]

| | Modified spider silk fibroin | Total drawing rate | Temperature of water (°C) | Shrinkage rate (%) (wet state) | Shrinkage rate (%) (dry state) |
|---|---|---|---|---|---|
| Manufacture Example 1 | 24wt% PRT799 | 1 | | 0 | 7.8 |
| Manufacture Example 2 | 24wt% PRT799 | 2 | | -1.2 | 10.3 |
| Manufacture Example 3 | 24wt% PRT799 | 3 | | 7.2 | 21.2 |
| Manufacture Example 4 | 24wt% PRT799 | 4 | 20 | 13.5 | 26.3 |
| Manufacture Example 5 | 18wt% PRT799 | 2 | | -2.3 | 9.5 |
| Manufacture Example 6 | 18wt% PRT799 | 3 | | 6 | 19.7 |
| Manufacture Example 7 | 18wt% PRT799 | 4 | | 14.3 | 27.5 |

(continued)

| | Modified spider silk fibroin | Total drawing rate | Temperature of water (°C) | Shrinkage rate (%) (wet state) | Shrinkage rate (%) (dry state) |
|---|---|---|---|---|---|
| Manufacture Example 2 | 24wt% PRT799 | 2 | 40 | -5.3 | 7.2 |
| Manufacture Example 3 | 24wt% PRT799 | 3 | | 8.7 | 21.3 |
| Manufacture Example 4 | 24wt% PRT799 | 4 | | 14.5 | 26 |
| Manufacture Example 5 | 18wt% PRT799 | 2 | | -4.3 | 7.3 |
| Manufacture Example 6 | 18wt% PRT799 | 3 | | 6.2 | 18.3 |
| Manufacture Example 7 | 18wt% PRT799 | 4 | | 16 | 28.7 |
| Manufacture Example 3 | 24wt% PRT799 | 3 | 60 | 6.8 | 21 |
| Manufacture Example 4 | 24wt% PRT799 | 4 | | 15 | 27.5 |
| Manufacture Example 5 | 18wt% PRT799 | 2 | | -1.5 | 10.7 |
| Manufacture Example 6 | 18wt% PRT799 | 3 | | 3.3 | 18.2 |
| Manufacture Example 7 | 18wt% PRT799 | 4 | | 16.2 | 29 |

[Table 7]

| | Modified spider silk fibroin | Total drawing rate | Temperature of water (°C) | Shrinkage rate (%) (wet state) | Shrinkage rate (%) (dry state) |
|---|---|---|---|---|---|
| Manufacture Example 8 | 24wt% PRT410 | 2 | 20 | -2.3 | 8.7 |
| Manufacture Example 9 | 24wt% PRT410 | 3 | | 4.7 | 16.7 |
| Manufacture Example 10 | 24wt% PRT410 | 4 | | 10.3 | 22.3 |
| Manufacture Example 9 | 24wt% PRT410 | 3 | 40 | 4.7 | 17.5 |
| Manufacture Example 10 | 24wt% PRT410 | 4 | | 11.5 | 24 |
| Manufacture Example 9 | 24wt% PRT410 | 3 | 60 | 2 | 16.5 |
| Manufacture Example 10 | 24wt% PRT410 | 4 | | 10.8 | 25 |

[Table 8]

|  | Modified spider silk fibroin | Total drawing rate | Temperature of water (°C) | Shrinkage rate (%) (wet state) | Shrinkage rate (%) (dry state) |
|---|---|---|---|---|---|
| Manufacture Example 11 | 24wt% PRT399 | 1 |  | -3.5 | 7.6 |
| Manufacture Example 12 | 24wt% PRT399 | 2 | 20 | 3.7 | 12.5 |
| Manufacture Example 13 | 24wt% PRT399 | 3 |  | 7.0 | 16.8 |
| Manufacture Example 12 | 24wt% PRT399 | 2 | 40 | 3.0 | 12.7 |
| Manufacture Example 13 | 24wt% PRT399 | 3 |  | 7.3 | 16.7 |
| Manufacture Example 12 | 24wt% PRT399 | 2 | 60 | 3.3 | 9.3 |
| Manufacture Example 13 | 24wt% PRT399 | 3 |  | 6.8 | 14.2 |

[Table 9]

|  | Modified spider silk fibroin | Total drawing rate | Temperature of water (°C) | Shrinkage rate (%) (wet state) | Shrinkage rate (%) (dry state) |
|---|---|---|---|---|---|
| Manufacture Example 14 | 24wt% PRT380 | 1 | 20 | -1.1 | 9.4 |
| Manufacture Example 15 | 24wt% PRT380 | 2 |  | 2.7 | 13.3 |
| Manufacture Example 16 | 24wt% PRT380 | 3 |  | 7.0 | 17.7 |
| Manufacture Example 17 | 24wt% PRT380 | 4 |  | 10.0 | 20.2 |
| Manufacture Example 15 | 24wt% PRT380 | 2 | 40 | 3.3 | 14.2 |
| Manufacture Example 16 | 24wt% PRT380 | 3 |  | 7.7 | 19.0 |
| Manufacture Example 17 | 24wt% PRT380 | 4 |  | 12.0 | 22.0 |
| Manufacture Example 15 | 24wt% PRT380 | 2 | 60 | 2.7 | 14.3 |
| Manufacture Example 16 | 24wt% PRT380 | 3 |  | 8.2 | 20.3 |
| Manufacture Example 17 | 24wt% PRT380 | 4 |  | 12.0 | 23.2 |

[(4) Examples]

<Manufacture of raw-material woven fabric>

Using a twisted yarn formed of the modified spider silk fibroin fiber, four kinds of raw-material woven fabrics having weaving densities different from each other as shown in Table 10 were woven in a plain weave by a rapier loom (Evergreen Automatic Sampling Loom: manufactured by CCI TECH INC.). The fineness of the twisted yarn formed of the modified fibroin fiber was 190 d, and the number of twists was 450 T/m.

[Manufacturing of high-density woven fabric]

Each of the four kinds of the raw-material woven fabrics obtained above was immersed in water at 40°C for 10 minutes and then dried to manufacture four kinds of high-density woven fabrics respectively formed of the modified fibroin fiber twisted yarns (Examples 1 to 4). Thereafter, the densities of the high-density woven fabrics of Examples 1 to 4 were examined. The results are shown in Table 10. In the table, weaving density is indicated in the form of warp thread density multiplied by weft thread density. For example, the weaving density "26 × 26" means a warp thread density of 26 (threads/in) and a weft thread density of 26 (threads/in).

[(5) Comparative Example]

Using a twisted yarn formed of a polyamide fiber instead of the twisted yarn formed of the modified fibroin fiber, four kinds of raw-material woven fabrics having densities different from each other as shown in Table 10 were woven in the same manner as in the manufacture of the raw-material woven fabric formed of the modified fibroin fiber twisted yarn. Here, the fineness of the twisted yarn formed of the polyamide fiber was 150 d, and the number of twists was 150 T/m.

After weaving, the four kinds of raw-material woven fabrics were immersed in water and then dried in the same manner as in Examples described above to manufacture four kinds of woven fabrics formed of the polyamide fiber twisted yarn (Comparative Examples 1 to 4). Thereafter, the densities of the woven fabrics of Comparative Examples 1 to 4 were examined. The results are shown together in Table 10.

[Table 10]

| | Weaving density before immersion | Weaving density after immersion | Rate of increase in weaving density |
|---|---|---|---|
| Example 1 | 26 × 26 | 60 × 74 | 2.57 |
| Example 2 | 51 × 39 | 92 × 88 | 2 |
| Example 3 | 51 × 65 | 92 × 107 | 1.71 |
| Example 4 | 102 × 78 | 119 × 93 | 1.17 |
| Comparative Example 1 | 26 × 26 | 26 × 26 | 1 |
| Comparative Example 2 | 51 × 39 | 51 × 39 | 1 |
| Comparative Example 3 | 51 × 65 | 51 × 65 | 1 |
| Comparative Example 4 | 110 × 87 | 110 × 87 | 1 |

Among the weaving densities of the warp thread and the weft thread used in Examples 1 to 4, the theoretical maximum value of the weaving density of the warp thread achievable by the conventional weaving step is 102 (threads/in), when the weaving density of the weft thread is 78 (threads/in). On the other hand, in the high-density woven fabric of Example 4, the weaving density of warp thread was 119 (threads/in), which exceeds the theoretical (weaving limit) maximum value of the weaving density of warp thread.

[(6) Reference Example 1: Evaluation of flame retardancy of modified spider silk fibroin]

A freeze-dried powder of the modified spider silk fibroin (PRT799) was obtained in the same manner as in (1) above. PRT799 is a hydrophilic modified spider silk fibroin having an average HI of 0 or less.

The freeze-dried powder of the modified spider silk fibroin (PRT799) was added to a DMSO solution containing LiCl (concentration: 4.0% by mass) such that the concentration of the modified spider silk fibroin was 24% by mass, and mixed for 3 hours to be dissolved using a shaker. Thereafter, insoluble bodies and bubbles were removed, whereby a modified spider silk fibroin solution (spinning stock solution) was obtained.

The spinning stock solution was filtered at 90°C with a metal filter having a mesh size of 5 $\mu$m, and then allowed to be left in a 30 mL stainless steel syringe, subjected to defoaming, and then discharged from a solid nozzle having a needle diameter of 0.2 mm into 100% by mass of methanol in the coagulation bath. The discharge temperature was 90°C. After coagulation, the obtained raw yarn was wound and subjected to air drying to obtain a modified spider silk fibroin fiber (raw material fiber).

A knitted fabric (thickness: 180 deniers, gauge number: 18) was manufactured by circular knitting using a circular knitting machine, using a twisted yarn in which raw material fibers were twisted with each other. 20 g of the obtained knitted fabric was cut out and used as a test piece.

A flammability test was performed in accordance with "Test Method for Granular or Low-Melting-Point Synthetic Resin" described in "Notice No. 50 of the Office of Hazardous Materials Regulation (as of May 31, 1995)". The test was performed under the conditions of a temperature of 22°C, a relative humidity of 45%, and an atmospheric pressure of 1021 hPa. The measurement results (Oxygen concentration (%), Combustion rate (%), and Converted combustion rate (%)) are shown in Table 11.

[Table 11]

| Oxygen concentration (%) | Combustion rate (%) | Converted combustion rate (%) |
|---|---|---|
| 20.0 | 39.1 | 40.1 |
| 27.0 | 48.1 | 49.3 |
| 28.0 | 51.9 | 53.2 |
| 30.0 | 53.6 | 54.9 |
| 50.0 | 61.2 | 62.7 |
| 70.0 | 91.1 | 93.3 |
| 100.0 | 97.6 | 100.0 |

As a result of the flammability test, the knitted fabric knitted with the modified spider silk fibroin (PRT799) fiber had a limiting oxygen index (LOI) value of 27.2. Generally, LOI value of 26 or more is considered as having flame retardancy. It can be seen that the modified spider silk fibroin has excellent flame retardancy.

[(7) Reference Example 2: Evaluation of hygroscopic exothermicity of modified spider silk fibroin]

Freeze-dried powders of the modified spider silk fibroins (PRT918 and PRT799) was obtained in the same manner as in (1) above. PRT918 is a hydrophobic modified spider silk fibroin having the amino acid sequence set forth in SEQ ID NO: 37 and having an average HI of more than 0.

The freeze-dried powder of the modified spider silk fibroin (PRT918 and PRT799) was added to a DMSO solution containing LiCl (concentration: 4.0% by mass) such that the concentration of the modified spider silk fibroin was 24% by mass, and mixed for 3 hours to be dissolved using a shaker. Thereafter, insoluble bodies and bubbles were removed, whereby a modified spider silk fibroin solution (spinning stock solution) was obtained.

The spinning stock solution was filtered at 60°C with a metal filter having a mesh size of 5 $\mu$m, and then allowed to be left in a 30 mL stainless steel syringe, subjected to defoaming, and then discharged from a solid nozzle having a needle diameter of 0.2 mm into 100% by mass of methanol in the coagulation bath. The discharge temperature was 60°C. After coagulation, the obtained raw yarn was wound and subjected to air drying to obtain a modified spider silk fibroin fiber (raw material fiber).

For comparison, commercially available wool fibers, cotton fibers, Tencel fibers, rayon fibers, and polyester fibers were prepared as raw material fibers.

Each raw material fiber was used to manufacture a knitted fabric by flat knitting using a flat knitting machine. Table 12 shows the thickness and the gauge number of the knitted fabric obtained by using the PRT918 fiber or the PRT799 fiber. The thickness and the gauge number of the knitted fabric using other raw material fibers were adjusted so that the cover factor was almost the same as the cover factor of the knitted fabric of the modified spider silk fibroin fiber. Specifically, the numerical values are as shown in Table 12.

[Table 12]

| Raw material fiber | Thickness [N] | Gauge number [GG] |
|---|---|---|
| PRT918 | 1/30 (metric count single yarn) | 18 |
| PRT799 | 1/30 (metric count single yarn) | 16 |
| Wool | 2/30 (two folded yarn) | 14 |
| Cotton | 2/34 (two folded yarn) | 14 |
| Tencel | 2/30 (two folded yarn) | 15 |
| Rayon | 1/38 (single yarn) | 14 |
| Polyester | 1/60 (single yarn) | 14 |

Two pieces of knitted fabric cut into 10 cm × 10 cm were put together and the four sides were sewn together to obtain a test piece (sample). After exposing the test piece to a low-humidity environment (temperature of 20°C ± 2°C, relative humidity of 40% ± 5%) for 4 hours or more, the test piece was transferred to a high-humidity environment (temperature of 20°C ± 2°C, relative humidity of 90% ± 5%), and the temperature was measured at an interval of 1 minute for 30 minutes with a temperature sensor attached in the center of the inside of the test piece.

From the measurement results, the maximum hygroscopic heat generation was determined according to the following Formula A.

$$\text{Formula A: Maximum hygroscopic heat generation} = \{(\text{Highest temperature of a sample when the sample has been transferred to a high humidity environment after being placed in a low humidity environment until a temperature of the sample reaches equilibrium}) - (\text{Temperature of the sample when the sample is being transferred to the high humidity environment after being placed in the low humidity environment until the temperature of the sample reaches equilibrium})\} \; (°C) \; / \; \text{Sample weight (g)}$$

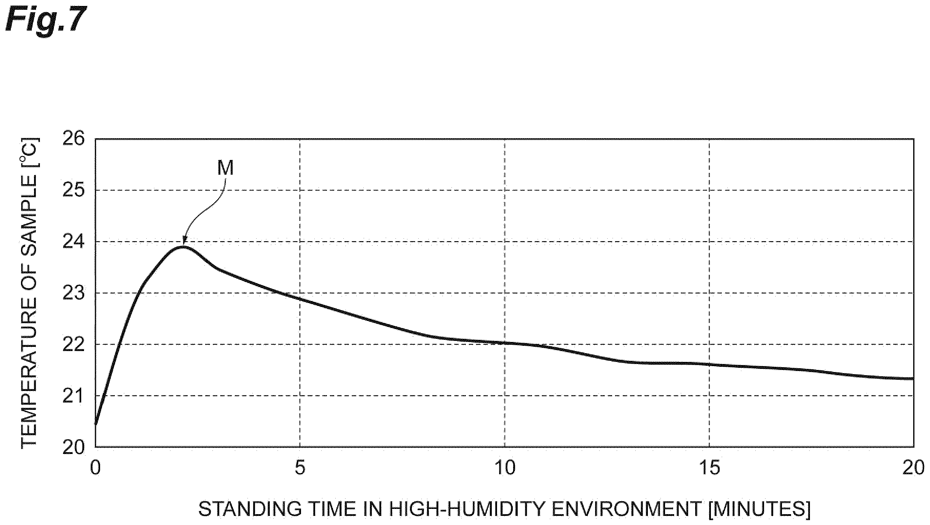

Fig. 7 is a graph showing one example of results of a hygroscopic exothermicity test. The horizontal axis of the graph indicates the time (minutes) left in the high-humidity environment, where 0 is the time when the sample was transferred from the low-humidity environment to the high-humidity environment. The vertical axis of the graph indicates the temperature (sample temperature) measured by the temperature sensor. In the graph shown in Fig. 7, a point indicated as M corresponds to a highest value of the sample temperature.

The results of calculation for the maximum hygroscopic heat generation are shown in Table 13.

[Table 13]

| Raw material fiber | Maximum hygroscopic heat generation (° C/g) |
|---|---|
| PRT918 | 0.040 |
| PRT799 | 0.031 |
| Wool | 0.020 |

(continued)

| Raw material fiber | Maximum hygroscopic heat generation (° C/g) |
|---|---|
| Cotton | 0.021 |
| Tencel | 0.018 |
| Rayon | 0.025 |
| Polyester | 0.010 |

As shown in Table 13, it can be seen that the modified spider silk fibroin (PRT918 and PRT799) fibers have a high value of the maximum hygroscopic heat generation and excellent hygroscopic exothermicity, as compared with the existing materials.

[(8) Reference Example 3: Evaluation of heat retention of modified spider silk fibroin]

Freeze-dried powders of the modified spider silk fibroins (PRT966 and PRT799) was obtained in the same manner as in (1) above. PRT966 is a hydrophobic modified spider silk fibroin having the amino acid sequence set forth in SEQ ID NO: 40 and having an average HI of more than 0.

The freeze-dried powder of the modified spider silk fibroin (PRT966 and PRT799) was added to a DMSO solution containing LiCl (concentration: 4.0% by mass) such that the concentration of the modified spider silk fibroin was 24% by mass, and mixed for 3 hours to be dissolved using a shaker. Thereafter, insoluble bodies and bubbles were removed, whereby a modified spider silk fibroin solution (spinning stock solution) was obtained.

The spinning stock solution was filtered at 60°C with a metal filter having a mesh size of 5 $\mu$m, and then allowed to be left in a 30 mL stainless steel syringe, subjected to defoaming, and then discharged from a solid nozzle having a needle diameter of 0.2 mm into 100% by mass of methanol in the coagulation bath. The discharge temperature was 60°C. After coagulation, the obtained raw yarn was wound and subjected to air drying to obtain a modified spider silk fibroin fiber (raw material fiber).

For comparison, commercially available wool fibers, silk fibers, cotton fibers, rayon fibers, and polyester fibers were prepared as raw material fibers.

Each raw material fiber was used to manufacture a knitted fabric by flat knitting using a flat knitting machine. The count, the number of twists, the gauge number, and the Basis weight of the knitted fabric using the PRT966 fiber and the PRT799 fiber are shown in Table 14. The thickness and the gauge number of the knitted fabric using other raw material fibers were adjusted so that the cover factor was almost the same as the cover factor of the knitted fabric of the modified spider silk fibroin fiber. Specifically, the numerical values are as shown in Table 14.

[Table 14]

| Raw material fiber | Count [Nm] | Number of twists | Gauge number GG | Basis weight [g/m$^2$] |
|---|---|---|---|---|
| PRT966 | 30 | 1 | 18 | 90.1 |
| PRT799 | 30 | 1 | 16 | 111.0 |
| Wool | 30 | 2 | 14 | 242.6 |
| Silk | 60 | 2 | 14 | 225.2 |
| Cotton | 34 | 2 | 14 | 194.1 |
| Rayon | 38 | 1 | 14 | 181.8 |
| Polyester | 60 | 1 | 14 | 184.7 |

The heat retention was evaluated by a dry contact method, using a KES-F7 Thermo Labo II tester manufactured by Kato Tech Co., Ltd. The dry contact method is a method assuming a direct contact between the skin and clothes in a dry state. One sheet of the knitted fabric cut into a rectangle of 20 cm $\times$ 20 cm was used as a test piece (sample). The test piece was set on a hot plate set to a constant temperature (30°C), and the quantity of heat (a) dissipated through the test piece was determined under the conditions of a wind velocity in a wind tunnel of 30 cm/sec. The quantity of heat (b) dissipated under the same conditions as described above was determined in a state where the test piece was not set, and the heat retention rate (%) was calculated according to the following formula.

$$\text{Heat retention rate } (\%) = (1 - a/b) \times 100$$

From the measurement results, the heat retention index was calculated according to the following Formula B.

$$\text{Formula B: Heat retention index} = \text{Heat retention rate } (\%) \text{ / Basis weight of sample } (g/m^2)$$

The results of calculation of the heat retention indices are shown in Table 15. It can be evaluated that the higher the heat retention index is, the more excellent the heat retention the material has.

[Table 15]

| Raw material fiber | Heat retention index |
|---|---|
| PRT966 | 0.33 |
| PRT799 | 0.22 |
| Wool | 0.16 |
| Silk | 0.11 |
| Cotton | 0.13 |
| Rayon | 0.02 |
| Polyester | 0.18 |

As shown in Table 15, it can be seen that the modified spider silk fibroin (PRT966 and PRT799) fibers have higher heat retention indices and excellent heat retention, as compared with the existing materials.

**Reference Signs List**

1···extrusion device, 2···undrawn yarn manufacturing device, 3···wet heat drawing device, 4···drying device, 6···doping liquid, 10 spinning device, 20···coagulation liquid bath, 21···drawing bath, 36···protein fiber.

**Sequence Listing**

SEQUENCE LISTING

<110> Spiber Inc.

<120> A woven fabric and a method for producing the same

<130> FP19-0338-00

<150> JP2018-071892
<151> 2018-04-03

<160> 44

<170> PatentIn version 3.5

<210> 1
<211> 50
<212> PRT
<213> Araneus diadematus

<400> 1

```
Ser Gly Cys Asp Val Leu Val Gln Ala Leu Leu Glu Val Val Ser Ala
1               5                   10                  15

Leu Val Ser Ile Leu Gly Ser Ser Ser Ile Gly Gln Ile Asn Tyr Gly
            20                  25                  30

Ala Ser Ala Gln Tyr Thr Gln Met Val Gly Gln Ser Val Ala Gln Ala
            35                  40                  45

Leu Ala
        50
```

<210> 2
<211> 30
<212> PRT
<213> Araneus diadematus

<400> 2

```
Ser Gly Cys Asp Val Leu Val Gln Ala Leu Leu Glu Val Val Ser Ala
1               5                   10                  15

Leu Val Ser Ile Leu Gly Ser Ser Ser Ile Gly Gln Ile Asn
            20                  25                  30
```

<210> 3
<211> 21
<212> PRT
<213> Araneus diadematus

<400> 3

```
Ser Gly Cys Asp Val Leu Val Gln Ala Leu Leu Glu Val Val Ser Ala
1               5                   10                  15
```

```
Leu Val Ser Ile Leu
            20


<210>  4
<211>  1154
<212>  PRT
<213>  Artificial Sequence

<220>
<223>  recombinant spider silk protein ADF3KaiLargeNRSH1

<400>  4

Met His His His His His His His His His His Ser Ser Gly Ser Ser
1                   5                   10                  15


Leu Glu Val Leu Phe Gln Gly Pro Ala Arg Ala Gly Ser Gly Gln Gln
            20                  25                  30


Gly Pro Gly Gln Gln Gly Pro Gly Gln Gln Gly Pro Gly Gln Gln Gly
            35                  40                  45


Pro Tyr Gly Pro Gly Ala Ser Ala Ala Ala Ala Ala Ala Gly Gly Tyr
    50                  55                  60


Gly Pro Gly Ser Gly Gln Gln Gly Pro Ser Gln Gln Gly Pro Gly Gln
65                  70                  75                  80


Gln Gly Pro Gly Gly Gln Gly Pro Tyr Gly Pro Gly Ala Ser Ala Ala
                85                  90                  95


Ala Ala Ala Ala Gly Gly Tyr Gly Pro Gly Ser Gly Gln Gln Gly Pro
            100                 105                 110


Gly Gly Gln Gly Pro Tyr Gly Pro Gly Ser Ser Ala Ala Ala Ala Ala
            115                 120                 125


Ala Gly Gly Asn Gly Pro Gly Ser Gly Gln Gln Gly Ala Gly Gln Gln
    130                 135                 140


Gly Pro Gly Gln Gln Gly Pro Gly Ala Ser Ala Ala Ala Ala Ala Ala
145                 150                 155                 160


Gly Gly Tyr Gly Pro Gly Ser Gly Gln Gln Gly Pro Gly Gln Gln Gly
                165                 170                 175


Pro Gly Gly Gln Gly Pro Tyr Gly Pro Gly Ala Ser Ala Ala Ala Ala
                180                 185                 190
```

42

Ala Ala Gly Gly Tyr Gly Pro Gly Ser Gly Gln Gly Pro Gly Gln Gln
        195                 200                 205

Gly Pro Gly Gly Gln Gly Pro Tyr Gly Pro Gly Ala Ser Ala Ala Ala
        210                 215                 220

Ala Ala Ala Gly Gly Tyr Gly Pro Gly Ser Gly Gln Gln Gly Pro Gly
225                 230                 235                 240

Gln Gln Gly Pro Gly Gln Gln Gly Pro Gly Gly Gln Gly Pro Tyr Gly
            245                 250                 255

Pro Gly Ala Ser Ala Ala Ala Ala Ala Ala Gly Gly Tyr Gly Pro Gly
            260                 265                 270

Tyr Gly Gln Gln Gly Pro Gly Gln Gln Gly Pro Gly Gly Gln Gly Pro
        275                 280                 285

Tyr Gly Pro Gly Ala Ser Ala Ala Ser Ala Ala Ser Gly Gly Tyr Gly
        290                 295                 300

Pro Gly Ser Gly Gln Gln Gly Pro Gly Gln Gln Gly Pro Gly Gly Gln
305                 310                 315                 320

Gly Pro Tyr Gly Pro Gly Ala Ser Ala Ala Ala Ala Ala Ala Gly Gly
            325                 330                 335

Tyr Gly Pro Gly Ser Gly Gln Gln Gly Pro Gly Gln Gln Gly Pro Gly
            340                 345                 350

Gln Gln Gly Pro Gly Gln Gln Gly Pro Gly Gly Gln Gly Pro Tyr Gly
        355                 360                 365

Pro Gly Ala Ser Ala Ala Ala Ala Ala Ala Gly Gly Tyr Gly Pro Gly
        370                 375                 380

Ser Gly Gln Gln Gly Pro Gly Gln Gln Gly Pro Gly Gln Gln Gly Pro
385                 390                 395                 400

Gly Gln Gln Gly Pro Gly Gln Gln Gly Pro Gly Gln Gln Gly Pro Gly
            405                 410                 415

Gln Gln Gly Pro Gly Gln Gln Gly Pro Gly Gln Gln Gly Pro Gly Gly
            420                 425                 430

Gln Gly Ala Tyr Gly Pro Gly Ala Ser Ala Ala Ala Gly Ala Ala Gly
        435                 440                 445

Gly Tyr Gly Pro Gly Ser Gly Gln Gln Gly Pro Gly Gln Gln Gly Pro
450 455 460

Gly Gln Gln Gly Pro Gly Gln Gln Gly Pro Gly Gln Gln Gly Pro Gly
465 470 475 480

Gln Gln Gly Pro Gly Gln Gln Gly Pro Gly Gln Gln Gly Pro Tyr Gly
485 490 495

Pro Gly Ala Ser Ala Ala Ala Ala Ala Gly Gly Tyr Gly Pro Gly
500 505 510

Ser Gly Gln Gln Gly Pro Gly Gln Gln Gly Pro Gly Gln Gln Gly Pro
515 520 525

Gly Gly Gln Gly Pro Tyr Gly Pro Gly Ala Ala Ser Ala Ala Val Ser
530 535 540

Val Ser Arg Ala Arg Ala Gly Ser Gly Gln Gln Gly Pro Gly Gln Gln
545 550 555 560

Gly Pro Gly Gln Gln Gly Pro Gly Gln Gln Gly Pro Tyr Gly Pro Gly
565 570 575

Ala Ser Ala Ala Ala Ala Ala Ala Gly Gly Tyr Gly Pro Gly Ser Gly
580 585 590

Gln Gln Gly Pro Ser Gln Gln Gly Pro Gly Gln Gln Gly Pro Gly Gly
595 600 605

Gln Gly Pro Tyr Gly Pro Gly Ala Ser Ala Ala Ala Ala Ala Ala Gly
610 615 620

Gly Tyr Gly Pro Gly Ser Gly Gln Gln Gly Pro Gly Gly Gln Gly Pro
625 630 635 640

Tyr Gly Pro Gly Ser Ser Ala Ala Ala Ala Ala Ala Gly Gly Asn Gly
645 650 655

Pro Gly Ser Gly Gln Gln Gly Ala Gly Gln Gln Gly Pro Gly Gln Gln
660 665 670

Gly Pro Gly Ala Ser Ala Ala Ala Ala Ala Ala Gly Gly Tyr Gly Pro
675 680 685

Gly Ser Gly Gln Gln Gly Pro Gly Gln Gln Gly Pro Gly Gly Gln Gly
690 695 700

Pro Tyr Gly Pro Gly Ala Ser Ala Ala Ala Ala Ala Ala Gly Gly Tyr
705                710                715                720

Gly Pro Gly Ser Gly Gln Gly Pro Gly Gln Gln Gly Pro Gly Gly Gln
                725                730                735

Gly Pro Tyr Gly Pro Gly Ala Ser Ala Ala Ala Ala Ala Ala Gly Gly
            740                745                750

Tyr Gly Pro Gly Ser Gly Gln Gln Gly Pro Gly Gln Gln Gly Pro Gly
        755                760                765

Gln Gln Gly Pro Gly Gly Gln Gly Pro Tyr Gly Pro Gly Ala Ser Ala
    770                775                780

Ala Ala Ala Ala Ala Gly Gly Tyr Gly Pro Gly Tyr Gly Gln Gln Gly
785                790                795                800

Pro Gly Gln Gln Gly Pro Gly Gly Gln Gly Pro Tyr Gly Pro Gly Ala
            805                810                815

Ser Ala Ala Ser Ala Ala Ser Gly Gly Tyr Gly Pro Gly Ser Gly Gln
            820                825                830

Gln Gly Pro Gly Gln Gln Gly Pro Gly Gly Gln Gly Pro Tyr Gly Pro
        835                840                845

Gly Ala Ser Ala Ala Ala Ala Ala Ala Gly Gly Tyr Gly Pro Gly Ser
    850                855                860

Gly Gln Gln Gly Pro Gly Gln Gln Gly Pro Gly Gln Gln Gly Pro Gly
865                870                875                880

Gln Gln Gly Pro Gly Gly Gln Gly Pro Tyr Gly Pro Gly Ala Ser Ala
            885                890                895

Ala Ala Ala Ala Gly Gly Tyr Gly Pro Gly Ser Gly Gln Gln Gly
        900                905                910

Pro Gly Gln Gln Gly Pro Gly Gln Gln Gly Pro Gly Gln Gln Gly Pro
            915                920                925

Gly Gln Gln Gly Pro Gly Gln Gln Gly Pro Gly Gln Gln Gly Pro Gly
    930                935                940

Gln Gln Gly Pro Gly Gln Gln Gly Pro Gly Gly Gln Gly Ala Tyr Gly

945                     950                     955                     960


Pro Gly Ala Ser Ala Ala Ala Gly Ala Ala Gly Gly Tyr Gly Pro Gly
                    965                     970                     975


Ser Gly Gln Gln Gly Pro Gly Gln Gln Gly Pro Gly Gln Gln Gly Pro
                980                     985                     990


Gly Gln Gln Gly Pro Gly Gln Gln  Gly Pro Gly Gln Gln  Gly Pro Gly
           995                     1000                    1005


Gln Gln  Gly Pro Gly Gln Gln  Gly Pro Tyr Gly Pro  Gly Ala Ser
      1010                    1015                    1020


Ala Ala  Ala Ala Ala Ala Gly  Gly Tyr Gly Pro Gly  Ser Gly Gln
      1025                    1030                    1035


Gln Gly  Pro Gly Gln Gln Gly  Pro Gly Gln Gln Gly  Pro Gly Gly
      1040                    1045                    1050


Gln Gly  Pro Tyr Gly Pro Gly  Ala Ala Ser Ala Ala  Val Ser Val
      1055                    1060                    1065


Gly Gly  Tyr Gly Pro Gln Ser  Ser Ser Val Pro Val  Ala Ser Ala
      1070                    1075                    1080


Val Ala  Ser Arg Leu Ser Ser  Pro Ala Ala Ser Ser  Arg Val Ser
      1085                    1090                    1095


Ser Ala  Val Ser Ser Leu Val  Ser Ser Gly Pro Thr  Lys His Ala
      1100                    1105                    1110


Ala Leu  Ser Asn Thr Ile Ser  Ser Val Val Ser Gln  Val Ser Ala
      1115                    1120                    1125


Ser Asn  Pro Gly Leu Ser Gly  Cys Asp Val Leu Val  Gln Ala Leu
      1130                    1135                    1140


Leu Glu  Val Val Ser Ala Leu  Val Ser Ile Leu
      1145                    1150


<210>  5
<211>  24
<212>  PRT
<213>  Artificial Sequence

<220>
<223>  His tag and start codon

<400> 5

Met His His His His His His His His His Ser Ser Gly Ser Ser
1               5                   10              15

Leu Glu Val Leu Phe Gln Gly Pro
            20


<210> 6
<211> 597
<212> PRT
<213> Artificial Sequence

<220>
<223> Met-PRT380

<400> 6

Met Gly Pro Gly Gln Gln Gly Pro Tyr Gly Pro Gly Ala Ser Ala Ala
1               5                   10              15

Ala Ala Ala Gly Gln Asn Gly Pro Gly Ser Gly Gln Gln Gly Pro Gly
            20                  25                  30

Gln Ser Ala Ala Ala Ala Ala Gly Gln Tyr Gly Pro Gly Gln Gln Gly
        35                  40                  45

Pro Gly Gln Gln Gly Pro Gly Ser Ser Ala Ala Ala Ala Ala Gly Pro
        50                  55                  60

Gly Gln Tyr Gly Pro Gly Gln Gln Gly Pro Ser Ala Ser Ala Ala Ala
65                  70                  75                  80

Ala Ala Gly Pro Gly Ser Gly Gln Gln Gly Pro Gly Ala Ser Ala Ala
                85                  90                  95

Ala Ala Ala Gly Gln Tyr Gly Pro Gly Gln Gln Gly Pro Gly Gln Gln
            100                 105                 110

Gly Pro Gly Ser Ser Ala Ala Ala Ala Ala Gly Gln Tyr Gly Ser Gly
        115                 120                 125

Pro Gly Gln Gln Gly Pro Tyr Gly Ser Ala Ala Ala Ala Ala Gly Pro
        130                 135                 140

Gly Ser Gly Gln Tyr Gly Gln Gly Pro Tyr Gly Pro Gly Ala Ser Ala
145                 150                 155                 160

Ala Ala Ala Gly Pro Gly Gln Tyr Gly Pro Gly Gln Gln Gly Pro
                165                 170                 175

Ser Ala Ser Ala Ala Ala Ala Ala Gly Ser Gly Gln Gln Gly Pro Gly
        180                 185                 190

Gln Tyr Gly Pro Tyr Ala Ser Ala Ala Ala Ala Ala Gly Gln Tyr Gly
        195                 200                 205

Ser Gly Pro Gly Gln Gln Gly Pro Tyr Gly Pro Gly Gln Ser Ala Ala
        210                 215                 220

Ala Ala Ala Gly Ser Gly Gln Gln Gly Pro Gly Gln Gln Gly Pro Tyr
225                 230                 235                 240

Ala Ser Ala Ala Ala Ala Ala Gly Pro Gly Gln Gln Gly Pro Tyr Gly
        245                 250                 255

Pro Gly Ser Ser Ala Ala Ala Ala Ala Gly Gln Tyr Gly Tyr Gly Pro
        260                 265                 270

Gly Gln Gln Gly Pro Tyr Gly Pro Gly Ala Ser Ala Ala Ala Ala Ala
        275                 280                 285

Gly Gln Asn Gly Pro Gly Ser Gly Gln Tyr Gly Pro Gly Gln Gln Gly
        290                 295                 300

Pro Gly Gln Ser Ala Ala Ala Ala Ala Gly Pro Gly Gln Gln Gly Pro
305                 310                 315                 320

Tyr Gly Pro Gly Ala Ser Ala Ala Ala Ala Ala Gly Gln Tyr Gly Pro
        325                 330                 335

Gly Gln Gln Gly Pro Gly Gln Tyr Gly Pro Gly Ser Ser Ala Ala Ala
        340                 345                 350

Ala Ala Gly Pro Gly Gln Gln Gly Pro Tyr Gly Pro Gly Ser Ser Ala
        355                 360                 365

Ala Ala Ala Gly Gln Tyr Gly Pro Gly Gln Gln Gly Pro Tyr Gly
        370                 375                 380

Pro Gly Gln Ser Ala Ala Ala Ala Ala Gly Gln Tyr Gln Gln Gly Pro
385                 390                 395                 400

Gly Gln Gln Gly Pro Tyr Gly Pro Gly Ala Ser Ala Ala Ala Ala Ala
        405                 410                 415

Gly Pro Gly Gln Gln Gly Pro Tyr Gly Pro Gly Ala Ser Ala Ala Ala
        420                 425                 430

48

```
Ala Ala Gly Pro Gly Gln Tyr Gly Pro Gly Gln Gln Gly Pro Ser Ala
        435                 440             445

Ser Ala Ala Ala Ala Ala Gly Gln Tyr Gly Ser Gly Pro Gly Gln Tyr
        450                 455             460

Gly Pro Tyr Gly Pro Gly Gln Ser Ala Ala Ala Ala Gly Pro Gly
465                 470             475             480

Ser Gly Gln Gln Gly Gln Gly Pro Tyr Gly Pro Gly Ala Ser Ala Ala
                485             490             495

Ala Ala Ala Gly Gln Tyr Gly Pro Gly Gln Gln Gly Pro Tyr Gly Pro
        500             505             510

Gly Gln Ser Ala Ala Ala Ala Ala Gly Pro Gly Ser Gly Gln Tyr Gly
        515             520             525

Pro Gly Ala Ser Ala Ala Ala Ala Gly Gln Asn Gly Pro Gly Ser
    530             535             540

Gly Gln Tyr Gly Pro Gly Gln Gln Gly Pro Gly Gln Ser Ala Ala Ala
545                 550             555             560

Ala Ala Gly Gln Tyr Gln Gln Gly Pro Gly Gln Gln Gly Pro Tyr Gly
            565             570             575

Pro Gly Ala Ser Ala Ala Ala Ala Ala Gly Pro Gly Ser Gly Gln Gln
        580             585             590

Gly Pro Gly Ala Ser
        595
```

<210>    7
<211>    590
<212>    PRT
<213>    Artificial Sequence

<220>
<223>    Met-PRT410

<400>    7

```
Met Gly Pro Gly Gln Gln Gly Pro Tyr Gly Pro Gly Ala Ser Ala Ala
1               5               10              15

Ala Ala Ala Gly Gln Asn Gly Pro Gly Ser Gly Gln Gln Gly Pro Gly
            20              25              30
```

```
Gln Ser Gly Gln Tyr Gly Pro Gly Gln Gln Gly Pro Gly Gln Gln Gly
        35              40              45

Pro Gly Ser Ser Ala Ala Ala Ala Ala Gly Pro Gly Gln Tyr Gly Pro
        50              55              60

Gly Gln Gln Gly Pro Ser Ala Ser Ala Ala Ala Ala Gly Pro Gly
65              70              75              80

Ser Gly Gln Gln Gly Pro Gly Ala Ser Gly Gln Tyr Gly Pro Gly Gln
                85              90              95

Gln Gly Pro Gly Gln Gln Gly Pro Gly Ser Ser Ala Ala Ala Ala Ala
            100             105             110

Gly Gln Tyr Gly Ser Gly Pro Gly Gln Gln Gly Pro Tyr Gly Ser Ala
        115             120             125

Ala Ala Ala Ala Gly Pro Gly Ser Gly Gln Tyr Gly Gln Gly Pro Tyr
    130             135             140

Gly Pro Gly Ala Ser Gly Pro Gly Gln Tyr Gly Pro Gly Gln Gln Gly
145             150             155             160

Pro Ser Ala Ser Ala Ala Ala Ala Gly Ser Gly Gln Gln Gly Pro
            165             170             175

Gly Gln Tyr Gly Pro Tyr Ala Ser Ala Ala Ala Ala Gly Gln Tyr
        180             185             190

Gly Ser Gly Pro Gly Gln Gln Gly Pro Tyr Gly Pro Gly Gln Ser Gly
        195             200             205

Ser Gly Gln Gln Gly Pro Gly Gln Gln Gly Pro Tyr Ala Ser Ala Ala
    210             215             220

Ala Ala Ala Gly Pro Gly Gln Gln Gly Pro Tyr Gly Pro Gly Ser Ser
225             230             235             240

Ala Ala Ala Ala Ala Gly Gln Tyr Gly Tyr Gly Pro Gly Gln Gln Gly
            245             250             255

Pro Tyr Gly Pro Gly Ala Ser Gly Gln Asn Gly Pro Gly Ser Gly Gln
        260             265             270

Tyr Gly Pro Gly Gln Gln Gly Pro Gly Gln Ser Ala Ala Ala Ala Ala
        275             280             285
```

Gly Pro Gly Gln Gln Gly Pro Tyr Gly Pro Gly Ala Ser Ala Ala Ala
    290                 295                 300

Ala Ala Gly Gln Tyr Gly Pro Gly Gln Gln Gly Pro Gly Gln Tyr Gly
    305                 310                 315                 320

Pro Gly Ser Ser Gly Pro Gly Gln Gln Gly Pro Tyr Gly Pro Gly Ser
            325                 330                 335

Ser Ala Ala Ala Ala Ala Gly Gln Tyr Gly Pro Gly Gln Gln Gly Pro
            340                 345                 350

Tyr Gly Pro Gly Gln Ser Ala Ala Ala Ala Ala Gly Gln Tyr Gln Gln
        355                 360                 365

Gly Pro Gly Gln Gln Gly Pro Tyr Gly Pro Gly Ala Ser Gly Pro Gly
    370                 375                 380

Gln Gln Gly Pro Tyr Gly Pro Gly Ala Ser Ala Ala Ala Ala Ala Gly
385                 390                 395                 400

Pro Gly Gln Tyr Gly Pro Gly Gln Gln Gly Pro Ser Ala Ser Ala Ala
            405                 410                 415

Ala Ala Ala Gly Gln Tyr Gly Ser Gly Pro Gly Gln Tyr Gly Pro Tyr
        420                 425                 430

Gly Pro Gly Gln Ser Gly Pro Gly Ser Gly Gln Gln Gly Gln Gly Pro
        435                 440                 445

Tyr Gly Pro Gly Ala Ser Ala Ala Ala Ala Gly Gln Tyr Gly Pro
        450                 455                 460

Gly Gln Gln Gly Pro Tyr Gly Pro Gly Gln Ser Ala Ala Ala Ala Ala
465                 470                 475                 480

Gly Pro Gly Ser Gly Gln Tyr Gly Pro Gly Ala Ser Gly Gln Asn Gly
            485                 490                 495

Pro Gly Ser Gly Gln Tyr Gly Pro Gly Gln Gln Gly Pro Gly Gln Ser
            500                 505                 510

Ala Ala Ala Ala Ala Gly Gln Tyr Gln Gln Gly Pro Gly Gln Gln Gly
        515                 520                 525

Pro Tyr Gly Pro Gly Ala Ser Ala Ala Ala Ala Ala Gly Gln Tyr Gly

51

```
            530                    535                      540


        Ser Gly Pro Gly Gln Gln Gly Pro Tyr Gly Pro Gly Gln Ser Gly Ser
        545                   550                   555                   560


        Gly Gln Gln Gly Pro Gly Gln Gln Gly Pro Tyr Ala Ser Ala Ala Ala
                          565                   570                   575


        Ala Ala Gly Pro Gly Ser Gly Gln Gln Gly Pro Gly Ala Ser
                      580                   585                   590


        <210>   8
        <211>   565
        <212>   PRT
        <213>   Artificial Sequence

        <220>
        <223>   Met-PRT525

        <400>   8

        Met Gly Pro Gly Gln Gln Gly Pro Tyr Gly Pro Gly Ala Ser Ala Ala
        1                   5                   10                    15


        Ala Ala Ala Ala Ala Gly Ser Asn Gly Pro Gly Ser Gly Gln Gln Gly
                      20                    25                    30


        Pro Gly Gln Ser Gly Gln Tyr Gly Pro Gly Gln Gln Gly Pro Gly Gln
                  35                    40                    45


        Gln Gly Pro Gly Ser Ser Ala Ala Ala Ala Ala Ala Gly Pro Gly
                  50                    55                    60


        Gln Tyr Gly Pro Gly Gln Gln Gly Pro Ser Ala Ser Ala Ala Ala Ala
        65                    70                    75                    80


        Ala Ala Ala Gly Pro Gly Ser Gly Gln Gln Gly Pro Gly Ala Ser Gly
                      85                    90                    95


        Gln Tyr Gly Pro Gly Gln Gln Gly Pro Gly Gln Gln Gly Pro Gly Ser
                  100                   105                   110


        Ser Ala Ala Ala Ala Ala Ala Ala Gly Ser Tyr Gly Ser Gly Pro Gly
                  115                   120                   125


        Gln Gln Gly Pro Tyr Gly Ser Ala Ala Ala Ala Ala Ala Ala Gly Pro
                  130                   135                   140


        Gly Ser Gly Gln Tyr Gly Gln Gly Pro Tyr Gly Pro Gly Ala Ser Gly
        145                   150                   155                   160
```

Pro Gly Gln Tyr Gly Pro Gly Gln Gln Gly Pro Ser Ala Ser Ala Ala
165 170 175

Ala Ala Ala Ala Ala Gly Ser Gly Gln Gln Gly Pro Gly Gln Tyr Gly
180 185 190

Pro Tyr Ala Ser Ala Ala Ala Ala Ala Ala Ala Gly Ser Tyr Gly Ser
195 200 205

Gly Pro Gly Gln Gln Gly Pro Tyr Gly Pro Gly Gln Ser Gly Ser Gly
210 215 220

Gln Gln Gly Pro Gly Gln Gln Gly Pro Tyr Ala Ser Ala Ala Ala Ala
225 230 235 240

Ala Ala Ala Gly Pro Gly Gln Gln Gly Pro Tyr Gly Pro Gly Ser Ser
245 250 255

Ala Ala Ala Ala Ala Ala Ala Gly Ser Tyr Gly Tyr Gly Pro Gly Gln
260 265 270

Gln Gly Pro Tyr Gly Pro Gly Ala Ser Gly Gln Asn Gly Pro Gly Ser
275 280 285

Gly Gln Tyr Gly Pro Gly Gln Gln Gly Pro Gly Pro Ser Ala Ala Ala
290 295 300

Ala Ala Ala Ala Gly Pro Gly Gln Gln Gly Pro Tyr Gly Pro Gly Ala
305 310 315 320

Ser Ala Ala Ala Ala Ala Ala Ala Gly Ser Tyr Gly Pro Gly Gln Gln
325 330 335

Gly Pro Gly Gln Tyr Gly Pro Gly Ser Ser Gly Pro Gly Gln Gln Gly
340 345 350

Pro Tyr Gly Pro Gly Ser Ser Ala Ala Ala Ala Ala Ala Ala Gly Ser
355 360 365

Tyr Gly Pro Gly Gln Gln Gly Pro Tyr Gly Pro Gly Pro Ser Ala Ala
370 375 380

Ala Ala Ala Ala Ala Gly Ser Tyr Gln Gln Gly Pro Gly Gln Gln Gly
385 390 395 400

Pro Tyr Gly Pro Gly Ala Ser Gly Pro Gly Gln Gln Gly Pro Tyr Gly

```
                            405                      410                         415


        Pro Gly Ala Ser Ala Ala Ala Ala Ala Ala Ala Gly Pro Gly Gln Tyr
                    420                 425                 430


        Gly Pro Gly Gln Gln Gly Pro Ser Ala Ser Ala Ala Ala Ala Ala Ala
                    435                 440                 445


        Ala Gly Ser Tyr Gly Ser Gly Pro Gly Gln Tyr Gly Pro Tyr Gly Pro
                    450                 455                 460


        Gly Gln Ser Gly Pro Gly Ser Gly Gln Gln Gly Gln Gly Pro Tyr Gly
        465                 470                 475                 480


        Pro Gly Ala Ser Ala Ala Ala Ala Ala Ala Ala Gly Ser Tyr Gly Pro
                    485                 490                 495


        Gly Gln Gln Gly Pro Tyr Gly Pro Gly Pro Ser Ala Ala Ala Ala Ala
                    500                 505                 510


        Ala Ala Gly Pro Gly Ser Gly Gln Tyr Gly Pro Gly Ala Ser Gly Gln
                    515                 520                 525


        Asn Gly Pro Gly Ser Gly Gln Tyr Gly Pro Gly Gln Gln Gly Pro Gly
                    530                 535                 540


        Pro Ser Ala Ala Ala Ala Ala Ala Ala Gly Pro Gly Ser Gly Gln Gln
        545                 550                 555                 560


        Gly Pro Gly Ala Ser
                    565


        <210>   9
        <211>   2364
        <212>   PRT
        <213>   Artificial Sequence

        <220>
        <223>   Met-PRT799

        <400>   9

        Met Gly Pro Gly Gln Gln Gly Pro Tyr Gly Pro Gly Ala Ser Ala Ala
        1                   5                   10                  15


        Ala Ala Ala Gly Gln Asn Gly Pro Gly Ser Gly Gln Gln Gly Pro Gly
                    20                  25                  30


        Gln Ser Gly Gln Tyr Gly Pro Gly Gln Gln Gly Pro Gly Gln Gln Gly
                    35                  40                  45
```

```
Pro Gly Ser Ser Ala Ala Ala Ala Ala Gly Pro Gly Gln Tyr Gly Pro
    50              55                  60

Gly Gln Gln Gly Pro Ser Ala Ser Ala Ala Ala Ala Gly Pro Gly
65              70                  75                  80

Ser Gly Gln Gln Gly Pro Gly Ala Ser Gly Gln Tyr Gly Pro Gly Gln
            85                  90                  95

Gln Gly Pro Gly Gln Gln Gly Pro Gly Ser Ser Ala Ala Ala Ala Ala
            100             105             110

Gly Gln Tyr Gly Ser Gly Pro Gly Gln Gln Gly Pro Tyr Gly Ser Ala
        115                 120             125

Ala Ala Ala Ala Gly Pro Gly Ser Gly Gln Tyr Gly Gln Gly Pro Tyr
    130                 135                 140

Gly Pro Gly Ala Ser Gly Pro Gly Gln Tyr Gly Pro Gly Gln Gln Gly
145                 150                 155                 160

Pro Ser Ala Ser Ala Ala Ala Ala Ala Gly Ser Gly Gln Gln Gly Pro
            165                 170                 175

Gly Gln Tyr Gly Pro Tyr Ala Ser Ala Ala Ala Ala Gly Gln Tyr
        180                 185                 190

Gly Ser Gly Pro Gly Gln Gln Gly Pro Tyr Gly Pro Gly Gln Ser Gly
        195                 200                 205

Ser Gly Gln Gln Gly Pro Gly Gln Gln Gly Pro Tyr Ala Ser Ala Ala
    210                 215                 220

Ala Ala Ala Gly Pro Gly Gln Gln Gly Pro Tyr Gly Pro Gly Ser Ser
225                 230                 235                 240

Ala Ala Ala Ala Ala Gly Gln Tyr Gly Tyr Gly Pro Gly Gln Gln Gly
            245                 250                 255

Pro Tyr Gly Pro Gly Ala Ser Gly Gln Asn Gly Pro Gly Ser Gly Gln
        260                 265                 270

Tyr Gly Pro Gly Gln Gln Gly Pro Gly Gln Ser Ala Ala Ala Ala Ala
        275                 280                 285

Gly Pro Gly Gln Gln Gly Pro Tyr Gly Pro Gly Ala Ser Ala Ala Ala
```

```
        290                    295                    300


Ala Ala Gly Gln Tyr Gly Pro Gly Gln Gln Gly Pro Gly Gln Tyr Gly
305                 310                 315                 320


Pro Gly Ser Ser Gly Pro Gly Gln Gln Gly Pro Tyr Gly Pro Gly Ser
            325                 330                 335


Ser Ala Ala Ala Ala Ala Gly Gln Tyr Gly Pro Gly Gln Gln Gly Pro
        340                 345                 350


Tyr Gly Pro Gly Gln Ser Ala Ala Ala Ala Ala Gly Gln Tyr Gln Gln
        355                 360                 365


Gly Pro Gly Gln Gln Gly Pro Tyr Gly Pro Gly Ala Ser Gly Pro Gly
    370                 375                 380


Gln Gln Gly Pro Tyr Gly Pro Gly Ala Ser Ala Ala Ala Ala Ala Gly
385                 390                 395                 400


Pro Gly Gln Tyr Gly Pro Gly Gln Gln Gly Pro Ser Ala Ser Ala Ala
            405                 410                 415


Ala Ala Ala Gly Gln Tyr Gly Ser Gly Pro Gly Gln Tyr Gly Pro Tyr
        420                 425                 430


Gly Pro Gly Gln Ser Gly Pro Gly Ser Gly Gln Gln Gly Gln Gly Pro
    435                 440                 445


Tyr Gly Pro Gly Ala Ser Ala Ala Ala Ala Gly Gln Tyr Gly Pro
    450                 455                 460


Gly Gln Gln Gly Pro Tyr Gly Pro Gly Gln Ser Ala Ala Ala Ala Ala
465                 470                 475                 480


Gly Pro Gly Ser Gly Gln Tyr Gly Pro Gly Ala Ser Gly Gln Asn Gly
            485                 490                 495


Pro Gly Ser Gly Gln Tyr Gly Pro Gly Gln Gln Gly Pro Gly Gln Ser
            500                 505                 510


Ala Ala Ala Ala Ala Gly Gln Tyr Gln Gln Gly Pro Gly Gln Gln Gly
        515                 520                 525


Pro Tyr Gly Pro Gly Ala Ser Ala Ala Ala Ala Ala Gly Gln Tyr Gly
    530                 535                 540
```

```
Ser Gly Pro Gly Gln Gln Gly Pro Tyr Gly Pro Gly Gln Ser Gly Ser
545                 550             555             560

Gly Gln Gln Gly Pro Gly Gln Gln Gly Pro Tyr Ala Ser Ala Ala Ala
                565             570             575

Ala Ala Gly Pro Gly Ser Gly Gln Gln Gly Pro Gly Ala Ser Gly Gln
            580             585             590

Gln Gly Pro Tyr Gly Pro Gly Ala Ser Ala Ala Ala Ala Ala Gly Gln
        595             600             605

Asn Gly Pro Gly Ser Gly Gln Gln Gly Pro Gly Gln Ser Gly Gln Tyr
        610             615             620

Gly Pro Gly Gln Gln Gly Pro Gly Gln Gln Gly Pro Gly Ser Ser Ala
625             630             635             640

Ala Ala Ala Ala Gly Pro Gly Gln Tyr Gly Pro Gly Gln Gln Gly Pro
            645             650             655

Ser Ala Ser Ala Ala Ala Ala Ala Gly Pro Gly Ser Gly Gln Gln Gly
        660             665             670

Pro Gly Ala Ser Gly Gln Tyr Gly Pro Gly Gln Gln Gly Pro Gly Gln
        675             680             685

Gln Gly Pro Gly Ser Ser Ala Ala Ala Ala Gly Gln Tyr Gly Ser
        690             695             700

Gly Pro Gly Gln Gln Gly Pro Tyr Gly Ser Ala Ala Ala Ala Ala Gly
705             710             715             720

Pro Gly Ser Gly Gln Tyr Gly Gln Gly Pro Tyr Gly Pro Gly Ala Ser
            725             730             735

Gly Pro Gly Gln Tyr Gly Pro Gly Gln Gln Gly Pro Ser Ala Ser Ala
            740             745             750

Ala Ala Ala Ala Gly Ser Gly Gln Gln Gly Pro Gly Gln Tyr Gly Pro
        755             760             765

Tyr Ala Ser Ala Ala Ala Ala Ala Gly Gln Tyr Gly Ser Gly Pro Gly
        770             775             780

Gln Gln Gly Pro Tyr Gly Pro Gly Gln Ser Gly Ser Gly Gln Gln Gly
785             790             795             800
```

57

Pro Gly Gln Gln Gly Pro Tyr Ala Ser Ala Ala Ala Ala Ala Gly Pro
805 810 815

Gly Gln Gln Gly Pro Tyr Gly Pro Gly Ser Ser Ala Ala Ala Ala Ala
820 825 830

Gly Gln Tyr Gly Tyr Gly Pro Gly Gln Gln Gly Pro Tyr Gly Pro Gly
835 840 845

Ala Ser Gly Gln Asn Gly Pro Gly Ser Gly Gln Tyr Gly Pro Gly Gln
850 855 860

Gln Gly Pro Gly Gln Ser Ala Ala Ala Ala Ala Gly Pro Gly Gln Gln
865 870 875 880

Gly Pro Tyr Gly Pro Gly Ala Ser Ala Ala Ala Ala Ala Gly Gln Tyr
885 890 895

Gly Pro Gly Gln Gln Gly Pro Gly Gln Tyr Gly Pro Gly Ser Ser Gly
900 905 910

Pro Gly Gln Gln Gly Pro Tyr Gly Pro Gly Ser Ser Ala Ala Ala Ala
915 920 925

Ala Gly Gln Tyr Gly Pro Gly Gln Gln Gly Pro Tyr Gly Pro Gly Gln
930 935 940

Ser Ala Ala Ala Ala Ala Gly Gln Tyr Gln Gln Gly Pro Gly Gln Gln
945 950 955 960

Gly Pro Tyr Gly Pro Gly Ala Ser Gly Pro Gly Gln Gln Gly Pro Tyr
965 970 975

Gly Pro Gly Ala Ser Ala Ala Ala Ala Gly Pro Gly Gln Tyr Gly
980 985 990

Pro Gly Gln Gln Gly Pro Ser Ala Ser Ala Ala Ala Ala Ala Gly Gln
995 1000 1005

Tyr Gly Ser Gly Pro Gly Gln Tyr Gly Pro Tyr Gly Pro Gly Gln
1010 1015 1020

Ser Gly Pro Gly Ser Gly Gln Gln Gly Gln Gly Pro Tyr Gly Pro
1025 1030 1035

Gly Ala Ser Ala Ala Ala Ala Ala Gly Gln Tyr Gly Pro Gly Gln
1040 1045 1050

58

```
Gln Gly Pro Tyr Gly Pro Gly  Gln Ser Ala Ala Ala  Ala Ala Gly
    1055              1060              1065

Pro Gly Ser Gly Gln Tyr Gly  Pro Gly Ala Ser Gly  Gln Asn Gly
    1070              1075              1080

Pro Gly Ser Gly Gln Tyr Gly  Pro Gly Gln Gln Gly  Pro Gly Gln
    1085              1090              1095

Ser Ala Ala Ala Ala Ala Gly  Gln Tyr Gln Gln Gly  Pro Gly Gln
    1100              1105              1110

Gln Gly Pro Tyr Gly Pro Gly  Ala Ser Ala Ala Ala  Ala Ala Gly
    1115              1120              1125

Gln Tyr Gly Ser Gly Pro Gly  Gln Gln Gly Pro Tyr  Gly Pro Gly
    1130              1135              1140

Gln Ser Gly Ser Gly Gln Gln  Gly Pro Gly Gln Gln  Gly Pro Tyr
    1145              1150              1155

Ala Ser Ala Ala Ala Ala Ala  Gly Pro Gly Ser Gly  Gln Gln Gly
    1160              1165              1170

Pro Gly Ala Ser Gly Gln Gln  Gly Pro Tyr Gly Pro  Gly Ala Ser
    1175              1180              1185

Ala Ala Ala Ala Ala Gly Gln  Asn Gly Pro Gly Ser  Gly Gln Gln
    1190              1195              1200

Gly Pro Gly Gln Ser Gly Gln  Tyr Gly Pro Gly Gln  Gln Gly Pro
    1205              1210              1215

Gly Gln Gln Gly Pro Gly Ser  Ser Ala Ala Ala Ala  Ala Gly Pro
    1220              1225              1230

Gly Gln Tyr Gly Pro Gly Gln  Gln Gly Pro Ser Ala  Ser Ala Ala
    1235              1240              1245

Ala Ala Ala Gly Pro Gly Ser  Gly Gln Gln Gly Pro  Gly Ala Ser
    1250              1255              1260

Gly Gln Tyr Gly Pro Gly Gln  Gln Gly Pro Gly Gln  Gln Gly Pro
    1265              1270              1275

Gly Ser Ser Ala Ala Ala Ala  Ala Gly Gln Tyr Gly  Ser Gly Pro
```

```
          1280                          1285                          1290


          Gly Gln   Gln Gly Pro Tyr Gly   Ser Ala Ala Ala Ala   Ala Gly Pro
              1295                  1300                  1305


          Gly Ser   Gly Gln Tyr Gly Gln   Gly Pro Tyr Gly Pro   Gly Ala Ser
              1310                  1315                  1320


          Gly Pro   Gly Gln Tyr Gly Pro   Gly Gln Gln Gly Pro   Ser Ala Ser
              1325                  1330                  1335


          Ala Ala   Ala Ala Ala Gly Ser   Gly Gln Gln Gly Pro   Gly Gln Tyr
              1340                  1345                  1350


          Gly Pro   Tyr Ala Ser Ala Ala   Ala Ala Ala Gly Gln   Tyr Gly Ser
              1355                  1360                  1365


          Gly Pro   Gly Gln Gln Gly Pro   Tyr Gly Pro Gly Gln   Ser Gly Ser
              1370                  1375                  1380


          Gly Gln   Gln Gly Pro Gly Gln   Gln Gly Pro Tyr Ala   Ser Ala Ala
              1385                  1390                  1395


          Ala Ala   Ala Gly Pro Gly Gln   Gln Gly Pro Tyr Gly   Pro Gly Ser
              1400                  1405                  1410


          Ser Ala   Ala Ala Ala Ala Gly   Gln Tyr Gly Tyr Gly   Pro Gly Gln
              1415                  1420                  1425


          Gln Gly   Pro Tyr Gly Pro Gly   Ala Ser Gly Gln Asn   Gly Pro Gly
              1430                  1435                  1440


          Ser Gly   Gln Tyr Gly Pro Gly   Gln Gln Gly Pro Gly   Gln Ser Ala
              1445                  1450                  1455


          Ala Ala   Ala Ala Gly Pro Gly   Gln Gln Gly Pro Tyr   Gly Pro Gly
              1460                  1465                  1470


          Ala Ser   Ala Ala Ala Ala Ala   Gly Gln Tyr Gly Pro   Gly Gln Gln
              1475                  1480                  1485


          Gly Pro   Gly Gln Tyr Gly Pro   Gly Ser Ser Gly Pro   Gly Gln Gln
              1490                  1495                  1500


          Gly Pro   Tyr Gly Pro Gly Ser   Ser Ala Ala Ala Ala   Ala Gly Gln
              1505                  1510                  1515
```

```
Tyr Gly  Pro Gly Gln Gln Gly   Pro Tyr Gly Pro Gly   Gln Ser Ala
    1520             1525               1530


Ala Ala  Ala Ala Gly Gln Tyr   Gln Gln Gly Pro Gly   Gln Gln Gly
    1535             1540               1545


Pro Tyr  Gly Pro Gly Ala Ser   Gly Pro Gly Gln Gln   Gly Pro Tyr
    1550             1555               1560


Gly Pro  Gly Ala Ser Ala Ala   Ala Ala Ala Gly Pro   Gly Gln Tyr
    1565             1570               1575


Gly Pro  Gly Gln Gln Gly Pro   Ser Ala Ser Ala Ala   Ala Ala Ala
    1580             1585               1590


Gly Gln  Tyr Gly Ser Gly Pro   Gly Gln Tyr Gly Pro   Tyr Gly Pro
    1595             1600               1605


Gly Gln  Ser Gly Pro Gly Ser   Gly Gln Gln Gly Gln   Gly Pro Tyr
    1610             1615               1620


Gly Pro  Gly Ala Ser Ala Ala   Ala Ala Ala Gly Gln   Tyr Gly Pro
    1625             1630               1635


Gly Gln  Gln Gly Pro Tyr Gly   Pro Gly Gln Ser Ala   Ala Ala Ala
    1640             1645               1650


Ala Gly  Pro Gly Ser Gly Gln   Tyr Gly Pro Gly Ala   Ser Gly Gln
    1655             1660               1665


Asn Gly  Pro Gly Ser Gly Gln   Tyr Gly Pro Gly Gln   Gln Gly Pro
    1670             1675               1680


Gly Gln  Ser Ala Ala Ala Ala   Ala Gly Gln Tyr Gln   Gln Gly Pro
    1685             1690               1695


Gly Gln  Gln Gly Pro Tyr Gly   Pro Gly Ala Ser Ala   Ala Ala Ala
    1700             1705               1710


Ala Gly  Gln Tyr Gly Ser Gly   Pro Gly Gln Gln Gly   Pro Tyr Gly
    1715             1720               1725


Pro Gly  Gln Ser Gly Ser Gly   Gln Gln Gly Pro Gly   Gln Gln Gly
    1730             1735               1740


Pro Tyr  Ala Ser Ala Ala Ala   Ala Ala Gly Pro Gly   Ser Gly Gln
    1745             1750               1755
```

```
Gln Gly Pro Gly Ala Ser Gly Gln Gln Gly Pro Tyr Gly Pro Gly
    1760              1765              1770

Ala Ser Ala Ala Ala Ala Ala Gly Gln Asn Gly Pro Gly Ser Gly
    1775              1780              1785

Gln Gln Gly Pro Gly Gln Ser Gly Gln Tyr Gly Pro Gly Gln Gln
    1790              1795              1800

Gly Pro Gly Gln Gln Gly Pro Gly Ser Ser Ala Ala Ala Ala Ala
    1805              1810              1815

Gly Pro Gly Gln Tyr Gly Pro Gly Gln Gln Gly Pro Ser Ala Ser
    1820              1825              1830

Ala Ala Ala Ala Ala Gly Pro Gly Ser Gly Gln Gln Gly Pro Gly
    1835              1840              1845

Ala Ser Gly Gln Tyr Gly Pro Gly Gln Gln Gly Pro Gly Gln Gln
    1850              1855              1860

Gly Pro Gly Ser Ser Ala Ala Ala Ala Ala Gly Gln Tyr Gly Ser
    1865              1870              1875

Gly Pro Gly Gln Gln Gly Pro Tyr Gly Ser Ala Ala Ala Ala Ala
    1880              1885              1890

Gly Pro Gly Ser Gly Gln Tyr Gly Gln Gly Pro Tyr Gly Pro Gly
    1895              1900              1905

Ala Ser Gly Pro Gly Gln Tyr Gly Pro Gly Gln Gln Gly Pro Ser
    1910              1915              1920

Ala Ser Ala Ala Ala Ala Ala Gly Ser Gly Gln Gln Gly Pro Gly
    1925              1930              1935

Gln Tyr Gly Pro Tyr Ala Ser Ala Ala Ala Ala Ala Gly Gln Tyr
    1940              1945              1950

Gly Ser Gly Pro Gly Gln Gln Gly Pro Tyr Gly Pro Gly Gln Ser
    1955              1960              1965

Gly Ser Gly Gln Gln Gly Pro Gly Gln Gln Gly Pro Tyr Ala Ser
    1970              1975              1980

Ala Ala Ala Ala Ala Gly Pro Gly Gln Gln Gly Pro Tyr Gly Pro
    1985              1990              1995
```

```
Gly Ser  Ser Ala Ala Ala Ala  Ala Gly Gln Tyr Gly  Tyr Gly Pro
    2000                 2005                 2010

Gly Gln  Gln Gly Pro Tyr Gly  Pro Gly Ala Ser Gly  Gln Asn Gly
    2015                 2020                 2025

Pro Gly  Ser Gly Gln Tyr Gly  Pro Gly Gln Gln Gly  Pro Gly Gln
    2030                 2035                 2040

Ser Ala  Ala Ala Ala Ala Gly  Pro Gly Gln Gln Gly  Pro Tyr Gly
    2045                 2050                 2055

Pro Gly  Ala Ser Ala Ala Ala  Ala Ala Gly Gln Tyr  Gly Pro Gly
    2060                 2065                 2070

Gln Gln  Gly Pro Gly Gln Tyr  Gly Pro Gly Ser Ser  Gly Pro Gly
    2075                 2080                 2085

Gln Gln  Gly Pro Tyr Gly Pro  Gly Ser Ser Ala Ala  Ala Ala Ala
    2090                 2095                 2100

Gly Gln  Tyr Gly Pro Gly Gln  Gln Gly Pro Tyr Gly  Pro Gly Gln
    2105                 2110                 2115

Ser Ala  Ala Ala Ala Ala Gly  Gln Tyr Gln Gln Gly  Pro Gly Gln
    2120                 2125                 2130

Gln Gly  Pro Tyr Gly Pro Gly  Ala Ser Gly Pro Gly  Gln Gln Gly
    2135                 2140                 2145

Pro Tyr  Gly Pro Gly Ala Ser  Ala Ala Ala Ala Ala  Gly Pro Gly
    2150                 2155                 2160

Gln Tyr  Gly Pro Gly Gln Gln  Gly Pro Ser Ala Ser  Ala Ala Ala
    2165                 2170                 2175

Ala Ala  Gly Gln Tyr Gly Ser  Gly Pro Gly Gln Tyr  Gly Pro Tyr
    2180                 2185                 2190

Gly Pro  Gly Gln Ser Gly Pro  Gly Ser Gly Gln Gln  Gly Gln Gly
    2195                 2200                 2205

Pro Tyr  Gly Pro Gly Ala Ser  Ala Ala Ala Ala Ala  Gly Gln Tyr
    2210                 2215                 2220

Gly Pro  Gly Gln Gln Gly Pro  Tyr Gly Pro Gly Gln  Ser Ala Ala
```

        2225                 2230                 2235

```
Ala Ala  Ala Gly Pro Gly Ser  Gly Gln Tyr Gly Pro  Gly Ala Ser
    2240                 2245                 2250

Gly Gln  Asn Gly Pro Gly Ser  Gly Gln Tyr Gly Pro  Gly Gln Gln
    2255                 2260                 2265

Gly Pro  Gly Gln Ser Ala Ala  Ala Ala Ala Gly Gln  Tyr Gln Gln
    2270                 2275                 2280

Gly Pro  Gly Gln Gln Gly Pro  Tyr Gly Pro Gly Ala  Ser Ala Ala
    2285                 2290                 2295

Ala Ala  Ala Gly Gln Tyr Gly  Ser Gly Pro Gly Gln  Gln Gly Pro
    2300                 2305                 2310

Tyr Gly  Pro Gly Gln Ser Gly  Ser Gly Gln Gln Gly  Pro Gly Gln
    2315                 2320                 2325

Gln Gly  Pro Tyr Ala Ser Ala  Ala Ala Ala Ala Gly  Pro Gly Ser
    2330                 2335                 2340

Gly Gln  Gln Gly Ser Ser Val  Asp Lys Leu Ala Ala  Ala Leu Glu
    2345                 2350                 2355

His His  His His His His
    2360
```

```
<210>  10
<211>  597
<212>  PRT
<213>  Artificial Sequence

<220>
<223>  Met-PRT313

<400>  10

Met Gly Pro Gly Gly Gln Gly Pro Tyr Gly Pro Gly Ala Ser Ala Ala
1               5                  10                 15

Ala Ala Ala Gly Gly Asn Gly Pro Gly Ser Gly Gln Gln Gly Pro Gly
            20                 25                 30

Gly Ser Ala Ala Ala Ala Ala Gly Gly Tyr Gly Pro Gly Gly Gln Gly
        35                 40                 45

Pro Gly Gln Gln Gly Pro Gly Ser Ser Ala Ala Ala Ala Ala Gly Pro
    50                 55                 60
```

Gly Gly Tyr Gly Pro Gly Gly Gln Gly Pro Ser Ala Ser Ala Ala Ala
65                    70              75                    80

Ala Ala Gly Pro Gly Ser Gly Gln Gln Gly Pro Gly Ala Ser Ala Ala
                    85              90                    95

Ala Ala Ala Gly Gly Tyr Gly Pro Gly Gly Gln Gly Pro Gly Gln Gln
            100              105              110

Gly Pro Gly Ser Ser Ala Ala Ala Ala Ala Gly Gly Tyr Gly Ser Gly
        115              120              125

Pro Gly Gln Gln Gly Pro Tyr Gly Ser Ala Ala Ala Ala Ala Gly Pro
    130              135              140

Gly Ser Gly Gly Tyr Gly Gln Gly Pro Tyr Gly Pro Gly Ala Ser Ala
145              150              155              160

Ala Ala Ala Ala Gly Pro Gly Gly Tyr Gly Pro Gly Gly Gln Gly Pro
            165              170              175

Ser Ala Ser Ala Ala Ala Ala Ala Gly Ser Gly Gln Gln Gly Pro Gly
        180              185              190

Gly Tyr Gly Pro Tyr Ala Ser Ala Ala Ala Ala Gly Gly Tyr Gly
        195              200              205

Ser Gly Pro Gly Gln Gln Gly Pro Tyr Gly Pro Gly Gly Ser Ala Ala
    210              215              220

Ala Ala Ala Gly Ser Gly Gln Gln Gly Pro Gly Gln Gln Gly Pro Tyr
225              230              235              240

Ala Ser Ala Ala Ala Ala Ala Gly Pro Gly Gly Gln Gly Pro Tyr Gly
            245              250              255

Pro Gly Ser Ser Ala Ala Ala Ala Ala Gly Gly Tyr Gly Tyr Gly Pro
        260              265              270

Gly Gly Gln Gly Pro Tyr Gly Pro Gly Ala Ser Ala Ala Ala Ala Ala
        275              280              285

Gly Gly Asn Gly Pro Gly Ser Gly Gly Tyr Gly Pro Gly Gln Gln Gly
        290              295              300

Pro Gly Gly Ser Ala Ala Ala Ala Ala Gly Pro Gly Gly Gln Gly Pro

|   | 305 |   |   |   | 310 |   |   |   | 315 |   |   |   | 320 |
|---|---|---|---|---|---|---|---|---|---|---|---|---|---|

Tyr Gly Pro Gly Ala Ser Ala Ala Ala Ala Ala Gly Gly Tyr Gly Pro
          325                330                335

Gly Gly Gln Gly Pro Gly Gly Tyr Gly Pro Gly Ser Ser Ala Ala Ala
          340                345              350

Ala Ala Gly Pro Gly Gly Gln Gly Pro Tyr Gly Pro Gly Ser Ser Ala
          355              360              365

Ala Ala Ala Ala Gly Gly Tyr Gly Pro Gly Gln Gln Gly Pro Tyr Gly
    370              375              380

Pro Gly Gly Ser Ala Ala Ala Ala Gly Gly Tyr Gln Gln Gly Pro
385              390            395              400

Gly Gly Gln Gly Pro Tyr Gly Pro Gly Ala Ser Ala Ala Ala Ala Ala
          405              410              415

Gly Pro Gly Gly Gln Gly Pro Tyr Gly Pro Gly Ala Ser Ala Ala Ala
          420              425              430

Ala Ala Gly Pro Gly Gly Tyr Gly Pro Gly Gly Gln Gly Pro Ser Ala
          435              440            445

Ser Ala Ala Ala Ala Ala Gly Gly Tyr Gly Ser Gly Pro Gly Gly Tyr
          450              455            460

Gly Pro Tyr Gly Pro Gly Gly Ser Ala Ala Ala Ala Ala Gly Pro Gly
465              470            475              480

Ser Gly Gln Gln Gly Gln Gly Pro Tyr Gly Pro Gly Ala Ser Ala Ala
          485              490            495

Ala Ala Ala Gly Gly Tyr Gly Pro Gly Gln Gln Gly Pro Tyr Gly Pro
          500              505            510

Gly Gly Ser Ala Ala Ala Ala Ala Gly Pro Gly Ser Gly Gly Tyr Gly
          515              520            525

Pro Gly Ala Ser Ala Ala Ala Ala Ala Gly Gly Asn Gly Pro Gly Ser
          530              535            540

Gly Gly Tyr Gly Pro Gly Gln Gln Gly Pro Gly Gly Ser Ala Ala Ala
          545              550            555            560

```
Ala Ala Gly Gly Tyr Gln Gln Gly Pro Gly Gly Gln Gly Pro Tyr Gly
                565             570             575

Pro Gly Ala Ser Ala Ala Ala Ala Ala Gly Pro Gly Ser Gly Gln Gln
                580             585             590

Gly Pro Gly Ala Ser
                595


<210> 11
<211> 12
<212> PRT
<213> Artificial Sequence

<220>
<223> HisTag

<400> 11

Met His His His His His His Ser Ser Gly Ser Ser
1               5               10


<210> 12
<211> 608
<212> PRT
<213> Artificial Sequence

<220>
<223> PRT380

<400> 12

Met His His His His His His Ser Ser Gly Ser Ser Gly Pro Gly Gln
1               5               10              15

Gln Gly Pro Tyr Gly Pro Gly Ala Ser Ala Ala Ala Ala Ala Gly Gln
            20              25              30

Asn Gly Pro Gly Ser Gly Gln Gln Gly Pro Gly Gln Ser Ala Ala Ala
            35              40              45

Ala Ala Gly Gln Tyr Gly Pro Gly Gln Gln Gly Pro Gly Gln Gln Gly
    50              55              60

Pro Gly Ser Ser Ala Ala Ala Ala Ala Gly Pro Gly Gln Tyr Gly Pro
65              70              75              80

Gly Gln Gln Gly Pro Ser Ala Ser Ala Ala Ala Ala Ala Gly Pro Gly
                85              90              95

Ser Gly Gln Gln Gly Pro Gly Ala Ser Ala Ala Ala Ala Ala Gly Gln
            100             105             110
```

67

```
Tyr Gly Pro Gly Gln Gln Gly Pro Gly Gln Gln Gly Pro Gly Ser Ser
    115             120             125

Ala Ala Ala Ala Ala Gly Gln Tyr Gly Ser Gly Pro Gly Gln Gln Gly
    130             135             140

Pro Tyr Gly Ser Ala Ala Ala Ala Ala Gly Pro Gly Ser Gly Gln Tyr
145             150             155             160

Gly Gln Gly Pro Tyr Gly Pro Gly Ala Ser Ala Ala Ala Ala Ala Gly
            165             170             175

Pro Gly Gln Tyr Gly Pro Gly Gln Gln Gly Pro Ser Ala Ser Ala Ala
            180             185             190

Ala Ala Ala Gly Ser Gly Gln Gln Gly Pro Gly Gln Tyr Gly Pro Tyr
    195             200             205

Ala Ser Ala Ala Ala Ala Ala Gly Gln Tyr Gly Ser Gly Pro Gly Gln
    210             215             220

Gln Gly Pro Tyr Gly Pro Gly Gln Ser Ala Ala Ala Ala Ala Gly Ser
225             230             235             240

Gly Gln Gln Gly Pro Gly Gln Gln Gly Pro Tyr Ala Ser Ala Ala Ala
            245             250             255

Ala Ala Gly Pro Gly Gln Gln Gly Pro Tyr Gly Pro Gly Ser Ser Ala
            260             265             270

Ala Ala Ala Ala Gly Gln Tyr Gly Tyr Gly Pro Gly Gln Gln Gly Pro
    275             280             285

Tyr Gly Pro Gly Ala Ser Ala Ala Ala Ala Gly Gln Asn Gly Pro
290             295             300

Gly Ser Gly Gln Tyr Gly Pro Gly Gln Gln Gly Pro Gly Gln Ser Ala
305             310             315             320

Ala Ala Ala Gly Pro Gly Gln Gln Gly Pro Tyr Gly Pro Gly Ala
    325             330             335

Ser Ala Ala Ala Ala Ala Gly Gln Tyr Gly Pro Gly Gln Gln Gly Pro
    340             345             350

Gly Gln Tyr Gly Pro Gly Ser Ser Ala Ala Ala Ala Ala Gly Pro Gly
    355             360             365
```

```
Gln Gln Gly Pro Tyr Gly Pro Gly Ser Ser Ala Ala Ala Ala Ala Gly
    370                 375             380

Gln Tyr Gly Pro Gly Gln Gln Gly Pro Tyr Gly Pro Gly Gln Ser Ala
    385                 390             395                 400

Ala Ala Ala Ala Gly Gln Tyr Gln Gln Gly Pro Gly Gln Gln Gly Pro
                405             410                 415

Tyr Gly Pro Gly Ala Ser Ala Ala Ala Ala Ala Gly Pro Gly Gln Gln
            420             425                 430

Gly Pro Tyr Gly Pro Gly Ala Ser Ala Ala Ala Ala Ala Gly Pro Gly
            435             440                 445

Gln Tyr Gly Pro Gly Gln Gln Gly Pro Ser Ala Ser Ala Ala Ala Ala
    450                 455             460

Ala Gly Gln Tyr Gly Ser Gly Pro Gly Gln Tyr Gly Pro Tyr Gly Pro
465             470             475                 480

Gly Gln Ser Ala Ala Ala Ala Ala Gly Pro Gly Ser Gly Gln Gln Gly
            485             490                 495

Gln Gly Pro Tyr Gly Pro Gly Ala Ser Ala Ala Ala Ala Ala Gly Gln
            500             505                 510

Tyr Gly Pro Gly Gln Gln Gly Pro Tyr Gly Pro Gly Gln Ser Ala Ala
            515             520                 525

Ala Ala Ala Gly Pro Gly Ser Gly Gln Tyr Gly Pro Gly Ala Ser Ala
    530                 535             540

Ala Ala Ala Gly Gln Asn Gly Pro Gly Ser Gly Gln Tyr Gly Pro
545             550             555                 560

Gly Gln Gln Gly Pro Gly Gln Ser Ala Ala Ala Ala Ala Gly Gln Tyr
            565             570                 575

Gln Gln Gly Pro Gly Gln Gln Gly Pro Tyr Gly Pro Gly Ala Ser Ala
            580             585                 590

Ala Ala Ala Gly Pro Gly Ser Gly Gln Gln Gly Pro Gly Ala Ser
    595                 600             605
```

<210>    13

<211> 601
<212> PRT
<213> Artificial Sequence

<220>
<223> PRT410

<400> 13

Met His His His His His His Ser Ser Gly Ser Ser Gly Pro Gly Gln
1               5                   10                  15

Gln Gly Pro Tyr Gly Pro Gly Ala Ser Ala Ala Ala Ala Ala Gly Gln
            20                  25                  30

Asn Gly Pro Gly Ser Gly Gln Gln Gly Pro Gly Gln Ser Gly Gln Tyr
            35                  40                  45

Gly Pro Gly Gln Gln Gly Pro Gly Gln Gln Gly Pro Gly Ser Ser Ala
        50                  55                  60

Ala Ala Ala Ala Gly Pro Gly Gln Tyr Gly Pro Gly Gln Gln Gly Pro
65                  70                  75                  80

Ser Ala Ser Ala Ala Ala Ala Ala Gly Pro Gly Ser Gly Gln Gln Gly
            85                  90                  95

Pro Gly Ala Ser Gly Gln Tyr Gly Pro Gly Gln Gln Gly Pro Gly Gln
            100                 105                 110

Gln Gly Pro Gly Ser Ser Ala Ala Ala Ala Ala Gly Gln Tyr Gly Ser
            115                 120                 125

Gly Pro Gly Gln Gln Gly Pro Tyr Gly Ser Ala Ala Ala Ala Ala Gly
        130                 135                 140

Pro Gly Ser Gly Gln Tyr Gly Gln Gly Pro Tyr Gly Pro Gly Ala Ser
145                 150                 155                 160

Gly Pro Gly Gln Tyr Gly Pro Gly Gln Gln Gly Pro Ser Ala Ser Ala
            165                 170                 175

Ala Ala Ala Gly Ser Gly Gln Gln Gly Pro Gly Gln Tyr Gly Pro
            180                 185                 190

Tyr Ala Ser Ala Ala Ala Ala Ala Gly Gln Tyr Gly Ser Gly Pro Gly
            195                 200                 205

Gln Gln Gly Pro Tyr Gly Pro Gly Gln Ser Gly Ser Gly Gln Gln Gly
        210                 215                 220

```
Pro Gly Gln Gln Gly Pro Tyr Ala Ser Ala Ala Ala Ala Ala Gly Pro
225                 230             235                 240


Gly Gln Gln Gly Pro Tyr Gly Pro Gly Ser Ser Ala Ala Ala Ala Ala
                245             250             255


Gly Gln Tyr Gly Tyr Gly Pro Gly Gln Gln Gly Pro Tyr Gly Pro Gly
        260             265             270


Ala Ser Gly Gln Asn Gly Pro Gly Ser Gly Gln Tyr Gly Pro Gly Gln
        275             280             285


Gln Gly Pro Gly Gln Ser Ala Ala Ala Ala Gly Pro Gly Gln Gln
    290             295             300


Gly Pro Tyr Gly Pro Gly Ala Ser Ala Ala Ala Ala Gly Gln Tyr
305             310             315             320


Gly Pro Gly Gln Gln Gly Pro Gly Gln Tyr Gly Pro Gly Ser Ser Gly
            325             330             335


Pro Gly Gln Gln Gly Pro Tyr Gly Pro Gly Ser Ser Ala Ala Ala Ala
            340             345             350


Ala Gly Gln Tyr Gly Pro Gly Gln Gln Gly Pro Tyr Gly Pro Gly Gln
    355             360             365


Ser Ala Ala Ala Ala Ala Gly Gln Tyr Gln Gln Gly Pro Gly Gln Gln
    370             375             380


Gly Pro Tyr Gly Pro Gly Ala Ser Gly Pro Gly Gln Gln Gly Pro Tyr
385             390             395             400


Gly Pro Gly Ala Ser Ala Ala Ala Ala Ala Gly Pro Gly Gln Tyr Gly
            405             410             415


Pro Gly Gln Gln Gly Pro Ser Ala Ser Ala Ala Ala Ala Ala Gly Gln
            420             425             430


Tyr Gly Ser Gly Pro Gly Gln Tyr Gly Pro Tyr Gly Pro Gly Gln Ser
        435             440             445


Gly Pro Gly Ser Gly Gln Gln Gly Gln Gly Pro Tyr Gly Pro Gly Ala
    450             455             460


Ser Ala Ala Ala Ala Ala Gly Gln Tyr Gly Pro Gly Gln Gln Gly Pro
```

71

```
                465                     470                     475                     480


        Tyr Gly Pro Gly Gln Ser Ala Ala Ala Ala Gly Pro Gly Ser Gly
                        485                 490                 495


        Gln Tyr Gly Pro Gly Ala Ser Gly Gln Asn Gly Pro Gly Ser Gly Gln
                    500                 505                 510


        Tyr Gly Pro Gly Gln Gln Gly Pro Gly Gln Ser Ala Ala Ala Ala Ala
                    515                 520                 525


        Gly Gln Tyr Gln Gln Gly Pro Gly Gln Gln Gly Pro Tyr Gly Pro Gly
                    530                 535                 540


        Ala Ser Ala Ala Ala Ala Ala Gly Gln Tyr Gly Ser Gly Pro Gly Gln
        545                 550                 555                 560


        Gln Gly Pro Tyr Gly Pro Gly Gln Ser Gly Ser Gly Gln Gln Gly Pro
                    565                 570                 575


        Gly Gln Gln Gly Pro Tyr Ala Ser Ala Ala Ala Ala Ala Gly Pro Gly
                    580                 585                 590


        Ser Gly Gln Gln Gly Pro Gly Ala Ser
                    595                 600


        <210>  14
        <211>  576
        <212>  PRT
        <213>  Artificial Sequence

        <220>
        <223>  PRT525

        <400>  14

        Met His His His His His His Ser Ser Gly Ser Ser Gly Pro Gly Gln
        1               5                   10                  15


        Gln Gly Pro Tyr Gly Pro Gly Ala Ser Ala Ala Ala Ala Ala Ala Ala
                    20                  25                  30


        Gly Ser Asn Gly Pro Gly Ser Gly Gln Gln Gly Pro Gly Gln Ser Gly
                    35                  40                  45


        Gln Tyr Gly Pro Gly Gln Gln Gly Pro Gly Gln Gln Gly Pro Gly Ser
                    50                  55                  60


        Ser Ala Ala Ala Ala Ala Ala Ala Gly Pro Gly Gln Tyr Gly Pro Gly
        65                  70                  75                  80
```

72

```
Gln Gln Gly Pro Ser Ala Ser Ala Ala Ala Ala Ala Ala Gly Pro
                85              90              95

Gly Ser Gly Gln Gln Gly Pro Gly Ala Ser Gly Gln Tyr Gly Pro Gly
            100             105             110

Gln Gln Gly Pro Gly Gln Gln Gly Pro Gly Ser Ser Ala Ala Ala Ala
        115             120             125

Ala Ala Ala Gly Ser Tyr Gly Ser Gly Pro Gly Gln Gln Gly Pro Tyr
    130             135             140

Gly Ser Ala Ala Ala Ala Ala Ala Gly Pro Gly Ser Gly Gln Tyr
145             150             155             160

Gly Gln Gly Pro Tyr Gly Pro Gly Ala Ser Gly Pro Gly Gln Tyr Gly
            165             170             175

Pro Gly Gln Gln Gly Pro Ser Ala Ser Ala Ala Ala Ala Ala Ala
        180             185             190

Gly Ser Gly Gln Gln Gly Pro Gly Gln Tyr Gly Pro Tyr Ala Ser Ala
        195             200             205

Ala Ala Ala Ala Ala Ala Gly Ser Tyr Gly Ser Gly Pro Gly Gln Gln
    210             215             220

Gly Pro Tyr Gly Pro Gly Gln Ser Gly Ser Gly Gln Gln Gly Pro Gly
225             230             235             240

Gln Gln Gly Pro Tyr Ala Ser Ala Ala Ala Ala Ala Ala Gly Pro
            245             250             255

Gly Gln Gln Gly Pro Tyr Gly Pro Gly Ser Ser Ala Ala Ala Ala Ala
        260             265             270

Ala Ala Gly Ser Tyr Gly Tyr Gly Pro Gly Gln Gln Gly Pro Tyr Gly
        275             280             285

Pro Gly Ala Ser Gly Gln Asn Gly Pro Gly Ser Gly Gln Tyr Gly Pro
    290             295             300

Gly Gln Gln Gly Pro Gly Pro Ser Ala Ala Ala Ala Ala Ala Gly
305             310             315             320

Pro Gly Gln Gln Gly Pro Tyr Gly Pro Gly Ala Ser Ala Ala Ala Ala
```

                    325                     330                     335

Ala Ala Ala Gly Ser Tyr Gly Pro Gly Gln Gln Gly Pro Gly Gln Tyr
            340                     345                     350

Gly Pro Gly Ser Ser Gly Pro Gly Gln Gln Gly Pro Tyr Gly Pro Gly
            355                     360                     365

Ser Ser Ala Ala Ala Ala Ala Ala Ala Gly Ser Tyr Gly Pro Gly Gln
    370                     375                     380

Gln Gly Pro Tyr Gly Pro Gly Pro Ser Ala Ala Ala Ala Ala Ala Ala
385                     390                     395                     400

Gly Ser Tyr Gln Gln Gly Pro Gly Gln Gln Gly Pro Tyr Gly Pro Gly
                405                     410                     415

Ala Ser Gly Pro Gly Gln Gln Gly Pro Tyr Gly Pro Gly Ala Ser Ala
            420                     425                     430

Ala Ala Ala Ala Ala Ala Gly Pro Gly Gln Tyr Gly Pro Gly Gln Gln
        435                     440                     445

Gly Pro Ser Ala Ser Ala Ala Ala Ala Ala Ala Gly Ser Tyr Gly
    450                     455                     460

Ser Gly Pro Gly Gln Tyr Gly Pro Tyr Gly Pro Gly Gln Ser Gly Pro
465                     470                     475                     480

Gly Ser Gly Gln Gln Gly Gln Gly Pro Tyr Gly Pro Gly Ala Ser Ala
            485                     490                     495

Ala Ala Ala Ala Ala Gly Ser Tyr Gly Pro Gly Gln Gln Gly Pro
        500                     505                     510

Tyr Gly Pro Gly Pro Ser Ala Ala Ala Ala Ala Ala Gly Pro Gly
        515                     520                     525

Ser Gly Gln Tyr Gly Pro Gly Ala Ser Gly Gln Asn Gly Pro Gly Ser
    530                     535                     540

Gly Gln Tyr Gly Pro Gly Gln Gln Gly Pro Gly Pro Ser Ala Ala Ala
545                     550                     555                     560

Ala Ala Ala Ala Gly Pro Gly Ser Gly Gln Gln Gly Pro Gly Ala Ser
            565                     570                     575

```
<210>   15
<211>   2375
<212>   PRT
<213>   Artificial Sequence

<220>
<223>   PRT799

<400>   15


Met His His His His His His Ser Ser Gly Ser Ser Gly Pro Gly Gln
1               5                   10                  15


Gln Gly Pro Tyr Gly Pro Gly Ala Ser Ala Ala Ala Ala Ala Gly Gln
                20                  25                  30


Asn Gly Pro Gly Ser Gly Gln Gln Gly Pro Gly Gln Ser Gly Gln Tyr
                35                  40                  45


Gly Pro Gly Gln Gln Gly Pro Gly Gln Gln Gly Pro Gly Ser Ser Ala
        50                  55                  60


Ala Ala Ala Ala Gly Pro Gly Gln Tyr Gly Pro Gly Gln Gln Gly Pro
65                  70                  75                  80


Ser Ala Ser Ala Ala Ala Ala Ala Gly Pro Gly Ser Gly Gln Gln Gly
                85                  90                  95


Pro Gly Ala Ser Gly Gln Tyr Gly Pro Gly Gln Gln Gly Pro Gly Gln
                100                 105                 110


Gln Gly Pro Gly Ser Ser Ala Ala Ala Ala Ala Gly Gln Tyr Gly Ser
                115                 120                 125


Gly Pro Gly Gln Gln Gly Pro Tyr Gly Ser Ala Ala Ala Ala Ala Gly
                130                 135                 140


Pro Gly Ser Gly Gln Tyr Gly Gln Gly Pro Tyr Gly Pro Gly Ala Ser
145                 150                 155                 160


Gly Pro Gly Gln Tyr Gly Pro Gly Gln Gln Gly Pro Ser Ala Ser Ala
                165                 170                 175


Ala Ala Ala Gly Ser Gly Gln Gln Gly Pro Gly Gln Tyr Gly Pro
                180                 185                 190


Tyr Ala Ser Ala Ala Ala Ala Ala Gly Gln Tyr Gly Ser Gly Pro Gly
                195                 200                 205


Gln Gln Gly Pro Tyr Gly Pro Gly Gln Ser Gly Ser Gly Gln Gln Gly
```

```
        210                           215                           220

Pro Gly Gln Gln Gly Pro Tyr Ala Ser Ala Ala Ala Ala Ala Gly Pro
225                 230                 235                     240

Gly Gln Gln Gly Pro Tyr Gly Pro Gly Ser Ser Ala Ala Ala Ala Ala
                245                 250                 255

Gly Gln Tyr Gly Tyr Gly Pro Gly Gln Gln Gly Pro Tyr Gly Pro Gly
                260                 265                 270

Ala Ser Gly Gln Asn Gly Pro Gly Ser Gly Gln Tyr Gly Pro Gly Gln
            275                 280                 285

Gln Gly Pro Gly Gln Ser Ala Ala Ala Ala Gly Pro Gly Gln Gln
            290                 295                 300

Gly Pro Tyr Gly Pro Gly Ala Ser Ala Ala Ala Ala Gly Gln Tyr
305                 310                 315                     320

Gly Pro Gly Gln Gln Gly Pro Gly Gln Tyr Gly Pro Gly Ser Ser Gly
                325                 330                     335

Pro Gly Gln Gln Gly Pro Tyr Gly Pro Gly Ser Ser Ala Ala Ala Ala
            340                 345                 350

Ala Gly Gln Tyr Gly Pro Gly Gln Gln Gly Pro Tyr Gly Pro Gly Gln
            355                 360                 365

Ser Ala Ala Ala Ala Ala Gly Gln Tyr Gln Gln Gly Pro Gly Gln Gln
            370                 375                 380

Gly Pro Tyr Gly Pro Gly Ala Ser Gly Pro Gly Gln Gln Gly Pro Tyr
385                 390                 395                     400

Gly Pro Gly Ala Ser Ala Ala Ala Ala Ala Gly Pro Gly Gln Tyr Gly
                405                 410                     415

Pro Gly Gln Gln Gly Pro Ser Ala Ser Ala Ala Ala Ala Ala Gly Gln
            420                 425                     430

Tyr Gly Ser Gly Pro Gly Gln Tyr Gly Pro Tyr Gly Pro Gly Gln Ser
            435                 440                     445

Gly Pro Gly Ser Gly Gln Gln Gly Gln Gly Pro Tyr Gly Pro Gly Ala
    450                 455                 460
```

Ser Ala Ala Ala Ala Ala Gly Gln Tyr Gly Pro Gly Gln Gln Gly Pro
465                 470             475                 480

Tyr Gly Pro Gly Gln Ser Ala Ala Ala Ala Ala Gly Pro Gly Ser Gly
            485                 490             495

Gln Tyr Gly Pro Gly Ala Ser Gly Gln Asn Gly Pro Gly Ser Gly Gln
            500                 505             510

Tyr Gly Pro Gly Gln Gln Gly Pro Gly Gln Ser Ala Ala Ala Ala Ala
            515                 520             525

Gly Gln Tyr Gln Gln Gly Pro Gly Gln Gln Gly Pro Tyr Gly Pro Gly
            530                 535             540

Ala Ser Ala Ala Ala Ala Ala Gly Gln Tyr Gly Ser Gly Pro Gly Gln
545                 550             555                 560

Gln Gly Pro Tyr Gly Pro Gly Gln Ser Gly Ser Gly Gln Gln Gly Pro
            565                 570             575

Gly Gln Gln Gly Pro Tyr Ala Ser Ala Ala Ala Ala Ala Gly Pro Gly
            580                 585             590

Ser Gly Gln Gln Gly Pro Gly Ala Ser Gly Gln Gln Gly Pro Tyr Gly
            595                 600             605

Pro Gly Ala Ser Ala Ala Ala Ala Ala Gly Gln Asn Gly Pro Gly Ser
            610                 615             620

Gly Gln Gln Gly Pro Gly Gln Ser Gly Gln Tyr Gly Pro Gly Gln Gln
625                 630             635                 640

Gly Pro Gly Gln Gln Gly Pro Gly Ser Ser Ala Ala Ala Ala Ala Gly
            645                 650             655

Pro Gly Gln Tyr Gly Pro Gly Gln Gln Gly Pro Ser Ala Ser Ala Ala
            660                 665             670

Ala Ala Ala Gly Pro Gly Ser Gly Gln Gln Gly Pro Gly Ala Ser Gly
            675                 680             685

Gln Tyr Gly Pro Gly Gln Gln Gly Pro Gly Gln Gln Gly Pro Gly Ser
            690                 695             700

Ser Ala Ala Ala Ala Ala Gly Gln Tyr Gly Ser Gly Pro Gly Gln Gln
705                 710             715                 720

77

```
Gly Pro Tyr Gly Ser Ala Ala Ala Ala Gly Pro Gly Ser Gly Gln
            725             730             735

Tyr Gly Gln Gly Pro Tyr Gly Pro Gly Ala Ser Gly Pro Gly Gln Tyr
            740             745             750

Gly Pro Gly Gln Gln Gly Pro Ser Ala Ser Ala Ala Ala Ala Ala Gly
            755             760             765

Ser Gly Gln Gln Gly Pro Gly Gln Tyr Gly Pro Tyr Ala Ser Ala Ala
            770             775             780

Ala Ala Ala Gly Gln Tyr Gly Ser Gly Pro Gly Gln Gln Gly Pro Tyr
785             790             795             800

Gly Pro Gly Gln Ser Gly Ser Gly Gln Gln Gly Pro Gly Gln Gln Gly
            805             810             815

Pro Tyr Ala Ser Ala Ala Ala Ala Ala Gly Pro Gly Gln Gln Gly Pro
            820             825             830

Tyr Gly Pro Gly Ser Ser Ala Ala Ala Ala Gly Gln Tyr Gly Tyr
            835             840             845

Gly Pro Gly Gln Gln Gly Pro Tyr Gly Pro Gly Ala Ser Gly Gln Asn
850             855             860

Gly Pro Gly Ser Gly Gln Tyr Gly Pro Gly Gln Gln Gly Pro Gly Gln
865             870             875             880

Ser Ala Ala Ala Ala Ala Gly Pro Gly Gln Gln Gly Pro Tyr Gly Pro
            885             890             895

Gly Ala Ser Ala Ala Ala Ala Ala Gly Gln Tyr Gly Pro Gly Gln Gln
            900             905             910

Gly Pro Gly Gln Tyr Gly Pro Gly Ser Ser Gly Pro Gly Gln Gln Gly
            915             920             925

Pro Tyr Gly Pro Gly Ser Ser Ala Ala Ala Ala Ala Gly Gln Tyr Gly
            930             935             940

Pro Gly Gln Gln Gly Pro Tyr Gly Pro Gly Gln Ser Ala Ala Ala Ala
945             950             955             960

Ala Gly Gln Tyr Gln Gln Gly Pro Gly Gln Gln Gly Pro Tyr Gly Pro
            965             970             975
```

78

```
Gly Ala Ser Gly Pro Gly Gln Gln Gly Pro Tyr Gly Pro Gly Ala Ser
        980                   985                   990

Ala Ala Ala Ala Ala Gly Pro Gly  Gln Tyr Gly Pro Gly  Gln Gln Gly
        995                  1000                  1005

Pro Ser  Ala Ser Ala Ala Ala  Ala Ala Gly Gln Tyr  Gly Ser Gly
        1010                  1015                  1020

Pro Gly  Gln Tyr Gly Pro Tyr  Gly Pro Gly Gln Ser  Gly Pro Gly
        1025                  1030                  1035

Ser Gly  Gln Gln Gly Gln Gly  Pro Tyr Gly Pro Gly  Ala Ser Ala
        1040                  1045                  1050

Ala Ala  Ala Ala Gly Gln Tyr  Gly Pro Gly Gln Gln  Gly Pro Tyr
        1055                  1060                  1065

Gly Pro  Gly Gln Ser Ala Ala  Ala Ala Ala Gly Pro  Gly Ser Gly
        1070                  1075                  1080

Gln Tyr  Gly Pro Gly Ala Ser  Gly Gln Asn Gly Pro  Gly Ser Gly
        1085                  1090                  1095

Gln Tyr  Gly Pro Gly Gln Gln  Gly Pro Gly Gln Ser  Ala Ala Ala
        1100                  1105                  1110

Ala Ala  Gly Gln Tyr Gln Gln  Gly Pro Gly Gln Gln  Gly Pro Tyr
        1115                  1120                  1125

Gly Pro  Gly Ala Ser Ala Ala  Ala Ala Ala Gly Gln  Tyr Gly Ser
        1130                  1135                  1140

Gly Pro  Gly Gln Gln Gly Pro  Tyr Gly Pro Gly Gln  Ser Gly Ser
        1145                  1150                  1155

Gly Gln  Gln Gly Pro Gly Gln  Gln Gly Pro Tyr Ala  Ser Ala Ala
        1160                  1165                  1170

Ala Ala  Ala Gly Pro Gly Ser  Gly Gln Gln Gly Pro  Gly Ala Ser
        1175                  1180                  1185

Gly Gln  Gln Gly Pro Tyr Gly  Pro Gly Ala Ser Ala  Ala Ala Ala
        1190                  1195                  1200

Ala Gly  Gln Asn Gly Pro Gly  Ser Gly Gln Gln Gly  Pro Gly Gln
```

                        1205                        1210                              1215


        Ser Gly   Gln Tyr Gly Pro Gly   Gln Gln Gly Pro Gly   Gln Gln Gly
                1220                    1225                  1230


        Pro Gly   Ser Ser Ala Ala Ala   Ala Ala Gly Pro Gly   Gln Tyr Gly
                1235                    1240                  1245


        Pro Gly   Gln Gln Gly Pro Ser   Ala Ser Ala Ala Ala   Ala Ala Gly
                1250                    1255                  1260


        Pro Gly   Ser Gly Gln Gln Gly   Pro Gly Ala Ser Gly   Gln Tyr Gly
                1265                    1270                  1275


        Pro Gly   Gln Gln Gly Pro Gly   Gln Gln Gly Pro Gly   Ser Ser Ala
                1280                    1285                  1290


        Ala Ala   Ala Ala Gly Gln Tyr   Gly Ser Gly Pro Gly   Gln Gln Gly
                1295                    1300                  1305


        Pro Tyr   Gly Ser Ala Ala Ala   Ala Ala Gly Pro Gly   Ser Gly Gln
                1310                    1315                  1320


        Tyr Gly   Gln Gly Pro Tyr Gly   Pro Gly Ala Ser Gly   Pro Gly Gln
                1325                    1330                  1335


        Tyr Gly   Pro Gly Gln Gln Gly   Pro Ser Ala Ser Ala   Ala Ala Ala
                1340                    1345                  1350


        Ala Gly   Ser Gly Gln Gln Gly   Pro Gly Gln Tyr Gly   Pro Tyr Ala
                1355                    1360                  1365


        Ser Ala   Ala Ala Ala Ala Gly   Gln Tyr Gly Ser Gly   Pro Gly Gln
                1370                    1375                  1380


        Gln Gly   Pro Tyr Gly Pro Gly   Gln Ser Gly Ser Gly   Gln Gln Gly
                1385                    1390                  1395


        Pro Gly   Gln Gln Gly Pro Tyr   Ala Ser Ala Ala Ala   Ala Ala Gly
                1400                    1405                  1410


        Pro Gly   Gln Gln Gly Pro Tyr   Gly Pro Gly Ser Ser   Ala Ala Ala
                1415                    1420                  1425


        Ala Ala   Gly Gln Tyr Gly Tyr   Gly Pro Gly Gln Gln   Gly Pro Tyr
                1430                    1435                  1440

Gly Pro Gly Ala Ser Gly Gln Asn Gly Pro Gly Ser Gly Gln Tyr
1445 1450 1455

Gly Pro Gly Gln Gln Gly Pro Gly Gln Ser Ala Ala Ala Ala Ala
1460 1465 1470

Gly Pro Gly Gln Gln Gly Pro Tyr Gly Pro Gly Ala Ser Ala Ala
1475 1480 1485

Ala Ala Ala Gly Gln Tyr Gly Pro Gly Gln Gln Gly Pro Gly Gln
1490 1495 1500

Tyr Gly Pro Gly Ser Ser Gly Pro Gly Gln Gln Gly Pro Tyr Gly
1505 1510 1515

Pro Gly Ser Ser Ala Ala Ala Ala Ala Gly Gln Tyr Gly Pro Gly
1520 1525 1530

Gln Gln Gly Pro Tyr Gly Pro Gly Gln Ser Ala Ala Ala Ala Ala
1535 1540 1545

Gly Gln Tyr Gln Gln Gly Pro Gly Gln Gln Gly Pro Tyr Gly Pro
1550 1555 1560

Gly Ala Ser Gly Pro Gly Gln Gln Gly Pro Tyr Gly Pro Gly Ala
1565 1570 1575

Ser Ala Ala Ala Ala Ala Gly Pro Gly Gln Tyr Gly Pro Gly Gln
1580 1585 1590

Gln Gly Pro Ser Ala Ser Ala Ala Ala Ala Ala Gly Gln Tyr Gly
1595 1600 1605

Ser Gly Pro Gly Gln Tyr Gly Pro Tyr Gly Pro Gly Gln Ser Gly
1610 1615 1620

Pro Gly Ser Gly Gln Gln Gly Gln Gly Pro Tyr Gly Pro Gly Ala
1625 1630 1635

Ser Ala Ala Ala Ala Gly Gln Tyr Gly Pro Gly Gln Gln Gly
1640 1645 1650

Pro Tyr Gly Pro Gly Gln Ser Ala Ala Ala Ala Ala Gly Pro Gly
1655 1660 1665

Ser Gly Gln Tyr Gly Pro Gly Ala Ser Gly Gln Asn Gly Pro Gly
1670 1675 1680

Ser Gly Gln Tyr Gly Pro Gly Gln Gln Gly Pro Gly Gln Ser Ala
    1685              1690              1695

Ala Ala Ala Ala Gly Gln Tyr Gln Gln Gly Pro Gly Gln Gln Gly
    1700              1705              1710

Pro Tyr Gly Pro Gly Ala Ser Ala Ala Ala Ala Gly Gln Tyr
    1715              1720              1725

Gly Ser Gly Pro Gly Gln Gln Gly Pro Tyr Gly Pro Gly Gln Ser
    1730              1735              1740

Gly Ser Gly Gln Gln Gly Pro Gly Gln Gln Gly Pro Tyr Ala Ser
    1745              1750              1755

Ala Ala Ala Ala Ala Gly Pro Gly Ser Gly Gln Gln Gly Pro Gly
    1760              1765              1770

Ala Ser Gly Gln Gln Gly Pro Tyr Gly Pro Gly Ala Ser Ala Ala
    1775              1780              1785

Ala Ala Ala Gly Gln Asn Gly Pro Gly Ser Gly Gln Gln Gly Pro
    1790              1795              1800

Gly Gln Ser Gly Gln Tyr Gly Pro Gly Gln Gln Gly Pro Gly Gln
    1805              1810              1815

Gln Gly Pro Gly Ser Ser Ala Ala Ala Ala Ala Gly Pro Gly Gln
    1820              1825              1830

Tyr Gly Pro Gly Gln Gln Gly Pro Ser Ala Ser Ala Ala Ala Ala
    1835              1840              1845

Ala Gly Pro Gly Ser Gly Gln Gln Gly Pro Gly Ala Ser Gly Gln
    1850              1855              1860

Tyr Gly Pro Gly Gln Gln Gly Pro Gly Gln Gln Gly Pro Gly Ser
    1865              1870              1875

Ser Ala Ala Ala Ala Ala Gly Gln Tyr Gly Ser Gly Pro Gly Gln
    1880              1885              1890

Gln Gly Pro Tyr Gly Ser Ala Ala Ala Ala Ala Gly Pro Gly Ser
    1895              1900              1905

Gly Gln Tyr Gly Gln Gly Pro Tyr Gly Pro Gly Ala Ser Gly Pro
    1910              1915              1920

```
Gly Gln  Tyr Gly Pro Gly Gln  Gln Gly Pro Ser Ala  Ser Ala Ala
    1925                 1930             1935


Ala Ala  Ala Gly Ser Gly Gln  Gln Gly Pro Gly Gln  Tyr Gly Pro
    1940                 1945             1950


Tyr Ala  Ser Ala Ala Ala Ala  Ala Gly Gln Tyr Gly  Ser Gly Pro
    1955                 1960             1965


Gly Gln  Gln Gly Pro Tyr Gly  Pro Gly Gln Ser Gly  Ser Gly Gln
    1970                 1975             1980


Gln Gly  Pro Gly Gln Gln Gly  Pro Tyr Ala Ser Ala  Ala Ala Ala
    1985                 1990             1995


Ala Gly  Pro Gly Gln Gln Gly  Pro Tyr Gly Pro Gly  Ser Ser Ala
    2000                 2005             2010


Ala Ala  Ala Ala Gly Gln Tyr  Gly Tyr Gly Pro Gly  Gln Gln Gly
    2015                 2020             2025


Pro Tyr  Gly Pro Gly Ala Ser  Gly Gln Asn Gly Pro  Gly Ser Gly
    2030                 2035             2040


Gln Tyr  Gly Pro Gly Gln Gln  Gly Pro Gly Gln Ser  Ala Ala Ala
    2045                 2050             2055


Ala Ala  Gly Pro Gly Gln Gln  Gly Pro Tyr Gly Pro  Gly Ala Ser
    2060                 2065             2070


Ala Ala  Ala Ala Ala Gly Gln  Tyr Gly Pro Gly Gln  Gln Gly Pro
    2075                 2080             2085


Gly Gln  Tyr Gly Pro Gly Ser  Ser Gly Pro Gly Gln  Gln Gly Pro
    2090                 2095             2100


Tyr Gly  Pro Gly Ser Ser Ala  Ala Ala Ala Ala Gly  Gln Tyr Gly
    2105                 2110             2115


Pro Gly  Gln Gln Gly Pro Tyr  Gly Pro Gly Gln Ser  Ala Ala Ala
    2120                 2125             2130


Ala Ala  Gly Gln Tyr Gln Gln  Gly Pro Gly Gln Gln  Gly Pro Tyr
    2135                 2140             2145


Gly Pro  Gly Ala Ser Gly Pro  Gly Gln Gln Gly Pro  Tyr Gly Pro
```

2150 2155 2160

Gly Ala Ser Ala Ala Ala Ala Ala Gly Pro Gly Gln Tyr Gly Pro
2165 2170 2175

Gly Gln Gln Gly Pro Ser Ala Ser Ala Ala Ala Ala Ala Gly Gln
2180 2185 2190

Tyr Gly Ser Gly Pro Gly Gln Tyr Gly Pro Tyr Gly Pro Gly Gln
2195 2200 2205

Ser Gly Pro Gly Ser Gly Gln Gln Gly Gln Gly Pro Tyr Gly Pro
2210 2215 2220

Gly Ala Ser Ala Ala Ala Ala Ala Gly Gln Tyr Gly Pro Gly Gln
2225 2230 2235

Gln Gly Pro Tyr Gly Pro Gly Gln Ser Ala Ala Ala Ala Ala Gly
2240 2245 2250

Pro Gly Ser Gly Gln Tyr Gly Pro Gly Ala Ser Gly Gln Asn Gly
2255 2260 2265

Pro Gly Ser Gly Gln Tyr Gly Pro Gly Gln Gln Gly Pro Gly Gln
2270 2275 2280

Ser Ala Ala Ala Ala Ala Gly Gln Tyr Gln Gln Gly Pro Gly Gln
2285 2290 2295

Gln Gly Pro Tyr Gly Pro Gly Ala Ser Ala Ala Ala Ala Ala Gly
2300 2305 2310

Gln Tyr Gly Ser Gly Pro Gly Gln Gln Gly Pro Tyr Gly Pro Gly
2315 2320 2325

Gln Ser Gly Ser Gly Gln Gln Gly Pro Gly Gln Gln Gly Pro Tyr
2330 2335 2340

Ala Ser Ala Ala Ala Ala Ala Gly Pro Gly Ser Gly Gln Gln Gly
2345 2350 2355

Ser Ser Val Asp Lys Leu Ala Ala Ala Leu Glu His His His His
2360 2365 2370

His His
2375

<210> 16
<211> 608
<212> PRT
<213> Artificial Sequence

<220>
<223> PRT313

<400> 16

Met His His His His His His Ser Ser Gly Ser Ser Gly Pro Gly Gly
1               5                   10                  15

Gln Gly Pro Tyr Gly Pro Gly Ala Ser Ala Ala Ala Ala Ala Gly Gly
            20                  25                  30

Asn Gly Pro Gly Ser Gly Gln Gln Gly Pro Gly Gly Ser Ala Ala Ala
            35                  40                  45

Ala Ala Gly Gly Tyr Gly Pro Gly Gly Gln Gly Pro Gly Gln Gln Gly
        50                  55                  60

Pro Gly Ser Ser Ala Ala Ala Ala Ala Gly Pro Gly Gly Tyr Gly Pro
65                  70                  75                  80

Gly Gly Gln Gly Pro Ser Ala Ser Ala Ala Ala Ala Ala Gly Pro Gly
                85                  90                  95

Ser Gly Gln Gln Gly Pro Gly Ala Ser Ala Ala Ala Ala Ala Gly Gly
            100                 105                 110

Tyr Gly Pro Gly Gly Gln Gly Pro Gly Gln Gln Gly Pro Gly Ser Ser
            115                 120                 125

Ala Ala Ala Ala Ala Gly Gly Tyr Gly Ser Gly Pro Gly Gln Gln Gly
        130                 135                 140

Pro Tyr Gly Ser Ala Ala Ala Ala Ala Gly Pro Gly Ser Gly Gly Tyr
145                 150                 155                 160

Gly Gln Gly Pro Tyr Gly Pro Gly Ala Ser Ala Ala Ala Ala Ala Gly
            165                 170                 175

Pro Gly Gly Tyr Gly Pro Gly Gly Gln Gly Pro Ser Ala Ser Ala Ala
            180                 185                 190

Ala Ala Ala Gly Ser Gly Gln Gln Gly Pro Gly Gly Tyr Gly Pro Tyr
        195                 200                 205

Ala Ser Ala Ala Ala Ala Ala Gly Gly Tyr Gly Ser Gly Pro Gly Gln

```
          210                      215                      220


Gln Gly Pro Tyr Gly Pro Gly Gly Ser Ala Ala Ala Ala Ala Gly Ser
225                 230                 235                 240


Gly Gln Gln Gly Pro Gly Gln Gln Gly Pro Tyr Ala Ser Ala Ala Ala
                245                 250                 255


Ala Ala Gly Pro Gly Gly Gln Gly Pro Tyr Gly Pro Gly Ser Ser Ala
            260                 265                 270


Ala Ala Ala Ala Gly Gly Tyr Gly Tyr Gly Pro Gly Gly Gln Gly Pro
            275                 280                 285


Tyr Gly Pro Gly Ala Ser Ala Ala Ala Ala Gly Gly Asn Gly Pro
    290                 295                 300


Gly Ser Gly Gly Tyr Gly Pro Gly Gln Gln Gly Pro Gly Gly Ser Ala
305                 310                 315                 320


Ala Ala Ala Ala Gly Pro Gly Gly Gln Gly Pro Tyr Gly Pro Gly Ala
            325                 330                 335


Ser Ala Ala Ala Ala Ala Gly Gly Tyr Gly Pro Gly Gly Gln Gly Pro
    340                 345                 350


Gly Gly Tyr Gly Pro Gly Ser Ser Ala Ala Ala Ala Ala Gly Pro Gly
    355                 360                 365


Gly Gln Gly Pro Tyr Gly Pro Gly Ser Ser Ala Ala Ala Ala Ala Gly
    370                 375                 380


Gly Tyr Gly Pro Gly Gln Gln Gly Pro Tyr Gly Pro Gly Gly Ser Ala
385                 390                 395                 400


Ala Ala Ala Gly Gly Tyr Gln Gln Gly Pro Gly Gly Gln Gly Pro
            405                 410                 415


Tyr Gly Pro Gly Ala Ser Ala Ala Ala Ala Gly Pro Gly Gly Gln
    420                 425                 430


Gly Pro Tyr Gly Pro Gly Ala Ser Ala Ala Ala Ala Gly Pro Gly
    435                 440                 445


Gly Tyr Gly Pro Gly Gly Gln Gly Pro Ser Ala Ser Ala Ala Ala Ala
    450                 455                 460
```

```
Ala Gly Gly Tyr Gly Ser Gly Pro Gly Gly Tyr Gly Pro Tyr Gly Pro
465                 470             475                 480

Gly Gly Ser Ala Ala Ala Ala Ala Gly Pro Gly Ser Gly Gln Gln Gly
            485             490                 495

Gln Gly Pro Tyr Gly Pro Gly Ala Ser Ala Ala Ala Ala Ala Gly Gly
        500             505             510

Tyr Gly Pro Gly Gln Gln Gly Pro Tyr Gly Pro Gly Gly Ser Ala Ala
        515             520             525

Ala Ala Ala Gly Pro Gly Ser Gly Gly Tyr Gly Pro Gly Ala Ser Ala
    530             535             540

Ala Ala Ala Gly Gly Asn Gly Pro Gly Ser Gly Gly Tyr Gly Pro
545             550             555             560

Gly Gln Gln Gly Pro Gly Gly Ser Ala Ala Ala Ala Ala Gly Gly Tyr
            565             570             575

Gln Gln Gly Pro Gly Gly Gln Gly Pro Tyr Gly Pro Gly Ala Ser Ala
        580             585             590

Ala Ala Ala Gly Pro Gly Ser Gly Gln Gln Gly Pro Gly Ala Ser
    595             600             605


<210>   17
<211>   590
<212>   PRT
<213>   Artificial Sequence

<220>
<223>   Met-PRT399

<400>   17

Met Gly Pro Gly Gly Gln Gly Pro Tyr Gly Pro Gly Ala Ser Ala Ala
1               5               10              15

Ala Ala Ala Gly Gly Asn Gly Pro Gly Ser Gly Gln Gln Gly Pro Gly
            20              25              30

Gly Ser Gly Gly Tyr Gly Pro Gly Gly Gln Gly Pro Gly Gln Gln Gly
        35              40              45

Pro Gly Ser Ser Ala Ala Ala Ala Ala Gly Pro Gly Gly Tyr Gly Pro
    50              55              60

Gly Gly Gln Gly Pro Ser Ala Ser Ala Ala Ala Ala Ala Gly Pro Gly
```

```
            65                          70                          75                          80


            Ser Gly Gln Gln Gly Pro Gly Ala Ser Gly Gly Tyr Gly Pro Gly Gly
                            85                  90                  95


            Gln Gly Pro Gly Gln Gln Gly Pro Gly Ser Ser Ala Ala Ala Ala Ala
                        100                 105                 110


            Gly Gly Tyr Gly Ser Gly Pro Gly Gln Gln Gly Pro Tyr Gly Ser Ala
                        115                 120                 125


            Ala Ala Ala Ala Gly Pro Gly Ser Gly Gly Tyr Gly Gln Gly Pro Tyr
                        130                 135                 140


            Gly Pro Gly Ala Ser Gly Pro Gly Gly Tyr Gly Pro Gly Gly Gln Gly
            145                 150                 155                 160


            Pro Ser Ala Ser Ala Ala Ala Ala Ala Gly Ser Gly Gln Gln Gly Pro
                        165                 170                 175


            Gly Gly Tyr Gly Pro Tyr Ala Ser Ala Ala Ala Ala Gly Gly Tyr
                        180                 185                 190


            Gly Ser Gly Pro Gly Gln Gln Gly Pro Tyr Gly Pro Gly Gly Ser Gly
                        195                 200                 205


            Ser Gly Gln Gln Gly Pro Gly Gln Gln Gly Pro Tyr Ala Ser Ala Ala
                210                 215                 220


            Ala Ala Ala Gly Pro Gly Gly Gln Gly Pro Tyr Gly Pro Gly Ser Ser
            225                 230                 235                 240


            Ala Ala Ala Ala Ala Gly Gly Tyr Gly Tyr Gly Pro Gly Gly Gln Gly
                        245                 250                 255


            Pro Tyr Gly Pro Gly Ala Ser Gly Gly Asn Gly Pro Gly Ser Gly Gly
                        260                 265                 270


            Tyr Gly Pro Gly Gln Gln Gly Pro Gly Gly Ser Ala Ala Ala Ala Ala
                        275                 280                 285


            Gly Pro Gly Gly Gln Gly Pro Tyr Gly Pro Gly Ala Ser Ala Ala Ala
                        290                 295                 300


            Ala Ala Gly Gly Tyr Gly Pro Gly Gly Gln Gly Pro Gly Gly Tyr Gly
            305                 310                 315                 320
```

Pro Gly Ser Ser Gly Pro Gly Gly Gln Gly Pro Tyr Gly Pro Gly Ser
325                 330                 335

Ser Ala Ala Ala Ala Ala Gly Gly Tyr Gly Pro Gly Gln Gln Gly Pro
340                 345                 350

Tyr Gly Pro Gly Gly Ser Ala Ala Ala Ala Ala Gly Gly Tyr Gln Gln
355                 360                 365

Gly Pro Gly Gly Gln Gly Pro Tyr Gly Pro Gly Ala Ser Gly Pro Gly
370                 375                 380

Gly Gln Gly Pro Tyr Gly Pro Gly Ala Ser Ala Ala Ala Ala Ala Gly
385                 390                 395                 400

Pro Gly Gly Tyr Gly Pro Gly Gly Gln Gly Pro Ser Ala Ser Ala Ala
405                 410                 415

Ala Ala Ala Gly Gly Tyr Gly Ser Gly Pro Gly Gly Tyr Gly Pro Tyr
420                 425                 430

Gly Pro Gly Gly Ser Gly Pro Gly Ser Gly Gln Gln Gly Gln Gly Pro
435                 440                 445

Tyr Gly Pro Gly Ala Ser Ala Ala Ala Ala Gly Gly Tyr Gly Pro
450                 455                 460

Gly Gln Gln Gly Pro Tyr Gly Pro Gly Gly Ser Ala Ala Ala Ala Ala
465                 470                 475                 480

Gly Pro Gly Ser Gly Gly Tyr Gly Pro Gly Ala Ser Gly Gly Asn Gly
485                 490                 495

Pro Gly Ser Gly Gly Tyr Gly Pro Gly Gln Gln Gly Pro Gly Gly Ser
500                 505                 510

Ala Ala Ala Ala Ala Gly Gly Tyr Gln Gln Gly Pro Gly Gly Gln Gly
515                 520                 525

Pro Tyr Gly Pro Gly Ala Ser Ala Ala Ala Ala Gly Gly Tyr Gly
530                 535                 540

Ser Gly Pro Gly Gln Gln Gly Pro Tyr Gly Pro Gly Gly Ser Gly Ser
545                 550                 555                 560

Gly Gln Gln Gly Pro Gly Gln Gln Gly Pro Tyr Ala Ser Ala Ala Ala
565                 570                 575

89

```
Ala Ala Gly Pro Gly Ser Gly Gln Gln Gly Pro Gly Ala Ser
            580               585               590


<210>  18
<211>  601
<212>  PRT
<213>  Artificial Sequence

<220>
<223>  PRT399

<400>  18

Met His His His His His His Ser Ser Gly Ser Ser Gly Pro Gly Gly
1               5                   10                  15


Gln Gly Pro Tyr Gly Pro Gly Ala Ser Ala Ala Ala Ala Ala Gly Gly
            20                  25                  30


Asn Gly Pro Gly Ser Gly Gln Gln Gly Pro Gly Gly Ser Gly Gly Tyr
            35                  40                  45


Gly Pro Gly Gly Gln Gly Pro Gly Gln Gln Gly Pro Gly Ser Ser Ala
        50                  55                  60


Ala Ala Ala Ala Gly Pro Gly Gly Tyr Gly Pro Gly Gly Gln Gly Pro
65                  70                  75                  80


Ser Ala Ser Ala Ala Ala Ala Ala Gly Pro Gly Ser Gly Gln Gln Gly
                85                  90                  95


Pro Gly Ala Ser Gly Gly Tyr Gly Pro Gly Gly Gln Gly Pro Gly Gln
            100                 105                 110


Gln Gly Pro Gly Ser Ser Ala Ala Ala Ala Ala Gly Gly Tyr Gly Ser
            115                 120                 125


Gly Pro Gly Gln Gln Gly Pro Tyr Gly Ser Ala Ala Ala Ala Ala Gly
        130                 135                 140


Pro Gly Ser Gly Gly Tyr Gly Gln Gly Pro Tyr Gly Pro Gly Ala Ser
145                 150                 155                 160


Gly Pro Gly Gly Tyr Gly Pro Gly Gly Gln Gly Pro Ser Ala Ser Ala
            165                 170                 175


Ala Ala Ala Ala Gly Ser Gly Gln Gln Gly Pro Gly Gly Tyr Gly Pro
            180                 185                 190
```

Tyr Ala Ser Ala Ala Ala Ala Ala Gly Gly Tyr Gly Ser Gly Pro Gly
    195               200              205

Gln Gln Gly Pro Tyr Gly Pro Gly Gly Ser Gly Ser Gly Gln Gln Gly
    210               215              220

Pro Gly Gln Gln Gly Pro Tyr Ala Ser Ala Ala Ala Ala Ala Gly Pro
225              230          235              240

Gly Gly Gln Gly Pro Tyr Gly Pro Gly Ser Ser Ala Ala Ala Ala Ala
        245             250             255

Gly Gly Tyr Gly Tyr Gly Pro Gly Gly Gln Gly Pro Tyr Gly Pro Gly
        260             265             270

Ala Ser Gly Gly Asn Gly Pro Gly Ser Gly Gly Tyr Gly Pro Gly Gln
    275               280          285

Gln Gly Pro Gly Gly Ser Ala Ala Ala Ala Gly Pro Gly Gly Gln
    290               295          300

Gly Pro Tyr Gly Pro Gly Ala Ser Ala Ala Ala Ala Ala Gly Gly Tyr
305              310          315              320

Gly Pro Gly Gly Gln Gly Pro Gly Gly Tyr Gly Pro Gly Ser Ser Gly
        325             330          335

Pro Gly Gly Gln Gly Pro Tyr Gly Pro Gly Ser Ser Ala Ala Ala Ala
        340             345          350

Ala Gly Gly Tyr Gly Pro Gly Gln Gln Gly Pro Tyr Gly Pro Gly Gly
        355             360          365

Ser Ala Ala Ala Ala Ala Gly Gly Tyr Gln Gln Gly Pro Gly Gly Gln
    370               375          380

Gly Pro Tyr Gly Pro Gly Ala Ser Gly Pro Gly Gly Gln Gly Pro Tyr
385              390          395              400

Gly Pro Gly Ala Ser Ala Ala Ala Ala Ala Gly Pro Gly Gly Tyr Gly
        405             410             415

Pro Gly Gly Gln Gly Pro Ser Ala Ser Ala Ala Ala Ala Ala Gly Gly
        420             425          430

Tyr Gly Ser Gly Pro Gly Gly Tyr Gly Pro Tyr Gly Pro Gly Gly Ser
        435             440          445

```
Gly Pro Gly Ser Gly Gln Gln Gly Gln Gly Pro Tyr Gly Pro Gly Ala
    450                 455                 460

Ser Ala Ala Ala Ala Ala Gly Gly Tyr Gly Pro Gly Gln Gln Gly Pro
465                 470                 475                 480

Tyr Gly Pro Gly Gly Ser Ala Ala Ala Ala Ala Gly Pro Gly Ser Gly
                485                 490                 495

Gly Tyr Gly Pro Gly Ala Ser Gly Gly Asn Gly Pro Gly Ser Gly Gly
            500                 505                 510

Tyr Gly Pro Gly Gln Gln Gly Pro Gly Gly Ser Ala Ala Ala Ala Ala
            515                 520                 525

Gly Gly Tyr Gln Gln Gly Pro Gly Gly Gln Gly Pro Tyr Gly Pro Gly
    530                 535                 540

Ala Ser Ala Ala Ala Ala Ala Gly Gly Tyr Gly Ser Gly Pro Gly Gln
545                 550                 555                 560

Gln Gly Pro Tyr Gly Pro Gly Gly Ser Gly Ser Gly Gln Gln Gly Pro
            565                 570                 575

Gly Gln Gln Gly Pro Tyr Ala Ser Ala Ala Ala Ala Ala Gly Pro Gly
            580                 585                 590

Ser Gly Gln Gln Gly Pro Gly Ala Ser
            595                 600
```

```
<210>  19
<211>  612
<212>  PRT
<213>  Artificial Sequence

<220>
<223>  Met-PRT720

<400>  19
```

```
Met Gly Pro Gly Gln Gln Gly Pro Tyr Gly Pro Gly Ala Ser Ala Ala
1               5                   10                  15

Ala Ala Ala Gly Gln Asn Gly Pro Gly Ser Gly Gln Gln Gly Pro Gly
            20                  25                  30

Gln Ser Gly Gln Tyr Gly Pro Gly Gln Gln Gly Pro Gly Gln Gln Gly
            35                  40                  45
```

```
Pro Gly Ser Ser Ala Ala Ala Ala Ala Gly Pro Gly Gln Tyr Val Leu
    50              55              60

Ile Gly Pro Gly Gln Gln Val Leu Ile Gly Pro Ser Ala Ser Ala Ala
65              70              75              80

Ala Ala Ala Gly Pro Gly Ser Gly Gln Gln Gly Pro Gly Ala Ser Gly
            85              90              95

Gln Tyr Gly Pro Gly Gln Gln Gly Pro Gly Gln Gln Gly Pro Gly Ser
        100             105             110

Ser Ala Ala Ala Ala Ala Gly Ser Tyr Gly Ser Val Leu Ile Gly Pro
    115             120             125

Gly Gln Gln Val Leu Ile Gly Pro Tyr Gly Ser Ala Ala Ala Ala Ala
    130             135             140

Gly Pro Gly Ser Gly Gln Tyr Gly Gln Gly Pro Tyr Gly Pro Gly Ala
145             150             155             160

Ser Gly Pro Gly Gln Tyr Gly Pro Gly Gln Gln Gly Pro Ser Ala Ser
            165             170             175

Ala Ala Ala Ala Gly Ser Gly Gln Gln Val Leu Ile Gly Pro Gly
            180             185             190

Gln Tyr Val Leu Ile Gly Pro Tyr Ala Ser Ala Ala Ala Ala Ala Gly
        195             200             205

Gln Tyr Gly Ser Gly Pro Gly Gln Gln Gly Pro Tyr Gly Pro Gly Gln
    210             215             220

Ser Gly Ser Gly Gln Gln Gly Pro Gly Gln Gln Gly Pro Tyr Ala Ser
225             230             235             240

Ala Ala Ala Ala Ala Gly Pro Gly Gln Gln Val Leu Ile Gly Pro Tyr
            245             250             255

Val Leu Ile Gly Pro Gly Ser Ser Ala Ala Ala Ala Gly Gln Tyr
        260             265             270

Gly Tyr Gly Pro Gly Gln Gln Gly Pro Tyr Gly Pro Gly Ala Ser Gly
        275             280             285

Gln Asn Gly Pro Gly Ser Gly Gln Tyr Gly Pro Gly Gln Gln Gly Pro
    290             295             300
```

93

Gly Gln Ser Ala Ala Ala Ala Ala Gly Pro Gly Gln Gln Val Leu Ile
305                 310                 315                 320

Gly Pro Tyr Val Leu Ile Gly Pro Gly Ala Ser Ala Ala Ala Ala Ala
            325                 330                 335

Gly Gln Tyr Gly Pro Gly Gln Gln Gly Pro Gly Gln Tyr Gly Pro Gly
            340                 345                 350

Ser Ser Gly Pro Gly Gln Gln Gly Pro Tyr Gly Pro Gly Ser Ser Ala
        355                 360                 365

Ala Ala Ala Ala Gly Ser Tyr Gly Pro Gly Gln Gln Val Leu Ile Gly
        370                 375                 380

Pro Tyr Val Leu Ile Gly Pro Gly Pro Ser Ala Ala Ala Ala Ala Gly
385                 390                 395                 400

Gln Tyr Gln Gln Gly Pro Gly Gln Gln Gly Pro Tyr Gly Pro Gly Ala
            405                 410                 415

Ser Gly Pro Gly Gln Gln Gly Pro Tyr Gly Pro Gly Ala Ser Ala Ala
            420                 425                 430

Ala Ala Ala Gly Pro Gly Gln Tyr Val Leu Ile Gly Pro Gly Gln Gln
        435                 440                 445

Val Leu Ile Gly Pro Ser Ala Ser Ala Ala Ala Ala Gly Gln Tyr
    450                 455                 460

Gly Ser Gly Pro Gly Gln Tyr Gly Pro Tyr Gly Pro Gly Gln Ser Gly
465                 470                 475                 480

Pro Gly Ser Gly Gln Gln Gly Gln Gly Pro Tyr Gly Pro Gly Ala Ser
            485                 490                 495

Ala Ala Ala Ala Ala Gly Ser Tyr Gly Pro Gly Gln Gln Val Leu Ile
        500                 505                 510

Gly Pro Tyr Val Leu Ile Gly Pro Gly Pro Ser Ala Ala Ala Ala Ala
        515                 520                 525

Gly Pro Gly Ser Gly Gln Tyr Gly Pro Gly Ala Ser Gly Gln Asn Gly
        530                 535                 540

Pro Gly Ser Gly Gln Tyr Gly Pro Gly Gln Gln Gly Pro Gly Gln Ser
545                 550                 555                 560

94

```
Ala Ala Ala Ala Ala Gly Gln Tyr Gln Gln Val Leu Ile Gly Pro Gly
              565                 570                 575

Gln Gln Gly Pro Tyr Val Leu Ile Gly Pro Gly Ala Ser Ala Ala Ala
              580                 585                 590

Ala Ala Gly Pro Gly Ser Gly Gln Gln Val Leu Ile Gly Pro Gly Ala
              595                 600                 605

Ser Val Leu Ile
              610
```

```
<210>  20
<211>  592
<212>  PRT
<213>  Artificial Sequence

<220>
<223>  Met-PRT665

<400>  20
```

```
Met Gly Pro Gly Gln Gln Gly Pro Tyr Gly Pro Gly Ala Ser Ala Ala
1                   5                 10                  15

Ala Ala Ala Ala Ala Gly Ser Asn Gly Pro Gly Ser Gly Gln Gln Gly
              20                  25                  30

Pro Gly Gln Ser Gly Gln Tyr Gly Pro Gly Gln Gln Gly Pro Gly Gln
              35                  40                  45

Gln Gly Pro Gly Ser Ser Ala Ala Ala Ala Ala Ala Gly Pro Gly
              50                  55                  60

Gln Tyr Val Leu Ile Gly Pro Gly Gln Gln Gly Pro Ser Ala Ser Ala
65                  70                  75                  80

Ala Ala Ala Ala Ala Gly Pro Gly Ser Gly Gln Gln Gly Pro Gly
              85                  90                  95

Ala Ser Gly Gln Tyr Gly Pro Gly Gln Gln Gly Pro Gly Gln Gln Gly
              100                 105                 110

Pro Gly Ser Ser Ala Ala Ala Ala Ala Ala Ala Gly Ser Tyr Gly Ser
              115                 120                 125

Val Leu Ile Gly Pro Gly Gln Gln Gly Pro Tyr Gly Ser Ala Ala Ala
              130                 135                 140
```

```
Ala Ala Ala Ala Gly Pro Gly Ser Gly Gln Tyr Gly Gln Gly Pro Tyr
145                 150                 155                 160

Gly Pro Gly Ala Ser Gly Pro Gly Gln Tyr Gly Pro Gly Gln Gln Gly
                165                 170                 175

Pro Ser Ala Ser Ala Ala Ala Ala Ala Ala Ala Gly Ser Gly Gln Gln
                180                 185                 190

Val Leu Ile Gly Pro Gly Gln Tyr Gly Pro Tyr Ala Ser Ala Ala Ala
            195                 200                 205

Ala Ala Ala Ala Gly Ser Tyr Gly Ser Gly Pro Gly Gln Gln Gly Pro
            210                 215                 220

Tyr Gly Pro Gly Gln Ser Gly Ser Gly Gln Gln Gly Pro Gly Gln Gln
225                 230                 235                 240

Gly Pro Tyr Ala Ser Ala Ala Ala Ala Ala Ala Gly Pro Gly Gln
            245                 250                 255

Gln Val Leu Ile Gly Pro Tyr Gly Pro Gly Ser Ser Ala Ala Ala Ala
            260                 265                 270

Ala Ala Ala Gly Ser Tyr Gly Tyr Gly Pro Gly Gln Gln Gly Pro Tyr
            275                 280                 285

Gly Pro Gly Ala Ser Gly Gln Asn Gly Pro Gly Ser Gly Gln Tyr Gly
    290                 295                 300

Pro Gly Gln Gln Gly Pro Gly Pro Ser Ala Ala Ala Ala Ala Ala Ala
305                 310                 315                 320

Gly Pro Gly Gln Gln Val Leu Ile Gly Pro Tyr Gly Pro Gly Ala Ser
            325                 330                 335

Ala Ala Ala Ala Ala Ala Ala Gly Ser Tyr Gly Pro Gly Gln Gln Gly
            340                 345                 350

Pro Gly Gln Tyr Gly Pro Gly Ser Ser Gly Pro Gly Gln Gln Gly Pro
            355                 360                 365

Tyr Gly Pro Gly Ser Ser Ala Ala Ala Ala Ala Ala Ala Gly Ser Tyr
    370                 375                 380

Gly Pro Gly Gln Gln Val Leu Ile Gly Pro Tyr Gly Pro Gly Pro Ser
385                 390                 395                 400
```

```
Ala Ala Ala Ala Ala Ala Ala Gly Ser Tyr Gln Gln Gly Pro Gly Gln
                405             410             415

Gln Gly Pro Tyr Gly Pro Gly Ala Ser Gly Pro Gly Gln Gln Gly Pro
                420             425             430

Tyr Gly Pro Gly Ala Ser Ala Ala Ala Ala Ala Ala Gly Pro Gly
                435             440             445

Gln Tyr Val Leu Ile Gly Pro Gly Gln Gln Gly Pro Ser Ala Ser Ala
    450             455             460

Ala Ala Ala Ala Ala Ala Gly Ser Tyr Gly Ser Gly Pro Gly Gln Tyr
465             470             475             480

Gly Pro Tyr Gly Pro Gly Gln Ser Gly Pro Gly Ser Gly Gln Gln Gly
                485             490             495

Gln Gly Pro Tyr Gly Pro Gly Ala Ser Ala Ala Ala Ala Ala Ala
                500             505             510

Gly Ser Tyr Gly Pro Gly Gln Gln Val Leu Ile Gly Pro Tyr Gly Pro
        515             520             525

Gly Pro Ser Ala Ala Ala Ala Ala Ala Ala Gly Pro Gly Ser Gly Gln
    530             535             540

Tyr Gly Pro Gly Ala Ser Gly Gln Asn Gly Pro Gly Ser Gly Gln Tyr
545             550             555             560

Gly Pro Gly Gln Gln Gly Pro Gly Pro Ser Ala Ala Ala Ala Ala Ala
                565             570             575

Ala Gly Pro Gly Ser Gly Gln Gln Gly Pro Gly Ala Ser Val Leu Ile
        580             585             590
```

```
<210>   21
<211>   619
<212>   PRT
<213>   Artificial Sequence

<220>
<223>   Met-PRT666

<400>   21

Met Gly Pro Gly Gln Gln Gly Pro Tyr Gly Pro Gly Ala Ser Ala Ala
1               5               10              15
```

Ala Ala Ala Ala Ala Gly Ser Asn Gly Pro Gly Ser Gly Gln Gln Gly
                  20                  25                  30

Pro Gly Gln Ser Gly Gln Tyr Gly Pro Gly Gln Gln Gly Pro Gly Gln
              35                  40                  45

Gln Gly Pro Gly Ser Ser Ala Ala Ala Ala Ala Ala Gly Pro Gly
          50                  55                  60

Gln Tyr Val Leu Ile Gly Pro Gly Gln Gln Val Leu Ile Gly Pro Ser
65                  70                  75                  80

Ala Ser Ala Ala Ala Ala Ala Ala Ala Gly Pro Gly Ser Gly Gln Gln
              85                  90                  95

Gly Pro Gly Ala Ser Gly Gln Tyr Gly Pro Gly Gln Gln Gly Pro Gly
          100                 105                 110

Gln Gln Gly Pro Gly Ser Ser Ala Ala Ala Ala Ala Ala Gly Ser
          115                 120                 125

Tyr Gly Ser Val Leu Ile Gly Pro Gly Gln Gln Val Leu Ile Gly Pro
          130                 135                 140

Tyr Gly Ser Ala Ala Ala Ala Ala Ala Gly Pro Gly Ser Gly Gln
145                 150                 155                 160

Tyr Gly Gln Gly Pro Tyr Gly Pro Gly Ala Ser Gly Pro Gly Gln Tyr
              165                 170                 175

Gly Pro Gly Gln Gln Gly Pro Ser Ala Ser Ala Ala Ala Ala Ala Ala
          180                 185                 190

Ala Gly Ser Gly Gln Gln Val Leu Ile Gly Pro Gly Gln Tyr Val Leu
          195                 200                 205

Ile Gly Pro Tyr Ala Ser Ala Ala Ala Ala Ala Ala Gly Ser Tyr
          210                 215                 220

Gly Ser Gly Pro Gly Gln Gln Gly Pro Tyr Gly Pro Gly Gln Ser Gly
225                 230                 235                 240

Ser Gly Gln Gln Gly Pro Gly Gln Gln Gly Pro Tyr Ala Ser Ala Ala
              245                 250                 255

Ala Ala Ala Ala Ala Gly Pro Gly Gln Gln Val Leu Ile Gly Pro Tyr
          260                 265                 270

```
Val Leu Ile Gly Pro Gly Ser Ser Ala Ala Ala Ala Ala Ala Gly
        275                 280                 285

Ser Tyr Gly Tyr Gly Pro Gly Gln Gln Gly Pro Tyr Gly Pro Gly Ala
        290                 295                 300

Ser Gly Gln Asn Gly Pro Gly Ser Gly Gln Tyr Gly Pro Gly Gln Gln
305                 310                 315                 320

Gly Pro Gly Pro Ser Ala Ala Ala Ala Ala Ala Gly Pro Gly Gln
                325                 330                 335

Gln Val Leu Ile Gly Pro Tyr Val Leu Ile Gly Pro Gly Ala Ser Ala
            340                 345                 350

Ala Ala Ala Ala Ala Ala Gly Ser Tyr Gly Pro Gly Gln Gln Gly Pro
        355                 360                 365

Gly Gln Tyr Gly Pro Gly Ser Ser Gly Pro Gly Gln Gln Gly Pro Tyr
    370                 375                 380

Gly Pro Gly Ser Ser Ala Ala Ala Ala Ala Ala Gly Ser Tyr Gly
385                 390                 395                 400

Pro Gly Gln Gln Val Leu Ile Gly Pro Tyr Val Leu Ile Gly Pro Gly
                405                 410                 415

Pro Ser Ala Ala Ala Ala Ala Ala Ala Gly Ser Tyr Gln Gln Gly Pro
        420                 425                 430

Gly Gln Gln Gly Pro Tyr Gly Pro Gly Ala Ser Gly Pro Gly Gln Gln
    435                 440                 445

Gly Pro Tyr Gly Pro Gly Ala Ser Ala Ala Ala Ala Ala Ala Gly
    450                 455                 460

Pro Gly Gln Tyr Val Leu Ile Gly Pro Gly Gln Gln Val Leu Ile Gly
465                 470                 475                 480

Pro Ser Ala Ser Ala Ala Ala Ala Ala Ala Gly Ser Tyr Gly Ser
        485                 490                 495

Gly Pro Gly Gln Tyr Gly Pro Tyr Gly Pro Gly Gln Ser Gly Pro Gly
        500                 505                 510

Ser Gly Gln Gln Gly Gln Gly Pro Tyr Gly Pro Gly Ala Ser Ala Ala
```

```
                    515                      520                      525


        Ala Ala Ala Ala Ala Gly Ser Tyr Gly Pro Gly Gln Gln Val Leu Ile
            530                     535                 540


        Gly Pro Tyr Val Leu Ile Gly Pro Gly Pro Ser Ala Ala Ala Ala Ala
        545                     550                 555                 560


        Ala Ala Gly Pro Gly Ser Gly Gln Tyr Gly Pro Gly Ala Ser Gly Gln
                        565                 570                 575


        Asn Gly Pro Gly Ser Gly Gln Tyr Gly Pro Gly Gln Gln Gly Pro Gly
                    580                 585                 590


        Pro Ser Ala Ala Ala Ala Ala Ala Ala Gly Pro Gly Ser Gly Gln Gln
                595                 600                 605


        Val Leu Ile Gly Pro Gly Ala Ser Val Leu Ile
            610                 615


        <210>   22
        <211>   623
        <212>   PRT
        <213>   Artificial Sequence

        <220>
        <223>   PRT720

        <400>   22

        Met His His His His His His Ser Ser Gly Ser Ser Gly Pro Gly Gln
        1               5                   10                  15


        Gln Gly Pro Tyr Gly Pro Gly Ala Ser Ala Ala Ala Ala Ala Gly Gln
                    20                  25                  30


        Asn Gly Pro Gly Ser Gly Gln Gln Gly Pro Gly Gln Ser Gly Gln Tyr
                35                  40                  45


        Gly Pro Gly Gln Gln Gly Pro Gly Gln Gln Gly Pro Gly Ser Ser Ala
            50                  55                  60


        Ala Ala Ala Ala Gly Pro Gly Gln Tyr Val Leu Ile Gly Pro Gly Gln
        65                  70                  75                  80


        Gln Val Leu Ile Gly Pro Ser Ala Ser Ala Ala Ala Ala Ala Gly Pro
                        85                  90                  95


        Gly Ser Gly Gln Gln Gly Pro Gly Ala Ser Gly Gln Tyr Gly Pro Gly
                    100                 105                 110
```

Gln Gln Gly Pro Gly Gln Gln Gly Pro Gly Ser Ser Ala Ala Ala Ala
    115            120            125

Ala Gly Ser Tyr Gly Ser Val Leu Ile Gly Pro Gly Gln Gln Val Leu
    130            135            140

Ile Gly Pro Tyr Gly Ser Ala Ala Ala Ala Ala Gly Pro Gly Ser Gly
145            150            155            160

Gln Tyr Gly Gln Gly Pro Tyr Gly Pro Gly Ala Ser Gly Pro Gly Gln
          165            170            175

Tyr Gly Pro Gly Gln Gln Gly Pro Ser Ala Ser Ala Ala Ala Ala Ala
          180            185            190

Gly Ser Gly Gln Gln Val Leu Ile Gly Pro Gly Gln Tyr Val Leu Ile
          195            200            205

Gly Pro Tyr Ala Ser Ala Ala Ala Ala Ala Gly Gln Tyr Gly Ser Gly
          210            215            220

Pro Gly Gln Gln Gly Pro Tyr Gly Pro Gly Gln Ser Gly Ser Gly Gln
225            230            235            240

Gln Gly Pro Gly Gln Gln Gly Pro Tyr Ala Ser Ala Ala Ala Ala Ala
          245            250            255

Gly Pro Gly Gln Gln Val Leu Ile Gly Pro Tyr Val Leu Ile Gly Pro
          260            265            270

Gly Ser Ser Ala Ala Ala Ala Ala Gly Gln Tyr Gly Tyr Gly Pro Gly
          275            280            285

Gln Gln Gly Pro Tyr Gly Pro Gly Ala Ser Gly Gln Asn Gly Pro Gly
          290            295            300

Ser Gly Gln Tyr Gly Pro Gly Gln Gln Gly Pro Gly Gln Ser Ala Ala
305            310            315            320

Ala Ala Ala Gly Pro Gly Gln Gln Val Leu Ile Gly Pro Tyr Val Leu
          325            330            335

Ile Gly Pro Gly Ala Ser Ala Ala Ala Ala Ala Gly Gln Tyr Gly Pro
          340            345            350

Gly Gln Gln Gly Pro Gly Gln Tyr Gly Pro Gly Ser Ser Gly Pro Gly

|  | 355 |  |  |  |  | 360 |  |  |  |  | 365 |  |  |  |
|---|---|---|---|---|---|---|---|---|---|---|---|---|---|---|

Gln Gln Gly Pro Tyr Gly Pro Gly Ser Ser Ala Ala Ala Ala Ala Gly
370 375 380

Ser Tyr Gly Pro Gly Gln Gln Val Leu Ile Gly Pro Tyr Val Leu Ile
385 390 395 400

Gly Pro Gly Pro Ser Ala Ala Ala Ala Ala Gly Gln Tyr Gln Gln Gly
405 410 415

Pro Gly Gln Gln Gly Pro Tyr Gly Pro Gly Ala Ser Gly Pro Gly Gln
420 425 430

Gln Gly Pro Tyr Gly Pro Gly Ala Ser Ala Ala Ala Ala Ala Gly Pro
435 440 445

Gly Gln Tyr Val Leu Ile Gly Pro Gly Gln Gln Val Leu Ile Gly Pro
450 455 460

Ser Ala Ser Ala Ala Ala Ala Ala Gly Gln Tyr Gly Ser Gly Pro Gly
465 470 475 480

Gln Tyr Gly Pro Tyr Gly Pro Gly Gln Ser Gly Pro Gly Ser Gly Gln
485 490 495

Gln Gly Gln Gly Pro Tyr Gly Pro Gly Ala Ser Ala Ala Ala Ala Ala
500 505 510

Gly Ser Tyr Gly Pro Gly Gln Gln Val Leu Ile Gly Pro Tyr Val Leu
515 520 525

Ile Gly Pro Gly Pro Ser Ala Ala Ala Ala Gly Pro Gly Ser Gly
530 535 540

Gln Tyr Gly Pro Gly Ala Ser Gly Gln Asn Gly Pro Gly Ser Gly Gln
545 550 555 560

Tyr Gly Pro Gly Gln Gln Gly Pro Gly Gln Ser Ala Ala Ala Ala Ala
565 570 575

Gly Gln Tyr Gln Gln Val Leu Ile Gly Pro Gly Gln Gln Gly Pro Tyr
580 585 590

Val Leu Ile Gly Pro Gly Ala Ser Ala Ala Ala Ala Ala Gly Pro Gly
595 600 605

Ser Gly Gln Gln Val Leu Ile Gly Pro Gly Ala Ser Val Leu Ile
    610                 615                 620

<210> 23
<211> 603
<212> PRT
<213> Artificial Sequence

<220>
<223> PRT665

<400> 23

Met His His His His His His Ser Ser Gly Ser Ser Gly Pro Gly Gln
1               5                   10                  15

Gln Gly Pro Tyr Gly Pro Gly Ala Ser Ala Ala Ala Ala Ala Ala Ala
            20                  25                  30

Gly Ser Asn Gly Pro Gly Ser Gly Gln Gln Gly Pro Gly Gln Ser Gly
        35                  40                  45

Gln Tyr Gly Pro Gly Gln Gln Gly Pro Gly Gln Gln Gly Pro Gly Ser
    50                  55                  60

Ser Ala Ala Ala Ala Ala Ala Ala Gly Pro Gly Gln Tyr Val Leu Ile
65                  70                  75                  80

Gly Pro Gly Gln Gln Gly Pro Ser Ala Ser Ala Ala Ala Ala Ala Ala
            85                  90                  95

Ala Gly Pro Gly Ser Gly Gln Gln Gly Pro Gly Ala Ser Gly Gln Tyr
            100                 105                 110

Gly Pro Gly Gln Gln Gly Pro Gly Gln Gln Gly Pro Gly Ser Ser Ala
            115                 120                 125

Ala Ala Ala Ala Ala Gly Ser Tyr Gly Ser Val Leu Ile Gly Pro
    130                 135                 140

Gly Gln Gln Gly Pro Tyr Gly Ser Ala Ala Ala Ala Ala Ala Ala Gly
145                 150                 155                 160

Pro Gly Ser Gly Gln Tyr Gly Gln Gly Pro Tyr Gly Pro Gly Ala Ser
            165                 170                 175

Gly Pro Gly Gln Tyr Gly Pro Gly Gln Gln Gly Pro Ser Ala Ser Ala
            180                 185                 190

Ala Ala Ala Ala Ala Gly Ser Gly Gln Gln Val Leu Ile Gly Pro

103

|     | 195 |     |     |     | 200 |     |     |     | 205 |     |     |     |     |
|-----|-----|-----|-----|-----|-----|-----|-----|-----|-----|-----|-----|-----|-----|

Gly Gln Tyr Gly Pro Tyr Ala Ser Ala Ala Ala Ala Ala Ala Gly
    210     215     220

Ser Tyr Gly Ser Gly Pro Gly Gln Gln Gly Pro Tyr Gly Pro Gly Gln
225     230     235     240

Ser Gly Ser Gly Gln Gln Gly Pro Gly Gln Gln Gly Pro Tyr Ala Ser
    245     250     255

Ala Ala Ala Ala Ala Ala Ala Gly Pro Gly Gln Gln Val Leu Ile Gly
    260     265     270

Pro Tyr Gly Pro Gly Ser Ser Ala Ala Ala Ala Ala Ala Gly Ser
    275     280     285

Tyr Gly Tyr Gly Pro Gly Gln Gln Gly Pro Tyr Gly Pro Gly Ala Ser
    290     295     300

Gly Gln Asn Gly Pro Gly Ser Gly Gln Tyr Gly Pro Gly Gln Gln Gly
305     310     315     320

Pro Gly Pro Ser Ala Ala Ala Ala Ala Ala Gly Pro Gly Gln Gln
    325     330     335

Val Leu Ile Gly Pro Tyr Gly Pro Gly Ala Ser Ala Ala Ala Ala Ala
    340     345     350

Ala Ala Gly Ser Tyr Gly Pro Gly Gln Gln Gly Pro Gly Gln Tyr Gly
    355     360     365

Pro Gly Ser Ser Gly Pro Gly Gln Gln Gly Pro Tyr Gly Pro Gly Ser
    370     375     380

Ser Ala Ala Ala Ala Ala Ala Gly Ser Tyr Gly Pro Gly Gln Gln
385     390     395     400

Val Leu Ile Gly Pro Tyr Gly Pro Gly Pro Ser Ala Ala Ala Ala Ala
    405     410     415

Ala Ala Gly Ser Tyr Gln Gln Gly Pro Gly Gln Gln Gly Pro Tyr Gly
    420     425     430

Pro Gly Ala Ser Gly Pro Gly Gln Gln Gly Pro Tyr Gly Pro Gly Ala
    435     440     445

Ser Ala Ala Ala Ala Ala Ala Ala Gly Pro Gly Gln Tyr Val Leu Ile
    450                 455                 460

Gly Pro Gly Gln Gln Gly Pro Ser Ala Ser Ala Ala Ala Ala Ala
465                 470                 475                 480

Ala Gly Ser Tyr Gly Ser Gly Pro Gly Gln Tyr Gly Pro Tyr Gly Pro
            485                 490                 495

Gly Gln Ser Gly Pro Gly Ser Gly Gln Gln Gly Gln Gly Pro Tyr Gly
        500                 505                 510

Pro Gly Ala Ser Ala Ala Ala Ala Ala Ala Ala Gly Ser Tyr Gly Pro
        515                 520                 525

Gly Gln Gln Val Leu Ile Gly Pro Tyr Gly Pro Gly Pro Ser Ala Ala
    530                 535                 540

Ala Ala Ala Ala Ala Gly Pro Gly Ser Gly Gln Tyr Gly Pro Gly Ala
545                 550                 555                 560

Ser Gly Gln Asn Gly Pro Gly Ser Gly Gln Tyr Gly Pro Gly Gln Gln
            565                 570                 575

Gly Pro Gly Pro Ser Ala Ala Ala Ala Ala Ala Ala Gly Pro Gly Ser
        580                 585                 590

Gly Gln Gln Gly Pro Gly Ala Ser Val Leu Ile
    595                 600

<210> 24
<211> 630
<212> PRT
<213> Artificial Sequence

<220>
<223> PRT666

<400> 24

Met His His His His His His Ser Ser Gly Ser Ser Gly Pro Gly Gln
1                 5                 10                  15

Gln Gly Pro Tyr Gly Pro Gly Ala Ser Ala Ala Ala Ala Ala Ala Ala
            20                  25                  30

Gly Ser Asn Gly Pro Gly Ser Gly Gln Gln Gly Pro Gly Gln Ser Gly
        35                  40                  45

Gln Tyr Gly Pro Gly Gln Gln Gly Pro Gly Gln Gln Gly Pro Gly Ser

50                          55                          60

Ser Ala Ala Ala Ala Ala Ala Ala Gly Pro Gly Gln Tyr Val Leu Ile
65                  70              75                      80

Gly Pro Gly Gln Gln Val Leu Ile Gly Pro Ser Ala Ser Ala Ala Ala
                85                  90                  95

Ala Ala Ala Ala Gly Pro Gly Ser Gly Gln Gln Gly Pro Gly Ala Ser
            100             105             110

Gly Gln Tyr Gly Pro Gly Gln Gln Gly Pro Gly Gln Gln Gly Pro Gly
        115             120             125

Ser Ser Ala Ala Ala Ala Ala Ala Gly Ser Tyr Gly Ser Val Leu
    130             135             140

Ile Gly Pro Gly Gln Gln Val Leu Ile Gly Pro Tyr Gly Ser Ala Ala
145             150             155             160

Ala Ala Ala Ala Ala Gly Pro Gly Ser Gly Gln Tyr Gly Gln Gly Pro
            165             170             175

Tyr Gly Pro Gly Ala Ser Gly Pro Gly Gln Tyr Gly Pro Gly Gln Gln
        180             185             190

Gly Pro Ser Ala Ser Ala Ala Ala Ala Ala Ala Gly Ser Gly Gln
        195             200             205

Gln Val Leu Ile Gly Pro Gly Gln Tyr Val Leu Ile Gly Pro Tyr Ala
    210             215             220

Ser Ala Ala Ala Ala Ala Ala Gly Ser Tyr Gly Ser Gly Pro Gly
225             230             235             240

Gln Gln Gly Pro Tyr Gly Pro Gly Gln Ser Gly Ser Gly Gln Gln Gly
        245             250             255

Pro Gly Gln Gln Gly Pro Tyr Ala Ser Ala Ala Ala Ala Ala Ala
        260             265             270

Gly Pro Gly Gln Gln Val Leu Ile Gly Pro Tyr Val Leu Ile Gly Pro
        275             280             285

Gly Ser Ser Ala Ala Ala Ala Ala Ala Gly Ser Tyr Gly Tyr Gly
    290             295             300

```
Pro Gly Gln Gln Gly Pro Tyr Gly Pro Gly Ala Ser Gly Gln Asn Gly
305             310             315             320


Pro Gly Ser Gly Gln Tyr Gly Pro Gly Gln Gln Gly Pro Gly Pro Ser
            325             330             335


Ala Ala Ala Ala Ala Ala Ala Gly Pro Gly Gln Gln Val Leu Ile Gly
        340             345             350


Pro Tyr Val Leu Ile Gly Pro Gly Ala Ser Ala Ala Ala Ala Ala Ala
        355             360             365


Ala Gly Ser Tyr Gly Pro Gly Gln Gln Gly Pro Gly Gln Tyr Gly Pro
    370             375             380


Gly Ser Ser Gly Pro Gly Gln Gln Gly Pro Tyr Gly Pro Gly Ser Ser
385             390             395             400


Ala Ala Ala Ala Ala Ala Ala Gly Ser Tyr Gly Pro Gly Gln Gln Val
            405             410             415


Leu Ile Gly Pro Tyr Val Leu Ile Gly Pro Gly Pro Ser Ala Ala Ala
        420             425             430


Ala Ala Ala Ala Gly Ser Tyr Gln Gln Gly Pro Gly Gln Gln Gly Pro
    435             440             445


Tyr Gly Pro Gly Ala Ser Gly Pro Gly Gln Gln Gly Pro Tyr Gly Pro
    450             455             460


Gly Ala Ser Ala Ala Ala Ala Ala Ala Gly Pro Gly Gln Tyr Val
465             470             475             480


Leu Ile Gly Pro Gly Gln Gln Val Leu Ile Gly Pro Ser Ala Ser Ala
            485             490             495


Ala Ala Ala Ala Ala Ala Gly Ser Tyr Gly Ser Gly Pro Gly Gln Tyr
        500             505             510


Gly Pro Tyr Gly Pro Gly Gln Ser Gly Pro Gly Ser Gly Gln Gln Gly
    515             520             525


Gln Gly Pro Tyr Gly Pro Gly Ala Ser Ala Ala Ala Ala Ala Ala Ala
    530             535             540


Gly Ser Tyr Gly Pro Gly Gln Gln Val Leu Ile Gly Pro Tyr Val Leu
545             550             555             560
```

107

```
Ile Gly Pro Gly Pro Ser Ala Ala Ala Ala Ala Ala Gly Pro Gly
            565             570             575

Ser Gly Gln Tyr Gly Pro Gly Ala Ser Gly Gln Asn Gly Pro Gly Ser
            580             585             590

Gly Gln Tyr Gly Pro Gly Gln Gln Gly Pro Gly Pro Ser Ala Ala Ala
            595             600             605

Ala Ala Ala Ala Gly Pro Gly Ser Gly Gln Gln Val Leu Ile Gly Pro
    610             615             620

Gly Ala Ser Val Leu Ile
625             630
```

```
<210>  25
<211>  593
<212>  PRT
<213>  Artificial Sequence

<220>
<223>  Met-PRT888

<400>  25
```

```
Met Gly Ser Ser Gly Pro Gly Val Leu Gly Pro Tyr Gly Pro Gly Ala
1               5               10              15

Ser Ala Ala Ala Ala Ala Gly Gln Asn Gly Pro Gly Ser Gly Val Leu
            20              25              30

Gly Pro Gly Gln Ser Gly Gln Tyr Gly Pro Gly Val Leu Gly Pro Gly
            35              40              45

Val Leu Gly Pro Gly Ser Ser Ala Ala Ala Ala Ala Gly Pro Gly Gln
    50              55              60

Tyr Gly Pro Gly Val Leu Gly Pro Ser Ala Ser Ala Ala Ala Ala Ala
65              70              75              80

Gly Pro Gly Ser Gly Val Leu Gly Pro Gly Ala Ser Gly Gln Tyr Gly
            85              90              95

Pro Gly Val Leu Gly Pro Gly Val Leu Gly Pro Gly Ser Ser Ala Ala
            100             105             110

Ala Ala Ala Gly Gln Tyr Gly Ser Gly Pro Gly Val Leu Gly Pro Tyr
            115             120             125
```

108

```
Gly Ser Ala Ala Ala Ala Ala Gly Pro Gly Ser Gly Gln Tyr Gly Gln
    130             135             140

Gly Pro Tyr Gly Pro Gly Ala Ser Gly Pro Gly Gln Tyr Gly Pro Gly
145             150             155             160

Val Leu Gly Pro Ser Ala Ser Ala Ala Ala Ala Ala Gly Ser Gly Val
                165             170             175

Leu Gly Pro Gly Gln Tyr Gly Pro Tyr Ala Ser Ala Ala Ala Ala Ala
            180             185             190

Gly Gln Tyr Gly Ser Gly Pro Gly Val Leu Gly Pro Tyr Gly Pro Gly
        195             200             205

Gln Ser Gly Ser Gly Val Leu Gly Pro Gly Val Leu Gly Pro Tyr Ala
    210             215             220

Ser Ala Ala Ala Ala Ala Gly Pro Gly Val Leu Gly Pro Tyr Gly Pro
225             230             235             240

Gly Ser Ser Ala Ala Ala Ala Ala Gly Gln Tyr Gly Tyr Gly Pro Gly
            245             250             255

Val Leu Gly Pro Tyr Gly Pro Gly Ala Ser Gly Gln Asn Gly Pro Gly
            260             265             270

Ser Gly Gln Tyr Gly Pro Gly Val Leu Gly Pro Gly Gln Ser Ala Ala
        275             280             285

Ala Ala Ala Gly Pro Gly Val Leu Gly Pro Tyr Gly Pro Gly Ala Ser
    290             295             300

Ala Ala Ala Ala Ala Gly Gln Tyr Gly Pro Gly Val Leu Gly Pro Gly
305             310             315             320

Gln Tyr Gly Pro Gly Ser Ser Gly Pro Gly Val Leu Gly Pro Tyr Gly
            325             330             335

Pro Gly Ser Ser Ala Ala Ala Ala Ala Gly Gln Tyr Gly Pro Gly Val
            340             345             350

Leu Gly Pro Tyr Gly Pro Gly Gln Ser Ala Ala Ala Ala Ala Gly Gln
        355             360             365

Tyr Val Leu Gly Pro Gly Val Leu Gly Pro Tyr Gly Pro Gly Ala Ser
370             375             380
```

```
Gly Pro Gly Val Leu Gly Pro Tyr Gly Pro Gly Ala Ser Ala Ala Ala
385             390             395             400

Ala Ala Gly Pro Gly Gln Tyr Gly Pro Gly Val Leu Gly Pro Ser Ala
            405             410             415

Ser Ala Ala Ala Ala Ala Gly Gln Tyr Gly Ser Gly Pro Gly Gln Tyr
        420             425             430

Gly Pro Tyr Gly Pro Gly Gln Ser Gly Pro Gly Ser Gly Val Leu Gly
        435             440             445

Gln Gly Pro Tyr Gly Pro Gly Ala Ser Ala Ala Ala Ala Ala Gly Gln
    450             455             460

Tyr Gly Pro Gly Val Leu Gly Pro Tyr Gly Pro Gly Gln Ser Ala Ala
465             470             475             480

Ala Ala Ala Gly Pro Gly Ser Gly Gln Tyr Gly Pro Gly Ala Ser Gly
            485             490             495

Gln Asn Gly Pro Gly Ser Gly Gln Tyr Gly Pro Gly Val Leu Gly Pro
            500             505             510

Gly Gln Ser Ala Ala Ala Ala Ala Gly Gln Tyr Val Leu Gly Pro Gly
        515             520             525

Val Leu Gly Pro Tyr Gly Pro Gly Ala Ser Ala Ala Ala Ala Ala Gly
    530             535             540

Gln Tyr Gly Ser Gly Pro Gly Val Leu Gly Pro Tyr Gly Pro Gly Gln
545             550             555             560

Ser Gly Ser Gly Val Leu Gly Pro Gly Val Leu Gly Pro Tyr Ala Ser
            565             570             575

Ala Ala Ala Ala Ala Gly Pro Gly Ser Gly Val Leu Gly Pro Gly Ala
        580             585             590

Ser
```

```
<210>  26
<211>  590
<212>  PRT
<213>  Artificial Sequence

<220>
```

<223> Met-PRT965

<400> 26

Met Gly Pro Gly Thr Ser Gly Pro Tyr Gly Pro Gly Ala Ser Ala Ala
1               5                   10                  15

Ala Ala Ala Gly Ala Asn Gly Pro Gly Ser Gly Thr Ser Gly Pro Gly
            20                  25                  30

Ala Ser Gly Ala Tyr Gly Pro Gly Thr Ser Gly Pro Gly Thr Ser Gly
            35                  40                  45

Pro Gly Ser Ser Ala Ala Ala Ala Ala Gly Pro Gly Ala Tyr Gly Pro
            50                  55                  60

Gly Thr Ser Gly Pro Ser Ala Ser Ala Ala Ala Ala Gly Pro Gly
65                  70                  75                  80

Ser Gly Thr Ser Gly Pro Gly Ala Ser Gly Ala Tyr Gly Pro Gly Thr
                85                  90                  95

Ser Gly Pro Gly Thr Ser Gly Pro Gly Ser Ser Ala Ala Ala Ala Ala
            100                 105                 110

Gly Ala Tyr Gly Ser Gly Pro Gly Thr Ser Gly Pro Tyr Gly Ser Ala
        115                 120                 125

Ala Ala Ala Ala Gly Pro Gly Ser Gly Ala Tyr Gly Ala Gly Pro Tyr
    130                 135                 140

Gly Pro Gly Ala Ser Gly Pro Gly Ala Tyr Gly Pro Gly Thr Ser Gly
145                 150                 155                 160

Pro Ser Ala Ser Ala Ala Ala Ala Gly Ser Gly Thr Ser Gly Pro
            165                 170                 175

Gly Ala Tyr Gly Pro Tyr Ala Ser Ala Ala Ala Ala Gly Ala Tyr
        180                 185                 190

Gly Ser Gly Pro Gly Thr Ser Gly Pro Tyr Gly Pro Gly Ala Ser Gly
        195                 200                 205

Ser Gly Thr Ser Gly Pro Gly Thr Ser Gly Pro Tyr Ala Ser Ala Ala
    210                 215                 220

Ala Ala Ala Gly Pro Gly Thr Ser Gly Pro Tyr Gly Pro Gly Ser Ser
225                 230                 235                 240

Ala Ala Ala Ala Ala Gly Ala Tyr Gly Tyr Gly Pro Gly Thr Ser Gly
            245             250             255

Pro Tyr Gly Pro Gly Ala Ser Gly Ala Asn Gly Pro Gly Ser Gly Ala
            260             265             270

Tyr Gly Pro Gly Thr Ser Gly Pro Gly Ala Ser Ala Ala Ala Ala Ala
            275             280             285

Gly Pro Gly Thr Ser Gly Pro Tyr Gly Pro Gly Ala Ser Ala Ala Ala
            290             295             300

Ala Ala Gly Ala Tyr Gly Pro Gly Thr Ser Gly Pro Gly Ala Tyr Gly
305             310             315             320

Pro Gly Ser Ser Gly Pro Gly Thr Ser Gly Pro Tyr Gly Pro Gly Ser
            325             330             335

Ser Ala Ala Ala Ala Ala Gly Ala Tyr Gly Pro Gly Thr Ser Gly Pro
            340             345             350

Tyr Gly Pro Gly Ala Ser Ala Ala Ala Ala Ala Gly Ala Tyr Thr Ser
            355             360             365

Gly Pro Gly Thr Ser Gly Pro Tyr Gly Pro Gly Ala Ser Gly Pro Gly
            370             375             380

Thr Ser Gly Pro Tyr Gly Pro Gly Ala Ser Ala Ala Ala Ala Ala Gly
385             390             395             400

Pro Gly Ala Tyr Gly Pro Gly Thr Ser Gly Pro Ser Ala Ser Ala Ala
            405             410             415

Ala Ala Ala Gly Ala Tyr Gly Ser Gly Pro Gly Ala Tyr Gly Pro Tyr
            420             425             430

Gly Pro Gly Ala Ser Gly Pro Gly Ser Gly Thr Ser Gly Ala Gly Pro
            435             440             445

Tyr Gly Pro Gly Ala Ser Ala Ala Ala Ala Gly Ala Tyr Gly Pro
            450             455             460

Gly Thr Ser Gly Pro Tyr Gly Pro Gly Ala Ser Ala Ala Ala Ala Ala
465             470             475             480

Gly Pro Gly Ser Gly Ala Tyr Gly Pro Gly Ala Ser Gly Ala Asn Gly
            485             490             495

112

```
Pro Gly Ser Gly Ala Tyr Gly Pro Gly Thr Ser Gly Pro Gly Ala Ser
            500                 505                 510

Ala Ala Ala Ala Ala Gly Ala Tyr Thr Ser Gly Pro Gly Thr Ser Gly
            515                 520                 525

Pro Tyr Gly Pro Gly Ala Ser Ala Ala Ala Ala Ala Gly Ala Tyr Gly
        530                 535                 540

Ser Gly Pro Gly Thr Ser Gly Pro Tyr Gly Pro Gly Ala Ser Gly Ser
545                 550                 555                 560

Gly Thr Ser Gly Pro Gly Thr Ser Gly Pro Tyr Ala Ser Ala Ala Ala
                565                 570                 575

Ala Ala Gly Pro Gly Ser Gly Thr Ser Gly Pro Gly Ala Ser
            580                 585                 590


<210>  27
<211>  593
<212>  PRT
<213>  Artificial Sequence

<220>
<223>  Met-PRT889

<400>  27

Met Gly Ser Ser Gly Pro Gly Val Leu Gly Pro Tyr Gly Pro Gly Ala
1                   5                   10                  15

Ser Ala Ala Ala Ala Ala Gly Ile Asn Gly Pro Gly Ser Gly Val Leu
            20                  25                  30

Gly Pro Gly Ile Ser Gly Ile Tyr Gly Pro Gly Val Leu Gly Pro Gly
            35                  40                  45

Val Leu Gly Pro Gly Ser Ser Ala Ala Ala Ala Ala Gly Pro Gly Ile
        50                  55                  60

Tyr Gly Pro Gly Val Leu Gly Pro Ser Ala Ser Ala Ala Ala Ala Ala
65                  70                  75                  80

Gly Pro Gly Ser Gly Val Leu Gly Pro Gly Ala Ser Gly Ile Tyr Gly
                85                  90                  95

Pro Gly Val Leu Gly Pro Gly Val Leu Gly Pro Gly Ser Ser Ala Ala
            100                 105                 110
```

```
Ala Ala Ala Gly Ile Tyr Gly Ser Gly Pro Gly Val Leu Gly Pro Tyr
        115                 120                 125

Gly Ser Ala Ala Ala Ala Ala Gly Pro Gly Ser Gly Ile Tyr Gly Ile
    130                 135                 140

Gly Pro Tyr Gly Pro Gly Ala Ser Gly Pro Gly Ile Tyr Gly Pro Gly
    145                 150                 155                 160

Val Leu Gly Pro Ser Ala Ser Ala Ala Ala Ala Gly Ser Gly Val
                165                 170                 175

Leu Gly Pro Gly Ile Tyr Gly Pro Tyr Ala Ser Ala Ala Ala Ala Ala
            180                 185                 190

Gly Ile Tyr Gly Ser Gly Pro Gly Val Leu Gly Pro Tyr Gly Pro Gly
        195                 200                 205

Ile Ser Gly Ser Gly Val Leu Gly Pro Gly Val Leu Gly Pro Tyr Ala
    210                 215                 220

Ser Ala Ala Ala Ala Ala Gly Pro Gly Val Leu Gly Pro Tyr Gly Pro
225                 230                 235                 240

Gly Ser Ser Ala Ala Ala Ala Ala Gly Ile Tyr Gly Tyr Gly Pro Gly
                245                 250                 255

Val Leu Gly Pro Tyr Gly Pro Gly Ala Ser Gly Ile Asn Gly Pro Gly
            260                 265                 270

Ser Gly Ile Tyr Gly Pro Gly Val Leu Gly Pro Gly Ile Ser Ala Ala
        275                 280                 285

Ala Ala Ala Gly Pro Gly Val Leu Gly Pro Tyr Gly Pro Gly Ala Ser
    290                 295                 300

Ala Ala Ala Ala Ala Gly Ile Tyr Gly Pro Gly Val Leu Gly Pro Gly
305                 310                 315                 320

Ile Tyr Gly Pro Gly Ser Ser Gly Pro Gly Val Leu Gly Pro Tyr Gly
            325                 330                 335

Pro Gly Ser Ser Ala Ala Ala Ala Ala Gly Ile Tyr Gly Pro Gly Val
        340                 345                 350

Leu Gly Pro Tyr Gly Pro Gly Ile Ser Ala Ala Ala Ala Ala Gly Ile
    355                 360                 365
```

```
Tyr Val Leu Gly Pro Gly Val Leu Gly Pro Tyr Gly Pro Gly Ala Ser
    370                 375                 380

Gly Pro Gly Val Leu Gly Pro Tyr Gly Pro Gly Ala Ser Ala Ala Ala
385                 390                 395                 400

Ala Ala Gly Pro Gly Ile Tyr Gly Pro Gly Val Leu Gly Pro Ser Ala
                405                 410                 415

Ser Ala Ala Ala Ala Ala Gly Ile Tyr Gly Ser Gly Pro Gly Ile Tyr
        420                 425                 430

Gly Pro Tyr Gly Pro Gly Ile Ser Gly Pro Gly Ser Gly Val Leu Gly
        435                 440                 445

Ile Gly Pro Tyr Gly Pro Gly Ala Ser Ala Ala Ala Ala Ala Gly Ile
    450                 455                 460

Tyr Gly Pro Gly Val Leu Gly Pro Tyr Gly Pro Gly Ile Ser Ala Ala
465                 470                 475                 480

Ala Ala Ala Gly Pro Gly Ser Gly Ile Tyr Gly Pro Gly Ala Ser Gly
            485                 490                 495

Ile Asn Gly Pro Gly Ser Gly Ile Tyr Gly Pro Gly Val Leu Gly Pro
        500                 505                 510

Gly Ile Ser Ala Ala Ala Ala Ala Gly Ile Tyr Val Leu Gly Pro Gly
        515                 520                 525

Val Leu Gly Pro Tyr Gly Pro Gly Ala Ser Ala Ala Ala Ala Ala Gly
    530                 535                 540

Ile Tyr Gly Ser Gly Pro Gly Val Leu Gly Pro Tyr Gly Pro Gly Ile
545                 550                 555                 560

Ser Gly Ser Gly Val Leu Gly Pro Gly Val Leu Gly Pro Tyr Ala Ser
                565                 570                 575

Ala Ala Ala Ala Ala Gly Pro Gly Ser Gly Val Leu Gly Pro Gly Ala
        580                 585                 590

Ser
```

<210> 28

<211> 590
<212> PRT
<213> Artificial Sequence

<220>
<223> Met-PRT916

<400> 28

```
Met Gly Pro Gly Val Ile Gly Pro Tyr Gly Pro Gly Ala Ser Ala Ala
1               5                   10                  15


Ala Ala Ala Gly Leu Asn Gly Pro Gly Ser Gly Val Ile Gly Pro Gly
            20                  25                  30


Leu Ser Gly Leu Tyr Gly Pro Gly Val Ile Gly Pro Gly Val Ile Gly
        35                  40                  45


Pro Gly Ser Ser Ala Ala Ala Ala Ala Gly Pro Gly Leu Tyr Gly Pro
        50                  55                  60


Gly Val Ile Gly Pro Ser Ala Ser Ala Ala Ala Ala Gly Pro Gly
65                  70                  75                  80


Ser Gly Val Ile Gly Pro Gly Ala Ser Gly Leu Tyr Gly Pro Gly Val
                85                  90                  95


Ile Gly Pro Gly Val Ile Gly Pro Gly Ser Ser Ala Ala Ala Ala Ala
            100                 105                 110


Gly Leu Tyr Gly Ser Gly Pro Gly Val Ile Gly Pro Tyr Gly Ser Ala
            115                 120                 125


Ala Ala Ala Ala Gly Pro Gly Ser Gly Leu Tyr Gly Leu Gly Pro Tyr
    130                 135                 140


Gly Pro Gly Ala Ser Gly Pro Gly Leu Tyr Gly Pro Gly Val Ile Gly
145                 150                 155                 160


Pro Ser Ala Ser Ala Ala Ala Ala Gly Ser Gly Val Ile Gly Pro
                165                 170                 175


Gly Leu Tyr Gly Pro Tyr Ala Ser Ala Ala Ala Ala Gly Leu Tyr
            180                 185                 190


Gly Ser Gly Pro Gly Val Ile Gly Pro Tyr Gly Pro Gly Leu Ser Gly
            195                 200                 205


Ser Gly Val Ile Gly Pro Gly Val Ile Gly Pro Tyr Ala Ser Ala Ala
    210                 215                 220
```

```
Ala Ala Ala Gly Pro Gly Val Ile Gly Pro Tyr Gly Pro Gly Ser Ser
225                 230                 235                 240

Ala Ala Ala Ala Ala Gly Leu Tyr Gly Tyr Gly Pro Gly Val Ile Gly
            245                 250                 255

Pro Tyr Gly Pro Gly Ala Ser Gly Leu Asn Gly Pro Gly Ser Gly Leu
            260                 265                 270

Tyr Gly Pro Gly Val Ile Gly Pro Gly Leu Ser Ala Ala Ala Ala Ala
            275                 280                 285

Gly Pro Gly Val Ile Gly Pro Tyr Gly Pro Gly Ala Ser Ala Ala Ala
    290                 295                 300

Ala Ala Gly Leu Tyr Gly Pro Gly Val Ile Gly Pro Gly Leu Tyr Gly
305                 310                 315                 320

Pro Gly Ser Ser Gly Pro Gly Val Ile Gly Pro Tyr Gly Pro Gly Ser
            325                 330                 335

Ser Ala Ala Ala Ala Ala Gly Leu Tyr Gly Pro Gly Val Ile Gly Pro
            340                 345                 350

Tyr Gly Pro Gly Leu Ser Ala Ala Ala Ala Ala Gly Leu Tyr Val Ile
            355                 360                 365

Gly Pro Gly Val Ile Gly Pro Tyr Gly Pro Gly Ala Ser Gly Pro Gly
    370                 375                 380

Val Ile Gly Pro Tyr Gly Pro Gly Ala Ser Ala Ala Ala Ala Ala Gly
385                 390                 395                 400

Pro Gly Leu Tyr Gly Pro Gly Val Ile Gly Pro Ser Ala Ser Ala Ala
            405                 410                 415

Ala Ala Ala Gly Leu Tyr Gly Ser Gly Pro Gly Leu Tyr Gly Pro Tyr
            420                 425                 430

Gly Pro Gly Leu Ser Gly Pro Gly Ser Gly Val Ile Gly Leu Gly Pro
            435                 440                 445

Tyr Gly Pro Gly Ala Ser Ala Ala Ala Ala Gly Leu Tyr Gly Pro
    450                 455                 460

Gly Val Ile Gly Pro Tyr Gly Pro Gly Leu Ser Ala Ala Ala Ala Ala
```

465                    470                    475                    480

Gly Pro Gly Ser Gly Leu Tyr Gly Pro Gly Ala Ser Gly Leu Asn Gly
                485                    490                    495

Pro Gly Ser Gly Leu Tyr Gly Pro Gly Val Ile Gly Pro Gly Leu Ser
            500                    505                    510

Ala Ala Ala Ala Ala Gly Leu Tyr Val Ile Gly Pro Gly Val Ile Gly
            515                    520                    525

Pro Tyr Gly Pro Gly Ala Ser Ala Ala Ala Ala Gly Leu Tyr Gly
    530                    535                    540

Ser Gly Pro Gly Val Ile Gly Pro Tyr Gly Pro Gly Leu Ser Gly Ser
545                    550                    555                    560

Gly Val Ile Gly Pro Gly Val Ile Gly Pro Tyr Ala Ser Ala Ala Ala
            565                    570                    575

Ala Ala Gly Pro Gly Ser Gly Val Ile Gly Pro Gly Ala Ser
            580                    585                    590

<210> 29
<211> 590
<212> PRT
<213> Artificial Sequence

<220>
<223> Met-PRT918

<400> 29

Met Gly Pro Gly Val Phe Gly Pro Tyr Gly Pro Gly Ala Ser Ala Ala
1               5                   10                  15

Ala Ala Ala Gly Ile Asn Gly Pro Gly Ser Gly Val Phe Gly Pro Gly
            20                  25                  30

Ile Ser Gly Ile Tyr Gly Pro Gly Val Phe Gly Pro Gly Val Phe Gly
        35                  40                  45

Pro Gly Ser Ser Ala Ala Ala Ala Ala Gly Pro Gly Ile Tyr Gly Pro
        50                  55                  60

Gly Val Phe Gly Pro Ser Ala Ser Ala Ala Ala Ala Gly Pro Gly
65                  70                  75                  80

Ser Gly Val Phe Gly Pro Gly Ala Ser Gly Ile Tyr Gly Pro Gly Val
                85                  90                  95

Phe Gly Pro Gly Val Phe Gly Pro Gly Ser Ser Ala Ala Ala Ala Ala
                100                 105                 110

Gly Ile Tyr Gly Ser Gly Pro Gly Val Phe Gly Pro Tyr Gly Ser Ala
        115                 120                 125

Ala Ala Ala Ala Gly Pro Gly Ser Gly Ile Tyr Gly Ile Gly Pro Tyr
        130                 135                 140

Gly Pro Gly Ala Ser Gly Pro Gly Ile Tyr Gly Pro Gly Val Phe Gly
145                 150                 155                 160

Pro Ser Ala Ser Ala Ala Ala Ala Ala Gly Ser Gly Val Phe Gly Pro
                165                 170                 175

Gly Ile Tyr Gly Pro Tyr Ala Ser Ala Ala Ala Ala Gly Ile Tyr
        180                 185                 190

Gly Ser Gly Pro Gly Val Phe Gly Pro Tyr Gly Pro Gly Ile Ser Gly
        195                 200                 205

Ser Gly Val Phe Gly Pro Gly Val Phe Gly Pro Tyr Ala Ser Ala Ala
        210                 215                 220

Ala Ala Ala Gly Pro Gly Val Phe Gly Pro Tyr Gly Pro Gly Ser Ser
225                 230                 235                 240

Ala Ala Ala Ala Ala Gly Ile Tyr Gly Tyr Gly Pro Gly Val Phe Gly
                245                 250                 255

Pro Tyr Gly Pro Gly Ala Ser Gly Ile Asn Gly Pro Gly Ser Gly Ile
        260                 265                 270

Tyr Gly Pro Gly Val Phe Gly Pro Gly Ile Ser Ala Ala Ala Ala Ala
        275                 280                 285

Gly Pro Gly Val Phe Gly Pro Tyr Gly Pro Gly Ala Ser Ala Ala Ala
    290                 295                 300

Ala Ala Gly Ile Tyr Gly Pro Gly Val Phe Gly Pro Gly Ile Tyr Gly
305                 310                 315                 320

Pro Gly Ser Ser Gly Pro Gly Val Phe Gly Pro Tyr Gly Pro Gly Ser
            325                 330                 335

Ser Ala Ala Ala Ala Ala Gly Ile Tyr Gly Pro Gly Val Phe Gly Pro

                        340                        345                            350


        Tyr Gly Pro Gly Ile Ser Ala Ala Ala Ala Ala Gly Ile Tyr Val Phe
                355                    360                    365


        Gly Pro Gly Val Phe Gly Pro Tyr Gly Pro Gly Ala Ser Gly Pro Gly
                370                    375                    380


        Val Phe Gly Pro Tyr Gly Pro Gly Ala Ser Ala Ala Ala Ala Ala Gly
        385                    390                    395                    400


        Pro Gly Ile Tyr Gly Pro Gly Val Phe Gly Pro Ser Ala Ser Ala Ala
                        405                    410                    415


        Ala Ala Ala Gly Ile Tyr Gly Ser Gly Pro Gly Ile Tyr Gly Pro Tyr
                420                    425                    430


        Gly Pro Gly Ile Ser Gly Pro Gly Ser Gly Val Phe Gly Ile Gly Pro
                435                    440                    445


        Tyr Gly Pro Gly Ala Ser Ala Ala Ala Ala Gly Ile Tyr Gly Pro
                450                    455                    460


        Gly Val Phe Gly Pro Tyr Gly Pro Gly Ile Ser Ala Ala Ala Ala Ala
        465                    470                    475                    480


        Gly Pro Gly Ser Gly Ile Tyr Gly Pro Gly Ala Ser Gly Ile Asn Gly
                        485                    490                    495


        Pro Gly Ser Gly Ile Tyr Gly Pro Gly Val Phe Gly Pro Gly Ile Ser
                500                    505                    510


        Ala Ala Ala Ala Ala Gly Ile Tyr Val Phe Gly Pro Gly Val Phe Gly
                515                    520                    525


        Pro Tyr Gly Pro Gly Ala Ser Ala Ala Ala Ala Gly Ile Tyr Gly
                530                    535                    540


        Ser Gly Pro Gly Val Phe Gly Pro Tyr Gly Pro Gly Ile Ser Gly Ser
        545                    550                    555                    560


        Gly Val Phe Gly Pro Gly Val Phe Gly Pro Tyr Ala Ser Ala Ala Ala
                        565                    570                    575


        Ala Ala Gly Pro Gly Ser Gly Val Phe Gly Pro Gly Ala Ser
                580                    585                    590

<210> 30
<211> 565
<212> PRT
<213> Artificial Sequence

<220>
<223> Met-PRT699

<400> 30

Met Gly Pro Gly Val Leu Gly Pro Tyr Gly Pro Gly Ala Ser Ala Ala
1               5                   10                  15

Ala Ala Ala Ala Ala Gly Ser Asn Gly Pro Gly Ser Gly Val Leu Gly
            20                  25                  30

Pro Gly Gln Ser Gly Gln Tyr Gly Pro Gly Val Leu Gly Pro Gly Val
        35                  40                  45

Leu Gly Pro Gly Ser Ser Ala Ala Ala Ala Ala Ala Gly Pro Gly
        50                  55                  60

Gln Tyr Gly Pro Gly Val Leu Gly Pro Ser Ala Ser Ala Ala Ala Ala
65                  70                  75                  80

Ala Ala Ala Gly Pro Gly Ser Gly Val Leu Gly Pro Gly Ala Ser Gly
            85                  90                  95

Gln Tyr Gly Pro Gly Val Leu Gly Pro Gly Val Leu Gly Pro Gly Ser
            100                 105                 110

Ser Ala Ala Ala Ala Ala Ala Ala Gly Ser Tyr Gly Ser Gly Pro Gly
        115                 120                 125

Val Leu Gly Pro Tyr Gly Ser Ala Ala Ala Ala Ala Ala Gly Pro
        130                 135                 140

Gly Ser Gly Gln Tyr Gly Gln Gly Pro Tyr Gly Pro Gly Ala Ser Gly
145                 150                 155                 160

Pro Gly Gln Tyr Gly Pro Gly Val Leu Gly Pro Ser Ala Ser Ala Ala
            165                 170                 175

Ala Ala Ala Ala Ala Gly Ser Gly Val Leu Gly Pro Gly Gln Tyr Gly
            180                 185                 190

Pro Tyr Ala Ser Ala Ala Ala Ala Ala Ala Ala Gly Ser Tyr Gly Ser
            195                 200                 205

Gly Pro Gly Val Leu Gly Pro Tyr Gly Pro Gly Gln Ser Gly Ser Gly

|     |     |     | 210 |     |     |     | 215 |     |     |     | 220 |     |     |     |
| --- | --- | --- | --- | --- | --- | --- | --- | --- | --- | --- | --- | --- | --- | --- |

Val Leu Gly Pro Gly Val Leu Gly Pro Tyr Ala Ser Ala Ala Ala
225                 230                 235                 240

Ala Ala Ala Gly Pro Gly Val Leu Gly Pro Tyr Gly Pro Gly Ser Ser
            245             250             255

Ala Ala Ala Ala Ala Ala Ala Gly Ser Tyr Gly Tyr Gly Pro Gly Val
        260             265             270

Leu Gly Pro Tyr Gly Pro Gly Ala Ser Gly Gln Asn Gly Pro Gly Ser
        275             280             285

Gly Gln Tyr Gly Pro Gly Val Leu Gly Pro Gly Pro Ser Ala Ala Ala
    290             295             300

Ala Ala Ala Ala Gly Pro Gly Val Leu Gly Pro Tyr Gly Pro Gly Ala
305             310             315             320

Ser Ala Ala Ala Ala Ala Ala Ala Gly Ser Tyr Gly Pro Gly Val Leu
            325             330             335

Gly Pro Gly Gln Tyr Gly Pro Gly Ser Ser Gly Pro Gly Val Leu Gly
        340             345             350

Pro Tyr Gly Pro Gly Ser Ser Ala Ala Ala Ala Ala Ala Ala Gly Ser
        355             360             365

Tyr Gly Pro Gly Val Leu Gly Pro Tyr Gly Pro Gly Pro Ser Ala Ala
        370             375             380

Ala Ala Ala Ala Ala Gly Ser Tyr Val Leu Gly Pro Gly Val Leu Gly
385             390             395             400

Pro Tyr Gly Pro Gly Ala Ser Gly Pro Gly Val Leu Gly Pro Tyr Gly
        405             410             415

Pro Gly Ala Ser Ala Ala Ala Ala Ala Ala Ala Gly Pro Gly Gln Tyr
        420             425             430

Gly Pro Gly Val Leu Gly Pro Ser Ala Ser Ala Ala Ala Ala Ala Ala
    435             440             445

Ala Gly Ser Tyr Gly Ser Gly Pro Gly Gln Tyr Gly Pro Tyr Gly Pro
    450             455             460

```
Gly Gln Ser Gly Pro Gly Ser Gly Val Leu Gly Gln Gly Pro Tyr Gly
465             470             475             480

Pro Gly Ala Ser Ala Ala Ala Ala Ala Ala Ala Gly Ser Tyr Gly Pro
                485             490             495

Gly Val Leu Gly Pro Tyr Gly Pro Gly Pro Ser Ala Ala Ala Ala Ala
            500             505             510

Ala Ala Gly Pro Gly Ser Gly Gln Tyr Gly Pro Gly Ala Ser Gly Gln
        515             520             525

Asn Gly Pro Gly Ser Gly Gln Tyr Gly Pro Gly Val Leu Gly Pro Gly
        530             535             540

Pro Ser Ala Ala Ala Ala Ala Ala Ala Gly Pro Gly Ser Gly Val Leu
545             550             555             560

Gly Pro Gly Ala Ser
                565


<210>   31
<211>   565
<212>   PRT
<213>   Artificial Sequence

<220>
<223>   Met-PRT698

<400>   31

Met Gly Pro Gly Val Leu Gly Pro Tyr Gly Pro Gly Ala Ser Ala Ala
1               5               10              15

Ala Ala Ala Ala Ala Gly Ser Asn Gly Pro Gly Ser Gly Val Leu Gly
            20              25              30

Pro Gly Ile Ser Gly Ile Tyr Gly Pro Gly Val Leu Gly Pro Gly Val
            35              40              45

Leu Gly Pro Gly Ser Ser Ala Ala Ala Ala Ala Ala Gly Pro Gly
        50              55              60

Ile Tyr Gly Pro Gly Val Leu Gly Pro Ser Ala Ser Ala Ala Ala Ala
65              70              75              80

Ala Ala Ala Gly Pro Gly Ser Gly Val Leu Gly Pro Gly Ala Ser Gly
            85              90              95

Ile Tyr Gly Pro Gly Val Leu Gly Pro Gly Val Leu Gly Pro Gly Ser
```

```
              100                      105                      110

     Ser Ala Ala Ala Ala Ala Ala Ala Gly Ser Tyr Gly Ser Gly Pro Gly
             115                  120              125

     Val Leu Gly Pro Tyr Gly Ser Ala Ala Ala Ala Ala Ala Ala Gly Pro
             130              135              140

     Gly Ser Gly Ile Tyr Gly Ile Gly Pro Tyr Gly Pro Gly Ala Ser Gly
     145              150              155                      160

     Pro Gly Ile Tyr Gly Pro Gly Val Leu Gly Pro Ser Ala Ser Ala Ala
                     165              170              175

     Ala Ala Ala Ala Ala Gly Ser Gly Val Leu Gly Pro Gly Ile Tyr Gly
                 180              185              190

     Pro Tyr Ala Ser Ala Ala Ala Ala Ala Ala Gly Ser Tyr Gly Ser
             195              200              205

     Gly Pro Gly Val Leu Gly Pro Tyr Gly Pro Gly Ile Ser Gly Ser Gly
             210              215              220

     Val Leu Gly Pro Gly Val Leu Gly Pro Tyr Ala Ser Ala Ala Ala Ala
     225              230              235                      240

     Ala Ala Ala Gly Pro Gly Val Leu Gly Pro Tyr Gly Pro Gly Ser Ser
                 245              250              255

     Ala Ala Ala Ala Ala Ala Ala Gly Ser Tyr Gly Tyr Gly Pro Gly Val
                 260              265              270

     Leu Gly Pro Tyr Gly Pro Gly Ala Ser Gly Ile Asn Gly Pro Gly Ser
             275              280              285

     Gly Ile Tyr Gly Pro Gly Val Leu Gly Pro Gly Pro Ser Ala Ala Ala
             290              295              300

     Ala Ala Ala Ala Gly Pro Gly Val Leu Gly Pro Tyr Gly Pro Gly Ala
     305              310              315                      320

     Ser Ala Ala Ala Ala Ala Ala Ala Gly Ser Tyr Gly Pro Gly Val Leu
                 325              330              335

     Gly Pro Gly Ile Tyr Gly Pro Gly Ser Ser Gly Pro Gly Val Leu Gly
             340              345              350
```

```
Pro Tyr Gly Pro Gly Ser Ser Ala Ala Ala Ala Ala Ala Ala Gly Ser
        355                 360             365

Tyr Gly Pro Gly Val Leu Gly Pro Tyr Gly Pro Gly Pro Ser Ala Ala
    370             375             380

Ala Ala Ala Ala Ala Gly Ser Tyr Val Leu Gly Pro Gly Val Leu Gly
385                 390             395                     400

Pro Tyr Gly Pro Gly Ala Ser Gly Pro Gly Val Leu Gly Pro Tyr Gly
            405             410                     415

Pro Gly Ala Ser Ala Ala Ala Ala Ala Ala Ala Gly Pro Gly Ile Tyr
        420                 425                 430

Gly Pro Gly Val Leu Gly Pro Ser Ala Ser Ala Ala Ala Ala Ala Ala
        435             440                 445

Ala Gly Ser Tyr Gly Ser Gly Pro Gly Ile Tyr Gly Pro Tyr Gly Pro
    450             455                 460

Gly Ile Ser Gly Pro Gly Ser Gly Val Leu Gly Ile Gly Pro Tyr Gly
465             470                 475                     480

Pro Gly Ala Ser Ala Ala Ala Ala Ala Ala Ala Gly Ser Tyr Gly Pro
        485                 490                     495

Gly Val Leu Gly Pro Tyr Gly Pro Gly Pro Ser Ala Ala Ala Ala Ala
        500             505                 510

Ala Ala Gly Pro Gly Ser Gly Ile Tyr Gly Pro Gly Ala Ser Gly Ile
    515             520                 525

Asn Gly Pro Gly Ser Gly Ile Tyr Gly Pro Gly Val Leu Gly Pro Gly
    530             535                 540

Pro Ser Ala Ala Ala Ala Ala Ala Ala Gly Pro Gly Ser Gly Val Leu
545                 550                 555                     560

Gly Pro Gly Ala Ser
            565
```

```
<210>   32
<211>   1179
<212>   PRT
<213>   Artificial Sequence

<220>
<223>   Met-PRT966
```

<400> 32

Met Gly Pro Gly Val Phe Gly Pro Tyr Gly Pro Gly Ala Ser Ala Ala
1               5                   10              15

Ala Ala Ala Gly Ile Asn Gly Pro Gly Ser Gly Val Phe Gly Pro Gly
            20                  25                  30

Ile Ser Gly Ile Tyr Gly Pro Gly Val Phe Gly Pro Gly Val Phe Gly
            35                  40                  45

Pro Gly Ser Ser Ala Ala Ala Ala Ala Gly Pro Gly Ile Tyr Gly Pro
        50                  55                  60

Gly Val Phe Gly Pro Ser Ala Ser Ala Ala Ala Ala Gly Pro Gly
65                  70                  75                  80

Ser Gly Val Phe Gly Pro Gly Ala Ser Gly Ile Tyr Gly Pro Gly Val
                85                  90                  95

Phe Gly Pro Gly Val Phe Gly Pro Gly Ser Ser Ala Ala Ala Ala Ala
            100                 105                 110

Gly Ile Tyr Gly Ser Gly Pro Gly Val Phe Gly Pro Tyr Gly Ser Ala
        115                 120                 125

Ala Ala Ala Ala Gly Pro Gly Ser Gly Ile Tyr Gly Ile Gly Pro Tyr
    130                 135                 140

Gly Pro Gly Ala Ser Gly Pro Gly Ile Tyr Gly Pro Gly Val Phe Gly
145                 150                 155                 160

Pro Ser Ala Ser Ala Ala Ala Ala Ala Gly Ser Gly Val Phe Gly Pro
            165                 170                 175

Gly Ile Tyr Gly Pro Tyr Ala Ser Ala Ala Ala Ala Ala Gly Ile Tyr
        180                 185                 190

Gly Ser Gly Pro Gly Val Phe Gly Pro Tyr Gly Pro Gly Ile Ser Gly
        195                 200                 205

Ser Gly Val Phe Gly Pro Gly Val Phe Gly Pro Tyr Ala Ser Ala Ala
    210                 215                 220

Ala Ala Ala Gly Pro Gly Val Phe Gly Pro Tyr Gly Pro Gly Ser Ser
225                 230                 235                 240

```
Ala Ala Ala Ala Ala Gly Ile Tyr Gly Tyr Gly Pro Gly Val Phe Gly
            245             250             255

Pro Tyr Gly Pro Gly Ala Ser Gly Ile Asn Gly Pro Gly Ser Gly Ile
            260             265             270

Tyr Gly Pro Gly Val Phe Gly Pro Gly Ile Ser Ala Ala Ala Ala Ala
            275             280             285

Gly Pro Gly Val Phe Gly Pro Tyr Gly Pro Gly Ala Ser Ala Ala Ala
            290             295             300

Ala Ala Gly Ile Tyr Gly Pro Gly Val Phe Gly Pro Gly Ile Tyr Gly
305             310             315             320

Pro Gly Ser Ser Gly Pro Gly Val Phe Gly Pro Tyr Gly Pro Gly Ser
            325             330             335

Ser Ala Ala Ala Ala Ala Gly Ile Tyr Gly Pro Gly Val Phe Gly Pro
            340             345             350

Tyr Gly Pro Gly Ile Ser Ala Ala Ala Ala Gly Ile Tyr Val Phe
            355             360             365

Gly Pro Gly Val Phe Gly Pro Tyr Gly Pro Gly Ala Ser Gly Pro Gly
            370             375             380

Val Phe Gly Pro Tyr Gly Pro Gly Ala Ser Ala Ala Ala Ala Ala Gly
385             390             395             400

Pro Gly Ile Tyr Gly Pro Gly Val Phe Gly Pro Ser Ala Ser Ala Ala
            405             410             415

Ala Ala Ala Gly Ile Tyr Gly Ser Gly Pro Gly Ile Tyr Gly Pro Tyr
            420             425             430

Gly Pro Gly Ile Ser Gly Pro Gly Ser Gly Val Phe Gly Ile Gly Pro
            435             440             445

Tyr Gly Pro Gly Ala Ser Ala Ala Ala Ala Gly Ile Tyr Gly Pro
            450             455             460

Gly Val Phe Gly Pro Tyr Gly Pro Gly Ile Ser Ala Ala Ala Ala Ala
465             470             475             480

Gly Pro Gly Ser Gly Ile Tyr Gly Pro Gly Ala Ser Gly Ile Asn Gly
            485             490             495
```

Pro Gly Ser Gly Ile Tyr Gly Pro Gly Val Phe Gly Pro Gly Ile Ser
        500                 505                 510

Ala Ala Ala Ala Ala Gly Ile Tyr Val Phe Gly Pro Gly Val Phe Gly
        515                 520                 525

Pro Tyr Gly Pro Gly Ala Ser Ala Ala Ala Ala Gly Ile Tyr Gly
        530                 535                 540

Ser Gly Pro Gly Val Phe Gly Pro Tyr Gly Pro Gly Ile Ser Gly Ser
545                 550                 555                 560

Gly Val Phe Gly Pro Gly Val Phe Gly Pro Tyr Ala Ser Ala Ala Ala
                565                 570                 575

Ala Ala Gly Pro Gly Ser Gly Val Phe Gly Pro Gly Ala Ser Gly Pro
                580                 585                 590

Gly Val Phe Gly Pro Tyr Gly Pro Gly Ala Ser Ala Ala Ala Ala Ala
        595                 600                 605

Gly Ile Asn Gly Pro Gly Ser Gly Val Phe Gly Pro Gly Ile Ser Gly
        610                 615                 620

Ile Tyr Gly Pro Gly Val Phe Gly Pro Gly Val Phe Gly Pro Gly Ser
625                 630                 635                 640

Ser Ala Ala Ala Ala Ala Gly Pro Gly Ile Tyr Gly Pro Gly Val Phe
                645                 650                 655

Gly Pro Ser Ala Ser Ala Ala Ala Ala Ala Gly Pro Gly Ser Gly Val
                660                 665                 670

Phe Gly Pro Gly Ala Ser Gly Ile Tyr Gly Pro Gly Val Phe Gly Pro
        675                 680                 685

Gly Val Phe Gly Pro Gly Ser Ser Ala Ala Ala Ala Gly Ile Tyr
        690                 695                 700

Gly Ser Gly Pro Gly Val Phe Gly Pro Tyr Gly Ser Ala Ala Ala Ala
705                 710                 715                 720

Ala Gly Pro Gly Ser Gly Ile Tyr Gly Ile Gly Pro Tyr Gly Pro Gly
                725                 730                 735

Ala Ser Gly Pro Gly Ile Tyr Gly Pro Gly Val Phe Gly Pro Ser Ala
        740                 745                 750

Ser Ala Ala Ala Ala Ala Gly Ser Gly Val Phe Gly Pro Gly Ile Tyr
        755                 760                 765

Gly Pro Tyr Ala Ser Ala Ala Ala Ala Ala Gly Ile Tyr Gly Ser Gly
        770                 775                 780

Pro Gly Val Phe Gly Pro Tyr Gly Pro Gly Ile Ser Gly Ser Gly Val
785                 790                 795                 800

Phe Gly Pro Gly Val Phe Gly Pro Tyr Ala Ser Ala Ala Ala Ala Ala
                805                 810                 815

Gly Pro Gly Val Phe Gly Pro Tyr Gly Pro Gly Ser Ser Ala Ala Ala
                820                 825                 830

Ala Ala Gly Ile Tyr Gly Tyr Gly Pro Gly Val Phe Gly Pro Tyr Gly
        835                 840                 845

Pro Gly Ala Ser Gly Ile Asn Gly Pro Gly Ser Gly Ile Tyr Gly Pro
        850                 855                 860

Gly Val Phe Gly Pro Gly Ile Ser Ala Ala Ala Ala Ala Gly Pro Gly
865                 870                 875                 880

Val Phe Gly Pro Tyr Gly Pro Gly Ala Ser Ala Ala Ala Ala Ala Gly
                885                 890                 895

Ile Tyr Gly Pro Gly Val Phe Gly Pro Gly Ile Tyr Gly Pro Gly Ser
                900                 905                 910

Ser Gly Pro Gly Val Phe Gly Pro Tyr Gly Pro Gly Ser Ser Ala Ala
        915                 920                 925

Ala Ala Ala Gly Ile Tyr Gly Pro Gly Val Phe Gly Pro Tyr Gly Pro
        930                 935                 940

Gly Ile Ser Ala Ala Ala Ala Ala Gly Ile Tyr Val Phe Gly Pro Gly
945                 950                 955                 960

Val Phe Gly Pro Tyr Gly Pro Gly Ala Ser Gly Pro Gly Val Phe Gly
                965                 970                 975

Pro Tyr Gly Pro Gly Ala Ser Ala Ala Ala Ala Gly Pro Gly Ile
        980                 985                 990

Tyr Gly Pro Gly Val Phe Gly Pro  Ser Ala Ser Ala Ala  Ala Ala Ala

```
              995                      1000                     1005
```

```
        Gly Ile  Tyr Gly Ser Gly Pro  Gly Ile Tyr Gly Pro  Tyr Gly Pro
            1010              1015              1020
```

```
        Gly Ile  Ser Gly Pro Gly Ser  Gly Val Phe Gly Ile  Gly Pro Tyr
            1025              1030              1035
```

```
        Gly Pro  Gly Ala Ser Ala Ala  Ala Ala Ala Gly Ile  Tyr Gly Pro
            1040              1045              1050
```

```
        Gly Val  Phe Gly Pro Tyr Gly  Pro Gly Ile Ser Ala  Ala Ala Ala
            1055              1060              1065
```

```
        Ala Gly  Pro Gly Ser Gly Ile  Tyr Gly Pro Gly Ala  Ser Gly Ile
            1070              1075              1080
```

```
        Asn Gly  Pro Gly Ser Gly Ile  Tyr Gly Pro Gly Val  Phe Gly Pro
            1085              1090              1095
```

```
        Gly Ile  Ser Ala Ala Ala Ala  Ala Gly Ile Tyr Val  Phe Gly Pro
            1100              1105              1110
```

```
        Gly Val  Phe Gly Pro Tyr Gly  Pro Gly Ala Ser Ala  Ala Ala Ala
            1115              1120              1125
```

```
        Ala Gly  Ile Tyr Gly Ser Gly  Pro Gly Val Phe Gly  Pro Tyr Gly
            1130              1135              1140
```

```
        Pro Gly  Ile Ser Gly Ser Gly  Val Phe Gly Pro Gly  Val Phe Gly
            1145              1150              1155
```

```
        Pro Tyr  Ala Ser Ala Ala Ala  Ala Ala Gly Pro Gly  Ser Gly Val
            1160              1165              1170
```

```
        Phe Gly  Pro Gly Ala Ser
            1175
```

```
        <210>  33
        <211>  601
        <212>  PRT
        <213>  Artificial Sequence

        <220>
        <223>  PRT888

        <400>  33

        Met His His His His His His Ser Ser Gly Ser Ser Gly Pro Gly Val
        1                   5                   10                  15
```

Leu Gly Pro Tyr Gly Pro Gly Ala Ser Ala Ala Ala Ala Ala Gly Gln
            20                      25                      30

Asn Gly Pro Gly Ser Gly Val Leu Gly Pro Gly Gln Ser Gly Gln Tyr
            35                      40                      45

Gly Pro Gly Val Leu Gly Pro Gly Val Leu Gly Pro Gly Ser Ser Ala
        50                      55                      60

Ala Ala Ala Ala Gly Pro Gly Gln Tyr Gly Pro Gly Val Leu Gly Pro
65                      70                      75                      80

Ser Ala Ser Ala Ala Ala Ala Ala Gly Pro Gly Ser Gly Val Leu Gly
                    85                      90                      95

Pro Gly Ala Ser Gly Gln Tyr Gly Pro Gly Val Leu Gly Pro Gly Val
                100                     105                     110

Leu Gly Pro Gly Ser Ser Ala Ala Ala Ala Ala Gly Gln Tyr Gly Ser
            115                     120                     125

Gly Pro Gly Val Leu Gly Pro Tyr Gly Ser Ala Ala Ala Ala Ala Gly
            130                     135                     140

Pro Gly Ser Gly Gln Tyr Gly Gln Gly Pro Tyr Gly Pro Gly Ala Ser
145                     150                     155                     160

Gly Pro Gly Gln Tyr Gly Pro Gly Val Leu Gly Pro Ser Ala Ser Ala
                165                     170                     175

Ala Ala Ala Gly Ser Gly Val Leu Gly Pro Gly Gln Tyr Gly Pro
            180                     185                     190

Tyr Ala Ser Ala Ala Ala Ala Ala Gly Gln Tyr Gly Ser Gly Pro Gly
            195                     200                     205

Val Leu Gly Pro Tyr Gly Pro Gly Gln Ser Gly Ser Gly Val Leu Gly
        210                     215                     220

Pro Gly Val Leu Gly Pro Tyr Ala Ser Ala Ala Ala Ala Gly Pro
225                     230                     235                     240

Gly Val Leu Gly Pro Tyr Gly Pro Gly Ser Ser Ala Ala Ala Ala Ala
                245                     250                     255

Gly Gln Tyr Gly Tyr Gly Pro Gly Val Leu Gly Pro Tyr Gly Pro Gly

131

```
                260                     265                         270


Ala Ser Gly Gln Asn Gly Pro Gly Ser Gly Gln Tyr Gly Pro Gly Val
        275                 280                 285


Leu Gly Pro Gly Gln Ser Ala Ala Ala Ala Gly Pro Gly Val Leu
        290                 295                 300


Gly Pro Tyr Gly Pro Gly Ala Ser Ala Ala Ala Ala Gly Gln Tyr
305                 310                 315                 320


Gly Pro Gly Val Leu Gly Pro Gly Gln Tyr Gly Pro Gly Ser Ser Gly
                325                 330                 335


Pro Gly Val Leu Gly Pro Tyr Gly Pro Gly Ser Ser Ala Ala Ala Ala
            340                 345                 350


Ala Gly Gln Tyr Gly Pro Gly Val Leu Gly Pro Tyr Gly Pro Gly Gln
        355                 360                 365


Ser Ala Ala Ala Ala Gly Gln Tyr Val Leu Gly Pro Gly Val Leu
    370                 375                 380


Gly Pro Tyr Gly Pro Gly Ala Ser Gly Pro Gly Val Leu Gly Pro Tyr
385                 390                 395                 400


Gly Pro Gly Ala Ser Ala Ala Ala Ala Gly Pro Gly Gln Tyr Gly
            405                 410                 415


Pro Gly Val Leu Gly Pro Ser Ala Ser Ala Ala Ala Ala Gly Gln
        420                 425                 430


Tyr Gly Ser Gly Pro Gly Gln Tyr Gly Pro Tyr Gly Pro Gly Gln Ser
        435                 440                 445


Gly Pro Gly Ser Gly Val Leu Gly Gln Gly Pro Tyr Gly Pro Gly Ala
    450                 455                 460


Ser Ala Ala Ala Ala Ala Gly Gln Tyr Gly Pro Gly Val Leu Gly Pro
465                 470                 475                 480


Tyr Gly Pro Gly Gln Ser Ala Ala Ala Ala Gly Pro Gly Ser Gly
            485                 490                 495


Gln Tyr Gly Pro Gly Ala Ser Gly Gln Asn Gly Pro Gly Ser Gly Gln
        500                 505                 510
```

```
Tyr Gly Pro Gly Val Leu Gly Pro Gly Gln Ser Ala Ala Ala Ala Ala
        515                 520             525

Gly Gln Tyr Val Leu Gly Pro Gly Val Leu Gly Pro Tyr Gly Pro Gly
        530                 535             540

Ala Ser Ala Ala Ala Ala Ala Gly Gln Tyr Gly Ser Gly Pro Gly Val
545                 550             555                     560

Leu Gly Pro Tyr Gly Pro Gly Gln Ser Gly Ser Gly Val Leu Gly Pro
            565             570                     575

Gly Val Leu Gly Pro Tyr Ala Ser Ala Ala Ala Ala Ala Gly Pro Gly
            580             585                 590

Ser Gly Val Leu Gly Pro Gly Ala Ser
        595             600
```

```
<210>  34
<211>  601
<212>  PRT
<213>  Artificial Sequence

<220>
<223>  PRT965

<400>  34
```

```
Met His His His His His His Ser Ser Gly Ser Ser Gly Pro Gly Thr
1                   5                   10                  15

Ser Gly Pro Tyr Gly Pro Gly Ala Ser Ala Ala Ala Ala Ala Gly Ala
            20              25                  30

Asn Gly Pro Gly Ser Gly Thr Ser Gly Pro Gly Ala Ser Gly Ala Tyr
        35              40                  45

Gly Pro Gly Thr Ser Gly Pro Gly Thr Ser Gly Pro Gly Ser Ser Ala
        50              55                  60

Ala Ala Ala Ala Gly Pro Gly Ala Tyr Gly Pro Gly Thr Ser Gly Pro
65              70              75                  80

Ser Ala Ser Ala Ala Ala Ala Ala Gly Pro Gly Ser Gly Thr Ser Gly
                85              90                  95

Pro Gly Ala Ser Gly Ala Tyr Gly Pro Gly Thr Ser Gly Pro Gly Thr
            100             105                 110

Ser Gly Pro Gly Ser Ser Ala Ala Ala Ala Ala Gly Ala Tyr Gly Ser
```

133

|     |     | 115 |     |     |     |     | 120 |     |     |     |     | 125 |     |     |     |

```
                 115                      120                      125

     Gly Pro Gly Thr Ser Gly Pro Tyr Gly Ser Ala Ala Ala Ala Ala Gly
         130                 135                 140

     Pro Gly Ser Gly Ala Tyr Gly Ala Gly Pro Tyr Gly Pro Gly Ala Ser
     145                 150                 155                 160

     Gly Pro Gly Ala Tyr Gly Pro Gly Thr Ser Gly Pro Ser Ala Ser Ala
                     165                 170                 175

     Ala Ala Ala Ala Gly Ser Gly Thr Ser Gly Pro Gly Ala Tyr Gly Pro
                     180                 185                 190

     Tyr Ala Ser Ala Ala Ala Ala Ala Gly Ala Tyr Gly Ser Gly Pro Gly
                     195                 200                 205

     Thr Ser Gly Pro Tyr Gly Pro Gly Ala Ser Gly Ser Gly Thr Ser Gly
         210                 215                 220

     Pro Gly Thr Ser Gly Pro Tyr Ala Ser Ala Ala Ala Ala Ala Gly Pro
     225                 230                 235                 240

     Gly Thr Ser Gly Pro Tyr Gly Pro Gly Ser Ser Ala Ala Ala Ala Ala
                     245                 250                 255

     Gly Ala Tyr Gly Tyr Gly Pro Gly Thr Ser Gly Pro Tyr Gly Pro Gly
                     260                 265                 270

     Ala Ser Gly Ala Asn Gly Pro Gly Ser Gly Ala Tyr Gly Pro Gly Thr
                     275                 280                 285

     Ser Gly Pro Gly Ala Ser Ala Ala Ala Ala Gly Pro Gly Thr Ser
         290                 295                 300

     Gly Pro Tyr Gly Pro Gly Ala Ser Ala Ala Ala Ala Gly Ala Tyr
     305                 310                 315                 320

     Gly Pro Gly Thr Ser Gly Pro Gly Ala Tyr Gly Pro Gly Ser Ser Gly
                     325                 330                 335

     Pro Gly Thr Ser Gly Pro Tyr Gly Pro Gly Ser Ser Ala Ala Ala Ala
                     340                 345                 350

     Ala Gly Ala Tyr Gly Pro Gly Thr Ser Gly Pro Tyr Gly Pro Gly Ala
                     355                 360                 365
```

```
        Ser Ala Ala Ala Ala Ala Gly Ala Tyr Thr Ser Gly Pro Gly Thr Ser
            370                 375                 380

        Gly Pro Tyr Gly Pro Gly Ala Ser Gly Pro Gly Thr Ser Gly Pro Tyr
        385                 390                 395                 400

        Gly Pro Gly Ala Ser Ala Ala Ala Ala Gly Pro Gly Ala Tyr Gly
                        405                 410                 415

        Pro Gly Thr Ser Gly Pro Ser Ala Ser Ala Ala Ala Ala Gly Ala
                    420                 425                 430

        Tyr Gly Ser Gly Pro Gly Ala Tyr Gly Pro Tyr Gly Pro Gly Ala Ser
                    435                 440                 445

        Gly Pro Gly Ser Gly Thr Ser Gly Ala Gly Pro Tyr Gly Pro Gly Ala
            450                 455                 460

        Ser Ala Ala Ala Ala Ala Gly Ala Tyr Gly Pro Gly Thr Ser Gly Pro
        465                 470                 475                 480

        Tyr Gly Pro Gly Ala Ser Ala Ala Ala Ala Gly Pro Gly Ser Gly
                    485                 490                 495

        Ala Tyr Gly Pro Gly Ala Ser Gly Ala Asn Gly Pro Gly Ser Gly Ala
                500                 505                 510

        Tyr Gly Pro Gly Thr Ser Gly Pro Gly Ala Ser Ala Ala Ala Ala Ala
            515                 520                 525

        Gly Ala Tyr Thr Ser Gly Pro Gly Thr Ser Gly Pro Tyr Gly Pro Gly
            530                 535                 540

        Ala Ser Ala Ala Ala Ala Ala Gly Ala Tyr Gly Ser Gly Pro Gly Thr
        545                 550                 555                 560

        Ser Gly Pro Tyr Gly Pro Gly Ala Ser Gly Ser Gly Thr Ser Gly Pro
                    565                 570                 575

        Gly Thr Ser Gly Pro Tyr Ala Ser Ala Ala Ala Ala Gly Pro Gly
                    580                 585                 590

        Ser Gly Thr Ser Gly Pro Gly Ala Ser
                595                 600


        <210>   35
        <211>   601
        <212>   PRT
```

135

<213>    Artificial Sequence

<220>
<223>    PRT889

<400>    35

Met His His His His His His Ser Ser Gly Ser Ser Gly Pro Gly Val
1                   5                   10                  15

Leu Gly Pro Tyr Gly Pro Gly Ala Ser Ala Ala Ala Ala Ala Gly Ile
              20                  25                  30

Asn Gly Pro Gly Ser Gly Val Leu Gly Pro Gly Ile Ser Gly Ile Tyr
          35                  40                  45

Gly Pro Gly Val Leu Gly Pro Gly Val Leu Gly Pro Gly Ser Ser Ala
      50                  55                  60

Ala Ala Ala Ala Gly Pro Gly Ile Tyr Gly Pro Gly Val Leu Gly Pro
65                  70                  75                  80

Ser Ala Ser Ala Ala Ala Ala Ala Gly Pro Gly Ser Gly Val Leu Gly
              85                  90                  95

Pro Gly Ala Ser Gly Ile Tyr Gly Pro Gly Val Leu Gly Pro Gly Val
              100                 105                 110

Leu Gly Pro Gly Ser Ser Ala Ala Ala Ala Ala Gly Ile Tyr Gly Ser
          115                 120                 125

Gly Pro Gly Val Leu Gly Pro Tyr Gly Ser Ala Ala Ala Ala Ala Gly
      130                 135                 140

Pro Gly Ser Gly Ile Tyr Gly Ile Gly Pro Tyr Gly Pro Gly Ala Ser
145                 150                 155                 160

Gly Pro Gly Ile Tyr Gly Pro Gly Val Leu Gly Pro Ser Ala Ser Ala
              165                 170                 175

Ala Ala Ala Ala Gly Ser Gly Val Leu Gly Pro Gly Ile Tyr Gly Pro
          180                 185                 190

Tyr Ala Ser Ala Ala Ala Ala Ala Gly Ile Tyr Gly Ser Gly Pro Gly
          195                 200                 205

Val Leu Gly Pro Tyr Gly Pro Gly Ile Ser Gly Ser Gly Val Leu Gly
      210                 215                 220

Pro Gly Val Leu Gly Pro Tyr Ala Ser Ala Ala Ala Ala Ala Gly Pro
225                 230             235                 240

Gly Val Leu Gly Pro Tyr Gly Pro Gly Ser Ser Ala Ala Ala Ala Ala
            245             250                 255

Gly Ile Tyr Gly Tyr Gly Pro Gly Val Leu Gly Pro Tyr Gly Pro Gly
            260             265                 270

Ala Ser Gly Ile Asn Gly Pro Gly Ser Gly Ile Tyr Gly Pro Gly Val
        275             280             285

Leu Gly Pro Gly Ile Ser Ala Ala Ala Ala Gly Pro Gly Val Leu
    290             295             300

Gly Pro Tyr Gly Pro Gly Ala Ser Ala Ala Ala Ala Gly Ile Tyr
305             310             315                 320

Gly Pro Gly Val Leu Gly Pro Gly Ile Tyr Gly Pro Gly Ser Ser Gly
            325             330             335

Pro Gly Val Leu Gly Pro Tyr Gly Pro Gly Ser Ser Ala Ala Ala Ala
            340             345             350

Ala Gly Ile Tyr Gly Pro Gly Val Leu Gly Pro Tyr Gly Pro Gly Ile
        355             360             365

Ser Ala Ala Ala Ala Ala Gly Ile Tyr Val Leu Gly Pro Gly Val Leu
    370             375             380

Gly Pro Tyr Gly Pro Gly Ala Ser Gly Pro Gly Val Leu Gly Pro Tyr
385             390             395             400

Gly Pro Gly Ala Ser Ala Ala Ala Ala Gly Pro Gly Ile Tyr Gly
            405             410             415

Pro Gly Val Leu Gly Pro Ser Ala Ser Ala Ala Ala Ala Gly Ile
        420             425             430

Tyr Gly Ser Gly Pro Gly Ile Tyr Gly Pro Tyr Gly Pro Gly Ile Ser
        435             440             445

Gly Pro Gly Ser Gly Val Leu Gly Ile Gly Pro Tyr Gly Pro Gly Ala
    450             455             460

Ser Ala Ala Ala Ala Ala Gly Ile Tyr Gly Pro Gly Val Leu Gly Pro
465             470             475             480

```
Tyr Gly Pro Gly Ile Ser Ala Ala Ala Ala Gly Pro Gly Ser Gly
                485             490             495

Ile Tyr Gly Pro Gly Ala Ser Gly Ile Asn Gly Pro Gly Ser Gly Ile
            500             505             510

Tyr Gly Pro Gly Val Leu Gly Pro Gly Ile Ser Ala Ala Ala Ala
            515             520             525

Gly Ile Tyr Val Leu Gly Pro Gly Val Leu Gly Pro Tyr Gly Pro Gly
    530             535             540

Ala Ser Ala Ala Ala Ala Ala Gly Ile Tyr Gly Ser Gly Pro Gly Val
545             550             555             560

Leu Gly Pro Tyr Gly Pro Gly Ile Ser Gly Ser Gly Val Leu Gly Pro
            565             570             575

Gly Val Leu Gly Pro Tyr Ala Ser Ala Ala Ala Ala Gly Pro Gly
            580             585             590

Ser Gly Val Leu Gly Pro Gly Ala Ser
        595             600
```

```
<210>  36
<211>  601
<212>  PRT
<213>  Artificial Sequence

<220>
<223>  PRT916

<400>  36
```

```
Met His His His His His His Ser Ser Gly Ser Ser Gly Pro Gly Val
1               5               10              15

Ile Gly Pro Tyr Gly Pro Gly Ala Ser Ala Ala Ala Ala Ala Gly Leu
            20              25              30

Asn Gly Pro Gly Ser Gly Val Ile Gly Pro Gly Leu Ser Gly Leu Tyr
            35              40              45

Gly Pro Gly Val Ile Gly Pro Gly Val Ile Gly Pro Gly Ser Ser Ala
        50              55              60

Ala Ala Ala Gly Pro Gly Leu Tyr Gly Pro Gly Val Ile Gly Pro
65              70              75              80
```

Ser Ala Ser Ala Ala Ala Ala Ala Gly Pro Gly Ser Gly Val Ile Gly
                85                      90                      95

Pro Gly Ala Ser Gly Leu Tyr Gly Pro Gly Val Ile Gly Pro Gly Val
                100                     105                     110

Ile Gly Pro Gly Ser Ser Ala Ala Ala Ala Gly Leu Tyr Gly Ser
                115                     120                     125

Gly Pro Gly Val Ile Gly Pro Tyr Gly Ser Ala Ala Ala Ala Gly
                130                     135                     140

Pro Gly Ser Gly Leu Tyr Gly Leu Gly Pro Tyr Gly Pro Gly Ala Ser
145                     150                     155                     160

Gly Pro Gly Leu Tyr Gly Pro Gly Val Ile Gly Pro Ser Ala Ser Ala
                165                     170                     175

Ala Ala Ala Ala Gly Ser Gly Val Ile Gly Pro Gly Leu Tyr Gly Pro
                180                     185                     190

Tyr Ala Ser Ala Ala Ala Ala Ala Gly Leu Tyr Gly Ser Gly Pro Gly
                195                     200                     205

Val Ile Gly Pro Tyr Gly Pro Gly Leu Ser Gly Ser Gly Val Ile Gly
                210                     215                     220

Pro Gly Val Ile Gly Pro Tyr Ala Ser Ala Ala Ala Ala Gly Pro
225                     230                     235                     240

Gly Val Ile Gly Pro Tyr Gly Pro Gly Ser Ser Ala Ala Ala Ala Ala
                245                     250                     255

Gly Leu Tyr Gly Tyr Gly Pro Gly Val Ile Gly Pro Tyr Gly Pro Gly
                260                     265                     270

Ala Ser Gly Leu Asn Gly Pro Gly Ser Gly Leu Tyr Gly Pro Gly Val
                275                     280                     285

Ile Gly Pro Gly Leu Ser Ala Ala Ala Ala Gly Pro Gly Val Ile
                290                     295                     300

Gly Pro Tyr Gly Pro Gly Ala Ser Ala Ala Ala Ala Gly Leu Tyr
305                     310                     315                     320

Gly Pro Gly Val Ile Gly Pro Gly Leu Tyr Gly Pro Gly Ser Ser Gly
                325                     330                     335

```
Pro Gly Val Ile Gly Pro Tyr Gly Pro Gly Ser Ser Ala Ala Ala Ala
            340             345             350

Ala Gly Leu Tyr Gly Pro Gly Val Ile Gly Pro Tyr Gly Pro Gly Leu
            355             360             365

Ser Ala Ala Ala Ala Ala Gly Leu Tyr Val Ile Gly Pro Gly Val Ile
            370             375             380

Gly Pro Tyr Gly Pro Gly Ala Ser Gly Pro Gly Val Ile Gly Pro Tyr
385             390             395             400

Gly Pro Gly Ala Ser Ala Ala Ala Ala Ala Gly Pro Gly Leu Tyr Gly
            405             410             415

Pro Gly Val Ile Gly Pro Ser Ala Ser Ala Ala Ala Ala Ala Gly Leu
            420             425             430

Tyr Gly Ser Gly Pro Gly Leu Tyr Gly Pro Tyr Gly Pro Gly Leu Ser
            435             440             445

Gly Pro Gly Ser Gly Val Ile Gly Leu Gly Pro Tyr Gly Pro Gly Ala
    450             455             460

Ser Ala Ala Ala Ala Ala Gly Leu Tyr Gly Pro Gly Val Ile Gly Pro
465             470             475             480

Tyr Gly Pro Gly Leu Ser Ala Ala Ala Ala Gly Pro Gly Ser Gly
            485             490             495

Leu Tyr Gly Pro Gly Ala Ser Gly Leu Asn Gly Pro Gly Ser Gly Leu
            500             505             510

Tyr Gly Pro Gly Val Ile Gly Pro Gly Leu Ser Ala Ala Ala Ala Ala
            515             520             525

Gly Leu Tyr Val Ile Gly Pro Gly Val Ile Gly Pro Tyr Gly Pro Gly
    530             535             540

Ala Ser Ala Ala Ala Ala Ala Gly Leu Tyr Gly Ser Gly Pro Gly Val
545             550             555             560

Ile Gly Pro Tyr Gly Pro Gly Leu Ser Gly Ser Gly Val Ile Gly Pro
            565             570             575

Gly Val Ile Gly Pro Tyr Ala Ser Ala Ala Ala Ala Ala Gly Pro Gly
    580             585             590
```

```
Ser Gly Val Ile Gly Pro Gly Ala Ser
        595                 600
```

<210> 37
<211> 601
<212> PRT
<213> Artificial Sequence

<220>
<223> PRT918

<400> 37

```
Met His His His His His His Ser Ser Gly Ser Ser Gly Pro Gly Val
1                   5                   10                  15
```

```
Phe Gly Pro Tyr Gly Pro Gly Ala Ser Ala Ala Ala Ala Ala Gly Ile
            20                  25                  30
```

```
Asn Gly Pro Gly Ser Gly Val Phe Gly Pro Gly Ile Ser Gly Ile Tyr
            35                  40                  45
```

```
Gly Pro Gly Val Phe Gly Pro Gly Val Phe Gly Pro Gly Ser Ser Ala
        50                  55                  60
```

```
Ala Ala Ala Ala Gly Pro Gly Ile Tyr Gly Pro Gly Val Phe Gly Pro
65                      70                  75                  80
```

```
Ser Ala Ser Ala Ala Ala Ala Ala Gly Pro Gly Ser Gly Val Phe Gly
            85                  90                  95
```

```
Pro Gly Ala Ser Gly Ile Tyr Gly Pro Gly Val Phe Gly Pro Gly Val
            100                 105                 110
```

```
Phe Gly Pro Gly Ser Ser Ala Ala Ala Ala Gly Ile Tyr Gly Ser
            115                 120                 125
```

```
Gly Pro Gly Val Phe Gly Pro Tyr Gly Ser Ala Ala Ala Ala Ala Gly
        130                 135                 140
```

```
Pro Gly Ser Gly Ile Tyr Gly Ile Gly Pro Tyr Gly Pro Gly Ala Ser
145                 150                 155                 160
```

```
Gly Pro Gly Ile Tyr Gly Pro Gly Val Phe Gly Pro Ser Ala Ser Ala
            165                 170                 175
```

```
Ala Ala Ala Ala Gly Ser Gly Val Phe Gly Pro Gly Ile Tyr Gly Pro
            180                 185                 190
```

141

Tyr Ala Ser Ala Ala Ala Ala Ala Gly Ile Tyr Gly Ser Gly Pro Gly
        195                 200             205

Val Phe Gly Pro Tyr Gly Pro Gly Ile Ser Gly Ser Gly Val Phe Gly
        210                 215             220

Pro Gly Val Phe Gly Pro Tyr Ala Ser Ala Ala Ala Ala Ala Gly Pro
225                 230             235                 240

Gly Val Phe Gly Pro Tyr Gly Pro Gly Ser Ser Ala Ala Ala Ala Ala
                245             250                 255

Gly Ile Tyr Gly Tyr Gly Pro Gly Val Phe Gly Pro Tyr Gly Pro Gly
            260             265             270

Ala Ser Gly Ile Asn Gly Pro Gly Ser Gly Ile Tyr Gly Pro Gly Val
        275             280             285

Phe Gly Pro Gly Ile Ser Ala Ala Ala Ala Gly Pro Gly Val Phe
        290             295             300

Gly Pro Tyr Gly Pro Gly Ala Ser Ala Ala Ala Ala Gly Ile Tyr
305                 310             315                 320

Gly Pro Gly Val Phe Gly Pro Gly Ile Tyr Gly Pro Gly Ser Ser Gly
                325             330             335

Pro Gly Val Phe Gly Pro Tyr Gly Pro Gly Ser Ser Ala Ala Ala Ala
            340             345             350

Ala Gly Ile Tyr Gly Pro Gly Val Phe Gly Pro Tyr Gly Pro Gly Ile
        355             360             365

Ser Ala Ala Ala Ala Gly Ile Tyr Val Phe Gly Pro Gly Val Phe
        370             375             380

Gly Pro Tyr Gly Pro Gly Ala Ser Gly Pro Gly Val Phe Gly Pro Tyr
385                 390             395                 400

Gly Pro Gly Ala Ser Ala Ala Ala Ala Gly Pro Gly Ile Tyr Gly
                405             410                 415

Pro Gly Val Phe Gly Pro Ser Ala Ser Ala Ala Ala Ala Gly Ile
            420             425             430

Tyr Gly Ser Gly Pro Gly Ile Tyr Gly Pro Tyr Gly Pro Gly Ile Ser
        435             440             445

142

```
Gly Pro Gly Ser Gly Val Phe Gly Ile Gly Pro Tyr Gly Pro Gly Ala
    450                 455                 460

Ser Ala Ala Ala Ala Ala Gly Ile Tyr Gly Pro Gly Val Phe Gly Pro
465                 470                 475                 480

Tyr Gly Pro Gly Ile Ser Ala Ala Ala Ala Gly Pro Gly Ser Gly
                485                 490                 495

Ile Tyr Gly Pro Gly Ala Ser Gly Ile Asn Gly Pro Gly Ser Gly Ile
            500                 505                 510

Tyr Gly Pro Gly Val Phe Gly Pro Gly Ile Ser Ala Ala Ala Ala Ala
            515                 520                 525

Gly Ile Tyr Val Phe Gly Pro Gly Val Phe Gly Pro Tyr Gly Pro Gly
    530                 535                 540

Ala Ser Ala Ala Ala Ala Ala Gly Ile Tyr Gly Ser Gly Pro Gly Val
545                 550                 555                 560

Phe Gly Pro Tyr Gly Pro Gly Ile Ser Gly Ser Gly Val Phe Gly Pro
            565                 570                 575

Gly Val Phe Gly Pro Tyr Ala Ser Ala Ala Ala Ala Ala Gly Pro Gly
            580                 585                 590

Ser Gly Val Phe Gly Pro Gly Ala Ser
        595                 600
```

<210> 38
<211> 576
<212> PRT
<213> Artificial Sequence

<220>
<223> PRT699

<400> 38

```
Met His His His His His His Ser Ser Gly Ser Ser Gly Pro Gly Val
1               5                   10                  15

Leu Gly Pro Tyr Gly Pro Gly Ala Ser Ala Ala Ala Ala Ala Ala Ala
            20                  25                  30

Gly Ser Asn Gly Pro Gly Ser Gly Val Leu Gly Pro Gly Gln Ser Gly
            35                  40                  45
```

Gln Tyr Gly Pro Gly Val Leu Gly Pro Gly Val Leu Gly Pro Gly Ser
50                      55                      60

Ser Ala Ala Ala Ala Ala Ala Ala Gly Pro Gly Gln Tyr Gly Pro Gly
65              70              75                      80

Val Leu Gly Pro Ser Ala Ser Ala Ala Ala Ala Ala Ala Gly Pro
85              90                      95

Gly Ser Gly Val Leu Gly Pro Gly Ala Ser Gly Gln Tyr Gly Pro Gly
100             105                     110

Val Leu Gly Pro Gly Val Leu Gly Pro Gly Ser Ser Ala Ala Ala Ala
115                     120                     125

Ala Ala Ala Gly Ser Tyr Gly Ser Gly Pro Gly Val Leu Gly Pro Tyr
130                     135                     140

Gly Ser Ala Ala Ala Ala Ala Ala Ala Gly Pro Gly Ser Gly Gln Tyr
145             150                     155                     160

Gly Gln Gly Pro Tyr Gly Pro Gly Ala Ser Gly Pro Gly Gln Tyr Gly
165                     170                     175

Pro Gly Val Leu Gly Pro Ser Ala Ser Ala Ala Ala Ala Ala Ala Ala
180                     185                     190

Gly Ser Gly Val Leu Gly Pro Gly Gln Tyr Gly Pro Tyr Ala Ser Ala
195                     200                     205

Ala Ala Ala Ala Ala Gly Ser Tyr Gly Ser Gly Pro Gly Val Leu
210                     215                     220

Gly Pro Tyr Gly Pro Gly Gln Ser Gly Ser Gly Val Leu Gly Pro Gly
225             230                     235                     240

Val Leu Gly Pro Tyr Ala Ser Ala Ala Ala Ala Ala Ala Gly Pro
245                     250                     255

Gly Val Leu Gly Pro Tyr Gly Pro Gly Ser Ser Ala Ala Ala Ala Ala
260                     265                     270

Ala Ala Gly Ser Tyr Gly Tyr Gly Pro Gly Val Leu Gly Pro Tyr Gly
275                     280                     285

Pro Gly Ala Ser Gly Gln Asn Gly Pro Gly Ser Gly Gln Tyr Gly Pro
290                     295                     300

144

Gly Val Leu Gly Pro Gly Pro Ser Ala Ala Ala Ala Ala Ala Ala Gly
305                 310             315                 320

Pro Gly Val Leu Gly Pro Tyr Gly Pro Gly Ala Ser Ala Ala Ala Ala
                325             330                 335

Ala Ala Ala Gly Ser Tyr Gly Pro Gly Val Leu Gly Pro Gly Gln Tyr
            340             345                 350

Gly Pro Gly Ser Ser Gly Pro Gly Val Leu Gly Pro Tyr Gly Pro Gly
            355             360                 365

Ser Ser Ala Ala Ala Ala Ala Ala Ala Gly Ser Tyr Gly Pro Gly Val
    370             375                 380

Leu Gly Pro Tyr Gly Pro Gly Pro Ser Ala Ala Ala Ala Ala Ala Ala
385             390                 395                 400

Gly Ser Tyr Val Leu Gly Pro Gly Val Leu Gly Pro Tyr Gly Pro Gly
            405             410                 415

Ala Ser Gly Pro Gly Val Leu Gly Pro Tyr Gly Pro Gly Ala Ser Ala
            420             425                 430

Ala Ala Ala Ala Ala Ala Gly Pro Gly Gln Tyr Gly Pro Gly Val Leu
    435             440                 445

Gly Pro Ser Ala Ser Ala Ala Ala Ala Ala Ala Gly Ser Tyr Gly
    450             455                 460

Ser Gly Pro Gly Gln Tyr Gly Pro Tyr Gly Pro Gly Gln Ser Gly Pro
465             470                 475                 480

Gly Ser Gly Val Leu Gly Gln Gly Pro Tyr Gly Pro Gly Ala Ser Ala
            485             490                 495

Ala Ala Ala Ala Ala Gly Ser Tyr Gly Pro Gly Val Leu Gly Pro
    500             505                 510

Tyr Gly Pro Gly Pro Ser Ala Ala Ala Ala Ala Ala Gly Pro Gly
    515             520                 525

Ser Gly Gln Tyr Gly Pro Gly Ala Ser Gly Gln Asn Gly Pro Gly Ser
    530             535                 540

Gly Gln Tyr Gly Pro Gly Val Leu Gly Pro Gly Pro Ser Ala Ala Ala

|   |   |   |   |   |   |   |   |   |   |   |   |   |   |   |
|---|---|---|---|---|---|---|---|---|---|---|---|---|---|---|
| 545 | | | | | 550 | | | | | 555 | | | | 560 |

Ala Ala Ala Ala Gly Pro Gly Ser Gly Val Leu Gly Pro Gly Ala Ser
                565                 570                 575

<210> 39
<211> 576
<212> PRT
<213> Artificial Sequence

<220>
<223> PRT698

<400> 39

Met His His His His His His Ser Ser Gly Ser Ser Gly Pro Gly Val
1                   5                   10                  15

Leu Gly Pro Tyr Gly Pro Gly Ala Ser Ala Ala Ala Ala Ala Ala Ala
            20                  25                  30

Gly Ser Asn Gly Pro Gly Ser Gly Val Leu Gly Pro Gly Ile Ser Gly
        35                  40                  45

Ile Tyr Gly Pro Gly Val Leu Gly Pro Gly Val Leu Gly Pro Gly Ser
        50                  55                  60

Ser Ala Ala Ala Ala Ala Ala Ala Gly Pro Gly Ile Tyr Gly Pro Gly
65                  70                  75                  80

Val Leu Gly Pro Ser Ala Ser Ala Ala Ala Ala Ala Ala Ala Gly Pro
                85                  90                  95

Gly Ser Gly Val Leu Gly Pro Gly Ala Ser Gly Ile Tyr Gly Pro Gly
            100                 105                 110

Val Leu Gly Pro Gly Val Leu Gly Pro Gly Ser Ser Ala Ala Ala Ala
            115                 120                 125

Ala Ala Ala Gly Ser Tyr Gly Ser Gly Pro Gly Val Leu Gly Pro Tyr
        130                 135                 140

Gly Ser Ala Ala Ala Ala Ala Ala Ala Gly Pro Gly Ser Gly Ile Tyr
145                 150                 155                 160

Gly Ile Gly Pro Tyr Gly Pro Gly Ala Ser Gly Pro Gly Ile Tyr Gly
            165                 170                 175

Pro Gly Val Leu Gly Pro Ser Ala Ser Ala Ala Ala Ala Ala Ala Ala
            180                 185                 190

```
Gly Ser Gly Val Leu Gly Pro Gly Ile Tyr Gly Pro Tyr Ala Ser Ala
        195                 200             205

Ala Ala Ala Ala Ala Ala Gly Ser Tyr Gly Ser Gly Pro Gly Val Leu
        210                 215             220

Gly Pro Tyr Gly Pro Gly Ile Ser Gly Ser Gly Val Leu Gly Pro Gly
225                 230                 235                 240

Val Leu Gly Pro Tyr Ala Ser Ala Ala Ala Ala Ala Ala Ala Gly Pro
                245                 250                 255

Gly Val Leu Gly Pro Tyr Gly Pro Gly Ser Ser Ala Ala Ala Ala Ala
                260                 265                 270

Ala Ala Gly Ser Tyr Gly Tyr Gly Pro Gly Val Leu Gly Pro Tyr Gly
        275                 280                 285

Pro Gly Ala Ser Gly Ile Asn Gly Pro Gly Ser Gly Ile Tyr Gly Pro
        290                 295                 300

Gly Val Leu Gly Pro Gly Pro Ser Ala Ala Ala Ala Ala Ala Ala Gly
305                 310                 315                 320

Pro Gly Val Leu Gly Pro Tyr Gly Pro Gly Ala Ser Ala Ala Ala Ala
                325                 330                 335

Ala Ala Ala Gly Ser Tyr Gly Pro Gly Val Leu Gly Pro Gly Ile Tyr
        340                 345                 350

Gly Pro Gly Ser Ser Gly Pro Gly Val Leu Gly Pro Tyr Gly Pro Gly
        355                 360                 365

Ser Ser Ala Ala Ala Ala Ala Ala Ala Gly Ser Tyr Gly Pro Gly Val
        370                 375                 380

Leu Gly Pro Tyr Gly Pro Gly Pro Ser Ala Ala Ala Ala Ala Ala Ala
385                 390                 395                 400

Gly Ser Tyr Val Leu Gly Pro Gly Val Leu Gly Pro Tyr Gly Pro Gly
                405                 410                 415

Ala Ser Gly Pro Gly Val Leu Gly Pro Tyr Gly Pro Gly Ala Ser Ala
                420                 425                 430

Ala Ala Ala Ala Ala Ala Gly Pro Gly Ile Tyr Gly Pro Gly Val Leu
```

```
                     435                       440                         445


     Gly Pro Ser Ala Ser Ala Ala Ala Ala Ala Ala Ala Gly Ser Tyr Gly
         450                 455                 460


     Ser Gly Pro Gly Ile Tyr Gly Pro Tyr Gly Pro Gly Ile Ser Gly Pro
     465                 470                 475                 480


     Gly Ser Gly Val Leu Gly Ile Gly Pro Tyr Gly Pro Gly Ala Ser Ala
                     485                 490                 495


     Ala Ala Ala Ala Ala Ala Gly Ser Tyr Gly Pro Gly Val Leu Gly Pro
                 500                 505                 510


     Tyr Gly Pro Gly Pro Ser Ala Ala Ala Ala Ala Ala Ala Gly Pro Gly
             515                 520                 525


     Ser Gly Ile Tyr Gly Pro Gly Ala Ser Gly Ile Asn Gly Pro Gly Ser
         530                 535                 540


     Gly Ile Tyr Gly Pro Gly Val Leu Gly Pro Gly Pro Ser Ala Ala Ala
     545                 550                 555                 560


     Ala Ala Ala Ala Gly Pro Gly Ser Gly Val Leu Gly Pro Gly Ala Ser
                 565                 570                 575


     <210>   40
     <211>   1190
     <212>   PRT
     <213>   Artificial Sequence

     <220>
     <223>   PRT966

     <400>   40

     Met His His His His His His Ser Ser Gly Ser Ser Gly Pro Gly Val
     1               5                   10                  15


     Phe Gly Pro Tyr Gly Pro Gly Ala Ser Ala Ala Ala Ala Ala Gly Ile
                     20                  25                  30


     Asn Gly Pro Gly Ser Gly Val Phe Gly Pro Gly Ile Ser Gly Ile Tyr
                 35                  40                  45


     Gly Pro Gly Val Phe Gly Pro Gly Val Phe Gly Pro Gly Ser Ser Ala
             50                  55                  60


     Ala Ala Ala Ala Gly Pro Gly Ile Tyr Gly Pro Gly Val Phe Gly Pro
     65                  70                  75                  80
```

148

```
Ser Ala Ser Ala Ala Ala Ala Ala Gly Pro Gly Ser Gly Val Phe Gly
                85                  90                  95

Pro Gly Ala Ser Gly Ile Tyr Gly Pro Gly Val Phe Gly Pro Gly Val
            100                 105                 110

Phe Gly Pro Gly Ser Ser Ala Ala Ala Ala Gly Ile Tyr Gly Ser
            115                 120                 125

Gly Pro Gly Val Phe Gly Pro Tyr Gly Ser Ala Ala Ala Ala Gly
        130                 135                 140

Pro Gly Ser Gly Ile Tyr Gly Ile Gly Pro Tyr Gly Pro Gly Ala Ser
145                 150                 155                 160

Gly Pro Gly Ile Tyr Gly Pro Gly Val Phe Gly Pro Ser Ala Ser Ala
                165                 170                 175

Ala Ala Ala Ala Gly Ser Gly Val Phe Gly Pro Gly Ile Tyr Gly Pro
            180                 185                 190

Tyr Ala Ser Ala Ala Ala Ala Ala Gly Ile Tyr Gly Ser Gly Pro Gly
            195                 200                 205

Val Phe Gly Pro Tyr Gly Pro Gly Ile Ser Gly Ser Gly Val Phe Gly
    210                 215                 220

Pro Gly Val Phe Gly Pro Tyr Ala Ser Ala Ala Ala Ala Ala Gly Pro
225                 230                 235                 240

Gly Val Phe Gly Pro Tyr Gly Pro Gly Ser Ser Ala Ala Ala Ala Ala
            245                 250                 255

Gly Ile Tyr Gly Tyr Gly Pro Gly Val Phe Gly Pro Tyr Gly Pro Gly
            260                 265                 270

Ala Ser Gly Ile Asn Gly Pro Gly Ser Gly Ile Tyr Gly Pro Gly Val
        275                 280                 285

Phe Gly Pro Gly Ile Ser Ala Ala Ala Ala Gly Pro Gly Val Phe
    290                 295                 300

Gly Pro Tyr Gly Pro Gly Ala Ser Ala Ala Ala Ala Gly Ile Tyr
305                 310                 315                 320

Gly Pro Gly Val Phe Gly Pro Gly Ile Tyr Gly Pro Gly Ser Ser Gly
```

<div align="center">325          330          335</div>

Pro Gly Val Phe Gly Pro Tyr Gly Pro Gly Ser Ser Ala Ala Ala Ala
      340        345        350

Ala Gly Ile Tyr Gly Pro Gly Val Phe Gly Pro Tyr Gly Pro Gly Ile
    355        360        365

Ser Ala Ala Ala Ala Ala Gly Ile Tyr Val Phe Gly Pro Gly Val Phe
    370        375        380

Gly Pro Tyr Gly Pro Gly Ala Ser Gly Pro Gly Val Phe Gly Pro Tyr
385        390        395        400

Gly Pro Gly Ala Ser Ala Ala Ala Ala Ala Gly Pro Gly Ile Tyr Gly
      405        410        415

Pro Gly Val Phe Gly Pro Ser Ala Ser Ala Ala Ala Ala Ala Gly Ile
    420        425        430

Tyr Gly Ser Gly Pro Gly Ile Tyr Gly Pro Tyr Gly Pro Gly Ile Ser
    435        440        445

Gly Pro Gly Ser Gly Val Phe Gly Ile Gly Pro Tyr Gly Pro Gly Ala
    450        455        460

Ser Ala Ala Ala Ala Ala Gly Ile Tyr Gly Pro Gly Val Phe Gly Pro
465        470        475        480

Tyr Gly Pro Gly Ile Ser Ala Ala Ala Ala Gly Pro Gly Ser Gly
      485        490        495

Ile Tyr Gly Pro Gly Ala Ser Gly Ile Asn Gly Pro Gly Ser Gly Ile
    500        505        510

Tyr Gly Pro Gly Val Phe Gly Pro Gly Ile Ser Ala Ala Ala Ala Ala
    515        520        525

Gly Ile Tyr Val Phe Gly Pro Gly Val Phe Gly Pro Tyr Gly Pro Gly
    530        535        540

Ala Ser Ala Ala Ala Ala Ala Gly Ile Tyr Gly Ser Gly Pro Gly Val
545        550        555        560

Phe Gly Pro Tyr Gly Pro Gly Ile Ser Gly Ser Gly Val Phe Gly Pro
      565        570        575

```
Gly Val Phe Gly Pro Tyr Ala Ser Ala Ala Ala Ala Gly Pro Gly
            580             585             590

Ser Gly Val Phe Gly Pro Gly Ala Ser Gly Pro Gly Val Phe Gly Pro
            595             600             605

Tyr Gly Pro Gly Ala Ser Ala Ala Ala Ala Gly Ile Asn Gly Pro
    610             615             620

Gly Ser Gly Val Phe Gly Pro Gly Ile Ser Gly Ile Tyr Gly Pro Gly
625             630             635             640

Val Phe Gly Pro Gly Val Phe Gly Pro Gly Ser Ser Ala Ala Ala Ala
            645             650             655

Ala Gly Pro Gly Ile Tyr Gly Pro Gly Val Phe Gly Pro Ser Ala Ser
            660             665             670

Ala Ala Ala Ala Ala Gly Pro Gly Ser Gly Val Phe Gly Pro Gly Ala
    675             680             685

Ser Gly Ile Tyr Gly Pro Gly Val Phe Gly Pro Gly Val Phe Gly Pro
    690             695             700

Gly Ser Ser Ala Ala Ala Ala Gly Ile Tyr Gly Ser Gly Pro Gly
705             710             715             720

Val Phe Gly Pro Tyr Gly Ser Ala Ala Ala Ala Gly Pro Gly Ser
            725             730             735

Gly Ile Tyr Gly Ile Gly Pro Tyr Gly Pro Gly Ala Ser Gly Pro Gly
            740             745             750

Ile Tyr Gly Pro Gly Val Phe Gly Pro Ser Ala Ser Ala Ala Ala Ala
    755             760             765

Ala Gly Ser Gly Val Phe Gly Pro Gly Ile Tyr Gly Pro Tyr Ala Ser
    770             775             780

Ala Ala Ala Ala Ala Gly Ile Tyr Gly Ser Gly Pro Gly Val Phe Gly
785             790             795             800

Pro Tyr Gly Pro Gly Ile Ser Gly Ser Gly Val Phe Gly Pro Gly Val
            805             810             815

Phe Gly Pro Tyr Ala Ser Ala Ala Ala Ala Gly Pro Gly Val Phe
    820             825             830
```

```
Gly Pro Tyr Gly Pro Gly Ser Ser Ala Ala Ala Ala Ala Gly Ile Tyr
        835                 840                 845


Gly Tyr Gly Pro Gly Val Phe Gly Pro Tyr Gly Pro Gly Ala Ser Gly
        850                 855                 860


Ile Asn Gly Pro Gly Ser Gly Ile Tyr Gly Pro Gly Val Phe Gly Pro
865                 870                 875                 880


Gly Ile Ser Ala Ala Ala Ala Ala Gly Pro Gly Val Phe Gly Pro Tyr
                885                 890                 895


Gly Pro Gly Ala Ser Ala Ala Ala Ala Gly Ile Tyr Gly Pro Gly
                900                 905                 910


Val Phe Gly Pro Gly Ile Tyr Gly Pro Gly Ser Ser Gly Pro Gly Val
            915                 920                 925


Phe Gly Pro Tyr Gly Pro Gly Ser Ser Ala Ala Ala Ala Ala Gly Ile
    930                 935                 940


Tyr Gly Pro Gly Val Phe Gly Pro Tyr Gly Pro Gly Ile Ser Ala Ala
945                 950                 955                 960


Ala Ala Ala Gly Ile Tyr Val Phe Gly Pro Gly Val Phe Gly Pro Tyr
                965                 970                 975


Gly Pro Gly Ala Ser Gly Pro Gly Val Phe Gly Pro Tyr Gly Pro Gly
                980                 985                 990


Ala Ser Ala Ala Ala Ala Ala Gly  Pro Gly Ile Tyr Gly  Pro Gly Val
        995                 1000                1005


Phe Gly  Pro Ser Ala Ser Ala  Ala Ala Ala Ala Gly  Ile Tyr Gly
    1010                1015                1020


Ser Gly  Pro Gly Ile Tyr Gly  Pro Tyr Gly Pro Gly  Ile Ser Gly
    1025                1030                1035


Pro Gly  Ser Gly Val Phe Gly  Ile Gly Pro Tyr Gly  Pro Gly Ala
    1040                1045                1050


Ser Ala  Ala Ala Ala Ala Gly  Ile Tyr Gly Pro Gly  Val Phe Gly
    1055                1060                1065


Pro Tyr  Gly Pro Gly Ile Ser  Ala Ala Ala Ala Ala  Gly Pro Gly
    1070                1075                1080
```

152

```
Ser Gly  Ile Tyr Gly Pro Gly  Ala Ser Gly Ile Asn  Gly Pro Gly
    1085                1090                1095

Ser Gly  Ile Tyr Gly Pro Gly  Val Phe Gly Pro Gly  Ile Ser Ala
    1100                1105                1110

Ala Ala  Ala Ala Gly Ile Tyr  Val Phe Gly Pro Gly  Val Phe Gly
    1115                1120                1125

Pro Tyr  Gly Pro Gly Ala Ser  Ala Ala Ala Ala Ala  Gly Ile Tyr
    1130                1135                1140

Gly Ser  Gly Pro Gly Val Phe  Gly Pro Tyr Gly Pro  Gly Ile Ser
    1145                1150                1155

Gly Ser  Gly Val Phe Gly Pro  Gly Val Phe Gly Pro  Tyr Ala Ser
    1160                1165                1170

Ala Ala  Ala Ala Ala Gly Pro  Gly Ser Gly Val Phe  Gly Pro Gly
    1175                1180                1185

Ala Ser
    1190


<210>  41
<211>  252
<212>  PRT
<213>  Artificial Sequence

<220>
<223>  Collagen-type4-Kai

<400>  41

Met His His His His His His Ser Ser Gly Ser Ser Lys Asp Gly Val
1           5               10              15

Pro Gly Phe Pro Gly Ser Glu Gly Val Lys Gly Asn Arg Gly Phe Pro
            20              25              30

Gly Leu Met Gly Glu Asp Gly Ile Lys Gly Gln Lys Gly Asp Ile Gly
        35              40              45

Pro Pro Gly Phe Arg Gly Pro Thr Glu Tyr Tyr Asp Thr Tyr Gln Glu
    50              55              60

Lys Gly Asp Glu Gly Thr Pro Gly Pro Pro Gly Pro Arg Gly Ala Arg
65              70              75              80
```

```
Gly Pro Gln Gly Pro Ser Gly Pro Pro Gly Val Pro Gly Ser Pro Gly
                85                  90                  95

Ser Ser Arg Pro Gly Leu Arg Gly Ala Pro Gly Trp Pro Gly Leu Lys
            100                 105                 110

Gly Ser Lys Gly Glu Arg Gly Arg Pro Gly Lys Asp Ala Met Gly Thr
            115                 120                 125

Pro Gly Ser Pro Gly Cys Ala Gly Ser Pro Gly Leu Pro Gly Ser Pro
    130                 135                 140

Gly Pro Pro Gly Pro Pro Gly Asp Ile Val Phe Arg Lys Gly Pro Pro
145                 150                 155                 160

Gly Asp His Gly Leu Pro Gly Tyr Leu Gly Ser Pro Gly Ile Pro Gly
            165                 170                 175

Val Asp Gly Pro Lys Gly Glu Pro Gly Leu Leu Cys Thr Gln Cys Pro
            180                 185                 190

Tyr Ile Pro Gly Pro Pro Gly Leu Pro Gly Leu Pro Gly Leu His Gly
        195                 200                 205

Val Lys Gly Ile Pro Gly Arg Gln Gly Ala Ala Gly Leu Lys Gly Ser
    210                 215                 220

Pro Gly Ser Pro Gly Asn Thr Gly Leu Pro Gly Phe Pro Gly Phe Pro
225                 230                 235                 240

Gly Ala Gln Gly Asp Pro Gly Leu Lys Gly Glu Lys
            245                 250
```

```
<210>  42
<211>  310
<212>  PRT
<213>  Artificial Sequence

<220>
<223>  Resilin-Kai

<400>  42
```

```
Met His His His His His His Pro Glu Pro Pro Val Asn Ser Tyr Leu
1               5                   10                  15

Pro Pro Ser Asp Ser Tyr Gly Ala Pro Gly Gln Ser Gly Pro Gly Gly
            20                  25                  30
```

```
Arg Pro Ser Asp Ser Tyr Gly Ala Pro Gly Gly Gly Asn Gly Gly Arg
        35              40              45

Pro Ser Asp Ser Tyr Gly Ala Pro Gly Gln Gly Gln Gly Gln Gly Gln
        50              55              60

Gly Gln Gly Gly Tyr Ala Gly Lys Pro Ser Asp Ser Tyr Gly Ala Pro
65              70              75              80

Gly Gly Gly Asp Gly Asn Gly Gly Arg Pro Ser Ser Ser Tyr Gly Ala
            85              90              95

Pro Gly Gly Gly Asn Gly Gly Arg Pro Ser Asp Thr Tyr Gly Ala Pro
        100             105             110

Gly Gly Gly Asn Gly Gly Arg Pro Ser Asp Thr Tyr Gly Ala Pro Gly
        115             120             125

Gly Gly Gly Asn Gly Asn Gly Gly Arg Pro Ser Ser Ser Tyr Gly Ala
    130             135             140

Pro Gly Gln Gly Gln Gly Asn Gly Asn Gly Gly Arg Pro Ser Ser Ser
145             150             155             160

Tyr Gly Ala Pro Gly Gly Gly Asn Gly Gly Arg Pro Ser Asp Thr Tyr
            165             170             175

Gly Ala Pro Gly Gly Gly Asn Gly Gly Arg Pro Ser Asp Thr Tyr Gly
        180             185             190

Ala Pro Gly Gly Gly Asn Asn Gly Gly Arg Pro Ser Ser Ser Tyr Gly
        195             200             205

Ala Pro Gly Gly Gly Asn Gly Gly Arg Pro Ser Asp Thr Tyr Gly Ala
        210             215             220

Pro Gly Gly Gly Asn Gly Asn Gly Ser Gly Gly Arg Pro Ser Ser Ser
225             230             235             240

Tyr Gly Ala Pro Gly Gln Gly Gln Gly Gly Phe Gly Gly Arg Pro Ser
            245             250             255

Asp Ser Tyr Gly Ala Pro Gly Gln Asn Gln Lys Pro Ser Asp Ser Tyr
        260             265             270

Gly Ala Pro Gly Ser Gly Asn Gly Asn Gly Gly Arg Pro Ser Ser Ser
        275             280             285
```

```
Tyr Gly Ala Pro Gly Ser Gly Pro Gly Gly Arg Pro Ser Asp Ser Tyr
    290             295             300

Gly Pro Pro Ala Ser Gly
305             310


<210>  43
<211>  282
<212>  PRT
<213>  Artificial Sequence

<220>
<223>  elastin short

<400>  43

Met His His His His His His Ser Ser Gly Ser Ser Leu Gly Val Ser
1               5               10              15

Ala Gly Ala Val Val Pro Gln Pro Gly Ala Gly Val Lys Pro Gly Lys
            20              25              30

Val Pro Gly Val Gly Leu Pro Gly Val Tyr Pro Gly Gly Val Leu Pro
        35              40              45

Gly Ala Arg Phe Pro Gly Val Gly Val Leu Pro Gly Val Pro Thr Gly
    50              55              60

Ala Gly Val Lys Pro Lys Ala Pro Gly Val Gly Gly Ala Phe Ala Gly
65              70              75              80

Ile Pro Gly Val Gly Pro Phe Gly Gly Pro Gln Pro Gly Val Pro Leu
            85              90              95

Gly Tyr Pro Ile Lys Ala Pro Lys Leu Pro Gly Gly Tyr Gly Leu Pro
            100             105             110

Tyr Thr Thr Gly Lys Leu Pro Tyr Gly Tyr Gly Pro Gly Gly Val Ala
        115             120             125

Gly Ala Ala Gly Lys Ala Gly Tyr Pro Thr Gly Thr Gly Val Gly Pro
    130             135             140

Gln Ala Ala Ala Ala Ala Ala Ala Lys Ala Ala Ala Lys Phe Gly Ala
145             150             155             160

Gly Ala Ala Gly Val Leu Pro Gly Val Gly Gly Ala Gly Val Pro Gly
            165             170             175
```

```
Val Pro Gly Ala Ile Pro Gly Ile Gly Gly Ile Ala Gly Val Gly Thr
            180             185             190


Pro Ala Ala Ala Ala Ala Ala Ala Ala Ala Ala Lys Ala Ala Lys Tyr
            195             200             205


Gly Ala Ala Ala Gly Leu Val Pro Gly Gly Pro Gly Phe Gly Pro Gly
    210             215             220


Val Val Gly Val Pro Gly Ala Gly Val Pro Gly Val Gly Val Pro Gly
225             230             235             240


Ala Gly Ile Pro Val Val Pro Gly Ala Gly Ile Pro Gly Ala Ala Val
            245             250             255


Pro Gly Val Val Ser Pro Glu Ala Ala Ala Lys Ala Ala Ala Lys Ala
            260             265             270


Ala Lys Tyr Gly Ala Arg Pro Gly Val Gly
        275             280
```

```
<210>   44
<211>   468
<212>   PRT
<213>   Artificial Sequence

<220>
<223>   type I keratin 26

<400>   44
```

```
Met Ser Phe Arg Leu Ser Gly Val Ser Arg Arg Leu Cys Ser Gln Ala
1               5               10              15


Gly Thr Gly Arg Leu Thr Gly Gly Arg Thr Gly Phe Arg Ala Gly Asn
            20              25              30


Val Cys Ser Gly Leu Gly Ala Gly Ser Ser Phe Ser Gly Pro Leu Gly
            35              40              45


Ser Val Ser Ser Lys Gly Ser Phe Ser His Gly Gly Gly Gly Leu Gly
        50              55              60


Ser Gly Val Cys Thr Gly Phe Leu Glu Asn Glu His Gly Leu Leu Pro
65              70              75              80


Gly Asn Glu Lys Val Thr Leu Gln Asn Leu Asn Asp Arg Leu Ala Ser
            85              90              95


Tyr Leu Asp His Val Cys Thr Leu Glu Glu Ala Asn Ala Asp Leu Glu
```

|  | 100 |  | 105 |  | 110 |  |
|---|---|---|---|---|---|---|

Gln Lys Ile Lys Gly Trp Tyr Glu Lys Tyr Gly Pro Gly Ser Gly Arg
115 120 125

Gln Leu Ala His Asp Tyr Ser Lys Tyr Phe Ser Val Thr Glu Asp Leu
130 135 140

Lys Arg Gln Ile Ile Ser Val Thr Thr Cys Asn Ala Ser Ile Val Leu
145 150 155 160

Gln Asn Glu Asn Ala Arg Leu Thr Ala Asp Asp Phe Arg Leu Lys Cys
165 170 175

Glu Asn Glu Leu Ala Leu His Gln Ser Val Glu Ala Asp Ile Asn Gly
180 185 190

Leu His Arg Val Met Asp Glu Leu Thr Leu Cys Thr Ser Asp Leu Glu
195 200 205

Met Gln Cys Glu Ala Leu Ser Glu Glu Leu Thr Tyr Leu Lys Lys Asn
210 215 220

His Gln Glu Glu Met Lys Val Met Gln Gly Ala Ala Arg Gly Asn Val
225 230 235 240

Asn Val Glu Ile Asn Ala Ala Pro Gly Val Asp Leu Thr Val Leu Leu
245 250 255

Asn Asn Met Arg Ala Glu Tyr Glu Asp Leu Ala Glu Gln Asn His Glu
260 265 270

Asp Ala Glu Ala Trp Phe Ser Glu Lys Ser Thr Ser Leu His Gln Gln
275 280 285

Ile Ser Asp Asp Ala Gly Ala Ala Met Ala Ala Arg Asn Glu Leu Met
290 295 300

Glu Leu Lys Arg Asn Leu Gln Thr Leu Glu Ile Glu Leu Gln Ser Leu
305 310 315 320

Leu Ala Met Lys His Ser Tyr Glu Cys Ser Leu Ala Glu Thr Glu Ser
325 330 335

Asn Tyr Cys His Gln Leu Gln Gln Ile Gln Glu Gln Ile Gly Ala Met
340 345 350

```
Glu Asp Gln Leu Gln Gln Ile Arg Met Glu Thr Glu Gly Gln Lys Leu
    355             360             365

Glu His Glu Arg Leu Leu Asp Val Lys Ile Phe Leu Glu Lys Glu Ile
    370             375             380

Glu Met Tyr Cys Lys Leu Ile Asp Gly Glu Gly Arg Lys Ser Lys Ser
385             390             395             400

Thr Cys Tyr Lys Ser Glu Gly Arg Gly Pro Lys Asn Ser Glu Asn Gln
            405             410             415

Val Lys Asp Ser Lys Glu Glu Ala Val Val Lys Thr Val Val Gly Glu
        420             425             430

Leu Asp Gln Leu Gly Ser Val Leu Ser Leu Arg Val His Ser Val Glu
    435             440             445

Glu Lys Ser Ser Lys Ile Ser Asn Ile Thr Met Glu Gln Arg Leu Pro
    450             455             460

Ser Lys Val Pro
465
```

SEQUENCE LISTING

<110>  Spiber Inc.

<120>  HIGH-DENSITY WOVEN FABRIC AND METHOD FOR MANUFACTURING SAME

<130>  MEH/97742EP1

<140>  EP19782456.8
<141>  2019-04-03

<150>  JP2018-071892
<151>  2018-04-03

<160>  44

<170>  PatentIn version 3.5

<210>  1
<211>  50
<212>  PRT
<213>  Araneus diadematus

<400>  1

Ser Gly Cys Asp Val Leu Val Gln Ala Leu Leu Glu Val Val Ser Ala
1               5                   10                  15

Leu Val Ser Ile Leu Gly Ser Ser Ser Ile Gly Gln Ile Asn Tyr Gly
            20                  25                  30

Ala Ser Ala Gln Tyr Thr Gln Met Val Gly Gln Ser Val Ala Gln Ala
            35                  40                  45

Leu Ala
    50

<210>  2
<211>  30
<212>  PRT
<213>  Araneus diadematus

<400>  2

Ser Gly Cys Asp Val Leu Val Gln Ala Leu Leu Glu Val Val Ser Ala
1               5                   10                  15

Leu Val Ser Ile Leu Gly Ser Ser Ser Ile Gly Gln Ile Asn
            20                  25                  30

<210>  3
<211>  21
<212>  PRT
<213>  Araneus diadematus

<400>  3

```
Ser Gly Cys Asp Val Leu Val Gln Ala Leu Leu Glu Val Val Ser Ala
1               5                   10                  15

Leu Val Ser Ile Leu
                20


<210>   4
<211>   1154
<212>   PRT
<213>   Artificial Sequence

<220>
<223>   recombinant spider silk protein ADF3KaiLargeNRSH1

<400>   4

Met His His His His His His His His His His Ser Ser Gly Ser Ser
1               5                   10                  15

Leu Glu Val Leu Phe Gln Gly Pro Ala Arg Ala Gly Ser Gly Gln Gln
                20                  25                  30

Gly Pro Gly Gln Gln Gly Pro Gly Gln Gln Gly Pro Gly Gln Gln Gly
            35                  40                  45

Pro Tyr Gly Pro Gly Ala Ser Ala Ala Ala Ala Ala Ala Gly Gly Tyr
    50                  55                  60

Gly Pro Gly Ser Gly Gln Gln Gly Pro Ser Gln Gln Gly Pro Gly Gln
65                  70                  75                  80

Gln Gly Pro Gly Gly Gln Gly Pro Tyr Gly Pro Gly Ala Ser Ala Ala
                85                  90                  95

Ala Ala Ala Ala Gly Gly Tyr Gly Pro Gly Ser Gly Gln Gln Gly Pro
            100                 105                 110

Gly Gly Gln Gly Pro Tyr Gly Pro Gly Ser Ser Ala Ala Ala Ala Ala
            115                 120                 125

Ala Gly Gly Asn Gly Pro Gly Ser Gly Gln Gln Gly Ala Gly Gln Gln
            130                 135                 140

Gly Pro Gly Gln Gln Gly Pro Gly Ala Ser Ala Ala Ala Ala Ala Ala
145                 150                 155                 160

Gly Gly Tyr Gly Pro Gly Ser Gly Gln Gln Gly Pro Gly Gln Gln Gly
                165                 170                 175

Pro Gly Gly Gln Gly Pro Tyr Gly Pro Gly Ala Ser Ala Ala Ala Ala
```

<pre>
                    180                      185                          190

         Ala Ala Gly Gly Tyr Gly Pro Gly Ser Gly Gln Gly Pro Gly Gln Gln
                 195             200                 205


         Gly Pro Gly Gly Gln Gly Pro Tyr Gly Pro Gly Ala Ser Ala Ala Ala
                 210             215                 220


         Ala Ala Ala Gly Gly Tyr Gly Pro Gly Ser Gly Gln Gln Gly Pro Gly
         225             230                 235                     240


         Gln Gln Gly Pro Gly Gln Gln Gly Pro Gly Gly Gln Gly Pro Tyr Gly
                     245                 250                     255


         Pro Gly Ala Ser Ala Ala Ala Ala Ala Ala Gly Gly Tyr Gly Pro Gly
                     260                 265                     270


         Tyr Gly Gln Gln Gly Pro Gly Gln Gln Gly Pro Gly Gly Gln Gly Pro
                 275                 280                 285


         Tyr Gly Pro Gly Ala Ser Ala Ala Ser Ala Ala Ser Gly Gly Tyr Gly
                 290                 295                 300


         Pro Gly Ser Gly Gln Gln Gly Pro Gly Gln Gln Gly Pro Gly Gly Gln
         305                 310                 315                     320


         Gly Pro Tyr Gly Pro Gly Ala Ser Ala Ala Ala Ala Ala Ala Gly Gly
                     325                 330                     335


         Tyr Gly Pro Gly Ser Gly Gln Gln Gly Pro Gly Gln Gln Gly Pro Gly
                     340                 345                     350


         Gln Gln Gly Pro Gly Gln Gln Gly Pro Gly Gly Gln Gly Pro Tyr Gly
                     355                 360                     365


         Pro Gly Ala Ser Ala Ala Ala Ala Ala Ala Gly Gly Tyr Gly Pro Gly
                 370                 375                 380


         Ser Gly Gln Gln Gly Pro Gly Gln Gln Gly Pro Gly Gln Gln Gly Pro
         385                 390                 395                     400


         Gly Gln Gln Gly Pro Gly Gln Gln Gly Pro Gly Gln Gln Gly Pro Gly
                     405                 410                     415


         Gln Gln Gly Pro Gly Gln Gln Gly Pro Gly Gln Gln Gly Pro Gly Gly
                     420                 425                     430
</pre>

```
Gln Gly Ala Tyr Gly Pro Gly Ala Ser Ala Ala Ala Gly Ala Ala Gly
        435             440             445

Gly Tyr Gly Pro Gly Ser Gly Gln Gln Gly Pro Gly Gln Gln Gly Pro
    450             455             460

Gly Gln Gln Gly Pro Gly Gln Gln Gly Pro Gly Gln Gln Gly Pro Gly
465             470             475             480

Gln Gln Gly Pro Gly Gln Gln Gly Pro Gly Gln Gln Gly Pro Tyr Gly
            485             490             495

Pro Gly Ala Ser Ala Ala Ala Ala Ala Gly Gly Tyr Gly Pro Gly
        500             505             510

Ser Gly Gln Gln Gly Pro Gly Gln Gln Gly Pro Gly Gln Gln Gly Pro
    515             520             525

Gly Gly Gln Gly Pro Tyr Gly Pro Gly Ala Ala Ser Ala Ala Val Ser
    530             535             540

Val Ser Arg Ala Arg Ala Gly Ser Gly Gln Gln Gly Pro Gly Gln Gln
545             550             555             560

Gly Pro Gly Gln Gln Gly Pro Gly Gln Gln Gly Pro Tyr Gly Pro Gly
            565             570             575

Ala Ser Ala Ala Ala Ala Ala Ala Gly Gly Tyr Gly Pro Gly Ser Gly
        580             585             590

Gln Gln Gly Pro Ser Gln Gln Gly Pro Gly Gln Gln Gly Pro Gly Gly
        595             600             605

Gln Gly Pro Tyr Gly Pro Gly Ala Ser Ala Ala Ala Ala Ala Ala Gly
    610             615             620

Gly Tyr Gly Pro Gly Ser Gly Gln Gln Gly Pro Gly Gly Gln Gly Pro
625             630             635             640

Tyr Gly Pro Gly Ser Ser Ala Ala Ala Ala Ala Ala Gly Gly Asn Gly
            645             650             655

Pro Gly Ser Gly Gln Gln Gly Ala Gly Gln Gln Gly Pro Gly Gln Gln
        660             665             670

Gly Pro Gly Ala Ser Ala Ala Ala Ala Ala Ala Gly Gly Tyr Gly Pro
    675             680             685
```

163

```
Gly Ser Gly Gln Gln Gly Pro Gly Gln Gln Gly Pro Gly Gly Gln Gly
    690             695             700

Pro Tyr Gly Pro Gly Ala Ser Ala Ala Ala Ala Ala Ala Gly Gly Tyr
705             710             715             720

Gly Pro Gly Ser Gly Gln Gly Pro Gly Gln Gln Gly Pro Gly Gly Gln
            725             730             735

Gly Pro Tyr Gly Pro Gly Ala Ser Ala Ala Ala Ala Ala Ala Gly Gly
            740             745             750

Tyr Gly Pro Gly Ser Gly Gln Gln Gly Pro Gly Gln Gln Gly Pro Gly
        755             760             765

Gln Gln Gly Pro Gly Gly Gln Gly Pro Tyr Gly Pro Gly Ala Ser Ala
    770             775             780

Ala Ala Ala Ala Gly Gly Tyr Gly Pro Gly Tyr Gly Gln Gln Gly
785             790             795             800

Pro Gly Gln Gln Gly Pro Gly Gly Gln Gly Pro Tyr Gly Pro Gly Ala
            805             810             815

Ser Ala Ala Ser Ala Ala Ser Gly Gly Tyr Gly Pro Gly Ser Gly Gln
            820             825             830

Gln Gly Pro Gly Gln Gln Gly Pro Gly Gly Gln Gly Pro Tyr Gly Pro
        835             840             845

Gly Ala Ser Ala Ala Ala Ala Ala Ala Gly Gly Tyr Gly Pro Gly Ser
    850             855             860

Gly Gln Gln Gly Pro Gly Gln Gln Gly Pro Gly Gln Gln Gly Pro Gly
865             870             875             880

Gln Gln Gly Pro Gly Gly Gln Gly Pro Tyr Gly Pro Gly Ala Ser Ala
        885             890             895

Ala Ala Ala Ala Gly Gly Tyr Gly Pro Gly Ser Gly Gln Gln Gly
    900             905             910

Pro Gly Gln Gln Gly Pro Gly Gln Gln Gly Pro Gly Gln Gln Gly Pro
    915             920             925

Gly Gln Gln Gly Pro Gly Gln Gln Gly Pro Gly Gln Gln Gly Pro Gly
    930             935             940
```

EP 3 779 031 A1

Gln Gln Gly Pro Gly Gln Gln Gly Pro Gly Gly Gln Gly Ala Tyr Gly
945                 950                 955                 960

Pro Gly Ala Ser Ala Ala Ala Gly Ala Ala Gly Gly Tyr Gly Pro Gly
                965                 970                 975

Ser Gly Gln Gln Gly Pro Gly Gln Gln Gly Pro Gly Gln Gln Gly Pro
                980                 985                 990

Gly Gln Gln Gly Pro Gly Gln Gln  Gly Pro Gly Gln Gln  Gly Pro Gly
        995                 1000                1005

Gln Gln  Gly Pro Gly Gln Gln  Gly Pro Tyr Gly Pro  Gly Ala Ser
        1010                1015                1020

Ala Ala  Ala Ala Ala Ala Gly  Gly Tyr Gly Pro Gly  Ser Gly Gln
        1025                1030                1035

Gln Gly  Pro Gly Gln Gln Gly  Pro Gly Gln Gln Gly  Pro Gly Gly
        1040                1045                1050

Gln Gly  Pro Tyr Gly Pro Gly  Ala Ala Ser Ala Ala  Val Ser Val
        1055                1060                1065

Gly Gly  Tyr Gly Pro Gln Ser  Ser Ser Val Pro Val  Ala Ser Ala
        1070                1075                1080

Val Ala  Ser Arg Leu Ser Ser  Pro Ala Ala Ser Ser  Arg Val Ser
        1085                1090                1095

Ser Ala  Val Ser Ser Leu Val  Ser Ser Gly Pro Thr  Lys His Ala
        1100                1105                1110

Ala Leu  Ser Asn Thr Ile Ser  Ser Val Val Ser Gln  Val Ser Ala
        1115                1120                1125

Ser Asn  Pro Gly Leu Ser Gly  Cys Asp Val Leu Val  Gln Ala Leu
        1130                1135                1140

Leu Glu  Val Val Ser Ala Leu  Val Ser Ile Leu
        1145                1150

<210>  5
<211>  24
<212>  PRT
<213>  Artificial Sequence

165

<220>
<223> His tag and start codon

<400> 5

Met His His His His His His His His His His Ser Ser Gly Ser Ser
1               5                   10                  15

Leu Glu Val Leu Phe Gln Gly Pro
            20

<210> 6
<211> 597
<212> PRT
<213> Artificial Sequence

<220>
<223> Met-PRT380

<400> 6

Met Gly Pro Gly Gln Gln Gly Pro Tyr Gly Pro Gly Ala Ser Ala Ala
1               5                   10                  15

Ala Ala Ala Gly Gln Asn Gly Pro Gly Ser Gly Gln Gln Gly Pro Gly
            20                  25                  30

Gln Ser Ala Ala Ala Ala Ala Gly Gln Tyr Gly Pro Gly Gln Gln Gly
        35                  40                  45

Pro Gly Gln Gln Gly Pro Gly Ser Ser Ala Ala Ala Ala Ala Gly Pro
    50                  55                  60

Gly Gln Tyr Gly Pro Gly Gln Gln Gly Pro Ser Ala Ser Ala Ala Ala
65                  70                  75                  80

Ala Ala Gly Pro Gly Ser Gly Gln Gln Gly Pro Gly Ala Ser Ala Ala
                85                  90                  95

Ala Ala Ala Gly Gln Tyr Gly Pro Gly Gln Gln Gly Pro Gly Gln Gln
            100                 105                 110

Gly Pro Gly Ser Ser Ala Ala Ala Ala Ala Gly Gln Tyr Gly Ser Gly
        115                 120                 125

Pro Gly Gln Gln Gly Pro Tyr Gly Ser Ala Ala Ala Ala Ala Gly Pro
    130                 135                 140

Gly Ser Gly Gln Tyr Gly Gln Gly Pro Tyr Gly Pro Gly Ala Ser Ala
145                 150                 155                 160

166

```
Ala Ala Ala Ala Gly Pro Gly Gln Tyr Gly Pro Gly Gln Gln Gly Pro
            165             170             175

Ser Ala Ser Ala Ala Ala Ala Ala Gly Ser Gly Gln Gln Gly Pro Gly
            180             185             190

Gln Tyr Gly Pro Tyr Ala Ser Ala Ala Ala Ala Gly Gln Tyr Gly
        195             200             205

Ser Gly Pro Gly Gln Gln Gly Pro Tyr Gly Pro Gly Gln Ser Ala Ala
    210             215             220

Ala Ala Ala Gly Ser Gly Gln Gln Gly Pro Gly Gln Gln Gly Pro Tyr
225             230             235             240

Ala Ser Ala Ala Ala Ala Ala Gly Pro Gly Gln Gln Gly Pro Tyr Gly
            245             250             255

Pro Gly Ser Ser Ala Ala Ala Ala Ala Gly Gln Tyr Gly Tyr Gly Pro
        260             265             270

Gly Gln Gln Gly Pro Tyr Gly Pro Gly Ala Ser Ala Ala Ala Ala Ala
    275             280             285

Gly Gln Asn Gly Pro Gly Ser Gly Gln Tyr Gly Pro Gly Gln Gln Gly
    290             295             300

Pro Gly Gln Ser Ala Ala Ala Ala Ala Gly Pro Gly Gln Gln Gly Pro
305             310             315             320

Tyr Gly Pro Gly Ala Ser Ala Ala Ala Ala Gly Gln Tyr Gly Pro
            325             330             335

Gly Gln Gln Gly Pro Gly Gln Tyr Gly Pro Gly Ser Ser Ala Ala Ala
        340             345             350

Ala Ala Gly Pro Gly Gln Gln Gly Pro Tyr Gly Pro Gly Ser Ser Ala
        355             360             365

Ala Ala Ala Ala Gly Gln Tyr Gly Pro Gly Gln Gln Gly Pro Tyr Gly
    370             375             380

Pro Gly Gln Ser Ala Ala Ala Ala Ala Gly Gln Tyr Gln Gln Gly Pro
385             390             395             400

Gly Gln Gln Gly Pro Tyr Gly Pro Gly Ala Ser Ala Ala Ala Ala Ala
            405             410             415
```

167

Gly Pro Gly Gln Gln Gly Pro Tyr Gly Pro Gly Ala Ser Ala Ala Ala
        420                 425             430

Ala Ala Gly Pro Gly Gln Tyr Gly Pro Gly Gln Gln Gly Pro Ser Ala
        435                 440             445

Ser Ala Ala Ala Ala Ala Gly Gln Tyr Gly Ser Gly Pro Gly Gln Tyr
    450                 455             460

Gly Pro Tyr Gly Pro Gly Gln Ser Ala Ala Ala Ala Gly Pro Gly
465             470             475             480

Ser Gly Gln Gln Gly Gln Gly Pro Tyr Gly Pro Gly Ala Ser Ala Ala
            485             490             495

Ala Ala Ala Gly Gln Tyr Gly Pro Gly Gln Gln Gly Pro Tyr Gly Pro
        500             505             510

Gly Gln Ser Ala Ala Ala Ala Ala Gly Pro Gly Ser Gly Gln Tyr Gly
        515             520             525

Pro Gly Ala Ser Ala Ala Ala Ala Gly Gln Asn Gly Pro Gly Ser
        530             535             540

Gly Gln Tyr Gly Pro Gly Gln Gln Gly Pro Gly Gln Ser Ala Ala Ala
545             550             555             560

Ala Ala Gly Gln Tyr Gln Gln Gly Pro Gly Gln Gln Gly Pro Tyr Gly
            565             570             575

Pro Gly Ala Ser Ala Ala Ala Ala Ala Gly Pro Gly Ser Gly Gln Gln
            580             585             590

Gly Pro Gly Ala Ser
        595

<210>   7
<211>   590
<212>   PRT
<213>   Artificial Sequence

<220>
<223>   Met-PRT410

<400>   7

Met Gly Pro Gly Gln Gln Gly Pro Tyr Gly Pro Gly Ala Ser Ala Ala
1               5               10              15

Ala Ala Ala Gly Gln Asn Gly Pro Gly Ser Gly Gln Gln Gly Pro Gly
                20                  25                  30

Gln Ser Gly Gln Tyr Gly Pro Gly Gln Gln Gly Pro Gly Gln Gln Gly
        35                  40                  45

Pro Gly Ser Ser Ala Ala Ala Ala Ala Gly Pro Gly Gln Tyr Gly Pro
        50                  55                  60

Gly Gln Gln Gly Pro Ser Ala Ser Ala Ala Ala Ala Gly Pro Gly
65                  70                  75                  80

Ser Gly Gln Gln Gly Pro Gly Ala Ser Gly Gln Tyr Gly Pro Gly Gln
                85                  90                  95

Gln Gly Pro Gly Gln Gln Gly Pro Gly Ser Ser Ala Ala Ala Ala Ala
            100                 105                 110

Gly Gln Tyr Gly Ser Gly Pro Gly Gln Gln Gly Pro Tyr Gly Ser Ala
        115                 120                 125

Ala Ala Ala Ala Gly Pro Gly Ser Gly Gln Tyr Gly Gln Gly Pro Tyr
    130                 135                 140

Gly Pro Gly Ala Ser Gly Pro Gly Gln Tyr Gly Pro Gly Gln Gln Gly
145                 150                 155                 160

Pro Ser Ala Ser Ala Ala Ala Ala Ala Gly Ser Gly Gln Gln Gly Pro
            165                 170                 175

Gly Gln Tyr Gly Pro Tyr Ala Ser Ala Ala Ala Ala Gly Gln Tyr
        180                 185                 190

Gly Ser Gly Pro Gly Gln Gln Gly Pro Tyr Gly Pro Gly Gln Ser Gly
        195                 200                 205

Ser Gly Gln Gln Gly Pro Gly Gln Gln Gly Pro Tyr Ala Ser Ala Ala
    210                 215                 220

Ala Ala Ala Gly Pro Gly Gln Gln Gly Pro Tyr Gly Pro Gly Ser Ser
225                 230                 235                 240

Ala Ala Ala Ala Ala Gly Gln Tyr Gly Tyr Gly Pro Gly Gln Gln Gly
            245                 250                 255

Pro Tyr Gly Pro Gly Ala Ser Gly Gln Asn Gly Pro Gly Ser Gly Gln
        260                 265                 270

169

```
Tyr Gly Pro Gly Gln Gln Gly Pro Gly Gln Ser Ala Ala Ala Ala Ala
        275                 280                 285

Gly Pro Gly Gln Gln Gly Pro Tyr Gly Pro Gly Ala Ser Ala Ala Ala
        290                 295                 300

Ala Ala Gly Gln Tyr Gly Pro Gly Gln Gln Gly Pro Gly Gln Tyr Gly
305                 310                 315                 320

Pro Gly Ser Ser Gly Pro Gly Gln Gln Gly Pro Tyr Gly Pro Gly Ser
                325                 330                 335

Ser Ala Ala Ala Ala Ala Gly Gln Tyr Gly Pro Gly Gln Gln Gly Pro
            340                 345                 350

Tyr Gly Pro Gly Gln Ser Ala Ala Ala Ala Ala Gly Gln Tyr Gln Gln
        355                 360                 365

Gly Pro Gly Gln Gln Gly Pro Tyr Gly Pro Gly Ala Ser Gly Pro Gly
    370                 375                 380

Gln Gln Gly Pro Tyr Gly Pro Gly Ala Ser Ala Ala Ala Ala Ala Gly
385                 390                 395                 400

Pro Gly Gln Tyr Gly Pro Gly Gln Gln Gly Pro Ser Ala Ser Ala Ala
        405                 410                 415

Ala Ala Ala Gly Gln Tyr Gly Ser Gly Pro Gly Gln Tyr Gly Pro Tyr
            420                 425                 430

Gly Pro Gly Gln Ser Gly Pro Gly Ser Gly Gln Gln Gly Gln Gly Pro
        435                 440                 445

Tyr Gly Pro Gly Ala Ser Ala Ala Ala Ala Gly Gln Tyr Gly Pro
        450                 455                 460

Gly Gln Gln Gly Pro Tyr Gly Pro Gly Gln Ser Ala Ala Ala Ala Ala
465                 470                 475                 480

Gly Pro Gly Ser Gly Gln Tyr Gly Pro Gly Ala Ser Gly Gln Asn Gly
            485                 490                 495

Pro Gly Ser Gly Gln Tyr Gly Pro Gly Gln Gln Gly Pro Gly Gln Ser
        500                 505                 510

Ala Ala Ala Ala Ala Gly Gln Tyr Gln Gln Gly Pro Gly Gln Gln Gly
        515                 520                 525
```

```
Pro Tyr Gly Pro Gly Ala Ser Ala Ala Ala Ala Gly Gln Tyr Gly
    530             535             540

Ser Gly Pro Gly Gln Gln Gly Pro Tyr Gly Pro Gly Gln Ser Gly Ser
545             550             555             560

Gly Gln Gln Gly Pro Gly Gln Gln Gly Pro Tyr Ala Ser Ala Ala Ala
                565             570             575

Ala Ala Gly Pro Gly Ser Gly Gln Gln Gly Pro Gly Ala Ser
            580             585             590
```

```
<210>   8
<211>   565
<212>   PRT
<213>   Artificial Sequence

<220>
<223>   Met-PRT525

<400>   8
```

```
Met Gly Pro Gly Gln Gln Gly Pro Tyr Gly Pro Gly Ala Ser Ala Ala
1               5               10              15

Ala Ala Ala Ala Ala Gly Ser Asn Gly Pro Gly Ser Gly Gln Gln Gly
            20              25              30

Pro Gly Gln Ser Gly Gln Tyr Gly Pro Gly Gln Gln Gly Pro Gly Gln
            35              40              45

Gln Gly Pro Gly Ser Ser Ala Ala Ala Ala Ala Ala Gly Pro Gly
        50              55              60

Gln Tyr Gly Pro Gly Gln Gln Gly Pro Ser Ala Ser Ala Ala Ala Ala
65              70              75              80

Ala Ala Ala Gly Pro Gly Ser Gly Gln Gln Gly Pro Gly Ala Ser Gly
            85              90              95

Gln Tyr Gly Pro Gly Gln Gln Gly Pro Gly Gln Gln Gly Pro Gly Ser
            100             105             110

Ser Ala Ala Ala Ala Ala Ala Ala Gly Ser Tyr Gly Ser Gly Pro Gly
        115             120             125

Gln Gln Gly Pro Tyr Gly Ser Ala Ala Ala Ala Ala Ala Gly Pro
    130             135             140
```

Gly Ser Gly Gln Tyr Gly Gln Gly Pro Tyr Gly Pro Gly Ala Ser Gly
145                 150                 155                 160

Pro Gly Gln Tyr Gly Pro Gly Gln Gln Gly Pro Ser Ala Ser Ala Ala
                165                 170                 175

Ala Ala Ala Ala Ala Gly Ser Gly Gln Gln Gly Pro Gly Gln Tyr Gly
                180                 185                 190

Pro Tyr Ala Ser Ala Ala Ala Ala Ala Ala Gly Ser Tyr Gly Ser
        195                 200                 205

Gly Pro Gly Gln Gln Gly Pro Tyr Gly Pro Gly Gln Ser Gly Ser Gly
        210                 215                 220

Gln Gln Gly Pro Gly Gln Gln Gly Pro Tyr Ala Ser Ala Ala Ala Ala
225                 230                 235                 240

Ala Ala Ala Gly Pro Gly Gln Gln Gly Pro Tyr Gly Pro Gly Ser Ser
                245                 250                 255

Ala Ala Ala Ala Ala Ala Ala Gly Ser Tyr Gly Tyr Gly Pro Gly Gln
                260                 265                 270

Gln Gly Pro Tyr Gly Pro Gly Ala Ser Gly Gln Asn Gly Pro Gly Ser
        275                 280                 285

Gly Gln Tyr Gly Pro Gly Gln Gln Gly Pro Gly Pro Ser Ala Ala Ala
        290                 295                 300

Ala Ala Ala Ala Gly Pro Gly Gln Gln Gly Pro Tyr Gly Pro Gly Ala
305                 310                 315                 320

Ser Ala Ala Ala Ala Ala Ala Ala Gly Ser Tyr Gly Pro Gly Gln Gln
                325                 330                 335

Gly Pro Gly Gln Tyr Gly Pro Gly Ser Ser Gly Pro Gly Gln Gln Gly
        340                 345                 350

Pro Tyr Gly Pro Gly Ser Ser Ala Ala Ala Ala Ala Ala Ala Gly Ser
        355                 360                 365

Tyr Gly Pro Gly Gln Gln Gly Pro Tyr Gly Pro Gly Pro Ser Ala Ala
        370                 375                 380

Ala Ala Ala Ala Ala Gly Ser Tyr Gln Gln Gly Pro Gly Gln Gln Gly
385                 390                 395                 400

```
Pro Tyr Gly Pro Gly Ala Ser Gly Pro Gly Gln Gln Gly Pro Tyr Gly
            405                 410                 415

Pro Gly Ala Ser Ala Ala Ala Ala Ala Ala Ala Gly Pro Gly Gln Tyr
            420                 425                 430

Gly Pro Gly Gln Gln Gly Pro Ser Ala Ser Ala Ala Ala Ala Ala Ala
            435                 440                 445

Ala Gly Ser Tyr Gly Ser Gly Pro Gly Gln Tyr Gly Pro Tyr Gly Pro
    450                 455                 460

Gly Gln Ser Gly Pro Gly Ser Gly Gln Gln Gly Gln Gly Pro Tyr Gly
465                 470                 475                 480

Pro Gly Ala Ser Ala Ala Ala Ala Ala Ala Ala Gly Ser Tyr Gly Pro
            485                 490                 495

Gly Gln Gln Gly Pro Tyr Gly Pro Gly Pro Ser Ala Ala Ala Ala Ala
            500                 505                 510

Ala Ala Gly Pro Gly Ser Gly Gln Tyr Gly Pro Gly Ala Ser Gly Gln
    515                 520                 525

Asn Gly Pro Gly Ser Gly Gln Tyr Gly Pro Gly Gln Gln Gly Pro Gly
    530                 535                 540

Pro Ser Ala Ala Ala Ala Ala Ala Ala Gly Pro Gly Ser Gly Gln Gln
545                 550                 555                 560

Gly Pro Gly Ala Ser
                565
```

```
<210>  9
<211>  2364
<212>  PRT
<213>  Artificial Sequence

<220>
<223>  Met-PRT799

<400>  9
```

```
Met Gly Pro Gly Gln Gln Gly Pro Tyr Gly Pro Gly Ala Ser Ala Ala
1               5                   10                  15

Ala Ala Ala Gly Gln Asn Gly Pro Gly Ser Gly Gln Gln Gly Pro Gly
            20                  25                  30
```

Gln Ser Gly Gln Tyr Gly Pro Gly Gln Gln Gly Pro Gly Gln Gln Gly
        35                  40                  45

Pro Gly Ser Ser Ala Ala Ala Ala Ala Gly Pro Gly Gln Tyr Gly Pro
        50                  55                  60

Gly Gln Gln Gly Pro Ser Ala Ser Ala Ala Ala Ala Gly Pro Gly
65                  70                  75                  80

Ser Gly Gln Gln Gly Pro Gly Ala Ser Gly Gln Tyr Gly Pro Gly Gln
                85                  90                  95

Gln Gly Pro Gly Gln Gln Gly Pro Gly Ser Ser Ala Ala Ala Ala Ala
            100                 105                 110

Gly Gln Tyr Gly Ser Gly Pro Gly Gln Gln Gly Pro Tyr Gly Ser Ala
        115                 120                 125

Ala Ala Ala Ala Gly Pro Gly Ser Gly Gln Tyr Gly Gln Gly Pro Tyr
    130                 135                 140

Gly Pro Gly Ala Ser Gly Pro Gly Gln Tyr Gly Pro Gly Gln Gln Gly
145                 150                 155                 160

Pro Ser Ala Ser Ala Ala Ala Ala Gly Ser Gly Gln Gln Gly Pro
            165                 170                 175

Gly Gln Tyr Gly Pro Tyr Ala Ser Ala Ala Ala Ala Gly Gln Tyr
        180                 185                 190

Gly Ser Gly Pro Gly Gln Gln Gly Pro Tyr Gly Pro Gly Gln Ser Gly
        195                 200                 205

Ser Gly Gln Gln Gly Pro Gly Gln Gln Gly Pro Tyr Ala Ser Ala Ala
    210                 215                 220

Ala Ala Ala Gly Pro Gly Gln Gln Gly Pro Tyr Gly Pro Gly Ser Ser
225                 230                 235                 240

Ala Ala Ala Ala Ala Gly Gln Tyr Gly Tyr Gly Pro Gly Gln Gln Gly
            245                 250                 255

Pro Tyr Gly Pro Gly Ala Ser Gly Gln Asn Gly Pro Gly Ser Gly Gln
        260                 265                 270

Tyr Gly Pro Gly Gln Gln Gly Pro Gly Gln Ser Ala Ala Ala Ala Ala
        275                 280                 285

Gly Pro Gly Gln Gln Gly Pro Tyr Gly Pro Gly Ala Ser Ala Ala Ala
290             295             300

Ala Ala Gly Gln Tyr Gly Pro Gly Gln Gln Gly Pro Gly Gln Tyr Gly
305             310             315             320

Pro Gly Ser Ser Gly Pro Gly Gln Gln Gly Pro Tyr Gly Pro Gly Ser
325             330             335

Ser Ala Ala Ala Ala Ala Gly Gln Tyr Gly Pro Gly Gln Gln Gly Pro
340             345             350

Tyr Gly Pro Gly Gln Ser Ala Ala Ala Ala Ala Gly Gln Tyr Gln Gln
355             360             365

Gly Pro Gly Gln Gln Gly Pro Tyr Gly Pro Gly Ala Ser Gly Pro Gly
370             375             380

Gln Gln Gly Pro Tyr Gly Pro Gly Ala Ser Ala Ala Ala Ala Ala Gly
385             390             395             400

Pro Gly Gln Tyr Gly Pro Gly Gln Gln Gly Pro Ser Ala Ser Ala Ala
405             410             415

Ala Ala Ala Gly Gln Tyr Gly Ser Gly Pro Gly Gln Tyr Gly Pro Tyr
420             425             430

Gly Pro Gly Gln Ser Gly Pro Gly Ser Gly Gln Gln Gly Gln Gly Pro
435             440             445

Tyr Gly Pro Gly Ala Ser Ala Ala Ala Ala Gly Gln Tyr Gly Pro
450             455             460

Gly Gln Gln Gly Pro Tyr Gly Pro Gly Gln Ser Ala Ala Ala Ala Ala
465             470             475             480

Gly Pro Gly Ser Gly Gln Tyr Gly Pro Gly Ala Ser Gly Gln Asn Gly
485             490             495

Pro Gly Ser Gly Gln Tyr Gly Pro Gly Gln Gln Gly Pro Gly Gln Ser
500             505             510

Ala Ala Ala Ala Ala Gly Gln Tyr Gln Gln Gly Pro Gly Gln Gln Gly
515             520             525

Pro Tyr Gly Pro Gly Ala Ser Ala Ala Ala Ala Ala Gly Gln Tyr Gly

530                        535                        540

Ser Gly Pro Gly Gln Gln Gly Pro Tyr Gly Pro Gly Gln Ser Gly Ser
545                        550                        555                        560

Gly Gln Gln Gly Pro Gly Gln Gln Gly Pro Tyr Ala Ser Ala Ala Ala
                565                        570                        575

Ala Ala Gly Pro Gly Ser Gly Gln Gln Gly Pro Gly Ala Ser Gly Gln
                580                        585                        590

Gln Gly Pro Tyr Gly Pro Gly Ala Ser Ala Ala Ala Ala Ala Gly Gln
                595                        600                        605

Asn Gly Pro Gly Ser Gly Gln Gln Gly Pro Gly Gln Ser Gly Gln Tyr
                610                        615                        620

Gly Pro Gly Gln Gln Gly Pro Gly Gln Gln Gly Pro Gly Ser Ser Ala
625                        630                        635                        640

Ala Ala Ala Ala Gly Pro Gly Gln Tyr Gly Pro Gly Gln Gln Gly Pro
                645                        650                        655

Ser Ala Ser Ala Ala Ala Ala Ala Gly Pro Gly Ser Gly Gln Gln Gly
                660                        665                        670

Pro Gly Ala Ser Gly Gln Tyr Gly Pro Gly Gln Gln Gly Pro Gly Gln
                675                        680                        685

Gln Gly Pro Gly Ser Ser Ala Ala Ala Ala Ala Gly Gln Tyr Gly Ser
                690                        695                        700

Gly Pro Gly Gln Gln Gly Pro Tyr Gly Ser Ala Ala Ala Ala Ala Gly
705                        710                        715                        720

Pro Gly Ser Gly Gln Tyr Gly Gln Gly Pro Tyr Gly Pro Gly Ala Ser
                725                        730                        735

Gly Pro Gly Gln Tyr Gly Pro Gly Gln Gln Gly Pro Ser Ala Ser Ala
                740                        745                        750

Ala Ala Ala Gly Ser Gly Gln Gln Gly Pro Gly Gln Tyr Gly Pro
                755                        760                        765

Tyr Ala Ser Ala Ala Ala Ala Ala Gly Gln Tyr Gly Ser Gly Pro Gly
                770                        775                        780

Gln Gln Gly Pro Tyr Gly Pro Gly Gln Ser Gly Ser Gly Gln Gln Gly
785                  790              795                  800

Pro Gly Gln Gln Gly Pro Tyr Ala Ser Ala Ala Ala Ala Ala Gly Pro
                  805              810                  815

Gly Gln Gln Gly Pro Tyr Gly Pro Gly Ser Ser Ala Ala Ala Ala Ala
             820              825              830

Gly Gln Tyr Gly Tyr Gly Pro Gly Gln Gln Gly Pro Tyr Gly Pro Gly
             835              840              845

Ala Ser Gly Gln Asn Gly Pro Gly Ser Gly Gln Tyr Gly Pro Gly Gln
    850              855              860

Gln Gly Pro Gly Gln Ser Ala Ala Ala Ala Ala Gly Pro Gly Gln Gln
865              870              875              880

Gly Pro Tyr Gly Pro Gly Ala Ser Ala Ala Ala Ala Gly Gln Tyr
        885              890              895

Gly Pro Gly Gln Gln Gly Pro Gly Gln Tyr Gly Pro Gly Ser Ser Gly
        900              905              910

Pro Gly Gln Gln Gly Pro Tyr Gly Pro Gly Ser Ser Ala Ala Ala Ala
        915              920              925

Ala Gly Gln Tyr Gly Pro Gly Gln Gln Gly Pro Tyr Gly Pro Gly Gln
    930              935              940

Ser Ala Ala Ala Ala Ala Gly Gln Tyr Gln Gln Gly Pro Gly Gln Gln
945              950              955              960

Gly Pro Tyr Gly Pro Gly Ala Ser Gly Pro Gly Gln Gln Gly Pro Tyr
        965              970              975

Gly Pro Gly Ala Ser Ala Ala Ala Ala Gly Pro Gly Gln Tyr Gly
        980              985              990

Pro Gly Gln Gln Gly Pro Ser Ala Ser Ala Ala Ala Ala Ala Gly Gln
        995              1000             1005

Tyr Gly Ser Gly Pro Gly Gln Tyr Gly Pro Tyr Gly Pro Gly Gln
    1010             1015             1020

Ser Gly Pro Gly Ser Gly Gln Gln Gly Gln Gly Pro Tyr Gly Pro
    1025             1030             1035

```
Gly Ala   Ser Ala Ala Ala Ala   Ala Gly Gln Tyr Gly   Pro Gly Gln
    1040                1045                    1050

Gln Gly   Pro Tyr Gly Pro Gly   Gln Ser Ala Ala Ala   Ala Ala Gly
    1055                1060                    1065

Pro Gly   Ser Gly Gln Tyr Gly   Pro Gly Ala Ser Gly   Gln Asn Gly
    1070                1075                    1080

Pro Gly   Ser Gly Gln Tyr Gly   Pro Gly Gln Gln Gly   Pro Gly Gln
    1085                1090                    1095

Ser Ala   Ala Ala Ala Ala Gly   Gln Tyr Gln Gln Gly   Pro Gly Gln
    1100                1105                    1110

Gln Gly   Pro Tyr Gly Pro Gly   Ala Ser Ala Ala Ala   Ala Ala Gly
    1115                1120                    1125

Gln Tyr   Gly Ser Gly Pro Gly   Gln Gln Gly Pro Tyr   Gly Pro Gly
    1130                1135                    1140

Gln Ser   Gly Ser Gly Gln Gln   Gly Pro Gly Gln Gln   Gly Pro Tyr
    1145                1150                    1155

Ala Ser   Ala Ala Ala Ala Ala   Gly Pro Gly Ser Gly   Gln Gln Gly
    1160                1165                    1170

Pro Gly   Ala Ser Gly Gln Gln   Gly Pro Tyr Gly Pro   Gly Ala Ser
    1175                1180                    1185

Ala Ala   Ala Ala Ala Gly Gln   Asn Gly Pro Gly Ser   Gly Gln Gln
    1190                1195                    1200

Gly Pro   Gly Gln Ser Gly Gln   Tyr Gly Pro Gly Gln   Gln Gly Pro
    1205                1210                    1215

Gly Gln   Gln Gly Pro Gly Ser   Ser Ala Ala Ala Ala   Ala Gly Pro
    1220                1225                    1230

Gly Gln   Tyr Gly Pro Gly Gln   Gln Gly Pro Ser Ala   Ser Ala Ala
    1235                1240                    1245

Ala Ala   Ala Gly Pro Gly Ser   Gly Gln Gln Gly Pro   Gly Ala Ser
    1250                1255                    1260

Gly Gln   Tyr Gly Pro Gly Gln   Gln Gly Pro Gly Gln   Gln Gly Pro
    1265                1270                    1275
```

178

```
Gly Ser   Ser Ala Ala Ala Ala   Ala Gly Gln Tyr Gly   Ser Gly Pro
    1280              1285               1290

Gly Gln   Gln Gly Pro Tyr Gly   Ser Ala Ala Ala Ala   Ala Gly Pro
    1295              1300               1305

Gly Ser   Gly Gln Tyr Gly Gln   Gly Pro Tyr Gly Pro   Gly Ala Ser
    1310              1315               1320

Gly Pro   Gly Gln Tyr Gly Pro   Gly Gln Gln Gly Pro   Ser Ala Ser
    1325              1330               1335

Ala Ala   Ala Ala Ala Gly Ser   Gly Gln Gln Gly Pro   Gly Gln Tyr
    1340              1345               1350

Gly Pro   Tyr Ala Ser Ala Ala   Ala Ala Ala Gly Gln   Tyr Gly Ser
    1355              1360               1365

Gly Pro   Gly Gln Gln Gly Pro   Tyr Gly Pro Gly Gln   Ser Gly Ser
    1370              1375               1380

Gly Gln   Gln Gly Pro Gly Gln   Gln Gly Pro Tyr Ala   Ser Ala Ala
    1385              1390               1395

Ala Ala   Ala Gly Pro Gly Gln   Gln Gly Pro Tyr Gly   Pro Gly Ser
    1400              1405               1410

Ser Ala   Ala Ala Ala Ala Gly   Gln Tyr Gly Tyr Gly   Pro Gly Gln
    1415              1420               1425

Gln Gly   Pro Tyr Gly Pro Gly   Ala Ser Gly Gln Asn   Gly Pro Gly
    1430              1435               1440

Ser Gly   Gln Tyr Gly Pro Gly   Gln Gln Gly Pro Gly   Gln Ser Ala
    1445              1450               1455

Ala Ala   Ala Ala Gly Pro Gly   Gln Gln Gly Pro Tyr   Gly Pro Gly
    1460              1465               1470

Ala Ser   Ala Ala Ala Ala Ala   Gly Gln Tyr Gly Pro   Gly Gln Gln
    1475              1480               1485

Gly Pro   Gly Gln Tyr Gly Pro   Gly Ser Ser Gly Pro   Gly Gln Gln
    1490              1495               1500

Gly Pro   Tyr Gly Pro Gly Ser   Ser Ala Ala Ala Ala   Ala Gly Gln
```

```
                1505                        1510                           1515


        Tyr Gly  Pro Gly Gln Gln Gly  Pro Tyr Gly Pro Gly  Gln Ser Ala
                 1520                   1525                 1530


        Ala Ala  Ala Ala Gly Gln Tyr  Gln Gln Gly Pro Gly  Gln Gln Gly
                 1535                   1540                 1545


        Pro Tyr  Gly Pro Gly Ala Ser  Gly Pro Gly Gln Gln  Gly Pro Tyr
                 1550                   1555                 1560


        Gly Pro  Gly Ala Ser Ala Ala  Ala Ala Ala Gly Pro  Gly Gln Tyr
                 1565                   1570                 1575


        Gly Pro  Gly Gln Gln Gly Pro  Ser Ala Ser Ala Ala  Ala Ala Ala
                 1580                   1585                 1590


        Gly Gln  Tyr Gly Ser Gly Pro  Gly Gln Tyr Gly Pro  Tyr Gly Pro
                 1595                   1600                 1605


        Gly Gln  Ser Gly Pro Gly Ser  Gly Gln Gln Gly Gln  Gly Pro Tyr
                 1610                   1615                 1620


        Gly Pro  Gly Ala Ser Ala Ala  Ala Ala Ala Gly Gln  Tyr Gly Pro
                 1625                   1630                 1635


        Gly Gln  Gln Gly Pro Tyr Gly  Pro Gly Gln Ser Ala  Ala Ala Ala
                 1640                   1645                 1650


        Ala Gly  Pro Gly Ser Gly Gln  Tyr Gly Pro Gly Ala  Ser Gly Gln
                 1655                   1660                 1665


        Asn Gly  Pro Gly Ser Gly Gln  Tyr Gly Pro Gly Gln  Gln Gly Pro
                 1670                   1675                 1680


        Gly Gln  Ser Ala Ala Ala Ala  Ala Gly Gln Tyr Gln  Gln Gly Pro
                 1685                   1690                 1695


        Gly Gln  Gln Gly Pro Tyr Gly  Pro Gly Ala Ser Ala  Ala Ala Ala
                 1700                   1705                 1710


        Ala Gly  Gln Tyr Gly Ser Gly  Pro Gly Gln Gln Gly  Pro Tyr Gly
                 1715                   1720                 1725


        Pro Gly  Gln Ser Gly Ser Gly  Gln Gln Gly Pro Gly  Gln Gln Gly
                 1730                   1735                 1740
```

```
Pro Tyr Ala Ser Ala Ala Ala   Ala Ala Gly Pro Gly   Ser Gly Gln
    1745                1750                1755

Gln Gly Pro Gly Ala Ser Gly   Gln Gln Gly Pro Tyr   Gly Pro Gly
    1760                1765                1770

Ala Ser Ala Ala Ala Ala Ala   Gly Gln Asn Gly Pro   Gly Ser Gly
    1775                1780                1785

Gln Gln Gly Pro Gly Gln Ser   Gly Gln Tyr Gly Pro   Gly Gln Gln
    1790                1795                1800

Gly Pro Gly Gln Gln Gly Pro   Gly Ser Ser Ala Ala   Ala Ala Ala
    1805                1810                1815

Gly Pro Gly Gln Tyr Gly Pro   Gly Gln Gln Gly Pro   Ser Ala Ser
    1820                1825                1830

Ala Ala Ala Ala Ala Gly Pro   Gly Ser Gly Gln Gln   Gly Pro Gly
    1835                1840                1845

Ala Ser Gly Gln Tyr Gly Pro   Gly Gln Gln Gly Pro   Gly Gln Gln
    1850                1855                1860

Gly Pro Gly Ser Ser Ala Ala   Ala Ala Ala Gly Gln   Tyr Gly Ser
    1865                1870                1875

Gly Pro Gly Gln Gln Gly Pro   Tyr Gly Ser Ala Ala   Ala Ala Ala
    1880                1885                1890

Gly Pro Gly Ser Gly Gln Tyr   Gly Gln Gly Pro Tyr   Gly Pro Gly
    1895                1900                1905

Ala Ser Gly Pro Gly Gln Tyr   Gly Pro Gly Gln Gln   Gly Pro Ser
    1910                1915                1920

Ala Ser Ala Ala Ala Ala Ala   Gly Ser Gly Gln Gln   Gly Pro Gly
    1925                1930                1935

Gln Tyr Gly Pro Tyr Ala Ser   Ala Ala Ala Ala Ala   Gly Gln Tyr
    1940                1945                1950

Gly Ser Gly Pro Gly Gln Gln   Gly Pro Tyr Gly Pro   Gly Gln Ser
    1955                1960                1965

Gly Ser Gly Gln Gln Gly Pro   Gly Gln Gln Gly Pro   Tyr Ala Ser
    1970                1975                1980
```

Ala Ala   Ala Ala Ala Gly Pro   Gly Gln Gln Gly Pro   Tyr Gly Pro
    1985                    1990                  1995

Gly Ser   Ser Ala Ala Ala Ala   Ala Gly Gln Tyr Gly   Tyr Gly Pro
    2000                    2005                  2010

Gly Gln   Gln Gly Pro Tyr Gly   Pro Gly Ala Ser Gly   Gln Asn Gly
    2015                    2020                  2025

Pro Gly   Ser Gly Gln Tyr Gly   Pro Gly Gln Gln Gly   Pro Gly Gln
    2030                    2035                  2040

Ser Ala   Ala Ala Ala Ala Gly   Pro Gly Gln Gln Gly   Pro Tyr Gly
    2045                    2050                  2055

Pro Gly   Ala Ser Ala Ala Ala   Ala Ala Gly Gln Tyr   Gly Pro Gly
    2060                    2065                  2070

Gln Gln   Gly Pro Gly Gln Tyr   Gly Pro Gly Ser Ser   Gly Pro Gly
    2075                    2080                  2085

Gln Gln   Gly Pro Tyr Gly Pro   Gly Ser Ser Ala Ala   Ala Ala Ala
    2090                    2095                  2100

Gly Gln   Tyr Gly Pro Gly Gln   Gln Gly Pro Tyr Gly   Pro Gly Gln
    2105                    2110                  2115

Ser Ala   Ala Ala Ala Ala Gly   Gln Tyr Gln Gln Gly   Pro Gly Gln
    2120                    2125                  2130

Gln Gly   Pro Tyr Gly Pro Gly   Ala Ser Gly Pro Gly   Gln Gln Gly
    2135                    2140                  2145

Pro Tyr   Gly Pro Gly Ala Ser   Ala Ala Ala Ala Ala   Gly Pro Gly
    2150                    2155                  2160

Gln Tyr   Gly Pro Gly Gln Gln   Gly Pro Ser Ala Ser   Ala Ala Ala
    2165                    2170                  2175

Ala Ala   Gly Gln Tyr Gly Ser   Gly Pro Gly Gln Tyr   Gly Pro Tyr
    2180                    2185                  2190

Gly Pro   Gly Gln Ser Gly Pro   Gly Ser Gly Gln Gln   Gly Gln Gly
    2195                    2200                  2205

Pro Tyr   Gly Pro Gly Ala Ser   Ala Ala Ala Ala Ala   Gly Gln Tyr
    2210                    2215                  2220

```
Gly Pro  Gly Gln Gln Gly Pro  Tyr Gly Pro Gly Gln  Ser Ala Ala
    2225              2230              2235

Ala Ala  Ala Gly Pro Gly Ser  Gly Gln Tyr Gly Pro  Gly Ala Ser
    2240              2245              2250

Gly Gln  Asn Gly Pro Gly Ser  Gly Gln Tyr Gly Pro  Gly Gln Gln
    2255              2260              2265

Gly Pro  Gly Gln Ser Ala Ala  Ala Ala Ala Gly Gln  Tyr Gln Gln
    2270              2275              2280

Gly Pro  Gly Gln Gln Gly Pro  Tyr Gly Pro Gly Ala  Ser Ala Ala
    2285              2290              2295

Ala Ala  Ala Gly Gln Tyr Gly  Ser Gly Pro Gly Gln  Gln Gly Pro
    2300              2305              2310

Tyr Gly  Pro Gly Gln Ser Gly  Ser Gly Gln Gln Gly  Pro Gly Gln
    2315              2320              2325

Gln Gly  Pro Tyr Ala Ser Ala  Ala Ala Ala Ala Gly  Pro Gly Ser
    2330              2335              2340

Gly Gln  Gln Gly Ser Ser Val  Asp Lys Leu Ala Ala  Ala Leu Glu
    2345              2350              2355

His His  His His His His
    2360


<210>  10
<211>  597
<212>  PRT
<213>  Artificial Sequence

<220>
<223>  Met-PRT313

<400>  10

Met Gly Pro Gly Gly Gln Gly Pro Tyr Gly Pro Gly Ala Ser Ala Ala
1               5               10              15

Ala Ala Ala Gly Gly Asn Gly Pro Gly Ser Gly Gln Gln Gly Pro Gly
            20              25              30

Gly Ser Ala Ala Ala Ala Ala Gly Gly Tyr Gly Pro Gly Gly Gln Gly
            35              40              45
```

Pro Gly Gln Gln Gly Pro Gly Ser Ser Ala Ala Ala Ala Ala Gly Pro
        50              55              60

Gly Gly Tyr Gly Pro Gly Gly Gln Gly Pro Ser Ala Ser Ala Ala Ala
65              70              75                      80

Ala Ala Gly Pro Gly Ser Gly Gln Gln Gly Pro Gly Ala Ser Ala Ala
            85              90                      95

Ala Ala Ala Gly Gly Tyr Gly Pro Gly Gly Gln Gly Pro Gly Gln Gln
            100             105             110

Gly Pro Gly Ser Ser Ala Ala Ala Ala Ala Gly Gly Tyr Gly Ser Gly
        115             120                     125

Pro Gly Gln Gln Gly Pro Tyr Gly Ser Ala Ala Ala Ala Ala Gly Pro
        130             135             140

Gly Ser Gly Gly Tyr Gly Gln Gly Pro Tyr Gly Pro Gly Ala Ser Ala
145             150             155                     160

Ala Ala Ala Gly Pro Gly Gly Tyr Gly Pro Gly Gly Gln Gly Pro
            165             170             175

Ser Ala Ser Ala Ala Ala Ala Ala Gly Ser Gly Gln Gln Gly Pro Gly
        180             185                     190

Gly Tyr Gly Pro Tyr Ala Ser Ala Ala Ala Ala Gly Gly Tyr Gly
        195             200             205

Ser Gly Pro Gly Gln Gln Gly Pro Tyr Gly Pro Gly Gly Ser Ala Ala
    210             215             220

Ala Ala Ala Gly Ser Gly Gln Gln Gly Pro Gly Gln Gln Gly Pro Tyr
225             230             235             240

Ala Ser Ala Ala Ala Ala Ala Gly Pro Gly Gly Gln Gly Pro Tyr Gly
            245             250             255

Pro Gly Ser Ser Ala Ala Ala Ala Ala Gly Gly Tyr Gly Tyr Gly Pro
        260             265             270

Gly Gly Gln Gly Pro Tyr Gly Pro Gly Ala Ser Ala Ala Ala Ala Ala
    275             280             285

Gly Gly Asn Gly Pro Gly Ser Gly Gly Tyr Gly Pro Gly Gln Gln Gly
    290             295             300

184

Pro Gly Gly Ser Ala Ala Ala Ala Ala Gly Pro Gly Gly Gln Gly Pro
305                     310                 315                 320

Tyr Gly Pro Gly Ala Ser Ala Ala Ala Ala Ala Gly Gly Tyr Gly Pro
                    325                 330                 335

Gly Gly Gln Gly Pro Gly Gly Tyr Gly Pro Gly Ser Ser Ala Ala Ala
                340                 345                 350

Ala Ala Gly Pro Gly Gly Gln Gly Pro Tyr Gly Pro Gly Ser Ser Ala
            355                 360                 365

Ala Ala Ala Ala Gly Gly Tyr Gly Pro Gly Gln Gln Gly Pro Tyr Gly
        370                 375                 380

Pro Gly Gly Ser Ala Ala Ala Ala Ala Gly Gly Tyr Gln Gln Gly Pro
385                 390                 395                 400

Gly Gly Gln Gly Pro Tyr Gly Pro Gly Ala Ser Ala Ala Ala Ala Ala
                405                 410                 415

Gly Pro Gly Gly Gln Gly Pro Tyr Gly Pro Gly Ala Ser Ala Ala Ala
            420                 425                 430

Ala Ala Gly Pro Gly Gly Tyr Gly Pro Gly Gly Gln Gly Pro Ser Ala
        435                 440                 445

Ser Ala Ala Ala Ala Ala Gly Gly Tyr Gly Ser Gly Pro Gly Gly Tyr
    450                 455                 460

Gly Pro Tyr Gly Pro Gly Gly Ser Ala Ala Ala Ala Ala Gly Pro Gly
465                 470                 475                 480

Ser Gly Gln Gln Gly Gln Gly Pro Tyr Gly Pro Gly Ala Ser Ala Ala
                485                 490                 495

Ala Ala Ala Gly Gly Tyr Gly Pro Gly Gln Gln Gly Pro Tyr Gly Pro
            500                 505                 510

Gly Gly Ser Ala Ala Ala Ala Ala Gly Pro Gly Ser Gly Gly Tyr Gly
        515                 520                 525

Pro Gly Ala Ser Ala Ala Ala Ala Gly Gly Asn Gly Pro Gly Ser
    530                 535                 540

Gly Gly Tyr Gly Pro Gly Gln Gln Gly Pro Gly Gly Ser Ala Ala Ala

185

```
                545                   550                   555                   560


                Ala Ala Gly Gly Tyr Gln Gln Gly Pro Gly Gly Gln Gly Pro Tyr Gly
                            565                   570                   575


                Pro Gly Ala Ser Ala Ala Ala Ala Ala Gly Pro Gly Ser Gly Gln Gln
                            580                   585                   590


                Gly Pro Gly Ala Ser
                            595



                <210>  11
                <211>  12
                <212>  PRT
                <213>  Artificial Sequence

                <220>
                <223>  HisTag

                <400>  11

                Met His His His His His His Ser Ser Gly Ser Ser
                1                   5                   10


                <210>  12
                <211>  608
                <212>  PRT
                <213>  Artificial Sequence

                <220>
                <223>  PRT380

                <400>  12

                Met His His His His His His Ser Ser Gly Ser Ser Gly Pro Gly Gln
                1                   5                   10                  15


                Gln Gly Pro Tyr Gly Pro Gly Ala Ser Ala Ala Ala Ala Ala Gly Gln
                            20                  25                  30


                Asn Gly Pro Gly Ser Gly Gln Gln Gly Pro Gly Gln Ser Ala Ala Ala
                            35                  40                  45


                Ala Ala Gly Gln Tyr Gly Pro Gly Gln Gln Gly Pro Gly Gln Gln Gly
                    50                  55                  60


                Pro Gly Ser Ser Ala Ala Ala Ala Ala Gly Pro Gly Gln Tyr Gly Pro
                65                  70                  75                  80


                Gly Gln Gln Gly Pro Ser Ala Ser Ala Ala Ala Ala Ala Gly Pro Gly
                            85                  90                  95
```

```
Ser Gly Gln Gln Gly Pro Gly Ala Ser Ala Ala Ala Ala Gly Gln
            100                 105                 110

Tyr Gly Pro Gly Gln Gln Gly Pro Gly Gln Gln Gly Pro Gly Ser Ser
            115                 120                 125

Ala Ala Ala Ala Ala Gly Gln Tyr Gly Ser Gly Pro Gly Gln Gln Gly
            130                 135                 140

Pro Tyr Gly Ser Ala Ala Ala Ala Ala Gly Pro Gly Ser Gly Gln Tyr
145                 150                 155                 160

Gly Gln Gly Pro Tyr Gly Pro Gly Ala Ser Ala Ala Ala Ala Ala Gly
            165                 170                 175

Pro Gly Gln Tyr Gly Pro Gly Gln Gln Gly Pro Ser Ala Ser Ala Ala
            180                 185                 190

Ala Ala Ala Gly Ser Gly Gln Gln Gly Pro Gly Gln Tyr Gly Pro Tyr
            195                 200                 205

Ala Ser Ala Ala Ala Ala Ala Gly Gln Tyr Gly Ser Gly Pro Gly Gln
            210                 215                 220

Gln Gly Pro Tyr Gly Pro Gly Gln Ser Ala Ala Ala Ala Ala Gly Ser
225                 230                 235                 240

Gly Gln Gln Gly Pro Gly Gln Gln Gly Pro Tyr Ala Ser Ala Ala Ala
            245                 250                 255

Ala Ala Gly Pro Gly Gln Gln Gly Pro Tyr Gly Pro Gly Ser Ser Ala
            260                 265                 270

Ala Ala Ala Gly Gln Tyr Gly Tyr Gly Pro Gly Gln Gln Gly Pro
            275                 280                 285

Tyr Gly Pro Gly Ala Ser Ala Ala Ala Ala Gly Gln Asn Gly Pro
290                 295                 300

Gly Ser Gly Gln Tyr Gly Pro Gly Gln Gln Gly Pro Gly Gln Ser Ala
305                 310                 315                 320

Ala Ala Ala Gly Pro Gly Gln Gln Gly Pro Tyr Gly Pro Gly Ala
            325                 330                 335

Ser Ala Ala Ala Ala Ala Gly Gln Tyr Gly Pro Gly Gln Gln Gly Pro
            340                 345                 350
```

Gly Gln Tyr Gly Pro Gly Ser Ser Ala Ala Ala Ala Ala Gly Pro Gly
        355                 360             365

Gln Gln Gly Pro Tyr Gly Pro Gly Ser Ser Ala Ala Ala Ala Ala Gly
        370                 375             380

Gln Tyr Gly Pro Gly Gln Gln Gly Pro Tyr Gly Pro Gly Gln Ser Ala
385                 390                 395                 400

Ala Ala Ala Ala Gly Gln Tyr Gln Gln Gly Pro Gly Gln Gln Gly Pro
                405                 410                 415

Tyr Gly Pro Gly Ala Ser Ala Ala Ala Ala Gly Pro Gly Gln Gln
        420                 425                 430

Gly Pro Tyr Gly Pro Gly Ala Ser Ala Ala Ala Ala Gly Pro Gly
        435                 440                 445

Gln Tyr Gly Pro Gly Gln Gln Gly Pro Ser Ala Ser Ala Ala Ala Ala
        450                 455                 460

Ala Gly Gln Tyr Gly Ser Gly Pro Gly Gln Tyr Gly Pro Tyr Gly Pro
465                 470                 475                 480

Gly Gln Ser Ala Ala Ala Ala Ala Gly Pro Gly Ser Gly Gln Gln Gly
                485                 490                 495

Gln Gly Pro Tyr Gly Pro Gly Ala Ser Ala Ala Ala Ala Ala Gly Gln
        500                 505                 510

Tyr Gly Pro Gly Gln Gln Gly Pro Tyr Gly Pro Gly Gln Ser Ala Ala
        515                 520                 525

Ala Ala Ala Gly Pro Gly Ser Gly Gln Tyr Gly Pro Gly Ala Ser Ala
        530                 535                 540

Ala Ala Ala Gly Gln Asn Gly Pro Gly Ser Gly Gln Tyr Gly Pro
545                 550                 555                 560

Gly Gln Gln Gly Pro Gly Gln Ser Ala Ala Ala Ala Ala Gly Gln Tyr
                565                 570                 575

Gln Gln Gly Pro Gly Gln Gln Gly Pro Tyr Gly Pro Gly Ala Ser Ala
        580                 585                 590

Ala Ala Ala Gly Pro Gly Ser Gly Gln Gln Gly Pro Gly Ala Ser
        595                 600                 605

```
<210>  13
<211>  601
<212>  PRT
<213>  Artificial Sequence

<220>
<223>  PRT410

<400>  13

Met His His His His His His Ser Ser Gly Ser Ser Gly Pro Gly Gln
1               5                   10                  15

Gln Gly Pro Tyr Gly Pro Gly Ala Ser Ala Ala Ala Ala Ala Gly Gln
            20                  25                  30

Asn Gly Pro Gly Ser Gly Gln Gln Gly Pro Gly Gln Ser Gly Gln Tyr
            35                  40                  45

Gly Pro Gly Gln Gln Gly Pro Gly Gln Gln Gly Pro Gly Ser Ser Ala
        50                  55                  60

Ala Ala Ala Ala Gly Pro Gly Gln Tyr Gly Pro Gly Gln Gln Gly Pro
65                  70                  75                  80

Ser Ala Ser Ala Ala Ala Ala Ala Gly Pro Gly Ser Gly Gln Gln Gly
                85                  90                  95

Pro Gly Ala Ser Gly Gln Tyr Gly Pro Gly Gln Gln Gly Pro Gly Gln
            100                 105                 110

Gln Gly Pro Gly Ser Ser Ala Ala Ala Ala Ala Gly Gln Tyr Gly Ser
            115                 120                 125

Gly Pro Gly Gln Gln Gly Pro Tyr Gly Ser Ala Ala Ala Ala Ala Gly
        130                 135                 140

Pro Gly Ser Gly Gln Tyr Gly Gln Gly Pro Tyr Gly Pro Gly Ala Ser
145                 150                 155                 160

Gly Pro Gly Gln Tyr Gly Pro Gly Gln Gln Gly Pro Ser Ala Ser Ala
                165                 170                 175

Ala Ala Ala Ala Gly Ser Gly Gln Gln Gly Pro Gly Gln Tyr Gly Pro
            180                 185                 190

Tyr Ala Ser Ala Ala Ala Ala Ala Gly Gln Tyr Gly Ser Gly Pro Gly
            195                 200                 205
```

Gln Gln Gly Pro Tyr Gly Pro Gly Gln Ser Gly Ser Gly Gln Gln Gly
    210                     215                 220

Pro Gly Gln Gln Gly Pro Tyr Ala Ser Ala Ala Ala Ala Ala Gly Pro
225                     230                 235                 240

Gly Gln Gln Gly Pro Tyr Gly Pro Gly Ser Ser Ala Ala Ala Ala Ala
                245                 250                 255

Gly Gln Tyr Gly Tyr Gly Pro Gly Gln Gln Gly Pro Tyr Gly Pro Gly
            260                 265                 270

Ala Ser Gly Gln Asn Gly Pro Gly Ser Gly Gln Tyr Gly Pro Gly Gln
        275                 280                 285

Gln Gly Pro Gly Gln Ser Ala Ala Ala Ala Gly Pro Gly Gln Gln
    290                 295                 300

Gly Pro Tyr Gly Pro Gly Ala Ser Ala Ala Ala Ala Ala Gly Gln Tyr
305                 310                 315                 320

Gly Pro Gly Gln Gln Gly Pro Gly Gln Tyr Gly Pro Gly Ser Ser Gly
            325                 330                 335

Pro Gly Gln Gln Gly Pro Tyr Gly Pro Gly Ser Ser Ala Ala Ala Ala
        340                 345                 350

Ala Gly Gln Tyr Gly Pro Gly Gln Gln Gly Pro Tyr Gly Pro Gly Gln
        355                 360                 365

Ser Ala Ala Ala Ala Ala Gly Gln Tyr Gln Gln Gly Pro Gly Gln Gln
    370                 375                 380

Gly Pro Tyr Gly Pro Gly Ala Ser Gly Pro Gly Gln Gln Gly Pro Tyr
385                 390                 395                 400

Gly Pro Gly Ala Ser Ala Ala Ala Ala Ala Gly Pro Gly Gln Tyr Gly
                405                 410                 415

Pro Gly Gln Gln Gly Pro Ser Ala Ser Ala Ala Ala Ala Ala Gly Gln
        420                 425                 430

Tyr Gly Ser Gly Pro Gly Gln Tyr Gly Pro Tyr Gly Pro Gly Gln Ser
        435                 440                 445

Gly Pro Gly Ser Gly Gln Gln Gly Gln Gly Pro Tyr Gly Pro Gly Ala
    450                 455                 460

```
Ser Ala Ala Ala Ala Ala Gly Gln Tyr Gly Pro Gly Gln Gln Gly Pro
465                 470             475                 480


Tyr Gly Pro Gly Gln Ser Ala Ala Ala Ala Ala Gly Pro Gly Ser Gly
            485                 490                 495


Gln Tyr Gly Pro Gly Ala Ser Gly Gln Asn Gly Pro Gly Ser Gly Gln
        500                 505                 510


Tyr Gly Pro Gly Gln Gln Gly Pro Gly Gln Ser Ala Ala Ala Ala Ala
        515                 520                 525


Gly Gln Tyr Gln Gln Gly Pro Gly Gln Gln Gly Pro Tyr Gly Pro Gly
    530                 535                 540


Ala Ser Ala Ala Ala Ala Ala Gly Gln Tyr Gly Ser Gly Pro Gly Gln
545                 550             555                 560


Gln Gly Pro Tyr Gly Pro Gly Gln Ser Gly Ser Gly Gln Gln Gly Pro
            565                 570                 575


Gly Gln Gln Gly Pro Tyr Ala Ser Ala Ala Ala Ala Ala Gly Pro Gly
        580                 585                 590


Ser Gly Gln Gln Gly Pro Gly Ala Ser
        595                 600
```

```
<210>  14
<211>  576
<212>  PRT
<213>  Artificial Sequence

<220>
<223>  PRT525

<400>  14
```

```
Met His His His His His His Ser Ser Gly Ser Ser Gly Pro Gly Gln
1               5                   10                  15


Gln Gly Pro Tyr Gly Pro Gly Ala Ser Ala Ala Ala Ala Ala Ala Ala
            20                  25                  30


Gly Ser Asn Gly Pro Gly Ser Gly Gln Gln Gly Pro Gly Gln Ser Gly
        35                  40                  45


Gln Tyr Gly Pro Gly Gln Gln Gly Pro Gly Gln Gln Gly Pro Gly Ser
    50                  55                  60
```

191

Ser Ala Ala Ala Ala Ala Ala Gly Pro Gly Gln Tyr Gly Pro Gly
65                  70              75                  80

Gln Gln Gly Pro Ser Ala Ser Ala Ala Ala Ala Ala Ala Gly Pro
            85                  90                  95

Gly Ser Gly Gln Gln Gly Pro Gly Ala Ser Gly Gln Tyr Gly Pro Gly
            100                 105                 110

Gln Gln Gly Pro Gly Gln Gln Gly Pro Gly Ser Ser Ala Ala Ala Ala
            115                 120                 125

Ala Ala Ala Gly Ser Tyr Gly Ser Gly Pro Gly Gln Gln Gly Pro Tyr
            130                 135                 140

Gly Ser Ala Ala Ala Ala Ala Ala Ala Gly Pro Gly Ser Gly Gln Tyr
145                 150                 155                 160

Gly Gln Gly Pro Tyr Gly Pro Gly Ala Ser Gly Pro Gly Gln Tyr Gly
            165                 170                 175

Pro Gly Gln Gln Gly Pro Ser Ala Ser Ala Ala Ala Ala Ala Ala Ala
            180                 185                 190

Gly Ser Gly Gln Gln Gly Pro Gly Gln Tyr Gly Pro Tyr Ala Ser Ala
            195                 200                 205

Ala Ala Ala Ala Ala Gly Ser Tyr Gly Ser Gly Pro Gly Gln Gln
            210                 215                 220

Gly Pro Tyr Gly Pro Gly Gln Ser Gly Ser Gly Gln Gln Gly Pro Gly
225                 230                 235                 240

Gln Gln Gly Pro Tyr Ala Ser Ala Ala Ala Ala Ala Ala Gly Pro
            245                 250                 255

Gly Gln Gln Gly Pro Tyr Gly Pro Gly Ser Ser Ala Ala Ala Ala
            260                 265                 270

Ala Ala Gly Ser Tyr Gly Tyr Gly Pro Gly Gln Gln Gly Pro Tyr Gly
            275                 280                 285

Pro Gly Ala Ser Gly Gln Asn Gly Pro Gly Ser Gly Gln Tyr Gly Pro
            290                 295                 300

Gly Gln Gln Gly Pro Gly Pro Ser Ala Ala Ala Ala Ala Ala Ala Gly
305                 310                 315                 320

192

Pro Gly Gln Gln Gly Pro Tyr Gly Pro Gly Ala Ser Ala Ala Ala Ala
              325                 330                 335

Ala Ala Ala Gly Ser Tyr Gly Pro Gly Gln Gln Gly Pro Gly Gln Tyr
              340                 345                 350

Gly Pro Gly Ser Ser Gly Pro Gly Gln Gln Gly Pro Tyr Gly Pro Gly
              355                 360                 365

Ser Ser Ala Ala Ala Ala Ala Ala Ala Gly Ser Tyr Gly Pro Gly Gln
              370                 375                 380

Gln Gly Pro Tyr Gly Pro Gly Pro Ser Ala Ala Ala Ala Ala Ala Ala
385               390                 395                 400

Gly Ser Tyr Gln Gln Gly Pro Gly Gln Gln Gly Pro Tyr Gly Pro Gly
              405                 410                 415

Ala Ser Gly Pro Gly Gln Gln Gly Pro Tyr Gly Pro Gly Ala Ser Ala
              420                 425                 430

Ala Ala Ala Ala Ala Ala Gly Pro Gly Gln Tyr Gly Pro Gly Gln Gln
              435                 440                 445

Gly Pro Ser Ala Ser Ala Ala Ala Ala Ala Ala Ala Gly Ser Tyr Gly
              450                 455                 460

Ser Gly Pro Gly Gln Tyr Gly Pro Tyr Gly Pro Gly Gln Ser Gly Pro
465               470                 475                 480

Gly Ser Gly Gln Gln Gly Gln Gly Pro Tyr Gly Pro Gly Ala Ser Ala
              485                 490                 495

Ala Ala Ala Ala Ala Gly Ser Tyr Gly Pro Gly Gln Gln Gly Pro
              500                 505                 510

Tyr Gly Pro Gly Pro Ser Ala Ala Ala Ala Ala Ala Gly Pro Gly
              515                 520                 525

Ser Gly Gln Tyr Gly Pro Gly Ala Ser Gly Gln Asn Gly Pro Gly Ser
              530                 535                 540

Gly Gln Tyr Gly Pro Gly Gln Gln Gly Pro Gly Pro Ser Ala Ala Ala
545               550                 555                 560

Ala Ala Ala Ala Gly Pro Gly Ser Gly Gln Gln Gly Pro Gly Ala Ser

565                          570                          575

<210> 15
<211> 2375
<212> PRT
<213> Artificial Sequence

<220>
<223> PRT799

<400> 15

Met His His His His His His Ser Ser Gly Ser Ser Gly Pro Gly Gln
1                   5                   10                  15

Gln Gly Pro Tyr Gly Pro Gly Ala Ser Ala Ala Ala Ala Ala Gly Gln
                20                  25                  30

Asn Gly Pro Gly Ser Gly Gln Gln Gly Pro Gly Gln Ser Gly Gln Tyr
            35                  40                  45

Gly Pro Gly Gln Gln Gly Pro Gly Gln Gln Gly Pro Gly Ser Ser Ala
        50                  55                  60

Ala Ala Ala Ala Gly Pro Gly Gln Tyr Gly Pro Gly Gln Gln Gly Pro
65                  70                  75                  80

Ser Ala Ser Ala Ala Ala Ala Ala Gly Pro Gly Ser Gly Gln Gln Gly
            85                  90                  95

Pro Gly Ala Ser Gly Gln Tyr Gly Pro Gly Gln Gln Gly Pro Gly Gln
            100                 105                 110

Gln Gly Pro Gly Ser Ser Ala Ala Ala Ala Ala Gly Gln Tyr Gly Ser
        115                 120                 125

Gly Pro Gly Gln Gln Gly Pro Tyr Gly Ser Ala Ala Ala Ala Ala Gly
    130                 135                 140

Pro Gly Ser Gly Gln Tyr Gly Gln Gly Pro Tyr Gly Pro Gly Ala Ser
145                 150                 155                 160

Gly Pro Gly Gln Tyr Gly Pro Gly Gln Gln Gly Pro Ser Ala Ser Ala
            165                 170                 175

Ala Ala Ala Gly Ser Gly Gln Gln Gly Pro Gly Gln Tyr Gly Pro
        180                 185                 190

Tyr Ala Ser Ala Ala Ala Ala Ala Gly Gln Tyr Gly Ser Gly Pro Gly
        195                 200                 205

Gln Gln Gly Pro Tyr Gly Pro Gly Gln Ser Gly Ser Gly Gln Gln Gly
    210               215              220

Pro Gly Gln Gln Gly Pro Tyr Ala Ser Ala Ala Ala Ala Ala Gly Pro
225               230              235                  240

Gly Gln Gln Gly Pro Tyr Gly Pro Gly Ser Ser Ala Ala Ala Ala Ala
                245              250                  255

Gly Gln Tyr Gly Tyr Gly Pro Gly Gln Gln Gly Pro Tyr Gly Pro Gly
            260              265              270

Ala Ser Gly Gln Asn Gly Pro Gly Ser Gly Gln Tyr Gly Pro Gly Gln
            275              280              285

Gln Gly Pro Gly Gln Ser Ala Ala Ala Ala Gly Pro Gly Gln Gln
    290              295              300

Gly Pro Tyr Gly Pro Gly Ala Ser Ala Ala Ala Ala Gly Gln Tyr
305              310              315              320

Gly Pro Gly Gln Gln Gly Pro Gly Gln Tyr Gly Pro Gly Ser Ser Gly
            325              330              335

Pro Gly Gln Gln Gly Pro Tyr Gly Pro Gly Ser Ser Ala Ala Ala Ala
    340              345              350

Ala Gly Gln Tyr Gly Pro Gly Gln Gln Gly Pro Tyr Gly Pro Gly Gln
    355              360              365

Ser Ala Ala Ala Ala Ala Gly Gln Tyr Gln Gln Gly Pro Gly Gln Gln
    370              375              380

Gly Pro Tyr Gly Pro Gly Ala Ser Gly Pro Gly Gln Gln Gly Pro Tyr
385              390              395              400

Gly Pro Gly Ala Ser Ala Ala Ala Ala Ala Gly Pro Gly Gln Tyr Gly
            405              410              415

Pro Gly Gln Gln Gly Pro Ser Ala Ser Ala Ala Ala Ala Ala Gly Gln
            420              425              430

Tyr Gly Ser Gly Pro Gly Gln Tyr Gly Pro Tyr Gly Pro Gly Gln Ser
    435              440              445

Gly Pro Gly Ser Gly Gln Gln Gly Gln Gly Pro Tyr Gly Pro Gly Ala

195

450                   455                  460

Ser Ala Ala Ala Ala Ala Gly Gln Tyr Gly Pro Gly Gln Gln Gly Pro
465             470           475           480

Tyr Gly Pro Gly Gln Ser Ala Ala Ala Ala Gly Pro Gly Ser Gly
         485         490         495

Gln Tyr Gly Pro Gly Ala Ser Gly Gln Asn Gly Pro Gly Ser Gly Gln
      500         505        510

Tyr Gly Pro Gly Gln Gln Gly Pro Gly Gln Ser Ala Ala Ala Ala Ala
      515         520        525

Gly Gln Tyr Gln Gln Gly Pro Gly Gln Gln Gly Pro Tyr Gly Pro Gly
    530         535        540

Ala Ser Ala Ala Ala Ala Ala Gly Gln Tyr Gly Ser Gly Pro Gly Gln
545           550         555        560

Gln Gly Pro Tyr Gly Pro Gly Gln Ser Gly Ser Gly Gln Gln Gly Pro
      565         570        575

Gly Gln Gln Gly Pro Tyr Ala Ser Ala Ala Ala Ala Gly Pro Gly
      580         585        590

Ser Gly Gln Gln Gly Pro Gly Ala Ser Gly Gln Gln Gly Pro Tyr Gly
      595         600        605

Pro Gly Ala Ser Ala Ala Ala Ala Ala Gly Gln Asn Gly Pro Gly Ser
      610         615        620

Gly Gln Gln Gly Pro Gly Gln Ser Gly Gln Tyr Gly Pro Gly Gln Gln
625           630         635        640

Gly Pro Gly Gln Gln Gly Pro Gly Ser Ser Ala Ala Ala Ala Gly
         645         650        655

Pro Gly Gln Tyr Gly Pro Gly Gln Gln Gly Pro Ser Ala Ser Ala Ala
      660         665        670

Ala Ala Ala Gly Pro Gly Ser Gly Gln Gln Gly Pro Gly Ala Ser Gly
      675         680        685

Gln Tyr Gly Pro Gly Gln Gln Gly Pro Gly Gln Gln Gly Pro Gly Ser
      690         695        700

Ser Ala Ala Ala Ala Ala Gly Gln Tyr Gly Ser Gly Pro Gly Gln Gln
705 710 715 720

Gly Pro Tyr Gly Ser Ala Ala Ala Ala Ala Gly Pro Gly Ser Gly Gln
725 730 735

Tyr Gly Gln Gly Pro Tyr Gly Pro Gly Ala Ser Gly Pro Gly Gln Tyr
740 745 750

Gly Pro Gly Gln Gln Gly Pro Ser Ala Ser Ala Ala Ala Ala Ala Gly
755 760 765

Ser Gly Gln Gln Gly Pro Gly Gln Tyr Gly Pro Tyr Ala Ser Ala Ala
770 775 780

Ala Ala Ala Gly Gln Tyr Gly Ser Gly Pro Gly Gln Gln Gly Pro Tyr
785 790 795 800

Gly Pro Gly Gln Ser Gly Ser Gly Gln Gln Gly Pro Gly Gln Gln Gly
805 810 815

Pro Tyr Ala Ser Ala Ala Ala Ala Ala Gly Pro Gly Gln Gln Gly Pro
820 825 830

Tyr Gly Pro Gly Ser Ser Ala Ala Ala Ala Gly Gln Tyr Gly Tyr
835 840 845

Gly Pro Gly Gln Gln Gly Pro Tyr Gly Pro Gly Ala Ser Gly Gln Asn
850 855 860

Gly Pro Gly Ser Gly Gln Tyr Gly Pro Gly Gln Gln Gly Pro Gly Gln
865 870 875 880

Ser Ala Ala Ala Ala Ala Gly Pro Gly Gln Gln Gly Pro Tyr Gly Pro
885 890 895

Gly Ala Ser Ala Ala Ala Ala Gly Gln Tyr Gly Pro Gly Gln Gln
900 905 910

Gly Pro Gly Gln Tyr Gly Pro Gly Ser Ser Gly Pro Gly Gln Gln Gly
915 920 925

Pro Tyr Gly Pro Gly Ser Ser Ala Ala Ala Ala Gly Gln Tyr Gly
930 935 940

Pro Gly Gln Gln Gly Pro Tyr Gly Pro Gly Gln Ser Ala Ala Ala Ala
945 950 955 960

197

```
Ala Gly Gln Tyr Gln Gln Gly Pro Gly Gln Gln Gly Pro Tyr Gly Pro
            965                     970                     975

Gly Ala Ser Gly Pro Gly Gln Gln Gly Pro Tyr Gly Pro Gly Ala Ser
            980                     985                     990

Ala Ala Ala Ala Ala Gly Pro Gly  Gln Tyr Gly Pro Gly  Gln Gln Gly
        995                 1000                    1005

Pro Ser  Ala Ser Ala Ala Ala  Ala Ala Gly Gln Tyr  Gly Ser Gly
    1010                 1015             1020

Pro Gly  Gln Tyr Gly Pro Tyr  Gly Pro Gly Gln Ser  Gly Pro Gly
    1025                 1030             1035

Ser Gly  Gln Gln Gly Gln Gly  Pro Tyr Gly Pro Gly  Ala Ser Ala
    1040                 1045             1050

Ala Ala  Ala Ala Gly Gln Tyr  Gly Pro Gly Gln Gln  Gly Pro Tyr
    1055                 1060             1065

Gly Pro  Gly Gln Ser Ala Ala  Ala Ala Ala Gly Pro  Gly Ser Gly
    1070                 1075             1080

Gln Tyr  Gly Pro Gly Ala Ser  Gly Gln Asn Gly Pro  Gly Ser Gly
    1085                 1090             1095

Gln Tyr  Gly Pro Gly Gln Gln  Gly Pro Gly Gln Ser  Ala Ala Ala
    1100                 1105             1110

Ala Ala  Gly Gln Tyr Gln Gln  Gly Pro Gly Gln Gln  Gly Pro Tyr
    1115                 1120             1125

Gly Pro  Gly Ala Ser Ala Ala  Ala Ala Ala Gly Gln  Tyr Gly Ser
    1130                 1135             1140

Gly Pro  Gly Gln Gln Gly Pro  Tyr Gly Pro Gly Gln  Ser Gly Ser
    1145                 1150             1155

Gly Gln  Gln Gly Pro Gly Gln  Gln Gly Pro Tyr Ala  Ser Ala Ala
    1160                 1165             1170

Ala Ala  Ala Gly Pro Gly Ser  Gly Gln Gln Gly Pro  Gly Ala Ser
    1175                 1180             1185

Gly Gln  Gln Gly Pro Tyr Gly  Pro Gly Ala Ser Ala  Ala Ala Ala
    1190                 1195             1200
```

198

```
Ala Gly  Gln Asn Gly Pro Gly  Ser Gly Gln Gln Gly  Pro Gly Gln
1205             1210                1215

Ser Gly  Gln Tyr Gly Pro Gly  Gln Gln Gly Pro Gly  Gln Gln Gly
1220             1225                1230

Pro Gly  Ser Ser Ala Ala Ala  Ala Ala Gly Pro Gly  Gln Tyr Gly
1235             1240                1245

Pro Gly  Gln Gln Gly Pro Ser  Ala Ser Ala Ala Ala  Ala Ala Gly
1250             1255                1260

Pro Gly  Ser Gly Gln Gln Gly  Pro Gly Ala Ser Gly  Gln Tyr Gly
1265             1270                1275

Pro Gly  Gln Gln Gly Pro Gly  Gln Gln Gly Pro Gly  Ser Ser Ala
1280             1285                1290

Ala Ala  Ala Ala Gly Gln Tyr  Gly Ser Gly Pro Gly  Gln Gln Gly
1295             1300                1305

Pro Tyr  Gly Ser Ala Ala Ala  Ala Ala Gly Pro Gly  Ser Gly Gln
1310             1315                1320

Tyr Gly  Gln Gly Pro Tyr Gly  Pro Gly Ala Ser Gly  Pro Gly Gln
1325             1330                1335

Tyr Gly  Pro Gly Gln Gln Gly  Pro Ser Ala Ser Ala  Ala Ala Ala
1340             1345                1350

Ala Gly  Ser Gly Gln Gln Gly  Pro Gly Gln Tyr Gly  Pro Tyr Ala
1355             1360                1365

Ser Ala  Ala Ala Ala Ala Gly  Gln Tyr Gly Ser Gly  Pro Gly Gln
1370             1375                1380

Gln Gly  Pro Tyr Gly Pro Gly  Gln Ser Gly Ser Gly  Gln Gln Gly
1385             1390                1395

Pro Gly  Gln Gln Gly Pro Tyr  Ala Ser Ala Ala Ala  Ala Ala Gly
1400             1405                1410

Pro Gly  Gln Gln Gly Pro Tyr  Gly Pro Gly Ser Ser  Ala Ala Ala
1415             1420                1425

Ala Ala  Gly Gln Tyr Gly Tyr  Gly Pro Gly Gln Gln  Gly Pro Tyr
```

1430                          1435                          1440

Gly Pro  Gly Ala Ser Gly Gln  Asn Gly Pro Gly Ser  Gly Gln Tyr
     1445              1450              1455

Gly Pro  Gly Gln Gln Gly Pro  Gly Gln Ser Ala Ala  Ala Ala Ala
     1460              1465              1470

Gly Pro  Gly Gln Gln Gly Pro  Tyr Gly Pro Gly Ala  Ser Ala Ala
     1475              1480              1485

Ala Ala  Ala Gly Gln Tyr Gly  Pro Gly Gln Gln Gly  Pro Gly Gln
     1490              1495              1500

Tyr Gly  Pro Gly Ser Ser Gly  Pro Gly Gln Gln Gly  Pro Tyr Gly
     1505              1510              1515

Pro Gly  Ser Ser Ala Ala Ala  Ala Ala Gly Gln Tyr  Gly Pro Gly
     1520              1525              1530

Gln Gln  Gly Pro Tyr Gly Pro  Gly Gln Ser Ala Ala  Ala Ala Ala
     1535              1540              1545

Gly Gln  Tyr Gln Gln Gly Pro  Gly Gln Gln Gly Pro  Tyr Gly Pro
     1550              1555              1560

Gly Ala  Ser Gly Pro Gly Gln  Gln Gly Pro Tyr Gly  Pro Gly Ala
     1565              1570              1575

Ser Ala  Ala Ala Ala Ala Gly  Pro Gly Gln Tyr Gly  Pro Gly Gln
     1580              1585              1590

Gln Gly  Pro Ser Ala Ser Ala  Ala Ala Ala Ala Gly  Gln Tyr Gly
     1595              1600              1605

Ser Gly  Pro Gly Gln Tyr Gly  Pro Tyr Gly Pro Gly  Gln Ser Gly
     1610              1615              1620

Pro Gly  Ser Gly Gln Gln Gly  Gln Gly Pro Tyr Gly  Pro Gly Ala
     1625              1630              1635

Ser Ala  Ala Ala Ala Ala Gly  Gln Tyr Gly Pro Gly  Gln Gln Gly
     1640              1645              1650

Pro Tyr  Gly Pro Gly Gln Ser  Ala Ala Ala Ala Ala  Gly Pro Gly
     1655              1660              1665

200

```
Ser Gly  Gln Tyr Gly Pro Gly  Ala Ser Gly Gln Asn  Gly Pro Gly
    1670             1675              1680

Ser Gly  Gln Tyr Gly Pro Gly  Gln Gln Gly Pro Gly  Gln Ser Ala
    1685             1690              1695

Ala Ala  Ala Ala Gly Gln Tyr  Gln Gln Gly Pro Gly  Gln Gln Gly
    1700             1705              1710

Pro Tyr  Gly Pro Gly Ala Ser  Ala Ala Ala Ala Ala  Gly Gln Tyr
    1715             1720              1725

Gly Ser  Gly Pro Gly Gln Gln  Gly Pro Tyr Gly Pro  Gly Gln Ser
    1730             1735              1740

Gly Ser  Gly Gln Gln Gly Pro  Gly Gln Gln Gly Pro  Tyr Ala Ser
    1745             1750              1755

Ala Ala  Ala Ala Ala Gly Pro  Gly Ser Gly Gln Gln  Gly Pro Gly
    1760             1765              1770

Ala Ser  Gly Gln Gln Gly Pro  Tyr Gly Pro Gly Ala  Ser Ala Ala
    1775             1780              1785

Ala Ala  Ala Gly Gln Asn Gly  Pro Gly Ser Gly Gln  Gln Gly Pro
    1790             1795              1800

Gly Gln  Ser Gly Gln Tyr Gly  Pro Gly Gln Gln Gly  Pro Gly Gln
    1805             1810              1815

Gln Gly  Pro Gly Ser Ser Ala  Ala Ala Ala Ala Gly  Pro Gly Gln
    1820             1825              1830

Tyr Gly  Pro Gly Gln Gln Gly  Pro Ser Ala Ser Ala  Ala Ala Ala
    1835             1840              1845

Ala Gly  Pro Gly Ser Gly Gln  Gln Gly Pro Gly Ala  Ser Gly Gln
    1850             1855              1860

Tyr Gly  Pro Gly Gln Gln Gly  Pro Gly Gln Gln Gly  Pro Gly Ser
    1865             1870              1875

Ser Ala  Ala Ala Ala Ala Gly  Gln Tyr Gly Ser Gly  Pro Gly Gln
    1880             1885              1890

Gln Gly  Pro Tyr Gly Ser Ala  Ala Ala Ala Ala Gly  Pro Gly Ser
    1895             1900              1905
```

Gly Gln Tyr Gly Gln Gly Pro Tyr Gly Pro Gly Ala Ser Gly Pro
    1910                1915            1920

Gly Gln Tyr Gly Pro Gly Gln Gln Gly Pro Ser Ala Ser Ala Ala
    1925                1930            1935

Ala Ala Ala Gly Ser Gly Gln Gln Gly Pro Gly Gln Tyr Gly Pro
    1940                1945            1950

Tyr Ala Ser Ala Ala Ala Ala Ala Gly Gln Tyr Gly Ser Gly Pro
    1955                1960            1965

Gly Gln Gln Gly Pro Tyr Gly Pro Gly Gln Ser Gly Ser Gly Gln
    1970                1975            1980

Gln Gly Pro Gly Gln Gln Gly Pro Tyr Ala Ser Ala Ala Ala Ala
    1985                1990            1995

Ala Gly Pro Gly Gln Gln Gly Pro Tyr Gly Pro Gly Ser Ser Ala
    2000                2005            2010

Ala Ala Ala Ala Gly Gln Tyr Gly Tyr Gly Pro Gly Gln Gln Gly
    2015                2020            2025

Pro Tyr Gly Pro Gly Ala Ser Gly Gln Asn Gly Pro Gly Ser Gly
    2030                2035            2040

Gln Tyr Gly Pro Gly Gln Gln Gly Pro Gly Gln Ser Ala Ala Ala
    2045                2050            2055

Ala Ala Gly Pro Gly Gln Gln Gly Pro Tyr Gly Pro Gly Ala Ser
    2060                2065            2070

Ala Ala Ala Ala Ala Gly Gln Tyr Gly Pro Gly Gln Gln Gly Pro
    2075                2080            2085

Gly Gln Tyr Gly Pro Gly Ser Ser Gly Pro Gly Gln Gln Gly Pro
    2090                2095            2100

Tyr Gly Pro Gly Ser Ser Ala Ala Ala Ala Ala Gly Gln Tyr Gly
    2105                2110            2115

Pro Gly Gln Gln Gly Pro Tyr Gly Pro Gly Gln Ser Ala Ala Ala
    2120                2125            2130

Ala Ala Gly Gln Tyr Gln Gln Gly Pro Gly Gln Gln Gly Pro Tyr
    2135                2140            2145

```
Gly Pro  Gly Ala Ser Gly Pro  Gly Gln Gln Gly Pro  Tyr Gly Pro
    2150                2155                2160

Gly Ala  Ser Ala Ala Ala Ala  Ala Gly Pro Gly Gln  Tyr Gly Pro
    2165                2170                2175

Gly Gln  Gln Gly Pro Ser Ala  Ser Ala Ala Ala Ala  Ala Gly Gln
    2180                2185                2190

Tyr Gly  Ser Gly Pro Gly Gln  Tyr Gly Pro Tyr Gly  Pro Gly Gln
    2195                2200                2205

Ser Gly  Pro Gly Ser Gly Gln  Gln Gly Gln Gly Pro  Tyr Gly Pro
    2210                2215                2220

Gly Ala  Ser Ala Ala Ala Ala  Ala Gly Gln Tyr Gly  Pro Gly Gln
    2225                2230                2235

Gln Gly  Pro Tyr Gly Pro Gly  Gln Ser Ala Ala Ala  Ala Ala Gly
    2240                2245                2250

Pro Gly  Ser Gly Gln Tyr Gly  Pro Gly Ala Ser Gly  Gln Asn Gly
    2255                2260                2265

Pro Gly  Ser Gly Gln Tyr Gly  Pro Gly Gln Gln Gly  Pro Gly Gln
    2270                2275                2280

Ser Ala  Ala Ala Ala Ala Gly  Gln Tyr Gln Gln Gly  Pro Gly Gln
    2285                2290                2295

Gln Gly  Pro Tyr Gly Pro Gly  Ala Ser Ala Ala Ala  Ala Ala Gly
    2300                2305                2310

Gln Tyr  Gly Ser Gly Pro Gly  Gln Gln Gly Pro Tyr  Gly Pro Gly
    2315                2320                2325

Gln Ser  Gly Ser Gly Gln Gln  Gly Pro Gly Gln Gln  Gly Pro Tyr
    2330                2335                2340

Ala Ser  Ala Ala Ala Ala Ala  Gly Pro Gly Ser Gly  Gln Gln Gly
    2345                2350                2355

Ser Ser  Val Asp Lys Leu Ala  Ala Ala Leu Glu His  His His His
    2360                2365                2370

His His
```

2375

```
<210>  16
<211>  608
<212>  PRT
<213>  Artificial Sequence

<220>
<223>  PRT313

<400>  16

Met His His His His His His Ser Ser Gly Ser Ser Gly Pro Gly Gly
1               5                   10                  15


Gln Gly Pro Tyr Gly Pro Gly Ala Ser Ala Ala Ala Ala Ala Gly Gly
                20                  25                  30


Asn Gly Pro Gly Ser Gly Gln Gln Gly Pro Gly Gly Ser Ala Ala Ala
            35                  40                  45


Ala Ala Gly Gly Tyr Gly Pro Gly Gly Gln Gly Pro Gly Gln Gln Gly
        50                  55                  60


Pro Gly Ser Ser Ala Ala Ala Ala Ala Gly Pro Gly Gly Tyr Gly Pro
65                  70                  75                  80


Gly Gly Gln Gly Pro Ser Ala Ser Ala Ala Ala Ala Ala Gly Pro Gly
                85                  90                  95


Ser Gly Gln Gln Gly Pro Gly Ala Ser Ala Ala Ala Ala Ala Gly Gly
            100                 105                 110


Tyr Gly Pro Gly Gly Gln Gly Pro Gly Gln Gln Gly Pro Gly Ser Ser
        115                 120                 125


Ala Ala Ala Ala Ala Gly Gly Tyr Gly Ser Gly Pro Gly Gln Gln Gly
        130                 135                 140


Pro Tyr Gly Ser Ala Ala Ala Ala Ala Gly Pro Gly Ser Gly Gly Tyr
145                 150                 155                 160


Gly Gln Gly Pro Tyr Gly Pro Gly Ala Ser Ala Ala Ala Ala Ala Gly
                165                 170                 175


Pro Gly Gly Tyr Gly Pro Gly Gly Gln Gly Pro Ser Ala Ser Ala Ala
            180                 185                 190


Ala Ala Ala Gly Ser Gly Gln Gln Gly Pro Gly Gly Tyr Gly Pro Tyr
        195                 200                 205
```

Ala Ser Ala Ala Ala Ala Ala Gly Gly Tyr Gly Ser Gly Pro Gly Gln
210 215 220

Gln Gly Pro Tyr Gly Pro Gly Gly Ser Ala Ala Ala Ala Ala Gly Ser
225 230 235 240

Gly Gln Gln Gly Pro Gly Gln Gln Gly Pro Tyr Ala Ser Ala Ala Ala
245 250 255

Ala Ala Gly Pro Gly Gly Gln Gly Pro Tyr Gly Pro Gly Ser Ser Ala
260 265 270

Ala Ala Ala Gly Gly Tyr Gly Tyr Gly Pro Gly Gly Gln Gly Pro
275 280 285

Tyr Gly Pro Gly Ala Ser Ala Ala Ala Ala Gly Gly Asn Gly Pro
290 295 300

Gly Ser Gly Gly Tyr Gly Pro Gly Gln Gln Gly Pro Gly Gly Ser Ala
305 310 315 320

Ala Ala Ala Gly Pro Gly Gly Gln Gly Pro Tyr Gly Pro Gly Ala
325 330 335

Ser Ala Ala Ala Ala Gly Gly Tyr Gly Pro Gly Gly Gln Gly Pro
340 345 350

Gly Gly Tyr Gly Pro Gly Ser Ser Ala Ala Ala Ala Ala Gly Pro Gly
355 360 365

Gly Gln Gly Pro Tyr Gly Pro Gly Ser Ser Ala Ala Ala Ala Gly
370 375 380

Gly Tyr Gly Pro Gly Gln Gln Gly Pro Tyr Gly Pro Gly Gly Ser Ala
385 390 395 400

Ala Ala Ala Gly Gly Tyr Gln Gln Gly Pro Gly Gly Gln Gly Pro
405 410 415

Tyr Gly Pro Gly Ala Ser Ala Ala Ala Ala Ala Gly Pro Gly Gly Gln
420 425 430

Gly Pro Tyr Gly Pro Gly Ala Ser Ala Ala Ala Ala Gly Pro Gly
435 440 445

Gly Tyr Gly Pro Gly Gly Gln Gly Pro Ser Ala Ser Ala Ala Ala Ala

```
                450                     455                         460


        Ala Gly Gly Tyr Gly Ser Gly Pro Gly Gly Tyr Gly Pro Tyr Gly Pro
        465                 470                 475                 480


        Gly Gly Ser Ala Ala Ala Ala Ala Gly Pro Gly Ser Gly Gln Gln Gly
                        485                 490                 495


        Gln Gly Pro Tyr Gly Pro Gly Ala Ser Ala Ala Ala Ala Ala Gly Gly
                    500                 505                 510


        Tyr Gly Pro Gly Gln Gln Gly Pro Tyr Gly Pro Gly Gly Ser Ala Ala
                    515                 520                 525


        Ala Ala Ala Gly Pro Gly Ser Gly Gly Tyr Gly Pro Gly Ala Ser Ala
            530                 535                 540


        Ala Ala Ala Gly Gly Asn Gly Pro Gly Ser Gly Gly Tyr Gly Pro
        545                 550                 555                 560


        Gly Gln Gln Gly Pro Gly Gly Ser Ala Ala Ala Ala Ala Gly Gly Tyr
                    565                 570                 575


        Gln Gln Gly Pro Gly Gly Gln Gly Pro Tyr Gly Pro Gly Ala Ser Ala
                    580                 585                 590


        Ala Ala Ala Gly Pro Gly Ser Gly Gln Gln Gly Pro Gly Ala Ser
            595                 600                 605
```

```
<210>   17
<211>   590
<212>   PRT
<213>   Artificial Sequence

<220>
<223>   Met-PRT399

<400>   17
```

```
Met Gly Pro Gly Gly Gln Gly Pro Tyr Gly Pro Gly Ala Ser Ala Ala
1               5               10                  15


Ala Ala Ala Gly Gly Asn Gly Pro Gly Ser Gly Gln Gln Gly Pro Gly
                20              25                  30


Gly Ser Gly Gly Tyr Gly Pro Gly Gly Gln Gly Pro Gly Gln Gln Gly
            35              40                  45


Pro Gly Ser Ser Ala Ala Ala Ala Ala Gly Pro Gly Gly Tyr Gly Pro
        50              55                  60
```

Gly Gly Gln Gly Pro Ser Ala Ser Ala Ala Ala Ala Ala Gly Pro Gly
65                  70              75                      80

Ser Gly Gln Gln Gly Pro Gly Ala Ser Gly Gly Tyr Gly Pro Gly Gly
                    85              90                  95

Gln Gly Pro Gly Gln Gln Gly Pro Gly Ser Ser Ala Ala Ala Ala Ala
            100             105             110

Gly Gly Tyr Gly Ser Gly Pro Gly Gln Gln Gly Pro Tyr Gly Ser Ala
        115             120             125

Ala Ala Ala Ala Gly Pro Gly Ser Gly Gly Tyr Gly Gln Gly Pro Tyr
    130             135             140

Gly Pro Gly Ala Ser Gly Pro Gly Gly Tyr Gly Pro Gly Gly Gln Gly
145             150             155                     160

Pro Ser Ala Ser Ala Ala Ala Ala Ala Gly Ser Gly Gln Gln Gly Pro
            165             170             175

Gly Gly Tyr Gly Pro Tyr Ala Ser Ala Ala Ala Ala Gly Gly Tyr
        180             185             190

Gly Ser Gly Pro Gly Gln Gln Gly Pro Tyr Gly Pro Gly Gly Ser Gly
        195             200             205

Ser Gly Gln Gln Gly Pro Gly Gln Gln Gly Pro Tyr Ala Ser Ala Ala
    210             215             220

Ala Ala Ala Gly Pro Gly Gly Gln Gly Pro Tyr Gly Pro Gly Ser Ser
225             230             235                     240

Ala Ala Ala Ala Ala Gly Gly Tyr Gly Tyr Gly Pro Gly Gly Gln Gly
            245             250             255

Pro Tyr Gly Pro Gly Ala Ser Gly Gly Asn Gly Pro Gly Ser Gly Gly
        260             265             270

Tyr Gly Pro Gly Gln Gln Gly Pro Gly Gly Ser Ala Ala Ala Ala Ala
        275             280             285

Gly Pro Gly Gly Gln Gly Pro Tyr Gly Pro Gly Ala Ser Ala Ala Ala
        290             295             300

Ala Ala Gly Gly Tyr Gly Pro Gly Gly Gln Gly Pro Gly Gly Tyr Gly

```
              305                    310                    315                    320

              Pro Gly Ser Ser Gly Pro Gly Gly Gln Gly Pro Tyr Gly Pro Gly Ser
                          325                    330                    335

              Ser Ala Ala Ala Ala Ala Gly Gly Tyr Gly Pro Gly Gln Gln Gly Pro
                          340                    345                    350

              Tyr Gly Pro Gly Gly Ser Ala Ala Ala Ala Ala Gly Gly Tyr Gln Gln
                          355                    360                    365

              Gly Pro Gly Gly Gln Gly Pro Tyr Gly Pro Gly Ala Ser Gly Pro Gly
                          370                    375                    380

              Gly Gln Gly Pro Tyr Gly Pro Gly Ala Ser Ala Ala Ala Ala Ala Gly
              385                    390                    395                    400

              Pro Gly Gly Tyr Gly Pro Gly Gly Gln Gly Pro Ser Ala Ser Ala Ala
                          405                    410                    415

              Ala Ala Ala Gly Gly Tyr Gly Ser Gly Pro Gly Gly Tyr Gly Pro Tyr
                          420                    425                    430

              Gly Pro Gly Gly Ser Gly Pro Gly Ser Gly Gln Gln Gly Gln Gly Pro
                          435                    440                    445

              Tyr Gly Pro Gly Ala Ser Ala Ala Ala Ala Gly Gly Tyr Gly Pro
                          450                    455                    460

              Gly Gln Gln Gly Pro Tyr Gly Pro Gly Gly Ser Ala Ala Ala Ala Ala
              465                    470                    475                    480

              Gly Pro Gly Ser Gly Gly Tyr Gly Pro Gly Ala Ser Gly Gly Asn Gly
                          485                    490                    495

              Pro Gly Ser Gly Gly Tyr Gly Pro Gly Gln Gln Gly Pro Gly Gly Ser
                          500                    505                    510

              Ala Ala Ala Ala Ala Gly Gly Tyr Gln Gln Gly Pro Gly Gly Gln Gly
                          515                    520                    525

              Pro Tyr Gly Pro Gly Ala Ser Ala Ala Ala Ala Gly Gly Tyr Gly
                          530                    535                    540

              Ser Gly Pro Gly Gln Gln Gly Pro Tyr Gly Pro Gly Gly Ser Gly Ser
              545                    550                    555                    560
```

```
Gly Gln Gln Gly Pro Gly Gln Gln Gly Pro Tyr Ala Ser Ala Ala Ala
            565             570             575


Ala Ala Gly Pro Gly Ser Gly Gln Gln Gly Pro Gly Ala Ser
            580             585             590
```

```
<210>  18
<211>  601
<212>  PRT
<213>  Artificial Sequence

<220>
<223>  PRT399

<400>  18
```

```
Met His His His His His His Ser Ser Gly Ser Ser Gly Pro Gly Gly
1               5                   10              15


Gln Gly Pro Tyr Gly Pro Gly Ala Ser Ala Ala Ala Ala Ala Gly Gly
            20              25              30


Asn Gly Pro Gly Ser Gly Gln Gln Gly Pro Gly Gly Ser Gly Gly Tyr
            35              40              45


Gly Pro Gly Gly Gln Gly Pro Gly Gln Gln Gly Pro Gly Ser Ser Ala
    50              55              60


Ala Ala Ala Ala Gly Pro Gly Gly Tyr Gly Pro Gly Gly Gln Gly Pro
65              70              75              80


Ser Ala Ser Ala Ala Ala Ala Ala Gly Pro Gly Ser Gly Gln Gln Gly
            85              90              95


Pro Gly Ala Ser Gly Gly Tyr Gly Pro Gly Gly Gln Gly Pro Gly Gln
            100             105             110


Gln Gly Pro Gly Ser Ser Ala Ala Ala Ala Ala Gly Gly Tyr Gly Ser
            115             120             125


Gly Pro Gly Gln Gln Gly Pro Tyr Gly Ser Ala Ala Ala Ala Ala Gly
    130             135             140


Pro Gly Ser Gly Gly Tyr Gly Gln Gly Pro Tyr Gly Pro Gly Ala Ser
145             150             155             160


Gly Pro Gly Gly Tyr Gly Pro Gly Gly Gln Gly Pro Ser Ala Ser Ala
    165             170             175


Ala Ala Ala Gly Ser Gly Gln Gln Gly Pro Gly Gly Tyr Gly Pro
```

|  | 180 |  |  | 185 |  |  | 190 |  |
|---|---|---|---|---|---|---|---|---|

Tyr Ala Ser Ala Ala Ala Ala Ala Gly Gly Tyr Gly Ser Gly Pro Gly
195 200 205

Gln Gln Gly Pro Tyr Gly Pro Gly Gly Ser Gly Ser Gly Gln Gln Gly
210 215 220

Pro Gly Gln Gln Gly Pro Tyr Ala Ser Ala Ala Ala Ala Ala Gly Pro
225 230 235 240

Gly Gly Gln Gly Pro Tyr Gly Pro Gly Ser Ser Ala Ala Ala Ala Ala
245 250 255

Gly Gly Tyr Gly Tyr Gly Pro Gly Gly Gln Gly Pro Tyr Gly Pro Gly
260 265 270

Ala Ser Gly Gly Asn Gly Pro Gly Ser Gly Gly Tyr Gly Pro Gly Gln
275 280 285

Gln Gly Pro Gly Gly Ser Ala Ala Ala Ala Gly Pro Gly Gly Gln
290 295 300

Gly Pro Tyr Gly Pro Gly Ala Ser Ala Ala Ala Ala Ala Gly Gly Tyr
305 310 315 320

Gly Pro Gly Gly Gln Gly Pro Gly Gly Tyr Gly Pro Gly Ser Ser Gly
325 330 335

Pro Gly Gly Gln Gly Pro Tyr Gly Pro Gly Ser Ser Ala Ala Ala Ala
340 345 350

Ala Gly Gly Tyr Gly Pro Gly Gln Gln Gly Pro Tyr Gly Pro Gly Gly
355 360 365

Ser Ala Ala Ala Ala Ala Gly Gly Tyr Gln Gln Gly Pro Gly Gly Gln
370 375 380

Gly Pro Tyr Gly Pro Gly Ala Ser Gly Pro Gly Gly Gln Gly Pro Tyr
385 390 395 400

Gly Pro Gly Ala Ser Ala Ala Ala Ala Ala Gly Pro Gly Gly Tyr Gly
405 410 415

Pro Gly Gly Gln Gly Pro Ser Ala Ser Ala Ala Ala Ala Ala Gly Gly
420 425 430

```
Tyr Gly Ser Gly Pro Gly Gly Tyr Gly Pro Tyr Gly Pro Gly Gly Ser
        435             440             445

Gly Pro Gly Ser Gly Gln Gln Gly Gln Gly Pro Tyr Gly Pro Gly Ala
    450             455             460

Ser Ala Ala Ala Ala Ala Gly Gly Tyr Gly Pro Gly Gln Gln Gly Pro
465             470             475             480

Tyr Gly Pro Gly Gly Ser Ala Ala Ala Ala Gly Pro Gly Ser Gly
        485             490             495

Gly Tyr Gly Pro Gly Ala Ser Gly Gly Asn Gly Pro Gly Ser Gly Gly
        500             505             510

Tyr Gly Pro Gly Gln Gln Gly Pro Gly Gly Ser Ala Ala Ala Ala Ala
        515             520             525

Gly Gly Tyr Gln Gln Gly Pro Gly Gly Gln Gly Pro Tyr Gly Pro Gly
    530             535             540

Ala Ser Ala Ala Ala Ala Gly Gly Tyr Gly Ser Gly Pro Gly Gln
545             550             555             560

Gln Gly Pro Tyr Gly Pro Gly Gly Ser Gly Ser Gly Gln Gln Gly Pro
        565             570             575

Gly Gln Gln Gly Pro Tyr Ala Ser Ala Ala Ala Ala Ala Gly Pro Gly
        580             585             590

Ser Gly Gln Gln Gly Pro Gly Ala Ser
        595             600
```

<210> 19
<211> 612
<212> PRT
<213> Artificial Sequence

<220>
<223> Met-PRT720

<400> 19

```
Met Gly Pro Gly Gln Gln Gly Pro Tyr Gly Pro Gly Ala Ser Ala Ala
1               5               10              15

Ala Ala Ala Gly Gln Asn Gly Pro Gly Ser Gly Gln Gln Gly Pro Gly
            20              25              30

Gln Ser Gly Gln Tyr Gly Pro Gly Gln Gln Gly Pro Gly Gln Gln Gly
```

211

| | | 35 | | | | | | 40 | | | | | | 45 | |

Pro Gly Ser Ser Ala Ala Ala Ala Ala Gly Pro Gly Gln Tyr Val Leu
    50               55              60

Ile Gly Pro Gly Gln Gln Val Leu Ile Gly Pro Ser Ala Ser Ala Ala
65             70             75              80

Ala Ala Ala Gly Pro Gly Ser Gly Gln Gln Gly Pro Gly Ala Ser Gly
          85            90             95

Gln Tyr Gly Pro Gly Gln Gln Gly Pro Gly Gln Gln Gly Pro Gly Ser
        100            105            110

Ser Ala Ala Ala Ala Ala Gly Ser Tyr Gly Ser Val Leu Ile Gly Pro
       115             120            125

Gly Gln Gln Val Leu Ile Gly Pro Tyr Gly Ser Ala Ala Ala Ala Ala
       130             135            140

Gly Pro Gly Ser Gly Gln Tyr Gly Gln Gly Pro Tyr Gly Pro Gly Ala
145           150           155           160

Ser Gly Pro Gly Gln Tyr Gly Pro Gly Gln Gln Gly Pro Ser Ala Ser
        165            170            175

Ala Ala Ala Ala Ala Gly Ser Gly Gln Gln Val Leu Ile Gly Pro Gly
       180             185           190

Gln Tyr Val Leu Ile Gly Pro Tyr Ala Ser Ala Ala Ala Ala Ala Gly
       195             200           205

Gln Tyr Gly Ser Gly Pro Gly Gln Gln Gly Pro Tyr Gly Pro Gly Gln
       210             215           220

Ser Gly Ser Gly Gln Gln Gly Pro Gly Gln Gln Gly Pro Tyr Ala Ser
225           230           235           240

Ala Ala Ala Ala Ala Gly Pro Gly Gln Gln Val Leu Ile Gly Pro Tyr
       245             250           255

Val Leu Ile Gly Pro Gly Ser Ser Ala Ala Ala Ala Gly Gln Tyr
       260             265           270

Gly Tyr Gly Pro Gly Gln Gln Gly Pro Tyr Gly Pro Gly Ala Ser Gly
       275             280           285

```
Gln Asn Gly Pro Gly Ser Gly Gln Tyr Gly Pro Gly Gln Gln Gly Pro
    290                 295             300

Gly Gln Ser Ala Ala Ala Ala Ala Gly Pro Gly Gln Gln Val Leu Ile
305             310             315                 320

Gly Pro Tyr Val Leu Ile Gly Pro Gly Ala Ser Ala Ala Ala Ala Ala
            325             330                 335

Gly Gln Tyr Gly Pro Gly Gln Gln Gly Pro Gly Gln Tyr Gly Pro Gly
        340             345             350

Ser Ser Gly Pro Gly Gln Gln Gly Pro Tyr Gly Pro Gly Ser Ser Ala
        355             360             365

Ala Ala Ala Ala Gly Ser Tyr Gly Pro Gly Gln Gln Val Leu Ile Gly
    370             375                 380

Pro Tyr Val Leu Ile Gly Pro Gly Pro Ser Ala Ala Ala Ala Ala Gly
385             390                 395             400

Gln Tyr Gln Gln Gly Pro Gly Gln Gln Gly Pro Tyr Gly Pro Gly Ala
            405             410             415

Ser Gly Pro Gly Gln Gln Gly Pro Tyr Gly Pro Gly Ala Ser Ala Ala
        420             425             430

Ala Ala Ala Gly Pro Gly Gln Tyr Val Leu Ile Gly Pro Gly Gln Gln
    435             440             445

Val Leu Ile Gly Pro Ser Ala Ser Ala Ala Ala Ala Gly Gln Tyr
    450             455             460

Gly Ser Gly Pro Gly Gln Tyr Gly Pro Tyr Gly Pro Gly Gln Ser Gly
465             470             475                 480

Pro Gly Ser Gly Gln Gln Gly Gln Gly Pro Tyr Gly Pro Gly Ala Ser
            485             490             495

Ala Ala Ala Ala Ala Gly Ser Tyr Gly Pro Gly Gln Gln Val Leu Ile
        500             505                 510

Gly Pro Tyr Val Leu Ile Gly Pro Gly Pro Ser Ala Ala Ala Ala Ala
        515             520             525

Gly Pro Gly Ser Gly Gln Tyr Gly Pro Gly Ala Ser Gly Gln Asn Gly
    530             535             540
```

```
Pro Gly Ser Gly Gln Tyr Gly Pro Gly Gln Gln Gly Pro Gly Gln Ser
545             550             555             560

Ala Ala Ala Ala Ala Gly Gln Tyr Gln Gln Val Leu Ile Gly Pro Gly
            565             570             575

Gln Gln Gly Pro Tyr Val Leu Ile Gly Pro Gly Ala Ser Ala Ala Ala
            580             585             590

Ala Ala Gly Pro Gly Ser Gly Gln Gln Val Leu Ile Gly Pro Gly Ala
            595             600             605

Ser Val Leu Ile
        610


<210>  20
<211>  592
<212>  PRT
<213>  Artificial Sequence

<220>
<223>  Met-PRT665

<400>  20

Met Gly Pro Gly Gln Gln Gly Pro Tyr Gly Pro Gly Ala Ser Ala Ala
1               5               10              15

Ala Ala Ala Ala Ala Gly Ser Asn Gly Pro Gly Ser Gly Gln Gln Gly
            20              25              30

Pro Gly Gln Ser Gly Gln Tyr Gly Pro Gly Gln Gln Gly Pro Gly Gln
            35              40              45

Gln Gly Pro Gly Ser Ser Ala Ala Ala Ala Ala Ala Gly Pro Gly
        50              55              60

Gln Tyr Val Leu Ile Gly Pro Gly Gln Gln Gly Pro Ser Ala Ser Ala
65              70              75              80

Ala Ala Ala Ala Ala Gly Pro Gly Ser Gly Gln Gln Gly Pro Gly
            85              90              95

Ala Ser Gly Gln Tyr Gly Pro Gly Gln Gln Gly Pro Gly Gln Gln Gly
            100             105             110

Pro Gly Ser Ser Ala Ala Ala Ala Ala Ala Ala Gly Ser Tyr Gly Ser
            115             120             125
```

Val Leu Ile Gly Pro Gly Gln Gln Gly Pro Tyr Gly Ser Ala Ala Ala
130                     135                 140

Ala Ala Ala Ala Gly Pro Gly Ser Gly Gln Tyr Gly Gln Gly Pro Tyr
145                 150                 155                 160

Gly Pro Gly Ala Ser Gly Pro Gly Gln Tyr Gly Pro Gly Gln Gln Gly
                165                 170                 175

Pro Ser Ala Ser Ala Ala Ala Ala Ala Ala Ala Gly Ser Gly Gln Gln
                180                 185                 190

Val Leu Ile Gly Pro Gly Gln Tyr Gly Pro Tyr Ala Ser Ala Ala Ala
            195                 200                 205

Ala Ala Ala Ala Gly Ser Tyr Gly Ser Gly Pro Gly Gln Gln Gly Pro
    210                 215                 220

Tyr Gly Pro Gly Gln Ser Gly Ser Gly Gln Gln Gly Pro Gly Gln Gln
225             230                 235                 240

Gly Pro Tyr Ala Ser Ala Ala Ala Ala Ala Ala Gly Pro Gly Gln
            245                 250                 255

Gln Val Leu Ile Gly Pro Tyr Gly Pro Gly Ser Ser Ala Ala Ala Ala
            260                 265                 270

Ala Ala Ala Gly Ser Tyr Gly Tyr Gly Pro Gly Gln Gln Gly Pro Tyr
    275                 280                 285

Gly Pro Gly Ala Ser Gly Gln Asn Gly Pro Gly Ser Gly Gln Tyr Gly
    290                 295                 300

Pro Gly Gln Gln Gly Pro Gly Pro Ser Ala Ala Ala Ala Ala Ala Ala
305             310                 315                 320

Gly Pro Gly Gln Gln Val Leu Ile Gly Pro Tyr Gly Pro Gly Ala Ser
            325                 330                 335

Ala Ala Ala Ala Ala Ala Ala Gly Ser Tyr Gly Pro Gly Gln Gln Gly
    340                 345                 350

Pro Gly Gln Tyr Gly Pro Gly Ser Ser Gly Pro Gly Gln Gln Gly Pro
    355                 360                 365

Tyr Gly Pro Gly Ser Ser Ala Ala Ala Ala Ala Ala Ala Gly Ser Tyr
370             375                 380

```
Gly Pro Gly Gln Gln Val Leu Ile Gly Pro Tyr Gly Pro Gly Pro Ser
385             390             395                 400

Ala Ala Ala Ala Ala Ala Ala Gly Ser Tyr Gln Gln Gly Pro Gly Gln
        405             410                 415

Gln Gly Pro Tyr Gly Pro Gly Ala Ser Gly Pro Gly Gln Gln Gly Pro
        420             425                 430

Tyr Gly Pro Gly Ala Ser Ala Ala Ala Ala Ala Ala Ala Gly Pro Gly
        435             440                 445

Gln Tyr Val Leu Ile Gly Pro Gly Gln Gln Gly Pro Ser Ala Ser Ala
    450             455                 460

Ala Ala Ala Ala Ala Ala Gly Ser Tyr Gly Ser Gly Pro Gly Gln Tyr
465             470                 475                 480

Gly Pro Tyr Gly Pro Gly Gln Ser Gly Pro Gly Ser Gly Gln Gln Gly
            485             490                 495

Gln Gly Pro Tyr Gly Pro Gly Ala Ser Ala Ala Ala Ala Ala Ala Ala
        500             505                 510

Gly Ser Tyr Gly Pro Gly Gln Gln Val Leu Ile Gly Pro Tyr Gly Pro
    515             520                 525

Gly Pro Ser Ala Ala Ala Ala Ala Ala Ala Gly Pro Gly Ser Gly Gln
    530             535                 540

Tyr Gly Pro Gly Ala Ser Gly Gln Asn Gly Pro Gly Ser Gly Gln Tyr
545             550                 555                 560

Gly Pro Gly Gln Gln Gly Pro Gly Pro Ser Ala Ala Ala Ala Ala Ala
            565             570                 575

Ala Gly Pro Gly Ser Gly Gln Gln Gly Pro Gly Ala Ser Val Leu Ile
            580             585                 590


<210>  21
<211>  619
<212>  PRT
<213>  Artificial Sequence

<220>
<223>  Met-PRT666

<400>  21
```

Met Gly Pro Gly Gln Gln Gly Pro Tyr Gly Pro Gly Ala Ser Ala Ala
1                   5                   10                  15

Ala Ala Ala Ala Ala Gly Ser Asn Gly Pro Gly Ser Gly Gln Gln Gly
            20                  25                  30

Pro Gly Gln Ser Gly Gln Tyr Gly Pro Gly Gln Gln Gly Pro Gly Gln
            35                  40                  45

Gln Gly Pro Gly Ser Ser Ala Ala Ala Ala Ala Ala Gly Pro Gly
            50                  55                  60

Gln Tyr Val Leu Ile Gly Pro Gly Gln Gln Val Leu Ile Gly Pro Ser
65                  70                  75                  80

Ala Ser Ala Ala Ala Ala Ala Ala Ala Gly Pro Gly Ser Gly Gln Gln
            85                  90                  95

Gly Pro Gly Ala Ser Gly Gln Tyr Gly Pro Gly Gln Gln Gly Pro Gly
            100                 105                 110

Gln Gln Gly Pro Gly Ser Ser Ala Ala Ala Ala Ala Ala Gly Ser
            115                 120                 125

Tyr Gly Ser Val Leu Ile Gly Pro Gly Gln Gln Val Leu Ile Gly Pro
    130                 135                 140

Tyr Gly Ser Ala Ala Ala Ala Ala Ala Gly Pro Gly Ser Gly Gln
145                 150                 155                 160

Tyr Gly Gln Gly Pro Tyr Gly Pro Gly Ala Ser Gly Pro Gly Gln Tyr
            165                 170                 175

Gly Pro Gly Gln Gln Gly Pro Ser Ala Ser Ala Ala Ala Ala Ala
            180                 185                 190

Ala Gly Ser Gly Gln Gln Val Leu Ile Gly Pro Gly Gln Tyr Val Leu
    195                 200                 205

Ile Gly Pro Tyr Ala Ser Ala Ala Ala Ala Ala Ala Gly Ser Tyr
    210                 215                 220

Gly Ser Gly Pro Gly Gln Gln Gly Pro Tyr Gly Pro Gly Gln Ser Gly
225                 230                 235                 240

Ser Gly Gln Gln Gly Pro Gly Gln Gln Gly Pro Tyr Ala Ser Ala Ala
            245                 250                 255

```
Ala Ala Ala Ala Ala Gly Pro Gly Gln Gln Val Leu Ile Gly Pro Tyr
            260             265             270

Val Leu Ile Gly Pro Gly Ser Ser Ala Ala Ala Ala Ala Ala Gly
        275             280             285

Ser Tyr Gly Tyr Gly Pro Gly Gln Gln Gly Pro Tyr Gly Pro Gly Ala
    290             295             300

Ser Gly Gln Asn Gly Pro Gly Ser Gly Gln Tyr Gly Pro Gly Gln Gln
305             310             315             320

Gly Pro Gly Pro Ser Ala Ala Ala Ala Ala Ala Gly Pro Gly Gln
            325             330             335

Gln Val Leu Ile Gly Pro Tyr Val Leu Ile Gly Pro Gly Ala Ser Ala
        340             345             350

Ala Ala Ala Ala Ala Gly Ser Tyr Gly Pro Gly Gln Gln Gly Pro
    355             360             365

Gly Gln Tyr Gly Pro Gly Ser Ser Gly Pro Gly Gln Gln Gly Pro Tyr
    370             375             380

Gly Pro Gly Ser Ser Ala Ala Ala Ala Ala Ala Gly Ser Tyr Gly
385             390             395             400

Pro Gly Gln Gln Val Leu Ile Gly Pro Tyr Val Leu Ile Gly Pro Gly
            405             410             415

Pro Ser Ala Ala Ala Ala Ala Ala Gly Ser Tyr Gln Gln Gly Pro
        420             425             430

Gly Gln Gln Gly Pro Tyr Gly Pro Gly Ala Ser Gly Pro Gly Gln Gln
    435             440             445

Gly Pro Tyr Gly Pro Gly Ala Ser Ala Ala Ala Ala Ala Ala Gly
    450             455             460

Pro Gly Gln Tyr Val Leu Ile Gly Pro Gly Gln Gln Val Leu Ile Gly
465             470             475             480

Pro Ser Ala Ser Ala Ala Ala Ala Ala Ala Gly Ser Tyr Gly Ser
            485             490             495

Gly Pro Gly Gln Tyr Gly Pro Tyr Gly Pro Gly Gln Ser Gly Pro Gly
        500             505             510
```

Ser Gly Gln Gln Gly Gln Gly Pro Tyr Gly Pro Gly Ala Ser Ala Ala
    515              520            525

Ala Ala Ala Ala Ala Gly Ser Tyr Gly Pro Gly Gln Gln Val Leu Ile
    530              535            540

Gly Pro Tyr Val Leu Ile Gly Pro Gly Pro Ser Ala Ala Ala Ala Ala
545             550            555            560

Ala Ala Gly Pro Gly Ser Gly Gln Tyr Gly Pro Gly Ala Ser Gly Gln
        565            570            575

Asn Gly Pro Gly Ser Gly Gln Tyr Gly Pro Gly Gln Gln Gly Pro Gly
      580            585            590

Pro Ser Ala Ala Ala Ala Ala Ala Ala Gly Pro Gly Ser Gly Gln Gln
      595            600            605

Val Leu Ile Gly Pro Gly Ala Ser Val Leu Ile
    610            615


<210> 22
<211> 623
<212> PRT
<213> Artificial Sequence

<220>
<223> PRT720

<400> 22

Met His His His His His His Ser Ser Gly Ser Ser Gly Pro Gly Gln
1              5             10            15

Gln Gly Pro Tyr Gly Pro Gly Ala Ser Ala Ala Ala Ala Gly Gln
        20            25            30

Asn Gly Pro Gly Ser Gly Gln Gln Gly Pro Gly Gln Ser Gly Gln Tyr
      35            40            45

Gly Pro Gly Gln Gln Gly Pro Gly Gln Gln Gly Pro Gly Ser Ser Ala
    50            55            60

Ala Ala Ala Ala Gly Pro Gly Gln Tyr Val Leu Ile Gly Pro Gly Gln
65             70            75            80

Gln Val Leu Ile Gly Pro Ser Ala Ser Ala Ala Ala Ala Ala Gly Pro
        85            90            95

```
Gly Ser Gly Gln Gln Gly Pro Gly Ala Ser Gly Gln Tyr Gly Pro Gly
        100             105             110

Gln Gln Gly Pro Gly Gln Gln Gly Pro Gly Ser Ser Ala Ala Ala Ala
        115             120             125

Ala Gly Ser Tyr Gly Ser Val Leu Ile Gly Pro Gly Gln Gln Val Leu
        130             135             140

Ile Gly Pro Tyr Gly Ser Ala Ala Ala Ala Ala Gly Pro Gly Ser Gly
145             150             155             160

Gln Tyr Gly Gln Gly Pro Tyr Gly Pro Gly Ala Ser Gly Pro Gly Gln
        165             170             175

Tyr Gly Pro Gly Gln Gln Gly Pro Ser Ala Ser Ala Ala Ala Ala Ala
        180             185             190

Gly Ser Gly Gln Gln Val Leu Ile Gly Pro Gly Gln Tyr Val Leu Ile
        195             200             205

Gly Pro Tyr Ala Ser Ala Ala Ala Ala Ala Gly Gln Tyr Gly Ser Gly
        210             215             220

Pro Gly Gln Gln Gly Pro Tyr Gly Pro Gly Gln Ser Gly Ser Gly Gln
225             230             235             240

Gln Gly Pro Gly Gln Gln Gly Pro Tyr Ala Ser Ala Ala Ala Ala Ala
        245             250             255

Gly Pro Gly Gln Gln Val Leu Ile Gly Pro Tyr Val Leu Ile Gly Pro
        260             265             270

Gly Ser Ser Ala Ala Ala Ala Ala Gly Gln Tyr Gly Tyr Gly Pro Gly
        275             280             285

Gln Gln Gly Pro Tyr Gly Pro Gly Ala Ser Gly Gln Asn Gly Pro Gly
        290             295             300

Ser Gly Gln Tyr Gly Pro Gly Gln Gln Gly Pro Gly Gln Ser Ala Ala
305             310             315             320

Ala Ala Ala Gly Pro Gly Gln Gln Val Leu Ile Gly Pro Tyr Val Leu
        325             330             335

Ile Gly Pro Gly Ala Ser Ala Ala Ala Ala Ala Gly Gln Tyr Gly Pro
        340             345             350
```

Gly Gln Gln Gly Pro Gly Gln Tyr Gly Pro Gly Ser Ser Gly Pro Gly
355 360 365

Gln Gln Gly Pro Tyr Gly Pro Gly Ser Ser Ala Ala Ala Ala Ala Gly
370 375 380

Ser Tyr Gly Pro Gly Gln Gln Val Leu Ile Gly Pro Tyr Val Leu Ile
385 390 395 400

Gly Pro Gly Pro Ser Ala Ala Ala Ala Ala Gly Gln Tyr Gln Gln Gly
405 410 415

Pro Gly Gln Gln Gly Pro Tyr Gly Pro Gly Ala Ser Gly Pro Gly Gln
420 425 430

Gln Gly Pro Tyr Gly Pro Gly Ala Ser Ala Ala Ala Ala Gly Pro
435 440 445

Gly Gln Tyr Val Leu Ile Gly Pro Gly Gln Gln Val Leu Ile Gly Pro
450 455 460

Ser Ala Ser Ala Ala Ala Ala Ala Gly Gln Tyr Gly Ser Gly Pro Gly
465 470 475 480

Gln Tyr Gly Pro Tyr Gly Pro Gly Gln Ser Gly Pro Gly Ser Gly Gln
485 490 495

Gln Gly Gln Gly Pro Tyr Gly Pro Gly Ala Ser Ala Ala Ala Ala Ala
500 505 510

Gly Ser Tyr Gly Pro Gly Gln Gln Val Leu Ile Gly Pro Tyr Val Leu
515 520 525

Ile Gly Pro Gly Pro Ser Ala Ala Ala Ala Gly Pro Gly Ser Gly
530 535 540

Gln Tyr Gly Pro Gly Ala Ser Gly Gln Asn Gly Pro Gly Ser Gly Gln
545 550 555 560

Tyr Gly Pro Gly Gln Gln Gly Pro Gly Gln Ser Ala Ala Ala Ala Ala
565 570 575

Gly Gln Tyr Gln Gln Val Leu Ile Gly Pro Gly Gln Gln Gly Pro Tyr
580 585 590

Val Leu Ile Gly Pro Gly Ala Ser Ala Ala Ala Ala Ala Gly Pro Gly

```
                595                    600                    605


          Ser Gly Gln Gln Val Leu Ile Gly Pro Gly Ala Ser Val Leu Ile
              610                    615                    620


<210>  23
<211>  603
<212>  PRT
<213>  Artificial Sequence

<220>
<223>  PRT665

<400>  23

Met His His His His His His Ser Ser Gly Ser Ser Gly Pro Gly Gln
1               5                   10                  15


Gln Gly Pro Tyr Gly Pro Gly Ala Ser Ala Ala Ala Ala Ala Ala Ala
            20                  25                  30


Gly Ser Asn Gly Pro Gly Ser Gly Gln Gln Gly Pro Gly Gln Ser Gly
            35                  40                  45


Gln Tyr Gly Pro Gly Gln Gln Gly Pro Gly Gln Gln Gly Pro Gly Ser
            50                  55                  60


Ser Ala Ala Ala Ala Ala Ala Ala Gly Pro Gly Gln Tyr Val Leu Ile
65                  70                  75                  80


Gly Pro Gly Gln Gln Gly Pro Ser Ala Ser Ala Ala Ala Ala Ala Ala
                85                  90                  95


Ala Gly Pro Gly Ser Gly Gln Gln Gly Pro Gly Ala Ser Gly Gln Tyr
            100                 105                 110


Gly Pro Gly Gln Gln Gly Pro Gly Gln Gln Gly Pro Gly Ser Ser Ala
            115                 120                 125


Ala Ala Ala Ala Ala Gly Ser Tyr Gly Ser Val Leu Ile Gly Pro
    130                 135                 140


Gly Gln Gln Gly Pro Tyr Gly Ser Ala Ala Ala Ala Ala Ala Ala Gly
145                 150                 155                 160


Pro Gly Ser Gly Gln Tyr Gly Gln Gly Pro Tyr Gly Pro Gly Ala Ser
                165                 170                 175


Gly Pro Gly Gln Tyr Gly Pro Gly Gln Gln Gly Pro Ser Ala Ser Ala
            180                 185                 190
```

Ala Ala Ala Ala Ala Ala Gly Ser Gly Gln Gln Val Leu Ile Gly Pro
            195                 200                 205

Gly Gln Tyr Gly Pro Tyr Ala Ser Ala Ala Ala Ala Ala Ala Ala Gly
            210                 215                 220

Ser Tyr Gly Ser Gly Pro Gly Gln Gln Gly Pro Tyr Gly Pro Gly Gln
225                 230                 235                 240

Ser Gly Ser Gly Gln Gln Gly Pro Gly Gln Gln Gly Pro Tyr Ala Ser
            245                 250                 255

Ala Ala Ala Ala Ala Ala Ala Gly Pro Gly Gln Gln Val Leu Ile Gly
            260                 265                 270

Pro Tyr Gly Pro Gly Ser Ser Ala Ala Ala Ala Ala Ala Ala Gly Ser
            275                 280                 285

Tyr Gly Tyr Gly Pro Gly Gln Gln Gly Pro Tyr Gly Pro Gly Ala Ser
            290                 295                 300

Gly Gln Asn Gly Pro Gly Ser Gly Gln Tyr Gly Pro Gly Gln Gln Gly
305                 310                 315                 320

Pro Gly Pro Ser Ala Ala Ala Ala Ala Ala Gly Pro Gly Gln Gln
            325                 330                 335

Val Leu Ile Gly Pro Tyr Gly Pro Gly Ala Ser Ala Ala Ala Ala Ala
            340                 345                 350

Ala Ala Gly Ser Tyr Gly Pro Gly Gln Gln Gly Pro Gly Gln Tyr Gly
            355                 360                 365

Pro Gly Ser Ser Gly Pro Gly Gln Gln Gly Pro Tyr Gly Pro Gly Ser
            370                 375                 380

Ser Ala Ala Ala Ala Ala Ala Ala Gly Ser Tyr Gly Pro Gly Gln Gln
385                 390                 395                 400

Val Leu Ile Gly Pro Tyr Gly Pro Gly Pro Ser Ala Ala Ala Ala Ala
            405                 410                 415

Ala Ala Gly Ser Tyr Gln Gln Gly Pro Gly Gln Gln Gly Pro Tyr Gly
            420                 425                 430

Pro Gly Ala Ser Gly Pro Gly Gln Gln Gly Pro Tyr Gly Pro Gly Ala

```
                435                    440                    445


        Ser Ala Ala Ala Ala Ala Ala Ala Gly Pro Gly Gln Tyr Val Leu Ile
            450                 455                 460


        Gly Pro Gly Gln Gln Gly Pro Ser Ala Ser Ala Ala Ala Ala Ala Ala
        465                 470                 475                 480


        Ala Gly Ser Tyr Gly Ser Gly Pro Gly Gln Tyr Gly Pro Tyr Gly Pro
                    485                 490                 495


        Gly Gln Ser Gly Pro Gly Ser Gly Gln Gln Gly Gln Gly Pro Tyr Gly
                500                 505                 510


        Pro Gly Ala Ser Ala Ala Ala Ala Ala Ala Ala Gly Ser Tyr Gly Pro
                515                 520                 525


        Gly Gln Gln Val Leu Ile Gly Pro Tyr Gly Pro Gly Pro Ser Ala Ala
                530                 535                 540


        Ala Ala Ala Ala Ala Gly Pro Gly Ser Gly Gln Tyr Gly Pro Gly Ala
        545                 550                 555                 560


        Ser Gly Gln Asn Gly Pro Gly Ser Gly Gln Tyr Gly Pro Gly Gln Gln
                    565                 570                 575


        Gly Pro Gly Pro Ser Ala Ala Ala Ala Ala Ala Ala Gly Pro Gly Ser
                    580                 585                 590


        Gly Gln Gln Gly Pro Gly Ala Ser Val Leu Ile
                595                 600


        <210>  24
        <211>  630
        <212>  PRT
        <213>  Artificial Sequence

        <220>
        <223>  PRT666

        <400>  24

        Met His His His His His His Ser Ser Gly Ser Ser Gly Pro Gly Gln
        1                   5                   10                  15


        Gln Gly Pro Tyr Gly Pro Gly Ala Ser Ala Ala Ala Ala Ala Ala Ala
                    20                  25                  30


        Gly Ser Asn Gly Pro Gly Ser Gly Gln Gln Gly Pro Gly Gln Ser Gly
                35                  40                  45
```

Gln Tyr Gly Pro Gly Gln Gln Gly Pro Gly Gln Gln Gly Pro Gly Ser
        50                  55                  60

Ser Ala Ala Ala Ala Ala Ala Ala Gly Pro Gly Gln Tyr Val Leu Ile
65                  70                  75                  80

Gly Pro Gly Gln Gln Val Leu Ile Gly Pro Ser Ala Ser Ala Ala Ala
                85                  90                  95

Ala Ala Ala Ala Gly Pro Gly Ser Gly Gln Gln Gly Pro Gly Ala Ser
            100                 105                 110

Gly Gln Tyr Gly Pro Gly Gln Gln Gly Pro Gly Gln Gln Gly Pro Gly
        115                 120                 125

Ser Ser Ala Ala Ala Ala Ala Ala Gly Ser Tyr Gly Ser Val Leu
    130                 135                 140

Ile Gly Pro Gly Gln Gln Val Leu Ile Gly Pro Tyr Gly Ser Ala Ala
145                 150                 155                 160

Ala Ala Ala Ala Ala Gly Pro Gly Ser Gly Gln Tyr Gly Gln Gly Pro
                165                 170                 175

Tyr Gly Pro Gly Ala Ser Gly Pro Gly Gln Tyr Gly Pro Gly Gln Gln
            180                 185                 190

Gly Pro Ser Ala Ser Ala Ala Ala Ala Ala Ala Gly Ser Gly Gln
            195                 200                 205

Gln Val Leu Ile Gly Pro Gly Gln Tyr Val Leu Ile Gly Pro Tyr Ala
    210                 215                 220

Ser Ala Ala Ala Ala Ala Ala Ala Gly Ser Tyr Gly Ser Gly Pro Gly
225                 230                 235                 240

Gln Gln Gly Pro Tyr Gly Pro Gly Gln Ser Gly Ser Gly Gln Gln Gly
            245                 250                 255

Pro Gly Gln Gln Gly Pro Tyr Ala Ser Ala Ala Ala Ala Ala Ala Ala
            260                 265                 270

Gly Pro Gly Gln Gln Val Leu Ile Gly Pro Tyr Val Leu Ile Gly Pro
        275                 280                 285

Gly Ser Ser Ala Ala Ala Ala Ala Ala Ala Gly Ser Tyr Gly Tyr Gly

225

290                    295                    300

Pro Gly Gln Gln Gly Pro Tyr Gly Pro Gly Ala Ser Gly Gln Asn Gly
305               310               315               320

Pro Gly Ser Gly Gln Tyr Gly Pro Gly Gln Gln Gly Pro Gly Pro Ser
               325               330               335

Ala Ala Ala Ala Ala Ala Ala Gly Pro Gly Gln Gln Val Leu Ile Gly
          340               345               350

Pro Tyr Val Leu Ile Gly Pro Gly Ala Ser Ala Ala Ala Ala Ala Ala
     355               360               365

Ala Gly Ser Tyr Gly Pro Gly Gln Gln Gly Pro Gly Gln Tyr Gly Pro
     370               375               380

Gly Ser Ser Gly Pro Gly Gln Gln Gly Pro Tyr Gly Pro Gly Ser Ser
385               390               395               400

Ala Ala Ala Ala Ala Ala Ala Gly Ser Tyr Gly Pro Gly Gln Gln Val
          405               410               415

Leu Ile Gly Pro Tyr Val Leu Ile Gly Pro Gly Pro Ser Ala Ala Ala
          420               425               430

Ala Ala Ala Ala Gly Ser Tyr Gln Gln Gly Pro Gly Gln Gln Gly Pro
     435               440               445

Tyr Gly Pro Gly Ala Ser Gly Pro Gly Gln Gln Gly Pro Tyr Gly Pro
     450               455               460

Gly Ala Ser Ala Ala Ala Ala Ala Ala Gly Pro Gly Gln Tyr Val
465               470               475               480

Leu Ile Gly Pro Gly Gln Gln Val Leu Ile Gly Pro Ser Ala Ser Ala
          485               490               495

Ala Ala Ala Ala Ala Gly Ser Tyr Gly Ser Gly Pro Gly Gln Tyr
          500               505               510

Gly Pro Tyr Gly Pro Gly Gln Ser Gly Pro Gly Ser Gly Gln Gln Gly
     515               520               525

Gln Gly Pro Tyr Gly Pro Gly Ala Ser Ala Ala Ala Ala Ala Ala Ala
     530               535               540

226

```
Gly Ser Tyr Gly Pro Gly Gln Gln Val Leu Ile Gly Pro Tyr Val Leu
545             550             555             560

Ile Gly Pro Gly Pro Ser Ala Ala Ala Ala Ala Ala Gly Pro Gly
            565             570             575

Ser Gly Gln Tyr Gly Pro Gly Ala Ser Gly Gln Asn Gly Pro Gly Ser
            580             585             590

Gly Gln Tyr Gly Pro Gly Gln Gln Gly Pro Gly Pro Ser Ala Ala Ala
        595             600             605

Ala Ala Ala Ala Gly Pro Gly Ser Gly Gln Gln Val Leu Ile Gly Pro
    610             615             620

Gly Ala Ser Val Leu Ile
625             630
```

```
<210>   25
<211>   593
<212>   PRT
<213>   Artificial Sequence

<220>
<223>   Met-PRT888

<400>   25
```

```
Met Gly Ser Ser Gly Pro Gly Val Leu Gly Pro Tyr Gly Pro Gly Ala
1               5               10              15

Ser Ala Ala Ala Ala Ala Gly Gln Asn Gly Pro Gly Ser Gly Val Leu
            20              25              30

Gly Pro Gly Gln Ser Gly Gln Tyr Gly Pro Gly Val Leu Gly Pro Gly
            35              40              45

Val Leu Gly Pro Gly Ser Ser Ala Ala Ala Ala Ala Gly Pro Gly Gln
        50              55              60

Tyr Gly Pro Gly Val Leu Gly Pro Ser Ala Ser Ala Ala Ala Ala Ala
65              70              75              80

Gly Pro Gly Ser Gly Val Leu Gly Pro Gly Ala Ser Gly Gln Tyr Gly
            85              90              95

Pro Gly Val Leu Gly Pro Gly Val Leu Gly Pro Gly Ser Ser Ala Ala
            100             105             110

Ala Ala Ala Gly Gln Tyr Gly Ser Gly Pro Gly Val Leu Gly Pro Tyr
```

227

|     | 115 |     |     |     | 120 |     |     |     | 125 |     |     |     |     |
|-----|-----|-----|-----|-----|-----|-----|-----|-----|-----|-----|-----|-----|-----|

Gly Ser Ala Ala Ala Ala Ala Gly Pro Gly Ser Gly Gln Tyr Gly Gln
130 135 140

Gly Pro Tyr Gly Pro Gly Ala Ser Gly Pro Gly Gln Tyr Gly Pro Gly
145 150 155 160

Val Leu Gly Pro Ser Ala Ser Ala Ala Ala Ala Ala Gly Ser Gly Val
165 170 175

Leu Gly Pro Gly Gln Tyr Gly Pro Tyr Ala Ser Ala Ala Ala Ala Ala
180 185 190

Gly Gln Tyr Gly Ser Gly Pro Gly Val Leu Gly Pro Tyr Gly Pro Gly
195 200 205

Gln Ser Gly Ser Gly Val Leu Gly Pro Gly Val Leu Gly Pro Tyr Ala
210 215 220

Ser Ala Ala Ala Ala Ala Gly Pro Gly Val Leu Gly Pro Tyr Gly Pro
225 230 235 240

Gly Ser Ser Ala Ala Ala Ala Ala Gly Gln Tyr Gly Tyr Gly Pro Gly
245 250 255

Val Leu Gly Pro Tyr Gly Pro Gly Ala Ser Gly Gln Asn Gly Pro Gly
260 265 270

Ser Gly Gln Tyr Gly Pro Gly Val Leu Gly Pro Gly Gln Ser Ala Ala
275 280 285

Ala Ala Ala Gly Pro Gly Val Leu Gly Pro Tyr Gly Pro Gly Ala Ser
290 295 300

Ala Ala Ala Ala Ala Gly Gln Tyr Gly Pro Gly Val Leu Gly Pro Gly
305 310 315 320

Gln Tyr Gly Pro Gly Ser Ser Gly Pro Gly Val Leu Gly Pro Tyr Gly
325 330 335

Pro Gly Ser Ser Ala Ala Ala Ala Ala Gly Gln Tyr Gly Pro Gly Val
340 345 350

Leu Gly Pro Tyr Gly Pro Gly Gln Ser Ala Ala Ala Ala Ala Gly Gln
355 360 365

Tyr Val Leu Gly Pro Gly Val Leu Gly Pro Tyr Gly Pro Gly Ala Ser
    370             375             380

Gly Pro Gly Val Leu Gly Pro Tyr Gly Pro Gly Ala Ser Ala Ala Ala
    385             390             395             400

Ala Ala Gly Pro Gly Gln Tyr Gly Pro Gly Val Leu Gly Pro Ser Ala
            405             410             415

Ser Ala Ala Ala Ala Ala Gly Gln Tyr Gly Ser Gly Pro Gly Gln Tyr
        420             425             430

Gly Pro Tyr Gly Pro Gly Gln Ser Gly Pro Gly Ser Gly Val Leu Gly
        435             440             445

Gln Gly Pro Tyr Gly Pro Gly Ala Ser Ala Ala Ala Ala Ala Gly Gln
    450             455             460

Tyr Gly Pro Gly Val Leu Gly Pro Tyr Gly Pro Gly Gln Ser Ala Ala
465             470             475             480

Ala Ala Ala Gly Pro Gly Ser Gly Gln Tyr Gly Pro Gly Ala Ser Gly
            485             490             495

Gln Asn Gly Pro Gly Ser Gly Gln Tyr Gly Pro Gly Val Leu Gly Pro
            500             505             510

Gly Gln Ser Ala Ala Ala Ala Ala Gly Gln Tyr Val Leu Gly Pro Gly
        515             520             525

Val Leu Gly Pro Tyr Gly Pro Gly Ala Ser Ala Ala Ala Ala Ala Gly
    530             535             540

Gln Tyr Gly Ser Gly Pro Gly Val Leu Gly Pro Tyr Gly Pro Gly Gln
545             550             555             560

Ser Gly Ser Gly Val Leu Gly Pro Gly Val Leu Gly Pro Tyr Ala Ser
            565             570             575

Ala Ala Ala Ala Ala Gly Pro Gly Ser Gly Val Leu Gly Pro Gly Ala
            580             585             590

Ser

<210> 26
<211> 590
<212> PRT

<213> Artificial Sequence

<220>
<223> Met-PRT965

<400> 26

Met Gly Pro Gly Thr Ser Gly Pro Tyr Gly Pro Gly Ala Ser Ala Ala
1               5                   10                  15

Ala Ala Ala Gly Ala Asn Gly Pro Gly Ser Gly Thr Ser Gly Pro Gly
            20                  25                  30

Ala Ser Gly Ala Tyr Gly Pro Gly Thr Ser Gly Pro Gly Thr Ser Gly
        35                  40                  45

Pro Gly Ser Ser Ala Ala Ala Ala Ala Gly Pro Gly Ala Tyr Gly Pro
        50                  55                  60

Gly Thr Ser Gly Pro Ser Ala Ser Ala Ala Ala Ala Gly Pro Gly
65                  70                  75                  80

Ser Gly Thr Ser Gly Pro Gly Ala Ser Gly Ala Tyr Gly Pro Gly Thr
                85                  90                  95

Ser Gly Pro Gly Thr Ser Gly Pro Gly Ser Ser Ala Ala Ala Ala Ala
            100                 105                 110

Gly Ala Tyr Gly Ser Gly Pro Gly Thr Ser Gly Pro Tyr Gly Ser Ala
        115                 120                 125

Ala Ala Ala Ala Gly Pro Gly Ser Gly Ala Tyr Gly Ala Gly Pro Tyr
        130                 135                 140

Gly Pro Gly Ala Ser Gly Pro Gly Ala Tyr Gly Pro Gly Thr Ser Gly
145                 150                 155                 160

Pro Ser Ala Ser Ala Ala Ala Ala Ala Gly Ser Gly Thr Ser Gly Pro
            165                 170                 175

Gly Ala Tyr Gly Pro Tyr Ala Ser Ala Ala Ala Ala Gly Ala Tyr
        180                 185                 190

Gly Ser Gly Pro Gly Thr Ser Gly Pro Tyr Gly Pro Gly Ala Ser Gly
        195                 200                 205

Ser Gly Thr Ser Gly Pro Gly Thr Ser Gly Pro Tyr Ala Ser Ala Ala
        210                 215                 220

Ala Ala Ala Gly Pro Gly Thr Ser Gly Pro Tyr Gly Pro Gly Ser Ser
225                     230                 235                 240

Ala Ala Ala Ala Ala Gly Ala Tyr Gly Tyr Gly Pro Gly Thr Ser Gly
                245                 250                 255

Pro Tyr Gly Pro Gly Ala Ser Gly Ala Asn Gly Pro Gly Ser Gly Ala
            260                 265                 270

Tyr Gly Pro Gly Thr Ser Gly Pro Gly Ala Ser Ala Ala Ala Ala Ala
        275                 280                 285

Gly Pro Gly Thr Ser Gly Pro Tyr Gly Pro Gly Ala Ser Ala Ala Ala
    290                 295                 300

Ala Ala Gly Ala Tyr Gly Pro Gly Thr Ser Gly Pro Gly Ala Tyr Gly
305                 310                 315                 320

Pro Gly Ser Ser Gly Pro Gly Thr Ser Gly Pro Tyr Gly Pro Gly Ser
            325                 330                 335

Ser Ala Ala Ala Ala Ala Gly Ala Tyr Gly Pro Gly Thr Ser Gly Pro
        340                 345                 350

Tyr Gly Pro Gly Ala Ser Ala Ala Ala Ala Ala Gly Ala Tyr Thr Ser
    355                 360                 365

Gly Pro Gly Thr Ser Gly Pro Tyr Gly Pro Gly Ala Ser Gly Pro Gly
    370                 375                 380

Thr Ser Gly Pro Tyr Gly Pro Gly Ala Ser Ala Ala Ala Ala Ala Gly
385                 390                 395                 400

Pro Gly Ala Tyr Gly Pro Gly Thr Ser Gly Pro Ser Ala Ser Ala Ala
            405                 410                 415

Ala Ala Ala Gly Ala Tyr Gly Ser Gly Pro Gly Ala Tyr Gly Pro Tyr
        420                 425                 430

Gly Pro Gly Ala Ser Gly Pro Gly Ser Gly Thr Ser Gly Ala Gly Pro
        435                 440                 445

Tyr Gly Pro Gly Ala Ser Ala Ala Ala Ala Ala Gly Ala Tyr Gly Pro
    450                 455                 460

Gly Thr Ser Gly Pro Tyr Gly Pro Gly Ala Ser Ala Ala Ala Ala Ala
465                 470                 475                 480

231

```
Gly Pro Gly Ser Gly Ala Tyr Gly Pro Gly Ala Ser Gly Ala Asn Gly
                485                 490                 495


Pro Gly Ser Gly Ala Tyr Gly Pro Gly Thr Ser Gly Pro Gly Ala Ser
                500                 505                 510


Ala Ala Ala Ala Ala Gly Ala Tyr Thr Ser Gly Pro Gly Thr Ser Gly
                515                 520                 525


Pro Tyr Gly Pro Gly Ala Ser Ala Ala Ala Ala Ala Gly Ala Tyr Gly
    530                 535                 540


Ser Gly Pro Gly Thr Ser Gly Pro Tyr Gly Pro Gly Ala Ser Gly Ser
545                 550                 555                 560


Gly Thr Ser Gly Pro Gly Thr Ser Gly Pro Tyr Ala Ser Ala Ala Ala
                565                 570                 575


Ala Ala Gly Pro Gly Ser Gly Thr Ser Gly Pro Gly Ala Ser
                580                 585                 590
```

<210> 27
<211> 593
<212> PRT
<213> Artificial Sequence

<220>
<223> Met-PRT889

<400> 27

```
Met Gly Ser Ser Gly Pro Gly Val Leu Gly Pro Tyr Gly Pro Gly Ala
1               5                   10                  15


Ser Ala Ala Ala Ala Ala Gly Ile Asn Gly Pro Gly Ser Gly Val Leu
                20                  25                  30


Gly Pro Gly Ile Ser Gly Ile Tyr Gly Pro Gly Val Leu Gly Pro Gly
                35                  40                  45


Val Leu Gly Pro Gly Ser Ser Ala Ala Ala Ala Ala Gly Pro Gly Ile
    50                  55                  60


Tyr Gly Pro Gly Val Leu Gly Pro Ser Ala Ser Ala Ala Ala Ala Ala
65                  70                  75                  80


Gly Pro Gly Ser Gly Val Leu Gly Pro Gly Ala Ser Gly Ile Tyr Gly
                85                  90                  95
```

Pro Gly Val Leu Gly Pro Gly Val Leu Gly Pro Gly Ser Ser Ala Ala
            100                     105                 110

Ala Ala Ala Gly Ile Tyr Gly Ser Gly Pro Gly Val Leu Gly Pro Tyr
            115                     120                 125

Gly Ser Ala Ala Ala Ala Ala Gly Pro Gly Ser Gly Ile Tyr Gly Ile
    130                     135                 140

Gly Pro Tyr Gly Pro Gly Ala Ser Gly Pro Gly Ile Tyr Gly Pro Gly
145                 150                 155                 160

Val Leu Gly Pro Ser Ala Ser Ala Ala Ala Ala Ala Gly Ser Gly Val
                165                 170                 175

Leu Gly Pro Gly Ile Tyr Gly Pro Tyr Ala Ser Ala Ala Ala Ala Ala
            180                 185                 190

Gly Ile Tyr Gly Ser Gly Pro Gly Val Leu Gly Pro Tyr Gly Pro Gly
        195                 200                 205

Ile Ser Gly Ser Gly Val Leu Gly Pro Gly Val Leu Gly Pro Tyr Ala
    210                 215                 220

Ser Ala Ala Ala Ala Ala Gly Pro Gly Val Leu Gly Pro Tyr Gly Pro
225                 230                 235                 240

Gly Ser Ser Ala Ala Ala Ala Ala Gly Ile Tyr Gly Tyr Gly Pro Gly
                245                 250                 255

Val Leu Gly Pro Tyr Gly Pro Gly Ala Ser Gly Ile Asn Gly Pro Gly
            260                 265                 270

Ser Gly Ile Tyr Gly Pro Gly Val Leu Gly Pro Gly Ile Ser Ala Ala
        275                 280                 285

Ala Ala Ala Gly Pro Gly Val Leu Gly Pro Tyr Gly Pro Gly Ala Ser
    290                 295                 300

Ala Ala Ala Ala Ala Gly Ile Tyr Gly Pro Gly Val Leu Gly Pro Gly
305                 310                 315                 320

Ile Tyr Gly Pro Gly Ser Ser Gly Pro Gly Val Leu Gly Pro Tyr Gly
            325                 330                 335

Pro Gly Ser Ser Ala Ala Ala Ala Ala Gly Ile Tyr Gly Pro Gly Val
        340                 345                 350

233

Leu Gly Pro Tyr Gly Pro Gly Ile Ser Ala Ala Ala Ala Gly Ile
        355                 360                 365

Tyr Val Leu Gly Pro Gly Val Leu Gly Pro Tyr Gly Pro Gly Ala Ser
        370                 375                 380

Gly Pro Gly Val Leu Gly Pro Tyr Gly Pro Gly Ala Ser Ala Ala Ala
385                 390                 395                 400

Ala Ala Gly Pro Gly Ile Tyr Gly Pro Gly Val Leu Gly Pro Ser Ala
                405                 410                 415

Ser Ala Ala Ala Ala Ala Gly Ile Tyr Gly Ser Gly Pro Gly Ile Tyr
            420                 425                 430

Gly Pro Tyr Gly Pro Gly Ile Ser Gly Pro Gly Ser Gly Val Leu Gly
        435                 440                 445

Ile Gly Pro Tyr Gly Pro Gly Ala Ser Ala Ala Ala Ala Ala Gly Ile
    450                 455                 460

Tyr Gly Pro Gly Val Leu Gly Pro Tyr Gly Pro Gly Ile Ser Ala Ala
465                 470                 475                 480

Ala Ala Ala Gly Pro Gly Ser Gly Ile Tyr Gly Pro Gly Ala Ser Gly
            485                 490                 495

Ile Asn Gly Pro Gly Ser Gly Ile Tyr Gly Pro Gly Val Leu Gly Pro
        500                 505                 510

Gly Ile Ser Ala Ala Ala Ala Ala Gly Ile Tyr Val Leu Gly Pro Gly
        515                 520                 525

Val Leu Gly Pro Tyr Gly Pro Gly Ala Ser Ala Ala Ala Ala Ala Gly
        530                 535                 540

Ile Tyr Gly Ser Gly Pro Gly Val Leu Gly Pro Tyr Gly Pro Gly Ile
545                 550                 555                 560

Ser Gly Ser Gly Val Leu Gly Pro Gly Val Leu Gly Pro Tyr Ala Ser
                565                 570                 575

Ala Ala Ala Ala Ala Gly Pro Gly Ser Gly Val Leu Gly Pro Gly Ala
            580                 585                 590

Ser

<210> 28
<211> 590
<212> PRT
<213> Artificial Sequence

<220>
<223> Met-PRT916

<400> 28

Met Gly Pro Gly Val Ile Gly Pro Tyr Gly Pro Gly Ala Ser Ala Ala
1               5                   10                  15

Ala Ala Ala Gly Leu Asn Gly Pro Gly Ser Gly Val Ile Gly Pro Gly
            20                  25                  30

Leu Ser Gly Leu Tyr Gly Pro Gly Val Ile Gly Pro Gly Val Ile Gly
        35                  40                  45

Pro Gly Ser Ser Ala Ala Ala Ala Ala Gly Pro Gly Leu Tyr Gly Pro
    50                  55                  60

Gly Val Ile Gly Pro Ser Ala Ser Ala Ala Ala Ala Gly Pro Gly
65                  70                  75                  80

Ser Gly Val Ile Gly Pro Gly Ala Ser Gly Leu Tyr Gly Pro Gly Val
            85                  90                  95

Ile Gly Pro Gly Val Ile Gly Pro Gly Ser Ser Ala Ala Ala Ala Ala
            100                 105                 110

Gly Leu Tyr Gly Ser Gly Pro Gly Val Ile Gly Pro Tyr Gly Ser Ala
        115                 120                 125

Ala Ala Ala Gly Pro Gly Ser Gly Leu Tyr Gly Leu Gly Pro Tyr
        130                 135                 140

Gly Pro Gly Ala Ser Gly Pro Gly Leu Tyr Gly Pro Gly Val Ile Gly
145                 150                 155                 160

Pro Ser Ala Ser Ala Ala Ala Ala Ala Gly Ser Gly Val Ile Gly Pro
            165                 170                 175

Gly Leu Tyr Gly Pro Tyr Ala Ser Ala Ala Ala Ala Gly Leu Tyr
        180                 185                 190

Gly Ser Gly Pro Gly Val Ile Gly Pro Tyr Gly Pro Gly Leu Ser Gly
        195                 200                 205

Ser Gly Val Ile Gly Pro Gly Val Ile Gly Pro Tyr Ala Ser Ala Ala
210             215             220

Ala Ala Ala Gly Pro Gly Val Ile Gly Pro Tyr Gly Pro Gly Ser Ser
225             230             235             240

Ala Ala Ala Ala Ala Gly Leu Tyr Gly Tyr Gly Pro Gly Val Ile Gly
        245             250             255

Pro Tyr Gly Pro Gly Ala Ser Gly Leu Asn Gly Pro Gly Ser Gly Leu
        260             265             270

Tyr Gly Pro Gly Val Ile Gly Pro Gly Leu Ser Ala Ala Ala Ala Ala
        275             280             285

Gly Pro Gly Val Ile Gly Pro Tyr Gly Pro Gly Ala Ser Ala Ala Ala
    290             295             300

Ala Ala Gly Leu Tyr Gly Pro Gly Val Ile Gly Pro Gly Leu Tyr Gly
305             310             315             320

Pro Gly Ser Ser Gly Pro Gly Val Ile Gly Pro Tyr Gly Pro Gly Ser
            325             330             335

Ser Ala Ala Ala Ala Ala Gly Leu Tyr Gly Pro Gly Val Ile Gly Pro
        340             345             350

Tyr Gly Pro Gly Leu Ser Ala Ala Ala Ala Gly Leu Tyr Val Ile
        355             360             365

Gly Pro Gly Val Ile Gly Pro Tyr Gly Pro Gly Ala Ser Gly Pro Gly
    370             375             380

Val Ile Gly Pro Tyr Gly Pro Gly Ala Ser Ala Ala Ala Ala Gly
385             390             395             400

Pro Gly Leu Tyr Gly Pro Gly Val Ile Gly Pro Ser Ala Ser Ala Ala
        405             410             415

Ala Ala Ala Gly Leu Tyr Gly Ser Gly Pro Gly Leu Tyr Gly Pro Tyr
        420             425             430

Gly Pro Gly Leu Ser Gly Pro Gly Ser Gly Val Ile Gly Leu Gly Pro
        435             440             445

Tyr Gly Pro Gly Ala Ser Ala Ala Ala Ala Gly Leu Tyr Gly Pro
450             455             460

236

```
Gly Val Ile Gly Pro Tyr Gly Pro Gly Leu Ser Ala Ala Ala Ala Ala
465             470             475                 480


Gly Pro Gly Ser Gly Leu Tyr Gly Pro Gly Ala Ser Gly Leu Asn Gly
                485             490                 495


Pro Gly Ser Gly Leu Tyr Gly Pro Gly Val Ile Gly Pro Gly Leu Ser
                500             505             510


Ala Ala Ala Ala Ala Gly Leu Tyr Val Ile Gly Pro Gly Val Ile Gly
            515             520             525


Pro Tyr Gly Pro Gly Ala Ser Ala Ala Ala Ala Gly Leu Tyr Gly
    530             535             540


Ser Gly Pro Gly Val Ile Gly Pro Tyr Gly Pro Gly Leu Ser Gly Ser
545             550             555             560


Gly Val Ile Gly Pro Gly Val Ile Gly Pro Tyr Ala Ser Ala Ala Ala
                565             570             575


Ala Ala Gly Pro Gly Ser Gly Val Ile Gly Pro Gly Ala Ser
            580             585             590


<210>   29
<211>   590
<212>   PRT
<213>   Artificial Sequence

<220>
<223>   Met-PRT918

<400>   29

Met Gly Pro Gly Val Phe Gly Pro Tyr Gly Pro Gly Ala Ser Ala Ala
1               5               10                  15


Ala Ala Ala Gly Ile Asn Gly Pro Gly Ser Gly Val Phe Gly Pro Gly
            20              25              30


Ile Ser Gly Ile Tyr Gly Pro Gly Val Phe Gly Pro Gly Val Phe Gly
            35              40              45


Pro Gly Ser Ser Ala Ala Ala Ala Ala Gly Pro Gly Ile Tyr Gly Pro
    50              55              60


Gly Val Phe Gly Pro Ser Ala Ser Ala Ala Ala Ala Gly Pro Gly
65              70              75                  80
```

```
Ser Gly Val Phe Gly Pro Gly Ala Ser Gly Ile Tyr Gly Pro Gly Val
              85                  90                  95

Phe Gly Pro Gly Val Phe Gly Pro Gly Ser Ser Ala Ala Ala Ala Ala
             100                 105                 110

Gly Ile Tyr Gly Ser Gly Pro Gly Val Phe Gly Pro Tyr Gly Ser Ala
             115                 120                 125

Ala Ala Ala Ala Gly Pro Gly Ser Gly Ile Tyr Gly Ile Gly Pro Tyr
    130                 135                 140

Gly Pro Gly Ala Ser Gly Pro Gly Ile Tyr Gly Pro Gly Val Phe Gly
145                 150                 155                 160

Pro Ser Ala Ser Ala Ala Ala Ala Ala Gly Ser Gly Val Phe Gly Pro
                165                 170                 175

Gly Ile Tyr Gly Pro Tyr Ala Ser Ala Ala Ala Ala Gly Ile Tyr
             180                 185                 190

Gly Ser Gly Pro Gly Val Phe Gly Pro Tyr Gly Pro Gly Ile Ser Gly
             195                 200                 205

Ser Gly Val Phe Gly Pro Gly Val Phe Gly Pro Tyr Ala Ser Ala Ala
    210                 215                 220

Ala Ala Ala Gly Pro Gly Val Phe Gly Pro Tyr Gly Pro Gly Ser Ser
225                 230                 235                 240

Ala Ala Ala Ala Ala Gly Ile Tyr Gly Tyr Gly Pro Gly Val Phe Gly
                245                 250                 255

Pro Tyr Gly Pro Gly Ala Ser Gly Ile Asn Gly Pro Gly Ser Gly Ile
             260                 265                 270

Tyr Gly Pro Gly Val Phe Gly Pro Gly Ile Ser Ala Ala Ala Ala Ala
             275                 280                 285

Gly Pro Gly Val Phe Gly Pro Tyr Gly Pro Gly Ala Ser Ala Ala Ala
    290                 295                 300

Ala Ala Gly Ile Tyr Gly Pro Gly Val Phe Gly Pro Gly Ile Tyr Gly
305                 310                 315                 320

Pro Gly Ser Ser Gly Pro Gly Val Phe Gly Pro Tyr Gly Pro Gly Ser
             325                 330                 335
```

238

```
Ser Ala Ala Ala Ala Ala Gly Ile Tyr Gly Pro Gly Val Phe Gly Pro
        340                 345             350

Tyr Gly Pro Gly Ile Ser Ala Ala Ala Ala Ala Gly Ile Tyr Val Phe
        355                 360             365

Gly Pro Gly Val Phe Gly Pro Tyr Gly Pro Gly Ala Ser Gly Pro Gly
        370                 375             380

Val Phe Gly Pro Tyr Gly Pro Gly Ala Ser Ala Ala Ala Ala Gly
385             390             395                     400

Pro Gly Ile Tyr Gly Pro Gly Val Phe Gly Pro Ser Ala Ser Ala Ala
                405             410             415

Ala Ala Ala Gly Ile Tyr Gly Ser Gly Pro Gly Ile Tyr Gly Pro Tyr
            420             425             430

Gly Pro Gly Ile Ser Gly Pro Gly Ser Gly Val Phe Gly Ile Gly Pro
        435             440             445

Tyr Gly Pro Gly Ala Ser Ala Ala Ala Ala Gly Ile Tyr Gly Pro
        450             455             460

Gly Val Phe Gly Pro Tyr Gly Pro Gly Ile Ser Ala Ala Ala Ala Ala
465             470             475                     480

Gly Pro Gly Ser Gly Ile Tyr Gly Pro Gly Ala Ser Gly Ile Asn Gly
            485             490             495

Pro Gly Ser Gly Ile Tyr Gly Pro Gly Val Phe Gly Pro Gly Ile Ser
        500             505             510

Ala Ala Ala Ala Ala Gly Ile Tyr Val Phe Gly Pro Gly Val Phe Gly
        515             520             525

Pro Tyr Gly Pro Gly Ala Ser Ala Ala Ala Ala Gly Ile Tyr Gly
        530             535             540

Ser Gly Pro Gly Val Phe Gly Pro Tyr Gly Pro Gly Ile Ser Gly Ser
545             550             555             560

Gly Val Phe Gly Pro Gly Val Phe Gly Pro Tyr Ala Ser Ala Ala Ala
            565             570             575

Ala Ala Gly Pro Gly Ser Gly Val Phe Gly Pro Gly Ala Ser
```

580                          585                          590

```
<210>  30
<211>  565
<212>  PRT
<213>  Artificial Sequence

<220>
<223>  Met-PRT699

<400>  30

Met Gly Pro Gly Val Leu Gly Pro Tyr Gly Pro Gly Ala Ser Ala Ala
1               5                  10                  15


Ala Ala Ala Ala Ala Gly Ser Asn Gly Pro Gly Ser Gly Val Leu Gly
            20                  25                  30


Pro Gly Gln Ser Gly Gln Tyr Gly Pro Gly Val Leu Gly Pro Gly Val
        35                  40                  45


Leu Gly Pro Gly Ser Ser Ala Ala Ala Ala Ala Ala Gly Pro Gly
        50                  55                  60


Gln Tyr Gly Pro Gly Val Leu Gly Pro Ser Ala Ser Ala Ala Ala Ala
65                  70                  75                  80


Ala Ala Ala Gly Pro Gly Ser Gly Val Leu Gly Pro Gly Ala Ser Gly
                85                  90                  95


Gln Tyr Gly Pro Gly Val Leu Gly Pro Gly Val Leu Gly Pro Gly Ser
            100                 105                 110


Ser Ala Ala Ala Ala Ala Ala Ala Gly Ser Tyr Gly Ser Gly Pro Gly
        115                 120                 125


Val Leu Gly Pro Tyr Gly Ser Ala Ala Ala Ala Ala Ala Gly Pro
    130                 135                 140


Gly Ser Gly Gln Tyr Gly Gln Gly Pro Tyr Gly Pro Gly Ala Ser Gly
145                 150                 155                 160


Pro Gly Gln Tyr Gly Pro Gly Val Leu Gly Pro Ser Ala Ser Ala Ala
            165                 170                 175


Ala Ala Ala Ala Ala Gly Ser Gly Val Leu Gly Pro Gly Gln Tyr Gly
            180                 185                 190


Pro Tyr Ala Ser Ala Ala Ala Ala Ala Ala Ala Gly Ser Tyr Gly Ser
        195                 200                 205
```

```
Gly Pro Gly Val Leu Gly Pro Tyr Gly Pro Gly Gln Ser Gly Ser Gly
    210                 215                 220

Val Leu Gly Pro Gly Val Leu Gly Pro Tyr Ala Ser Ala Ala Ala Ala
225                 230                 235                 240

Ala Ala Ala Gly Pro Gly Val Leu Gly Pro Tyr Gly Pro Gly Ser Ser
            245                 250                 255

Ala Ala Ala Ala Ala Ala Ala Gly Ser Tyr Gly Tyr Gly Pro Gly Val
            260                 265                 270

Leu Gly Pro Tyr Gly Pro Gly Ala Ser Gly Gln Asn Gly Pro Gly Ser
            275                 280                 285

Gly Gln Tyr Gly Pro Gly Val Leu Gly Pro Gly Pro Ser Ala Ala Ala
    290                 295                 300

Ala Ala Ala Ala Gly Pro Gly Val Leu Gly Pro Tyr Gly Pro Gly Ala
305                 310                 315                 320

Ser Ala Ala Ala Ala Ala Ala Ala Gly Ser Tyr Gly Pro Gly Val Leu
            325                 330                 335

Gly Pro Gly Gln Tyr Gly Pro Gly Ser Ser Gly Pro Gly Val Leu Gly
    340                 345                 350

Pro Tyr Gly Pro Gly Ser Ser Ala Ala Ala Ala Ala Ala Ala Gly Ser
    355                 360                 365

Tyr Gly Pro Gly Val Leu Gly Pro Tyr Gly Pro Gly Pro Ser Ala Ala
    370                 375                 380

Ala Ala Ala Ala Ala Gly Ser Tyr Val Leu Gly Pro Gly Val Leu Gly
385                 390                 395                 400

Pro Tyr Gly Pro Gly Ala Ser Gly Pro Gly Val Leu Gly Pro Tyr Gly
            405                 410                 415

Pro Gly Ala Ser Ala Ala Ala Ala Ala Ala Gly Pro Gly Gln Tyr
            420                 425                 430

Gly Pro Gly Val Leu Gly Pro Ser Ala Ser Ala Ala Ala Ala Ala Ala
    435                 440                 445

Ala Gly Ser Tyr Gly Ser Gly Pro Gly Gln Tyr Gly Pro Tyr Gly Pro
```

```
                    450                      455                        460


       Gly Gln Ser Gly Pro Gly Ser Gly Val Leu Gly Gln Gly Pro Tyr Gly
       465                  470                  475                  480


       Pro Gly Ala Ser Ala Ala Ala Ala Ala Ala Ala Gly Ser Tyr Gly Pro
                       485                  490                  495


       Gly Val Leu Gly Pro Tyr Gly Pro Gly Pro Ser Ala Ala Ala Ala Ala
                   500                  505                  510


       Ala Ala Gly Pro Gly Ser Gly Gln Tyr Gly Pro Gly Ala Ser Gly Gln
                   515                  520                  525


       Asn Gly Pro Gly Ser Gly Gln Tyr Gly Pro Gly Val Leu Gly Pro Gly
                   530                  535                  540


       Pro Ser Ala Ala Ala Ala Ala Ala Ala Gly Pro Gly Ser Gly Val Leu
       545                  550                  555                  560


       Gly Pro Gly Ala Ser
                       565


       <210>  31
       <211>  565
       <212>  PRT
       <213>  Artificial Sequence

       <220>
       <223>  Met-PRT698

       <400>  31

       Met Gly Pro Gly Val Leu Gly Pro Tyr Gly Pro Gly Ala Ser Ala Ala
       1               5                  10                  15


       Ala Ala Ala Ala Ala Gly Ser Asn Gly Pro Gly Ser Gly Val Leu Gly
                   20                  25                  30


       Pro Gly Ile Ser Gly Ile Tyr Gly Pro Gly Val Leu Gly Pro Gly Val
                   35                  40                  45


       Leu Gly Pro Gly Ser Ser Ala Ala Ala Ala Ala Ala Gly Pro Gly
                   50                  55                  60


       Ile Tyr Gly Pro Gly Val Leu Gly Pro Ser Ala Ser Ala Ala Ala Ala
       65                  70                  75                  80


       Ala Ala Ala Gly Pro Gly Ser Gly Val Leu Gly Pro Gly Ala Ser Gly
                       85                  90                  95
```

```
Ile Tyr Gly Pro Gly Val Leu Gly Pro Gly Val Leu Gly Pro Gly Ser
        100                 105                 110

Ser Ala Ala Ala Ala Ala Ala Ala Gly Ser Tyr Gly Ser Gly Pro Gly
        115                 120                 125

Val Leu Gly Pro Tyr Gly Ser Ala Ala Ala Ala Ala Ala Ala Gly Pro
        130                 135                 140

Gly Ser Gly Ile Tyr Gly Ile Gly Pro Tyr Gly Pro Gly Ala Ser Gly
145                 150                 155                 160

Pro Gly Ile Tyr Gly Pro Gly Val Leu Gly Pro Ser Ala Ser Ala Ala
                165                 170                 175

Ala Ala Ala Ala Ala Gly Ser Gly Val Leu Gly Pro Gly Ile Tyr Gly
                180                 185                 190

Pro Tyr Ala Ser Ala Ala Ala Ala Ala Ala Gly Ser Tyr Gly Ser
        195                 200                 205

Gly Pro Gly Val Leu Gly Pro Tyr Gly Pro Gly Ile Ser Gly Ser Gly
        210                 215                 220

Val Leu Gly Pro Gly Val Leu Gly Pro Tyr Ala Ser Ala Ala Ala Ala
225                 230                 235                 240

Ala Ala Ala Gly Pro Gly Val Leu Gly Pro Tyr Gly Pro Gly Ser Ser
                245                 250                 255

Ala Ala Ala Ala Ala Ala Ala Gly Ser Tyr Gly Tyr Gly Pro Gly Val
                260                 265                 270

Leu Gly Pro Tyr Gly Pro Gly Ala Ser Gly Ile Asn Gly Pro Gly Ser
        275                 280                 285

Gly Ile Tyr Gly Pro Gly Val Leu Gly Pro Gly Pro Ser Ala Ala Ala
        290                 295                 300

Ala Ala Ala Ala Gly Pro Gly Val Leu Gly Pro Tyr Gly Pro Gly Ala
305                 310                 315                 320

Ser Ala Ala Ala Ala Ala Ala Ala Gly Ser Tyr Gly Pro Gly Val Leu
                325                 330                 335

Gly Pro Gly Ile Tyr Gly Pro Gly Ser Ser Gly Pro Gly Val Leu Gly
```

```
                340                         345                         350


        Pro Tyr Gly Pro Gly Ser Ser Ala Ala Ala Ala Ala Ala Ala Gly Ser
                355                 360                 365


        Tyr Gly Pro Gly Val Leu Gly Pro Tyr Gly Pro Gly Pro Ser Ala Ala
                370                 375                 380


        Ala Ala Ala Ala Ala Gly Ser Tyr Val Leu Gly Pro Gly Val Leu Gly
        385                 390                 395                 400


        Pro Tyr Gly Pro Gly Ala Ser Gly Pro Gly Val Leu Gly Pro Tyr Gly
                        405                 410                 415


        Pro Gly Ala Ser Ala Ala Ala Ala Ala Ala Ala Gly Pro Gly Ile Tyr
                    420                 425                 430


        Gly Pro Gly Val Leu Gly Pro Ser Ala Ser Ala Ala Ala Ala Ala Ala
                435                 440                 445


        Ala Gly Ser Tyr Gly Ser Gly Pro Gly Ile Tyr Gly Pro Tyr Gly Pro
            450                 455                 460


        Gly Ile Ser Gly Pro Gly Ser Gly Val Leu Gly Ile Gly Pro Tyr Gly
        465                 470                 475                 480


        Pro Gly Ala Ser Ala Ala Ala Ala Ala Ala Ala Gly Ser Tyr Gly Pro
                    485                 490                 495


        Gly Val Leu Gly Pro Tyr Gly Pro Gly Pro Ser Ala Ala Ala Ala Ala
                500                 505                 510


        Ala Ala Gly Pro Gly Ser Gly Ile Tyr Gly Pro Gly Ala Ser Gly Ile
                515                 520                 525


        Asn Gly Pro Gly Ser Gly Ile Tyr Gly Pro Gly Val Leu Gly Pro Gly
            530                 535                 540


        Pro Ser Ala Ala Ala Ala Ala Ala Ala Gly Pro Gly Ser Gly Val Leu
        545                 550                 555                 560


        Gly Pro Gly Ala Ser
                        565



        <210>   32
        <211>   1179
        <212>   PRT
        <213>   Artificial Sequence
```

<220>
<223>  Met-PRT966

<400>  32

Met Gly Pro Gly Val Phe Gly Pro Tyr Gly Pro Gly Ala Ser Ala Ala
1               5                   10                  15

Ala Ala Ala Gly Ile Asn Gly Pro Gly Ser Gly Val Phe Gly Pro Gly
            20                  25                  30

Ile Ser Gly Ile Tyr Gly Pro Gly Val Phe Gly Pro Gly Val Phe Gly
        35                  40                  45

Pro Gly Ser Ser Ala Ala Ala Ala Ala Gly Pro Gly Ile Tyr Gly Pro
    50                  55                  60

Gly Val Phe Gly Pro Ser Ala Ser Ala Ala Ala Ala Gly Pro Gly
65                  70                  75                  80

Ser Gly Val Phe Gly Pro Gly Ala Ser Gly Ile Tyr Gly Pro Gly Val
                85                  90                  95

Phe Gly Pro Gly Val Phe Gly Pro Gly Ser Ser Ala Ala Ala Ala Ala
            100                 105                 110

Gly Ile Tyr Gly Ser Gly Pro Gly Val Phe Gly Pro Tyr Gly Ser Ala
        115                 120                 125

Ala Ala Ala Ala Gly Pro Gly Ser Gly Ile Tyr Gly Ile Gly Pro Tyr
    130                 135                 140

Gly Pro Gly Ala Ser Gly Pro Gly Ile Tyr Gly Pro Gly Val Phe Gly
145                 150                 155                 160

Pro Ser Ala Ser Ala Ala Ala Ala Gly Ser Gly Val Phe Gly Pro
            165                 170                 175

Gly Ile Tyr Gly Pro Tyr Ala Ser Ala Ala Ala Ala Gly Ile Tyr
        180                 185                 190

Gly Ser Gly Pro Gly Val Phe Gly Pro Tyr Gly Pro Gly Ile Ser Gly
        195                 200                 205

Ser Gly Val Phe Gly Pro Gly Val Phe Gly Pro Tyr Ala Ser Ala Ala
        210                 215                 220

Ala Ala Ala Gly Pro Gly Val Phe Gly Pro Tyr Gly Pro Gly Ser Ser

225                     230                     235                     240

Ala Ala Ala Ala Ala Gly Ile Tyr Gly Tyr Gly Pro Gly Val Phe Gly
              245                 250                 255

Pro Tyr Gly Pro Gly Ala Ser Gly Ile Asn Gly Pro Gly Ser Gly Ile
              260                 265                 270

Tyr Gly Pro Gly Val Phe Gly Pro Gly Ile Ser Ala Ala Ala Ala Ala
              275                 280                 285

Gly Pro Gly Val Phe Gly Pro Tyr Gly Pro Gly Ala Ser Ala Ala Ala
              290                 295                 300

Ala Ala Gly Ile Tyr Gly Pro Gly Val Phe Gly Pro Gly Ile Tyr Gly
305                 310                 315                 320

Pro Gly Ser Ser Gly Pro Gly Val Phe Gly Pro Tyr Gly Pro Gly Ser
              325                 330                 335

Ser Ala Ala Ala Ala Ala Gly Ile Tyr Gly Pro Gly Val Phe Gly Pro
              340                 345                 350

Tyr Gly Pro Gly Ile Ser Ala Ala Ala Ala Ala Gly Ile Tyr Val Phe
              355                 360                 365

Gly Pro Gly Val Phe Gly Pro Tyr Gly Pro Gly Ala Ser Gly Pro Gly
              370                 375                 380

Val Phe Gly Pro Tyr Gly Pro Gly Ala Ser Ala Ala Ala Ala Ala Gly
385                 390                 395                 400

Pro Gly Ile Tyr Gly Pro Gly Val Phe Gly Pro Ser Ala Ser Ala Ala
              405                 410                 415

Ala Ala Ala Gly Ile Tyr Gly Ser Gly Pro Gly Ile Tyr Gly Pro Tyr
              420                 425                 430

Gly Pro Gly Ile Ser Gly Pro Gly Ser Gly Val Phe Gly Ile Gly Pro
              435                 440                 445

Tyr Gly Pro Gly Ala Ser Ala Ala Ala Ala Gly Ile Tyr Gly Pro
              450                 455                 460

Gly Val Phe Gly Pro Tyr Gly Pro Gly Ile Ser Ala Ala Ala Ala Ala
465                 470                 475                 480

Gly Pro Gly Ser Gly Ile Tyr Gly Pro Gly Ala Ser Gly Ile Asn Gly
          485                     490              495

Pro Gly Ser Gly Ile Tyr Gly Pro Gly Val Phe Gly Pro Gly Ile Ser
          500                     505              510

Ala Ala Ala Ala Ala Gly Ile Tyr Val Phe Gly Pro Gly Val Phe Gly
          515                 520              525

Pro Tyr Gly Pro Gly Ala Ser Ala Ala Ala Ala Gly Ile Tyr Gly
    530              535              540

Ser Gly Pro Gly Val Phe Gly Pro Tyr Gly Pro Gly Ile Ser Gly Ser
545              550              555                  560

Gly Val Phe Gly Pro Gly Val Phe Gly Pro Tyr Ala Ser Ala Ala Ala
          565              570              575

Ala Ala Gly Pro Gly Ser Gly Val Phe Gly Pro Gly Ala Ser Gly Pro
          580              585              590

Gly Val Phe Gly Pro Tyr Gly Pro Gly Ala Ser Ala Ala Ala Ala Ala
          595              600              605

Gly Ile Asn Gly Pro Gly Ser Gly Val Phe Gly Pro Gly Ile Ser Gly
    610              615              620

Ile Tyr Gly Pro Gly Val Phe Gly Pro Gly Val Phe Gly Pro Gly Ser
625              630              635                  640

Ser Ala Ala Ala Ala Ala Gly Pro Gly Ile Tyr Gly Pro Gly Val Phe
              645              650              655

Gly Pro Ser Ala Ser Ala Ala Ala Ala Gly Pro Gly Ser Gly Val
          660              665              670

Phe Gly Pro Gly Ala Ser Gly Ile Tyr Gly Pro Gly Val Phe Gly Pro
      675              680              685

Gly Val Phe Gly Pro Gly Ser Ser Ala Ala Ala Ala Gly Ile Tyr
    690              695              700

Gly Ser Gly Pro Gly Val Phe Gly Pro Tyr Gly Ser Ala Ala Ala Ala
705              710              715                  720

Ala Gly Pro Gly Ser Gly Ile Tyr Gly Ile Gly Pro Tyr Gly Pro Gly
          725              730              735

247

```
Ala Ser Gly Pro Gly Ile Tyr Gly Pro Gly Val Phe Gly Pro Ser Ala
            740                 745             750

Ser Ala Ala Ala Ala Ala Gly Ser Gly Val Phe Gly Pro Gly Ile Tyr
            755                 760             765

Gly Pro Tyr Ala Ser Ala Ala Ala Ala Ala Gly Ile Tyr Gly Ser Gly
            770                 775             780

Pro Gly Val Phe Gly Pro Tyr Gly Pro Gly Ile Ser Gly Ser Gly Val
785                 790                 795                 800

Phe Gly Pro Gly Val Phe Gly Pro Tyr Ala Ser Ala Ala Ala Ala Ala
                805                 810                 815

Gly Pro Gly Val Phe Gly Pro Tyr Gly Pro Gly Ser Ser Ala Ala Ala
                820                 825                 830

Ala Ala Gly Ile Tyr Gly Tyr Gly Pro Gly Val Phe Gly Pro Tyr Gly
            835                 840                 845

Pro Gly Ala Ser Gly Ile Asn Gly Pro Gly Ser Gly Ile Tyr Gly Pro
            850                 855                 860

Gly Val Phe Gly Pro Gly Ile Ser Ala Ala Ala Ala Gly Pro Gly
865                 870                 875                 880

Val Phe Gly Pro Tyr Gly Pro Gly Ala Ser Ala Ala Ala Ala Ala Gly
                885                 890                 895

Ile Tyr Gly Pro Gly Val Phe Gly Pro Gly Ile Tyr Gly Pro Gly Ser
            900                 905                 910

Ser Gly Pro Gly Val Phe Gly Pro Tyr Gly Pro Gly Ser Ser Ala Ala
            915                 920                 925

Ala Ala Ala Gly Ile Tyr Gly Pro Gly Val Phe Gly Pro Tyr Gly Pro
            930                 935                 940

Gly Ile Ser Ala Ala Ala Ala Gly Ile Tyr Val Phe Gly Pro Gly
945                 950                 955                 960

Val Phe Gly Pro Tyr Gly Pro Gly Ala Ser Gly Pro Gly Val Phe Gly
                965                 970                 975

Pro Tyr Gly Pro Gly Ala Ser Ala Ala Ala Ala Ala Gly Pro Gly Ile
            980                 985                 990
```

248

```
Tyr Gly Pro Gly Val Phe Gly Pro  Ser Ala Ser Ala Ala  Ala Ala Ala
        995               1000              1005


Gly Ile  Tyr Gly Ser Gly Pro  Gly Ile Tyr Gly Pro  Tyr Gly Pro
        1010              1015              1020


Gly Ile  Ser Gly Pro Gly Ser  Gly Val Phe Gly Ile  Gly Pro Tyr
        1025              1030              1035


Gly Pro  Gly Ala Ser Ala Ala  Ala Ala Ala Gly Ile  Tyr Gly Pro
        1040              1045              1050


Gly Val  Phe Gly Pro Tyr Gly  Pro Gly Ile Ser Ala  Ala Ala Ala
        1055              1060              1065


Ala Gly  Pro Gly Ser Gly Ile  Tyr Gly Pro Gly Ala  Ser Gly Ile
        1070              1075              1080


Asn Gly  Pro Gly Ser Gly Ile  Tyr Gly Pro Gly Val  Phe Gly Pro
        1085              1090              1095


Gly Ile  Ser Ala Ala Ala Ala  Ala Gly Ile Tyr Val  Phe Gly Pro
        1100              1105              1110


Gly Val  Phe Gly Pro Tyr Gly  Pro Gly Ala Ser Ala  Ala Ala Ala
        1115              1120              1125


Ala Gly  Ile Tyr Gly Ser Gly  Pro Gly Val Phe Gly  Pro Tyr Gly
        1130              1135              1140


Pro Gly  Ile Ser Gly Ser Gly  Val Phe Gly Pro Gly  Val Phe Gly
        1145              1150              1155


Pro Tyr  Ala Ser Ala Ala Ala  Ala Ala Gly Pro Gly  Ser Gly Val
        1160              1165              1170


Phe Gly  Pro Gly Ala Ser
        1175


<210>   33
<211>   601
<212>   PRT
<213>   Artificial Sequence

<220>
<223>   PRT888

<400>   33
```

```
Met His His His His His His Ser Ser Gly Ser Ser Gly Pro Gly Val
1                   5                   10                  15

Leu Gly Pro Tyr Gly Pro Gly Ala Ser Ala Ala Ala Ala Ala Gly Gln
            20                  25                  30

Asn Gly Pro Gly Ser Gly Val Leu Gly Pro Gly Gln Ser Gly Gln Tyr
            35                  40                  45

Gly Pro Gly Val Leu Gly Pro Gly Val Leu Gly Pro Gly Ser Ser Ala
        50                  55                  60

Ala Ala Ala Ala Gly Pro Gly Gln Tyr Gly Pro Gly Val Leu Gly Pro
65                  70                  75                  80

Ser Ala Ser Ala Ala Ala Ala Ala Gly Pro Gly Ser Gly Val Leu Gly
                85                  90                  95

Pro Gly Ala Ser Gly Gln Tyr Gly Pro Gly Val Leu Gly Pro Gly Val
            100                 105                 110

Leu Gly Pro Gly Ser Ser Ala Ala Ala Ala Ala Gly Gln Tyr Gly Ser
            115                 120                 125

Gly Pro Gly Val Leu Gly Pro Tyr Gly Ser Ala Ala Ala Ala Ala Gly
        130                 135                 140

Pro Gly Ser Gly Gln Tyr Gly Gln Gly Pro Tyr Gly Pro Gly Ala Ser
145                 150                 155                 160

Gly Pro Gly Gln Tyr Gly Pro Gly Val Leu Gly Pro Ser Ala Ser Ala
            165                 170                 175

Ala Ala Ala Ala Gly Ser Gly Val Leu Gly Pro Gly Gln Tyr Gly Pro
            180                 185                 190

Tyr Ala Ser Ala Ala Ala Ala Ala Gly Gln Tyr Gly Ser Gly Pro Gly
            195                 200                 205

Val Leu Gly Pro Tyr Gly Pro Gly Gln Ser Gly Ser Gly Val Leu Gly
            210                 215                 220

Pro Gly Val Leu Gly Pro Tyr Ala Ser Ala Ala Ala Ala Ala Gly Pro
225                 230                 235                 240

Gly Val Leu Gly Pro Tyr Gly Pro Gly Ser Ser Ala Ala Ala Ala Ala
            245                 250                 255
```

250

Gly Gln Tyr Gly Tyr Gly Pro Gly Val Leu Gly Pro Tyr Gly Pro Gly
        260             265             270

Ala Ser Gly Gln Asn Gly Pro Gly Ser Gly Gln Tyr Gly Pro Gly Val
        275             280             285

Leu Gly Pro Gly Gln Ser Ala Ala Ala Ala Ala Gly Pro Gly Val Leu
        290             295             300

Gly Pro Tyr Gly Pro Gly Ala Ser Ala Ala Ala Ala Ala Gly Gln Tyr
305             310             315             320

Gly Pro Gly Val Leu Gly Pro Gly Gln Tyr Gly Pro Gly Ser Ser Gly
            325             330             335

Pro Gly Val Leu Gly Pro Tyr Gly Pro Gly Ser Ser Ala Ala Ala Ala
        340             345             350

Ala Gly Gln Tyr Gly Pro Gly Val Leu Gly Pro Tyr Gly Pro Gly Gln
        355             360             365

Ser Ala Ala Ala Ala Ala Gly Gln Tyr Val Leu Gly Pro Gly Val Leu
    370             375             380

Gly Pro Tyr Gly Pro Gly Ala Ser Gly Pro Gly Val Leu Gly Pro Tyr
385             390             395             400

Gly Pro Gly Ala Ser Ala Ala Ala Ala Ala Gly Pro Gly Gln Tyr Gly
            405             410             415

Pro Gly Val Leu Gly Pro Ser Ala Ser Ala Ala Ala Ala Ala Gly Gln
        420             425             430

Tyr Gly Ser Gly Pro Gly Gln Tyr Gly Pro Tyr Gly Pro Gly Gln Ser
        435             440             445

Gly Pro Gly Ser Gly Val Leu Gly Gln Gly Pro Tyr Gly Pro Gly Ala
    450             455             460

Ser Ala Ala Ala Ala Ala Gly Gln Tyr Gly Pro Gly Val Leu Gly Pro
465             470             475             480

Tyr Gly Pro Gly Gln Ser Ala Ala Ala Ala Gly Pro Gly Ser Gly Gln
            485             490             495

Gln Tyr Gly Pro Gly Ala Ser Gly Gln Asn Gly Pro Gly Ser Gly Gln

```
          500                    505                    510


    Tyr Gly Pro Gly Val Leu Gly Pro Gly Gln Ser Ala Ala Ala Ala Ala
            515                520                525


    Gly Gln Tyr Val Leu Gly Pro Gly Val Leu Gly Pro Tyr Gly Pro Gly
        530                535                540


    Ala Ser Ala Ala Ala Ala Ala Gly Gln Tyr Gly Ser Gly Pro Gly Val
    545             550                555                560


    Leu Gly Pro Tyr Gly Pro Gly Gln Ser Gly Ser Gly Val Leu Gly Pro
                565                570                575


    Gly Val Leu Gly Pro Tyr Ala Ser Ala Ala Ala Ala Ala Gly Pro Gly
                580                585                590


    Ser Gly Val Leu Gly Pro Gly Ala Ser
            595                600


    <210>  34
    <211>  601
    <212>  PRT
    <213>  Artificial Sequence

    <220>
    <223>  PRT965

    <400>  34

    Met His His His His His His Ser Ser Gly Ser Ser Gly Pro Gly Thr
    1                   5                   10                  15


    Ser Gly Pro Tyr Gly Pro Gly Ala Ser Ala Ala Ala Ala Ala Gly Ala
                20                 25                 30


    Asn Gly Pro Gly Ser Gly Thr Ser Gly Pro Gly Ala Ser Gly Ala Tyr
            35                 40                 45


    Gly Pro Gly Thr Ser Gly Pro Gly Thr Ser Gly Pro Gly Ser Ser Ala
        50                 55                 60


    Ala Ala Ala Ala Gly Pro Gly Ala Tyr Gly Pro Gly Thr Ser Gly Pro
    65                 70                 75                 80


    Ser Ala Ser Ala Ala Ala Ala Ala Gly Pro Gly Ser Gly Thr Ser Gly
                85                 90                 95


    Pro Gly Ala Ser Gly Ala Tyr Gly Pro Gly Thr Ser Gly Pro Gly Thr
                100                105                110
```

```
Ser Gly Pro Gly Ser Ser Ala Ala Ala Ala Ala Gly Ala Tyr Gly Ser
        115             120             125

Gly Pro Gly Thr Ser Gly Pro Tyr Gly Ser Ala Ala Ala Ala Ala Gly
        130             135             140

Pro Gly Ser Gly Ala Tyr Gly Ala Gly Pro Tyr Gly Pro Gly Ala Ser
145             150             155             160

Gly Pro Gly Ala Tyr Gly Pro Gly Thr Ser Gly Pro Ser Ala Ser Ala
                165             170             175

Ala Ala Ala Ala Gly Ser Gly Thr Ser Gly Pro Gly Ala Tyr Gly Pro
            180             185             190

Tyr Ala Ser Ala Ala Ala Ala Ala Gly Ala Tyr Gly Ser Gly Pro Gly
            195             200             205

Thr Ser Gly Pro Tyr Gly Pro Gly Ala Ser Gly Ser Gly Thr Ser Gly
    210             215             220

Pro Gly Thr Ser Gly Pro Tyr Ala Ser Ala Ala Ala Ala Ala Gly Pro
225             230             235             240

Gly Thr Ser Gly Pro Tyr Gly Pro Gly Ser Ser Ala Ala Ala Ala Ala
            245             250             255

Gly Ala Tyr Gly Tyr Gly Pro Gly Thr Ser Gly Pro Tyr Gly Pro Gly
            260             265             270

Ala Ser Gly Ala Asn Gly Pro Gly Ser Gly Ala Tyr Gly Pro Gly Thr
    275             280             285

Ser Gly Pro Gly Ala Ser Ala Ala Ala Ala Gly Pro Gly Thr Ser
    290             295             300

Gly Pro Tyr Gly Pro Gly Ala Ser Ala Ala Ala Ala Gly Ala Tyr
305             310             315             320

Gly Pro Gly Thr Ser Gly Pro Gly Ala Tyr Gly Pro Gly Ser Ser Gly
            325             330             335

Pro Gly Thr Ser Gly Pro Tyr Gly Pro Gly Ser Ser Ala Ala Ala Ala
    340             345             350

Ala Gly Ala Tyr Gly Pro Gly Thr Ser Gly Pro Tyr Gly Pro Gly Ala
```

EP 3 779 031 A1

355                          360                          365

Ser Ala Ala Ala Ala Ala Gly Ala Tyr Thr Ser Gly Pro Gly Thr Ser
    370                  375              380

Gly Pro Tyr Gly Pro Gly Ala Ser Gly Pro Gly Thr Ser Gly Pro Tyr
385              390              395                  400

Gly Pro Gly Ala Ser Ala Ala Ala Ala Ala Gly Pro Gly Ala Tyr Gly
             405              410                  415

Pro Gly Thr Ser Gly Pro Ser Ala Ser Ala Ala Ala Ala Ala Gly Ala
             420              425                  430

Tyr Gly Ser Gly Pro Gly Ala Tyr Gly Pro Tyr Gly Pro Gly Ala Ser
         435              440              445

Gly Pro Gly Ser Gly Thr Ser Gly Ala Gly Pro Tyr Gly Pro Gly Ala
    450              455              460

Ser Ala Ala Ala Ala Ala Gly Ala Tyr Gly Pro Gly Thr Ser Gly Pro
465              470              475                  480

Tyr Gly Pro Gly Ala Ser Ala Ala Ala Ala Gly Pro Gly Ser Gly
             485              490                  495

Ala Tyr Gly Pro Gly Ala Ser Gly Ala Asn Gly Pro Gly Ser Gly Ala
    500              505              510

Tyr Gly Pro Gly Thr Ser Gly Pro Gly Ala Ser Ala Ala Ala Ala Ala
    515              520              525

Gly Ala Tyr Thr Ser Gly Pro Gly Thr Ser Gly Pro Tyr Gly Pro Gly
    530              535              540

Ala Ser Ala Ala Ala Ala Ala Gly Ala Tyr Gly Ser Gly Pro Gly Thr
545              550              555                  560

Ser Gly Pro Tyr Gly Pro Gly Ala Ser Gly Ser Gly Thr Ser Gly Pro
             565              570              575

Gly Thr Ser Gly Pro Tyr Ala Ser Ala Ala Ala Ala Gly Pro Gly
             580              585                  590

Ser Gly Thr Ser Gly Pro Gly Ala Ser
    595              600

254

```
<210>   35
<211>   601
<212>   PRT
<213>   Artificial Sequence

<220>
<223>   PRT889

<400>   35


Met His His His His His His Ser Ser Gly Ser Ser Gly Pro Gly Val
1               5                   10                  15


Leu Gly Pro Tyr Gly Pro Gly Ala Ser Ala Ala Ala Ala Ala Gly Ile
            20                  25                  30


Asn Gly Pro Gly Ser Gly Val Leu Gly Pro Gly Ile Ser Gly Ile Tyr
            35                  40                  45


Gly Pro Gly Val Leu Gly Pro Gly Val Leu Gly Pro Gly Ser Ser Ala
        50                  55                  60


Ala Ala Ala Ala Gly Pro Gly Ile Tyr Gly Pro Gly Val Leu Gly Pro
65                  70                  75                  80


Ser Ala Ser Ala Ala Ala Ala Ala Gly Pro Gly Ser Gly Val Leu Gly
                85                  90                  95


Pro Gly Ala Ser Gly Ile Tyr Gly Pro Gly Val Leu Gly Pro Gly Val
            100                 105                 110


Leu Gly Pro Gly Ser Ser Ala Ala Ala Ala Ala Gly Ile Tyr Gly Ser
            115                 120                 125


Gly Pro Gly Val Leu Gly Pro Tyr Gly Ser Ala Ala Ala Ala Ala Gly
            130                 135                 140


Pro Gly Ser Gly Ile Tyr Gly Ile Gly Pro Tyr Gly Pro Gly Ala Ser
145                 150                 155                 160


Gly Pro Gly Ile Tyr Gly Pro Gly Val Leu Gly Pro Ser Ala Ser Ala
                165                 170                 175


Ala Ala Ala Ala Gly Ser Gly Val Leu Gly Pro Gly Ile Tyr Gly Pro
                180                 185                 190


Tyr Ala Ser Ala Ala Ala Ala Ala Gly Ile Tyr Gly Ser Gly Pro Gly
            195                 200                 205


Val Leu Gly Pro Tyr Gly Pro Gly Ile Ser Gly Ser Gly Val Leu Gly
```

210                     215                     220

Pro Gly Val Leu Gly Pro Tyr Ala Ser Ala Ala Ala Ala Ala Gly Pro
225                 230             235                 240


Gly Val Leu Gly Pro Tyr Gly Pro Gly Ser Ser Ala Ala Ala Ala Ala
                245             250             255


Gly Ile Tyr Gly Tyr Gly Pro Gly Val Leu Gly Pro Tyr Gly Pro Gly
            260             265             270


Ala Ser Gly Ile Asn Gly Pro Gly Ser Gly Ile Tyr Gly Pro Gly Val
        275             280             285


Leu Gly Pro Gly Ile Ser Ala Ala Ala Ala Gly Pro Gly Val Leu
    290             295             300


Gly Pro Tyr Gly Pro Gly Ala Ser Ala Ala Ala Ala Gly Ile Tyr
305             310             315             320


Gly Pro Gly Val Leu Gly Pro Gly Ile Tyr Gly Pro Gly Ser Ser Gly
            325             330             335


Pro Gly Val Leu Gly Pro Tyr Gly Pro Gly Ser Ser Ala Ala Ala Ala
    340             345             350


Ala Gly Ile Tyr Gly Pro Gly Val Leu Gly Pro Tyr Gly Pro Gly Ile
    355             360             365


Ser Ala Ala Ala Ala Ala Gly Ile Tyr Val Leu Gly Pro Gly Val Leu
    370             375             380


Gly Pro Tyr Gly Pro Gly Ala Ser Gly Pro Gly Val Leu Gly Pro Tyr
385             390             395             400


Gly Pro Gly Ala Ser Ala Ala Ala Ala Gly Pro Gly Ile Tyr Gly
            405             410             415


Pro Gly Val Leu Gly Pro Ser Ala Ser Ala Ala Ala Ala Gly Ile
        420             425             430


Tyr Gly Ser Gly Pro Gly Ile Tyr Gly Pro Tyr Gly Pro Gly Ile Ser
    435             440             445


Gly Pro Gly Ser Gly Val Leu Gly Ile Gly Pro Tyr Gly Pro Gly Ala
    450             455             460

```
Ser Ala Ala Ala Ala Ala Gly Ile Tyr Gly Pro Gly Val Leu Gly Pro
465             470             475             480

Tyr Gly Pro Gly Ile Ser Ala Ala Ala Ala Gly Pro Gly Ser Gly
            485             490             495

Ile Tyr Gly Pro Gly Ala Ser Gly Ile Asn Gly Pro Gly Ser Gly Ile
            500             505             510

Tyr Gly Pro Gly Val Leu Gly Pro Gly Ile Ser Ala Ala Ala Ala Ala
            515             520             525

Gly Ile Tyr Val Leu Gly Pro Gly Val Leu Gly Pro Tyr Gly Pro Gly
    530             535             540

Ala Ser Ala Ala Ala Ala Ala Gly Ile Tyr Gly Ser Gly Pro Gly Val
545             550             555             560

Leu Gly Pro Tyr Gly Pro Gly Ile Ser Gly Ser Gly Val Leu Gly Pro
            565             570             575

Gly Val Leu Gly Pro Tyr Ala Ser Ala Ala Ala Ala Ala Gly Pro Gly
            580             585             590

Ser Gly Val Leu Gly Pro Gly Ala Ser
        595             600
```

```
<210>   36
<211>   601
<212>   PRT
<213>   Artificial Sequence

<220>
<223>   PRT916

<400>   36
```

```
Met His His His His His His Ser Ser Gly Ser Ser Gly Pro Gly Val
1               5               10              15

Ile Gly Pro Tyr Gly Pro Gly Ala Ser Ala Ala Ala Ala Ala Gly Leu
            20              25              30

Asn Gly Pro Gly Ser Gly Val Ile Gly Pro Gly Leu Ser Gly Leu Tyr
            35              40              45

Gly Pro Gly Val Ile Gly Pro Gly Val Ile Gly Pro Gly Ser Ser Ala
    50              55              60

Ala Ala Ala Ala Gly Pro Gly Leu Tyr Gly Pro Gly Val Ile Gly Pro
```

|     |     |     | 65  |     |     |     | 70  |     |     |     | 75  |     |     |     | 80  |

Ser Ala Ser Ala Ala Ala Ala Ala Gly Pro Gly Ser Gly Val Ile Gly
            85                  90                  95

Pro Gly Ala Ser Gly Leu Tyr Gly Pro Gly Val Ile Gly Pro Gly Val
            100                 105                 110

Ile Gly Pro Gly Ser Ser Ala Ala Ala Ala Gly Leu Tyr Gly Ser
            115                 120                 125

Gly Pro Gly Val Ile Gly Pro Tyr Gly Ser Ala Ala Ala Ala Gly
            130                 135                 140

Pro Gly Ser Gly Leu Tyr Gly Leu Gly Pro Tyr Gly Pro Gly Ala Ser
145                 150                 155                 160

Gly Pro Gly Leu Tyr Gly Pro Gly Val Ile Gly Pro Ser Ala Ser Ala
            165                 170                 175

Ala Ala Ala Ala Gly Ser Gly Val Ile Gly Pro Gly Leu Tyr Gly Pro
            180                 185                 190

Tyr Ala Ser Ala Ala Ala Ala Ala Gly Leu Tyr Gly Ser Gly Pro Gly
            195                 200                 205

Val Ile Gly Pro Tyr Gly Pro Gly Leu Ser Gly Ser Gly Val Ile Gly
            210                 215                 220

Pro Gly Val Ile Gly Pro Tyr Ala Ser Ala Ala Ala Ala Gly Pro
225                 230                 235                 240

Gly Val Ile Gly Pro Tyr Gly Pro Gly Ser Ser Ala Ala Ala Ala Ala
            245                 250                 255

Gly Leu Tyr Gly Tyr Gly Pro Gly Val Ile Gly Pro Tyr Gly Pro Gly
            260                 265                 270

Ala Ser Gly Leu Asn Gly Pro Gly Ser Gly Leu Tyr Gly Pro Gly Val
            275                 280                 285

Ile Gly Pro Gly Leu Ser Ala Ala Ala Ala Gly Pro Gly Val Ile
            290                 295                 300

Gly Pro Tyr Gly Pro Gly Ala Ser Ala Ala Ala Ala Gly Leu Tyr
305                 310                 315                 320

258

Gly Pro Gly Val Ile Gly Pro Gly Leu Tyr Gly Pro Gly Ser Ser Gly
                325                 330                 335

Pro Gly Val Ile Gly Pro Tyr Gly Pro Gly Ser Ser Ala Ala Ala Ala
                340                 345                 350

Ala Gly Leu Tyr Gly Pro Gly Val Ile Gly Pro Tyr Gly Pro Gly Leu
                355                 360                 365

Ser Ala Ala Ala Ala Ala Gly Leu Tyr Val Ile Gly Pro Gly Val Ile
    370                 375                 380

Gly Pro Tyr Gly Pro Gly Ala Ser Gly Pro Gly Val Ile Gly Pro Tyr
385                 390                 395                 400

Gly Pro Gly Ala Ser Ala Ala Ala Ala Ala Gly Pro Gly Leu Tyr Gly
                405                 410                 415

Pro Gly Val Ile Gly Pro Ser Ala Ser Ala Ala Ala Ala Gly Leu
                420                 425                 430

Tyr Gly Ser Gly Pro Gly Leu Tyr Gly Pro Tyr Gly Pro Gly Leu Ser
    435                 440                 445

Gly Pro Gly Ser Gly Val Ile Gly Leu Gly Pro Tyr Gly Pro Gly Ala
    450                 455                 460

Ser Ala Ala Ala Ala Ala Gly Leu Tyr Gly Pro Gly Val Ile Gly Pro
465                 470                 475                 480

Tyr Gly Pro Gly Leu Ser Ala Ala Ala Ala Gly Pro Gly Ser Gly
                485                 490                 495

Leu Tyr Gly Pro Gly Ala Ser Gly Leu Asn Gly Pro Gly Ser Gly Leu
            500                 505                 510

Tyr Gly Pro Gly Val Ile Gly Pro Gly Leu Ser Ala Ala Ala Ala Ala
        515                 520                 525

Gly Leu Tyr Val Ile Gly Pro Gly Val Ile Gly Pro Tyr Gly Pro Gly
    530                 535                 540

Ala Ser Ala Ala Ala Ala Gly Leu Tyr Gly Ser Gly Pro Gly Val
545                 550                 555                 560

Ile Gly Pro Tyr Gly Pro Gly Leu Ser Gly Ser Gly Val Ile Gly Pro
                565                 570                 575

259

```
Gly Val Ile Gly Pro Tyr Ala Ser Ala Ala Ala Ala Gly Pro Gly
        580             585             590


Ser Gly Val Ile Gly Pro Gly Ala Ser
        595             600


<210> 37
<211> 601
<212> PRT
<213> Artificial Sequence

<220>
<223> PRT918

<400> 37

Met His His His His His His Ser Ser Gly Ser Ser Gly Pro Gly Val
1               5               10              15


Phe Gly Pro Tyr Gly Pro Gly Ala Ser Ala Ala Ala Ala Gly Ile
        20              25              30


Asn Gly Pro Gly Ser Gly Val Phe Gly Pro Gly Ile Ser Gly Ile Tyr
        35              40              45


Gly Pro Gly Val Phe Gly Pro Gly Val Phe Gly Pro Gly Ser Ser Ala
        50              55              60


Ala Ala Ala Ala Gly Pro Gly Ile Tyr Gly Pro Gly Val Phe Gly Pro
65              70              75              80


Ser Ala Ser Ala Ala Ala Ala Ala Gly Pro Gly Ser Gly Val Phe Gly
                85              90              95


Pro Gly Ala Ser Gly Ile Tyr Gly Pro Gly Val Phe Gly Pro Gly Val
        100             105             110


Phe Gly Pro Gly Ser Ser Ala Ala Ala Ala Gly Ile Tyr Gly Ser
        115             120             125


Gly Pro Gly Val Phe Gly Pro Tyr Gly Ser Ala Ala Ala Ala Gly
        130             135             140


Pro Gly Ser Gly Ile Tyr Gly Ile Gly Pro Tyr Gly Pro Gly Ala Ser
145             150             155             160


Gly Pro Gly Ile Tyr Gly Pro Gly Val Phe Gly Pro Ser Ala Ser Ala
                165             170             175
```

260

Ala Ala Ala Ala Gly Ser Gly Val Phe Gly Pro Gly Ile Tyr Gly Pro
            180                 185                 190

Tyr Ala Ser Ala Ala Ala Ala Ala Gly Ile Tyr Gly Ser Gly Pro Gly
            195                 200                 205

Val Phe Gly Pro Tyr Gly Pro Gly Ile Ser Gly Ser Gly Val Phe Gly
            210                 215                 220

Pro Gly Val Phe Gly Pro Tyr Ala Ser Ala Ala Ala Ala Ala Gly Pro
225                 230                 235                 240

Gly Val Phe Gly Pro Tyr Gly Pro Gly Ser Ser Ala Ala Ala Ala Ala
            245                 250                 255

Gly Ile Tyr Gly Tyr Gly Pro Gly Val Phe Gly Pro Tyr Gly Pro Gly
            260                 265                 270

Ala Ser Gly Ile Asn Gly Pro Gly Ser Gly Ile Tyr Gly Pro Gly Val
            275                 280                 285

Phe Gly Pro Gly Ile Ser Ala Ala Ala Ala Gly Pro Gly Val Phe
            290                 295                 300

Gly Pro Tyr Gly Pro Gly Ala Ser Ala Ala Ala Ala Ala Gly Ile Tyr
305                 310                 315                 320

Gly Pro Gly Val Phe Gly Pro Gly Ile Tyr Gly Pro Gly Ser Ser Gly
            325                 330                 335

Pro Gly Val Phe Gly Pro Tyr Gly Pro Gly Ser Ser Ala Ala Ala Ala
            340                 345                 350

Ala Gly Ile Tyr Gly Pro Gly Val Phe Gly Pro Tyr Gly Pro Gly Ile
            355                 360                 365

Ser Ala Ala Ala Ala Ala Gly Ile Tyr Val Phe Gly Pro Gly Val Phe
370                 375                 380

Gly Pro Tyr Gly Pro Gly Ala Ser Gly Pro Gly Val Phe Gly Pro Tyr
385                 390                 395                 400

Gly Pro Gly Ala Ser Ala Ala Ala Ala Ala Gly Pro Gly Ile Tyr Gly
            405                 410                 415

Pro Gly Val Phe Gly Pro Ser Ala Ser Ala Ala Ala Ala Ala Gly Ile
            420                 425                 430

Tyr Gly Ser Gly Pro Gly Ile Tyr Gly Pro Tyr Gly Pro Gly Ile Ser
435                 440             445

Gly Pro Gly Ser Gly Val Phe Gly Ile Gly Pro Tyr Gly Pro Gly Ala
    450                 455             460

Ser Ala Ala Ala Ala Ala Gly Ile Tyr Gly Pro Gly Val Phe Gly Pro
465                 470             475                 480

Tyr Gly Pro Gly Ile Ser Ala Ala Ala Ala Gly Pro Gly Ser Gly
                485             490             495

Ile Tyr Gly Pro Gly Ala Ser Gly Ile Asn Gly Pro Gly Ser Gly Ile
        500             505             510

Tyr Gly Pro Gly Val Phe Gly Pro Gly Ile Ser Ala Ala Ala Ala Ala
    515             520             525

Gly Ile Tyr Val Phe Gly Pro Gly Val Phe Gly Pro Tyr Gly Pro Gly
    530             535             540

Ala Ser Ala Ala Ala Ala Gly Ile Tyr Gly Ser Gly Pro Gly Val
545             550             555                 560

Phe Gly Pro Tyr Gly Pro Gly Ile Ser Gly Ser Gly Val Phe Gly Pro
            565             570             575

Gly Val Phe Gly Pro Tyr Ala Ser Ala Ala Ala Ala Gly Pro Gly
            580             585             590

Ser Gly Val Phe Gly Pro Gly Ala Ser
        595             600

<210> 38
<211> 576
<212> PRT
<213> Artificial Sequence

<220>
<223> PRT699

<400> 38

Met His His His His His His Ser Ser Gly Ser Ser Gly Pro Gly Val
1               5                   10                  15

Leu Gly Pro Tyr Gly Pro Gly Ala Ser Ala Ala Ala Ala Ala Ala
        20                  25                  30

```
Gly Ser Asn Gly Pro Gly Ser Gly Val Leu Gly Pro Gly Gln Ser Gly
        35              40              45

Gln Tyr Gly Pro Gly Val Leu Gly Pro Gly Val Leu Gly Pro Gly Ser
        50              55              60

Ser Ala Ala Ala Ala Ala Ala Ala Gly Pro Gly Gln Tyr Gly Pro Gly
65              70              75              80

Val Leu Gly Pro Ser Ala Ser Ala Ala Ala Ala Ala Ala Gly Pro
            85              90              95

Gly Ser Gly Val Leu Gly Pro Gly Ala Ser Gly Gln Tyr Gly Pro Gly
            100             105             110

Val Leu Gly Pro Gly Val Leu Gly Pro Gly Ser Ser Ala Ala Ala Ala
            115             120             125

Ala Ala Ala Gly Ser Tyr Gly Ser Gly Pro Gly Val Leu Gly Pro Tyr
    130             135             140

Gly Ser Ala Ala Ala Ala Ala Ala Ala Gly Pro Gly Ser Gly Gln Tyr
145             150             155             160

Gly Gln Gly Pro Tyr Gly Pro Gly Ala Ser Gly Pro Gly Gln Tyr Gly
            165             170             175

Pro Gly Val Leu Gly Pro Ser Ala Ser Ala Ala Ala Ala Ala Ala Ala
            180             185             190

Gly Ser Gly Val Leu Gly Pro Gly Gln Tyr Gly Pro Tyr Ala Ser Ala
            195             200             205

Ala Ala Ala Ala Ala Gly Ser Tyr Gly Ser Gly Pro Gly Val Leu
    210             215             220

Gly Pro Tyr Gly Pro Gly Gln Ser Gly Ser Gly Val Leu Gly Pro Gly
225             230             235             240

Val Leu Gly Pro Tyr Ala Ser Ala Ala Ala Ala Ala Ala Gly Pro
            245             250             255

Gly Val Leu Gly Pro Tyr Gly Pro Gly Ser Ser Ala Ala Ala Ala Ala
            260             265             270

Ala Ala Gly Ser Tyr Gly Tyr Gly Pro Gly Val Leu Gly Pro Tyr Gly
    275             280             285
```

Pro Gly Ala Ser Gly Gln Asn Gly Pro Gly Ser Gly Gln Tyr Gly Pro
    290                 295                 300

Gly Val Leu Gly Pro Gly Pro Ser Ala Ala Ala Ala Ala Ala Ala Gly
305                 310                 315                 320

Pro Gly Val Leu Gly Pro Tyr Gly Pro Gly Ala Ser Ala Ala Ala Ala
                325                 330                 335

Ala Ala Ala Gly Ser Tyr Gly Pro Gly Val Leu Gly Pro Gly Gln Tyr
            340                 345                 350

Gly Pro Gly Ser Ser Gly Pro Gly Val Leu Gly Pro Tyr Gly Pro Gly
            355                 360                 365

Ser Ser Ala Ala Ala Ala Ala Ala Ala Gly Ser Tyr Gly Pro Gly Val
    370                 375                 380

Leu Gly Pro Tyr Gly Pro Gly Pro Ser Ala Ala Ala Ala Ala Ala Ala
385                 390                 395                 400

Gly Ser Tyr Val Leu Gly Pro Gly Val Leu Gly Pro Tyr Gly Pro Gly
            405                 410                 415

Ala Ser Gly Pro Gly Val Leu Gly Pro Tyr Gly Pro Gly Ala Ser Ala
            420                 425                 430

Ala Ala Ala Ala Ala Gly Pro Gly Gln Tyr Gly Pro Gly Val Leu
    435                 440                 445

Gly Pro Ser Ala Ser Ala Ala Ala Ala Ala Ala Gly Ser Tyr Gly
    450                 455                 460

Ser Gly Pro Gly Gln Tyr Gly Pro Tyr Gly Pro Gly Gln Ser Gly Pro
465                 470                 475                 480

Gly Ser Gly Val Leu Gly Gln Gly Pro Tyr Gly Pro Gly Ala Ser Ala
            485                 490                 495

Ala Ala Ala Ala Ala Gly Ser Tyr Gly Pro Gly Val Leu Gly Pro
    500                 505                 510

Tyr Gly Pro Gly Pro Ser Ala Ala Ala Ala Ala Ala Gly Pro Gly
    515                 520                 525

Ser Gly Gln Tyr Gly Pro Gly Ala Ser Gly Gln Asn Gly Pro Gly Ser
    530                 535                 540

**264**

Gly Gln Tyr Gly Pro Gly Val Leu Gly Pro Gly Pro Ser Ala Ala Ala
545 550 555 560

Ala Ala Ala Ala Gly Pro Gly Ser Gly Val Leu Gly Pro Gly Ala Ser
565 570 575

<210> 39
<211> 576
<212> PRT
<213> Artificial Sequence

<220>
<223> PRT698

<400> 39

Met His His His His His His Ser Ser Gly Ser Ser Gly Pro Gly Val
1 5 10 15

Leu Gly Pro Tyr Gly Pro Gly Ala Ser Ala Ala Ala Ala Ala Ala Ala
20 25 30

Gly Ser Asn Gly Pro Gly Ser Gly Val Leu Gly Pro Gly Ile Ser Gly
35 40 45

Ile Tyr Gly Pro Gly Val Leu Gly Pro Gly Val Leu Gly Pro Gly Ser
50 55 60

Ser Ala Ala Ala Ala Ala Ala Ala Gly Pro Gly Ile Tyr Gly Pro Gly
65 70 75 80

Val Leu Gly Pro Ser Ala Ser Ala Ala Ala Ala Ala Ala Ala Gly Pro
85 90 95

Gly Ser Gly Val Leu Gly Pro Gly Ala Ser Gly Ile Tyr Gly Pro Gly
100 105 110

Val Leu Gly Pro Gly Val Leu Gly Pro Gly Ser Ser Ala Ala Ala Ala
115 120 125

Ala Ala Ala Gly Ser Tyr Gly Ser Gly Pro Gly Val Leu Gly Pro Tyr
130 135 140

Gly Ser Ala Ala Ala Ala Ala Ala Ala Gly Pro Gly Ser Gly Ile Tyr
145 150 155 160

Gly Ile Gly Pro Tyr Gly Pro Gly Ala Ser Gly Pro Gly Ile Tyr Gly
165 170 175

```
Pro Gly Val Leu Gly Pro Ser Ala Ser Ala Ala Ala Ala Ala Ala
        180             185             190

Gly Ser Gly Val Leu Gly Pro Gly Ile Tyr Gly Pro Tyr Ala Ser Ala
        195             200             205

Ala Ala Ala Ala Ala Ala Gly Ser Tyr Gly Ser Gly Pro Gly Val Leu
        210             215             220

Gly Pro Tyr Gly Pro Gly Ile Ser Gly Ser Gly Val Leu Gly Pro Gly
225             230             235             240

Val Leu Gly Pro Tyr Ala Ser Ala Ala Ala Ala Ala Ala Gly Pro
        245             250             255

Gly Val Leu Gly Pro Tyr Gly Pro Gly Ser Ser Ala Ala Ala Ala Ala
        260             265             270

Ala Ala Gly Ser Tyr Gly Tyr Gly Pro Gly Val Leu Gly Pro Tyr Gly
        275             280             285

Pro Gly Ala Ser Gly Ile Asn Gly Pro Gly Ser Gly Ile Tyr Gly Pro
        290             295             300

Gly Val Leu Gly Pro Gly Pro Ser Ala Ala Ala Ala Ala Ala Gly
305             310             315             320

Pro Gly Val Leu Gly Pro Tyr Gly Pro Gly Ala Ser Ala Ala Ala Ala
        325             330             335

Ala Ala Ala Gly Ser Tyr Gly Pro Gly Val Leu Gly Pro Gly Ile Tyr
        340             345             350

Gly Pro Gly Ser Ser Gly Pro Gly Val Leu Gly Pro Tyr Gly Pro Gly
        355             360             365

Ser Ser Ala Ala Ala Ala Ala Ala Gly Ser Tyr Gly Pro Gly Val
370             375             380

Leu Gly Pro Tyr Gly Pro Gly Pro Ser Ala Ala Ala Ala Ala Ala
385             390             395             400

Gly Ser Tyr Val Leu Gly Pro Gly Val Leu Gly Pro Tyr Gly Pro Gly
        405             410             415

Ala Ser Gly Pro Gly Val Leu Gly Pro Tyr Gly Pro Gly Ala Ser Ala
        420             425             430
```

266

```
Ala Ala Ala Ala Ala Ala Gly Pro Gly Ile Tyr Gly Pro Gly Val Leu
        435                 440             445

Gly Pro Ser Ala Ser Ala Ala Ala Ala Ala Ala Gly Ser Tyr Gly
    450             455             460

Ser Gly Pro Gly Ile Tyr Gly Pro Tyr Gly Pro Gly Ile Ser Gly Pro
465                 470             475                 480

Gly Ser Gly Val Leu Gly Ile Gly Pro Tyr Gly Pro Gly Ala Ser Ala
                485             490             495

Ala Ala Ala Ala Ala Ala Gly Ser Tyr Gly Pro Gly Val Leu Gly Pro
        500             505             510

Tyr Gly Pro Gly Pro Ser Ala Ala Ala Ala Ala Ala Ala Gly Pro Gly
    515             520             525

Ser Gly Ile Tyr Gly Pro Gly Ala Ser Gly Ile Asn Gly Pro Gly Ser
    530             535             540

Gly Ile Tyr Gly Pro Gly Val Leu Gly Pro Gly Pro Ser Ala Ala Ala
545             550             555             560

Ala Ala Ala Ala Gly Pro Gly Ser Gly Val Leu Gly Pro Gly Ala Ser
            565             570             575
```

<210> 40
<211> 1190
<212> PRT
<213> Artificial Sequence

<220>
<223> PRT966

<400> 40

```
Met His His His His His His Ser Ser Gly Ser Ser Gly Pro Gly Val
1                   5                   10                  15

Phe Gly Pro Tyr Gly Pro Gly Ala Ser Ala Ala Ala Ala Ala Gly Ile
                20                  25                  30

Asn Gly Pro Gly Ser Gly Val Phe Gly Pro Gly Ile Ser Gly Ile Tyr
            35                  40                  45

Gly Pro Gly Val Phe Gly Pro Gly Val Phe Gly Pro Gly Ser Ser Ala
    50                  55                  60
```

EP 3 779 031 A1

```
Ala Ala Ala Ala Gly Pro Gly Ile Tyr Gly Pro Gly Val Phe Gly Pro
65              70              75              80

Ser Ala Ser Ala Ala Ala Ala Ala Gly Pro Gly Ser Gly Val Phe Gly
                85              90              95

Pro Gly Ala Ser Gly Ile Tyr Gly Pro Gly Val Phe Gly Pro Gly Val
            100             105             110

Phe Gly Pro Gly Ser Ser Ala Ala Ala Ala Gly Ile Tyr Gly Ser
            115             120             125

Gly Pro Gly Val Phe Gly Pro Tyr Gly Ser Ala Ala Ala Ala Gly
    130             135             140

Pro Gly Ser Gly Ile Tyr Gly Ile Gly Pro Tyr Gly Pro Gly Ala Ser
145             150             155             160

Gly Pro Gly Ile Tyr Gly Pro Gly Val Phe Gly Pro Ser Ala Ser Ala
            165             170             175

Ala Ala Ala Ala Gly Ser Gly Val Phe Gly Pro Gly Ile Tyr Gly Pro
            180             185             190

Tyr Ala Ser Ala Ala Ala Ala Ala Gly Ile Tyr Gly Ser Gly Pro Gly
    195             200             205

Val Phe Gly Pro Tyr Gly Pro Gly Ile Ser Gly Ser Gly Val Phe Gly
    210             215             220

Pro Gly Val Phe Gly Pro Tyr Ala Ser Ala Ala Ala Ala Gly Pro
225             230             235             240

Gly Val Phe Gly Pro Tyr Gly Pro Gly Ser Ser Ala Ala Ala Ala
            245             250             255

Gly Ile Tyr Gly Tyr Gly Pro Gly Val Phe Gly Pro Tyr Gly Pro Gly
    260             265             270

Ala Ser Gly Ile Asn Gly Pro Gly Ser Gly Ile Tyr Gly Pro Gly Val
    275             280             285

Phe Gly Pro Gly Ile Ser Ala Ala Ala Ala Gly Pro Gly Val Phe
    290             295             300

Gly Pro Tyr Gly Pro Gly Ala Ser Ala Ala Ala Ala Gly Ile Tyr
305             310             315             320
```

Gly Pro Gly Val Phe Gly Pro Gly Ile Tyr Gly Pro Gly Ser Ser Gly
                325                      330                   335

Pro Gly Val Phe Gly Pro Tyr Gly Pro Gly Ser Ser Ala Ala Ala Ala
            340                   345                   350

Ala Gly Ile Tyr Gly Pro Gly Val Phe Gly Pro Tyr Gly Pro Gly Ile
        355                   360                   365

Ser Ala Ala Ala Ala Ala Gly Ile Tyr Val Phe Gly Pro Gly Val Phe
    370                   375                   380

Gly Pro Tyr Gly Pro Gly Ala Ser Gly Pro Gly Val Phe Gly Pro Tyr
385                   390                   395                   400

Gly Pro Gly Ala Ser Ala Ala Ala Ala Gly Pro Gly Ile Tyr Gly
                405                   410                   415

Pro Gly Val Phe Gly Pro Ser Ala Ser Ala Ala Ala Ala Gly Ile
            420                   425                   430

Tyr Gly Ser Gly Pro Gly Ile Tyr Gly Pro Tyr Gly Pro Gly Ile Ser
        435                   440                   445

Gly Pro Gly Ser Gly Val Phe Gly Ile Gly Pro Tyr Gly Pro Gly Ala
    450                   455                   460

Ser Ala Ala Ala Ala Ala Gly Ile Tyr Gly Pro Gly Val Phe Gly Pro
465                   470                   475                   480

Tyr Gly Pro Gly Ile Ser Ala Ala Ala Ala Gly Pro Gly Ser Gly
                485                   490                   495

Ile Tyr Gly Pro Gly Ala Ser Gly Ile Asn Gly Pro Gly Ser Gly Ile
        500                   505                   510

Tyr Gly Pro Gly Val Phe Gly Pro Gly Ile Ser Ala Ala Ala Ala Ala
        515                   520                   525

Gly Ile Tyr Val Phe Gly Pro Gly Val Phe Gly Pro Tyr Gly Pro Gly
    530                   535                   540

Ala Ser Ala Ala Ala Ala Ala Gly Ile Tyr Gly Ser Gly Pro Gly Val
545                   550                   555                   560

Phe Gly Pro Tyr Gly Pro Gly Ile Ser Gly Ser Gly Val Phe Gly Pro

565 570 575

Gly Val Phe Gly Pro Tyr Ala Ser Ala Ala Ala Ala Ala Gly Pro Gly
580 585 590

Ser Gly Val Phe Gly Pro Gly Ala Ser Gly Pro Gly Val Phe Gly Pro
595 600 605

Tyr Gly Pro Gly Ala Ser Ala Ala Ala Ala Gly Ile Asn Gly Pro
610 615 620

Gly Ser Gly Val Phe Gly Pro Gly Ile Ser Gly Ile Tyr Gly Pro Gly
625 630 635 640

Val Phe Gly Pro Gly Val Phe Gly Pro Gly Ser Ser Ala Ala Ala Ala
645 650 655

Ala Gly Pro Gly Ile Tyr Gly Pro Gly Val Phe Gly Pro Ser Ala Ser
660 665 670

Ala Ala Ala Ala Ala Gly Pro Gly Ser Gly Val Phe Gly Pro Gly Ala
675 680 685

Ser Gly Ile Tyr Gly Pro Gly Val Phe Gly Pro Gly Val Phe Gly Pro
690 695 700

Gly Ser Ser Ala Ala Ala Ala Ala Gly Ile Tyr Gly Ser Gly Pro Gly
705 710 715 720

Val Phe Gly Pro Tyr Gly Ser Ala Ala Ala Ala Gly Pro Gly Ser
725 730 735

Gly Ile Tyr Gly Ile Gly Pro Tyr Gly Pro Gly Ala Ser Gly Pro Gly
740 745 750

Ile Tyr Gly Pro Gly Val Phe Gly Pro Ser Ala Ser Ala Ala Ala Ala
755 760 765

Ala Gly Ser Gly Val Phe Gly Pro Gly Ile Tyr Gly Pro Tyr Ala Ser
770 775 780

Ala Ala Ala Ala Ala Gly Ile Tyr Gly Ser Gly Pro Gly Val Phe Gly
785 790 795 800

Pro Tyr Gly Pro Gly Ile Ser Gly Ser Gly Val Phe Gly Pro Gly Val
805 810 815

```
Phe Gly Pro Tyr Ala Ser Ala Ala Ala Ala Ala Gly Pro Gly Val Phe
        820                 825                 830

Gly Pro Tyr Gly Pro Gly Ser Ser Ala Ala Ala Ala Ala Gly Ile Tyr
        835                 840                 845

Gly Tyr Gly Pro Gly Val Phe Gly Pro Tyr Gly Pro Gly Ala Ser Gly
        850                 855                 860

Ile Asn Gly Pro Gly Ser Gly Ile Tyr Gly Pro Gly Val Phe Gly Pro
865                 870                 875                 880

Gly Ile Ser Ala Ala Ala Ala Ala Gly Pro Gly Val Phe Gly Pro Tyr
                885                 890                 895

Gly Pro Gly Ala Ser Ala Ala Ala Ala Gly Ile Tyr Gly Pro Gly
                900                 905                 910

Val Phe Gly Pro Gly Ile Tyr Gly Pro Gly Ser Ser Gly Pro Gly Val
        915                 920                 925

Phe Gly Pro Tyr Gly Pro Gly Ser Ser Ala Ala Ala Ala Ala Gly Ile
        930                 935                 940

Tyr Gly Pro Gly Val Phe Gly Pro Tyr Gly Pro Gly Ile Ser Ala Ala
945                 950                 955                 960

Ala Ala Ala Gly Ile Tyr Val Phe Gly Pro Gly Val Phe Gly Pro Tyr
                965                 970                 975

Gly Pro Gly Ala Ser Gly Pro Gly Val Phe Gly Pro Tyr Gly Pro Gly
                980                 985                 990

Ala Ser Ala Ala Ala Ala Ala Gly Pro Gly Ile Tyr Gly Pro Gly Val
                995                 1000                1005

Phe Gly Pro Ser Ala Ser Ala Ala Ala Ala Gly Ile Tyr Gly
        1010                1015                1020

Ser Gly Pro Gly Ile Tyr Gly Pro Tyr Gly Pro Gly Ile Ser Gly
        1025                1030                1035

Pro Gly Ser Gly Val Phe Gly Ile Gly Pro Tyr Gly Pro Gly Ala
        1040                1045                1050

Ser Ala Ala Ala Ala Ala Gly Ile Tyr Gly Pro Gly Val Phe Gly
        1055                1060                1065
```

```
Pro Tyr  Gly Pro Gly Ile Ser  Ala Ala Ala Ala Ala  Gly Pro Gly
    1070             1075                 1080


Ser Gly  Ile Tyr Gly Pro Gly  Ala Ser Gly Ile Asn  Gly Pro Gly
    1085             1090                 1095


Ser Gly  Ile Tyr Gly Pro Gly  Val Phe Gly Pro Gly  Ile Ser Ala
    1100             1105                 1110


Ala Ala  Ala Ala Gly Ile Tyr  Val Phe Gly Pro Gly  Val Phe Gly
    1115             1120                 1125


Pro Tyr  Gly Pro Gly Ala Ser  Ala Ala Ala Ala Ala  Gly Ile Tyr
    1130             1135                 1140


Gly Ser  Gly Pro Gly Val Phe  Gly Pro Tyr Gly Pro  Gly Ile Ser
    1145             1150                 1155


Gly Ser  Gly Val Phe Gly Pro  Gly Val Phe Gly Pro  Tyr Ala Ser
    1160             1165                 1170


Ala Ala  Ala Ala Ala Gly Pro  Gly Ser Gly Val Phe  Gly Pro Gly
    1175             1180                 1185


Ala Ser
    1190



<210>   41
<211>   252
<212>   PRT
<213>   Artificial Sequence

<220>
<223>   Collagen-type4-Kai

<400>   41

Met His His His His His His Ser Ser Gly Ser Ser Lys Asp Gly Val
1               5                   10                  15


Pro Gly Phe Pro Gly Ser Glu Gly Val Lys Gly Asn Arg Gly Phe Pro
            20                  25                  30


Gly Leu Met Gly Glu Asp Gly Ile Lys Gly Gln Lys Gly Asp Ile Gly
        35                  40                  45


Pro Pro Gly Phe Arg Gly Pro Thr Glu Tyr Tyr Asp Thr Tyr Gln Glu
    50                  55                  60
```

Lys Gly Asp Glu Gly Thr Pro Gly Pro Pro Gly Pro Arg Gly Ala Arg
65                  70              75                  80

Gly Pro Gln Gly Pro Ser Gly Pro Pro Gly Val Pro Gly Ser Pro Gly
                85              90                  95

Ser Ser Arg Pro Gly Leu Arg Gly Ala Pro Gly Trp Pro Gly Leu Lys
            100             105             110

Gly Ser Lys Gly Glu Arg Gly Arg Pro Gly Lys Asp Ala Met Gly Thr
            115             120             125

Pro Gly Ser Pro Gly Cys Ala Gly Ser Pro Gly Leu Pro Gly Ser Pro
    130             135             140

Gly Pro Pro Gly Pro Pro Gly Asp Ile Val Phe Arg Lys Gly Pro Pro
145             150             155             160

Gly Asp His Gly Leu Pro Gly Tyr Leu Gly Ser Pro Gly Ile Pro Gly
            165             170             175

Val Asp Gly Pro Lys Gly Glu Pro Gly Leu Leu Cys Thr Gln Cys Pro
            180             185             190

Tyr Ile Pro Gly Pro Pro Gly Leu Pro Gly Leu Pro Gly Leu His Gly
        195             200             205

Val Lys Gly Ile Pro Gly Arg Gln Gly Ala Ala Gly Leu Lys Gly Ser
    210             215             220

Pro Gly Ser Pro Gly Asn Thr Gly Leu Pro Gly Phe Pro Gly Phe Pro
225             230             235             240

Gly Ala Gln Gly Asp Pro Gly Leu Lys Gly Glu Lys
            245             250

<210>  42
<211>  310
<212>  PRT
<213>  Artificial Sequence

<220>
<223>  Resilin-Kai

<400>  42

Met His His His His His His Pro Glu Pro Pro Val Asn Ser Tyr Leu
1               5                   10                  15

Pro Pro Ser Asp Ser Tyr Gly Ala Pro Gly Gln Ser Gly Pro Gly Gly

273

|     |     |     | 20  |     |     |     | 25  |     |     |     | 30  |     |     |
|-----|-----|-----|-----|-----|-----|-----|-----|-----|-----|-----|-----|-----|-----|

Arg Pro Ser Asp Ser Tyr Gly Ala Pro Gly Gly Gly Asn Gly Gly Arg
        35              40              45

Pro Ser Asp Ser Tyr Gly Ala Pro Gly Gln Gly Gln Gly Gln Gly Gln
        50              55              60

Gly Gln Gly Gly Tyr Ala Gly Lys Pro Ser Asp Ser Tyr Gly Ala Pro
65              70              75              80

Gly Gly Gly Asp Gly Asn Gly Gly Arg Pro Ser Ser Ser Tyr Gly Ala
            85              90              95

Pro Gly Gly Gly Asn Gly Gly Arg Pro Ser Asp Thr Tyr Gly Ala Pro
        100             105             110

Gly Gly Gly Asn Gly Gly Arg Pro Ser Asp Thr Tyr Gly Ala Pro Gly
        115             120             125

Gly Gly Gly Asn Gly Asn Gly Gly Arg Pro Ser Ser Ser Tyr Gly Ala
    130             135             140

Pro Gly Gln Gly Gln Gly Asn Gly Asn Gly Gly Arg Pro Ser Ser Ser
145             150             155             160

Tyr Gly Ala Pro Gly Gly Gly Asn Gly Gly Arg Pro Ser Asp Thr Tyr
            165             170             175

Gly Ala Pro Gly Gly Gly Asn Gly Gly Arg Pro Ser Asp Thr Tyr Gly
        180             185             190

Ala Pro Gly Gly Gly Asn Asn Gly Gly Arg Pro Ser Ser Ser Tyr Gly
        195             200             205

Ala Pro Gly Gly Gly Asn Gly Gly Arg Pro Ser Asp Thr Tyr Gly Ala
210             215             220

Pro Gly Gly Gly Asn Gly Asn Gly Ser Gly Gly Arg Pro Ser Ser Ser
225             230             235             240

Tyr Gly Ala Pro Gly Gln Gly Gln Gly Gly Phe Gly Gly Arg Pro Ser
            245             250             255

Asp Ser Tyr Gly Ala Pro Gly Gln Asn Gln Lys Pro Ser Asp Ser Tyr
        260             265             270

```
Gly Ala Pro Gly Ser Gly Asn Gly Asn Gly Gly Arg Pro Ser Ser Ser
        275                 280                 285


Tyr Gly Ala Pro Gly Ser Gly Pro Gly Gly Arg Pro Ser Asp Ser Tyr
        290                 295                 300


Gly Pro Pro Ala Ser Gly
305                 310
```

```
<210>   43
<211>   282
<212>   PRT
<213>   Artificial Sequence

<220>
<223>   elastin short

<400>   43
```

```
Met His His His His His His Ser Ser Gly Ser Ser Leu Gly Val Ser
1                   5                   10                  15


Ala Gly Ala Val Val Pro Gln Pro Gly Ala Gly Val Lys Pro Gly Lys
            20                  25                  30


Val Pro Gly Val Gly Leu Pro Gly Val Tyr Pro Gly Gly Val Leu Pro
            35                  40                  45


Gly Ala Arg Phe Pro Gly Val Gly Val Leu Pro Gly Val Pro Thr Gly
        50                  55                  60


Ala Gly Val Lys Pro Lys Ala Pro Gly Val Gly Gly Ala Phe Ala Gly
65                  70                  75                  80


Ile Pro Gly Val Gly Pro Phe Gly Gly Pro Gln Pro Gly Val Pro Leu
            85                  90                  95


Gly Tyr Pro Ile Lys Ala Pro Lys Leu Pro Gly Gly Tyr Gly Leu Pro
            100                 105                 110


Tyr Thr Thr Gly Lys Leu Pro Tyr Gly Tyr Gly Pro Gly Gly Val Ala
        115                 120                 125


Gly Ala Ala Gly Lys Ala Gly Tyr Pro Thr Gly Thr Gly Val Gly Pro
        130                 135                 140


Gln Ala Ala Ala Ala Ala Ala Lys Ala Ala Ala Lys Phe Gly Ala
145                 150                 155                 160


Gly Ala Ala Gly Val Leu Pro Gly Val Gly Gly Ala Gly Val Pro Gly
```

165                          170                          175

Val Pro Gly Ala Ile Pro Gly Ile Gly Gly Ile Ala Gly Val Gly Thr
            180                 185                 190

Pro Ala Ala Ala Ala Ala Ala Ala Ala Ala Ala Lys Ala Ala Lys Tyr
            195                 200                 205

Gly Ala Ala Ala Gly Leu Val Pro Gly Gly Pro Gly Phe Gly Pro Gly
    210                 215                 220

Val Val Gly Val Pro Gly Ala Gly Val Pro Gly Val Gly Val Pro Gly
225                 230                 235                 240

Ala Gly Ile Pro Val Val Pro Gly Ala Gly Ile Pro Gly Ala Ala Val
            245                 250                 255

Pro Gly Val Val Ser Pro Glu Ala Ala Ala Lys Ala Ala Ala Lys Ala
            260                 265                 270

Ala Lys Tyr Gly Ala Arg Pro Gly Val Gly
        275                 280

<210>  44
<211>  468
<212>  PRT
<213>  Artificial Sequence

<220>
<223>  type I keratin 26

<400>  44

Met Ser Phe Arg Leu Ser Gly Val Ser Arg Arg Leu Cys Ser Gln Ala
1               5                   10                  15

Gly Thr Gly Arg Leu Thr Gly Gly Arg Thr Gly Phe Arg Ala Gly Asn
            20                  25                  30

Val Cys Ser Gly Leu Gly Ala Gly Ser Ser Phe Ser Gly Pro Leu Gly
        35                  40                  45

Ser Val Ser Ser Lys Gly Ser Phe Ser His Gly Gly Gly Gly Leu Gly
    50                  55                  60

Ser Gly Val Cys Thr Gly Phe Leu Glu Asn Glu His Gly Leu Leu Pro
65                  70                  75                  80

Gly Asn Glu Lys Val Thr Leu Gln Asn Leu Asn Asp Arg Leu Ala Ser
                85                  90                  95

Tyr Leu Asp His Val Cys Thr Leu Glu Glu Ala Asn Ala Asp Leu Glu
100                105              110

Gln Lys Ile Lys Gly Trp Tyr Glu Lys Tyr Gly Pro Gly Ser Gly Arg
115                120              125

Gln Leu Ala His Asp Tyr Ser Lys Tyr Phe Ser Val Thr Glu Asp Leu
130                135              140

Lys Arg Gln Ile Ile Ser Val Thr Thr Cys Asn Ala Ser Ile Val Leu
145                150              155              160

Gln Asn Glu Asn Ala Arg Leu Thr Ala Asp Asp Phe Arg Leu Lys Cys
165                170              175

Glu Asn Glu Leu Ala Leu His Gln Ser Val Glu Ala Asp Ile Asn Gly
180                185              190

Leu His Arg Val Met Asp Glu Leu Thr Leu Cys Thr Ser Asp Leu Glu
195                200              205

Met Gln Cys Glu Ala Leu Ser Glu Glu Leu Thr Tyr Leu Lys Lys Asn
210                215              220

His Gln Glu Glu Met Lys Val Met Gln Gly Ala Ala Arg Gly Asn Val
225                230              235              240

Asn Val Glu Ile Asn Ala Ala Pro Gly Val Asp Leu Thr Val Leu Leu
245                250              255

Asn Asn Met Arg Ala Glu Tyr Glu Asp Leu Ala Glu Gln Asn His Glu
260                265              270

Asp Ala Glu Ala Trp Phe Ser Glu Lys Ser Thr Ser Leu His Gln Gln
275                280              285

Ile Ser Asp Asp Ala Gly Ala Ala Met Ala Ala Arg Asn Glu Leu Met
290                295              300

Glu Leu Lys Arg Asn Leu Gln Thr Leu Glu Ile Glu Leu Gln Ser Leu
305                310                315              320

Leu Ala Met Lys His Ser Tyr Glu Cys Ser Leu Ala Glu Thr Glu Ser
325                330                335

Asn Tyr Cys His Gln Leu Gln Gln Ile Gln Glu Gln Ile Gly Ala Met

```
              340                    345                        350

        Glu Asp Gln Leu Gln Gln Ile Arg Met Glu Thr Glu Gly Gln Lys Leu
                355                 360                 365


        Glu His Glu Arg Leu Leu Asp Val Lys Ile Phe Leu Glu Lys Glu Ile
            370                 375                 380


        Glu Met Tyr Cys Lys Leu Ile Asp Gly Glu Gly Arg Lys Ser Lys Ser
        385                 390                 395                 400


        Thr Cys Tyr Lys Ser Glu Gly Arg Gly Pro Lys Asn Ser Glu Asn Gln
                        405                 410                 415


        Val Lys Asp Ser Lys Glu Glu Ala Val Val Lys Thr Val Val Gly Glu
                420                 425                 430


        Leu Asp Gln Leu Gly Ser Val Leu Ser Leu Arg Val His Ser Val Glu
                435                 440                 445


        Glu Lys Ser Ser Lys Ile Ser Asn Ile Thr Met Glu Gln Arg Leu Pro
            450                 455                 460


        Ser Lys Val Pro
        465
```

**Claims**

1. A method for manufacturing a high-density woven fabric, comprising steps of:

   weaving a fiber that at least partially comprises a protein fiber to obtain a raw-material woven fabric; and
   shrinking the raw-material woven fabric by bringing the raw-material woven fabric into contact with aqueous
   moisture to obtain a high-density woven fabric.

2. The manufacturing method according to claim 1, wherein the protein fiber is capable of shrinking by 2% or more when being brought into contact with the aqueous moisture.

3. The manufacturing method according to claim 1 or 2, wherein the protein fiber is capable of shrinking by more than 7% when being brought into contact with the aqueous moisture and subsequently dried.

4. The manufacturing method according to any one of claims 1 to 3, wherein a temperature of the aqueous moisture is lower than a boiling point thereof.

5. The manufacturing method according to any one of claims 1 to 4, wherein the weaving step comprises adjusting a weaving density of the raw-material woven fabric.

6. The manufacturing method according to any one of claims 1 to 5, wherein the protein fiber is a fibroin fiber.

7. The manufacturing method according to claim 6, wherein the fibroin fiber is a modified spider silk fibroin fiber.

8. A high-density woven fabric, which is a woven fabric comprising a fiber that at least partially comprises a protein fiber, wherein the fiber is shrunk by aqueous moisture.

9. A high-density woven fabric, which is a woven fabric comprising a fiber that at least partially comprises a protein fiber, wherein the woven fabric is shrunk by aqueous moisture.

10. A high-density woven fabric, which is a woven fabric comprising a fiber that at least partially comprises a protein fiber, wherein a weaving density of the woven fabric exceeds a maximum value of a weaving density achievable by weaving the fiber.

11. The high-density woven fabric according to any one of claims 8 to 10, wherein the protein fiber is a fibroin fiber.

12. The high-density woven fabric according to claim 11, wherein the fibroin fiber is a modified spider silk fibroin fiber.

13. The manufacturing method according to any one of claims 1 to 7, wherein

the protein fiber comprises a modified fibroin,
the modified fibroin comprises a domain sequence represented by

Formula 1:          $[(A)_n \text{motif-REP}]_m$,

and
the domain sequence has an amino acid sequence with a reduced content of $(A)_n$ motifs, which corresponds to an amino acid sequence in which at least one or a plurality of $(A)_n$ motifs are deleted, as compared with a naturally occurring fibroin,
wherein in Formula 1, the $(A)_n$ motif represents an amino acid sequence composed of 2 to 27 amino acid residues and the number of alanine residues with respect to a total number of amino acid residues in the $(A)_n$ motif is 83% or more;
REP represents an amino acid sequence composed of 10 to 200 amino acid residues;
m represents an integer of 2 to 300;
a plurality of the $(A)_n$ motifs may have the same amino acid sequence or amino acid sequences different from each other; and
a plurality of REPs may have the same amino acid sequence or amino acid sequences different from each other.

14. The manufacturing method according to any one of claims 1 to 7, wherein

the protein fiber comprises a modified fibroin,
the modified fibroin comprises a domain sequence represented by

Formula 1:          $[(A)_n \text{ motif-REP}]_m$,

and
the domain sequence has an amino acid sequence with a reduced content of glycine residues, which corresponds to an amino acid sequence in which at least one or a plurality of glycine residues in REP are substituted with other amino acid residues, as compared with a naturally occurring fibroin,
wherein in Formula 1, the $(A)_n$ motif represents an amino acid sequence composed of 2 to 27 amino acid residues and the number of alanine residues with respect to a total number of amino acid residues in the $(A)_n$ motif is 83% or more;
REP represents an amino acid sequence composed of 10 to 200 amino acid residues;
m represents an integer of 2 to 300;
a plurality of the $(A)_n$ motifs may have the same amino acid sequence or amino acid sequences different from each other; and
a plurality of REPs may have the same amino acid sequence or amino acid sequences different from each other.

15. The manufacturing method according to any one of claims 1 to 7, wherein

the protein fiber comprises a modified fibroin,
the modified fibroin comprises a domain sequence represented by

Formula 1:    [(A)$_n$ motif-REP]$_m$,

and

the domain sequence has an amino acid sequence comprising a region having a locally high hydropathy index, which corresponds to an amino acid sequence in which one or a plurality of amino acid residues in REP are substituted with an amino acid residue having a high hydropathy index and/or one or a plurality of amino acid residues having a high hydropathy index is inserted into REP, as compared with a naturally occurring fibroin, wherein in Formula 1, the (A)$_n$ motif represents an amino acid sequence composed of 2 to 27 amino acid residues and the number of alanine residues with respect to a total number of amino acid residues in the (A)$_n$ motif is 83% or more;

REP represents an amino acid sequence composed of 10 to 200 amino acid residues;

m represents an integer of 2 to 300;

a plurality of the (A)$_n$ motifs may have the same amino acid sequence or amino acid sequences different from each other; and

a plurality of REPs may have the same amino acid sequence or amino acid sequences different from each other.

16. The manufacturing method according to any one of claims 1 to 7, wherein

the protein fiber comprises a modified fibroin,

the modified fibroin comprises a domain sequence represented by

Formula 1:    [(A)$_n$ motif-REP]$_m$

or

Formula 2:    [(A)$_n$ motif-REP]$_m$-(A)$_n$

motif, and

the domain sequence has an amino acid sequence with a reduced content of glutamine residues, which corresponds to an amino acid sequence in which one or a plurality of glutamine residues in REP are deleted or substituted with other amino acid residues, as compared with a naturally occurring fibroin, wherein in Fonnula 1 and Formula 2, the (A)$_n$ motif represents an amino acid sequence composed of 2 to 27 amino acid residues and the number of alanine residues with respect to a total number of amino acid residues in the (A)$_n$ motif is 80% or more;

REP represents an amino acid sequence composed of 10 to 200 amino acid residues;

m represents an integer of 2 to 300;

a plurality of the (A)$_n$ motifs may have the same amino acid sequence or amino acid sequences different from each other; and

a plurality of REPs may have the same amino acid sequence or amino acid sequences different from each other.

17. The manufacturing method according to any one of claims 1 to 7, wherein

the protein fiber comprises a modified fibroin, and

the modified fibroin has a limiting oxygen index (LOI) value of 26.0 or more.

18. The manufacturing method according to any one of claims 1 to 7, wherein

the protein fiber comprises a modified fibroin, and

a maximum hygroscopic heat generation, as determined according to the following Formula A, of the modified fibroin is more than 0.025°C/g:

Formula A: Maximum hygroscopic heat generation = {(Highest temperature of a sample when the sample has been transferred to a high humidity environment after being placed in a low humidity environment until a temperature of the sample reaches equilibrium) - (Temperature of the sample when the sample is being transferred to the high humidity environment after being placed in the low humidity environment until the temperature of the sample reaches equilibrium)} (°C) / Sample weight (g),

wherein in Formula A, the low humidity environment means an environment of a temperature of 20°C and a relative humidity of 40%, and the high humidity environment means an environment of a temperature of 20°C and a relative humidity of 90%.

Fig.1

EP 3 779 031 A1

**Fig.2**

Fig.3

EP 3 779 031 A1

Fig.4

# Fig.5

REGION A

50      40      10      20      30

AAAA      AAAA      AAAA      AAAA      AAAA      AAAA

GPGXX   GPGXX    GPGXX   GPGXX      GPGXX     GPGXX   GPGXX

EP 3 779 031 A1

# Fig.6

Fig.7

EP 3 779 031 A1

## INTERNATIONAL SEARCH REPORT

| International application No. |
| --- |
| PCT/JP2019/014897 |

**A. CLASSIFICATION OF SUBJECT MATTER**
Int.Cl. D06M11/01(2006.01)i, D01F4/02(2006.01)i, D03D15/00(2006.01)i, D06B5/00(2006.01)i

According to International Patent Classification (IPC) or to both national classification and IPC

**B. FIELDS SEARCHED**

Minimum documentation searched (classification system followed by classification symbols)
Int.Cl. D06M11/01, D01F4/02, D03D15/00, D06B5/00

Documentation searched other than minimum documentation to the extent that such documents are included in the fields searched

| | |
| --- | --- |
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)
JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
| --- | --- | --- |
| X | JP 6213 C1 (SAKANE, Kiyoichi) 05 May 1903, entire text (Family: none) | 1–6, 8–11 |
| X | JP 111700 C2 (KENGYO SIKENSHOCHO) 21 August 1935, entire text (Family: none) | 1–6, 8–11 |
| X | JP 09-31798 A (AGE CO., LTD.) 04 February 1997, claims, paragraph [0008], fig. 1 & US 5598615 A, claims, column 2, lines 1–12, fig. 1 | 1–6, 8–11 |

☒ Further documents are listed in the continuation of Box C. ☐ See patent family annex.

| | | |
| --- | --- | --- |
| * | Special categories of cited documents: | |
| "A" | document defining the general state of the art which is not considered to be of particular relevance | |
| "E" | earlier application or patent but published on or after the international filing date | |
| "L" | document which may throw doubts on priority claim(s) or which is cited to establish the publication date of another citation or other special reason (as specified) | |
| "O" | document referring to an oral disclosure, use, exhibition or other means | |
| "P" | document published prior to the international filing date but later than the priority date claimed | |

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
| --- | --- |
| 13 June 2019 (13.06.2019) | 25 June 2019 (25.06.2019) |

| Name and mailing address of the ISA/ | Authorized officer |
| --- | --- |
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

**INTERNATIONAL SEARCH REPORT**

International application No.

PCT/JP2019/014897

C (Continuation). DOCUMENTS CONSIDERED TO BE RELEVANT

| Category* | Citation of document, with indication, where appropriate, of the relevant passages | Relevant to claim No. |
|---|---|---|
| X | JP 07-252746 A (AGE CO., LTD.) 03 October 1995, claims, examples, fig. 1 (Family: none) | 1-6, 8-11 |
| X<br>Y | WO 2013/147590 A2 (ESSAIDI, Jalila) 03 October 2013, claims, pp. 6, 7, 9, 11, fig. 3-10 & US 2015/0056256 A1 & EP 2831333 A2 & NL 1039495 C | 1-12, 17, 18<br>13-16 |
| Y | WO 2017/188434 A1 (SPIBER INC.) 02 November 2017, claims, examples, fig. 1, paragraphs [0057], [0059] & EP 3450452 A1, claims, examples, fig. 1, paragraphs [0058], [0060] & CA 3012821 A & CN 109071619 A & KR 10-2019-0003583 A | 13-16 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013209756 A

- JP 2002238569 A

**Non-patent literature cited in the description**

- *Nucleic Acid Res.,* 1982, vol. 10, 6487
- *Methods in Enzymology,* 1983, vol. 100, 448
- **KYTE J ; DOOLITTLE R.** A simple method for displaying the hydropathic character of a protein. *J. Mol. Biol.,* 1982, vol. 157, 105-132

- *Notice No. 50 of the Office of Hazardous Materials Regulation,* 31 May 1995
- *Proc. Natl. Acad. Sci. USA,* 1972, vol. 69, 2110